(19) Europäisches Patentamt
European Patent Office
Office européen des brevets

(11) EP 4 452 256 B1

(12) EUROPEAN PATENT SPECIFICATION

(45) Date of publication and mention of the grant of the patent:
**10.12.2025 Bulletin 2025/50**

(21) Application number: **22843653.1**

(22) Date of filing: **20.12.2022**

(51) International Patent Classification (IPC):
***A61K 31/336** (2006.01)* ***A61K 31/495** (2006.01)*
***A61K 31/498** (2006.01)* ***A61K 45/06** (2006.01)*
***A61P 35/00** (2006.01)* ***A61K 41/00** (2020.01)*

(52) Cooperative Patent Classification (CPC):
(C-Sets available)
**A61K 31/498; A61K 31/336; A61K 31/495; A61K 41/0038; A61K 45/06; A61P 35/00** (Cont.)

(86) International application number:
**PCT/EP2022/086913**

(87) International publication number:
**WO 2023/118085 (29.06.2023 Gazette 2023/26)**

# (54) METHODS OF TREATING BRAIN TUMOURS AND NEUROBLASTOMAS

VERFAHREN ZUR BEHANDLUNG VON GEHIRNTUMOREN UND NEUROBLASTOMEN

MÉTHODES DE TRAITEMENT DE TUMEURS CÉRÉBRALES ET DE NEUROBLASTOMES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**

(30) Priority: **21.12.2021 US 202163292029 P**

(43) Date of publication of application:
**30.10.2024 Bulletin 2024/44**

(73) Proprietor: **Astrazeneca AB**
**151 85 Södertälje (SE)**

(72) Inventor: **HAMERLIK, Petra**
**Cambridge, Cambridgeshire CB2 0AA (GB)**

(74) Representative: **AstraZeneca Intellectual Property**
**Eastbrook House**
**Shaftesbury Road**
**Cambridge CB2 8BF (GB)**

(56) References cited:
**WO-A1-2021/013735** **WO-A1-2021/260092**

- **NABORS LOUIS BURT ET AL: "Central Nervous System Cancers, Version 3.2020, NCCN Clinical Practice Guidelines in Oncology", JNCCN. JOURNAL OF THE NATIONAL COMPREHENSIVE CANCER NETWORK, vol. 18, no. 11, 1 November 2020 (2020-11-01), US, pages 1537 - 1570, XP093029277, ISSN: 1540-1405, DOI: 10.6004/jnccn.2020.0052**
- **ANONYMOUS: "Treating Neuroblastoma", AMERICAN CANCER SOCIETY, 28 April 2021 (2021-04-28), American Cancer Society website, pages 1 - 28, XP093029299, Retrieved from the Internet <URL:https://www.cancer.org/content/dam/CRC/PDF/Public/8761.00.pdf> [retrieved on 20230306]**
- **J. MURAI ET AL: "Rationale for Poly(ADP-ribose) Polymerase (PARP) Inhibitors in Combination Therapy with Camptothecins or Temozolomide Based on PARP Trapping versus Catalytic Inhibition", JOURNAL OF PHARMACOLOGY AND EXPERIMENTAL THERAPEUTICS, vol. 349, no. 3, 1 May 2014 (2014-05-01), US, pages 408 - 416, XP055173940, ISSN: 0022-3565, DOI: 10.1124/jpet.113.210146**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PIKE ANDY ET AL: "Abstract 5076: Evaluation of the CNS penetration of a next generation PARP inhibitor, AZD9574, in cynomolgus monkey using positron emission tomography", 2ASTRAZENECA, WALTHAM, MA;, vol. 82, no. 12_Supplement, 15 June 2022 (2022-06-15), pages 5076 - 5076, XP093028867, Retrieved from the Internet <URL:https://aacrjournals.org/cancerres/article/82/12_Supplement/5076/700301/Abstract-5076-Evaluation-of-the-CNS-penetration-of> DOI: 10.1158/1538-7445.AM2022-5076**
- **ANONYMOUS: "Study Record Versions History of Changes for Study: NCT05417594 Study of AZD9574 as Monotherapy and in Combination With Anti-cancer Agents in Participants With Advanced Solid Malignancies (CERTIS1)", CLINICALTRIALS.GOV, 18 November 2022 (2022-11-18), clinicaltrials.gov, pages 1 - 20, XP093028489, Retrieved from the Internet <URL:https://clinicaltrials.gov/ct2/history/NCT05417594?V_5=View#StudyPageTop>**
- **ANONYMOUS CAT: "AZD-9574 Molecular Formula: C 21 Product DataSheet Inhibitors @BULLET Screening Libraries @BULLET Proteins", MEDCHEMEXPRESS, 1 January 2023 (2023-01-01), pages 1 - 2, XP093029075, Retrieved from the Internet <URL:https://file.medchemexpress.com/batch_PDF/HY-145804/AZD-9574-DataSheet-MedChemExpress.pdf>**


(52) Cooperative Patent Classification (CPC): (Cont.)

C-Sets
**A61K 31/336, A61K 2300/00;**
**A61K 31/495, A61K 2300/00;**
**A61K 31/498, A61K 2300/00;**
**A61K 41/0038, A61K 2300/00**

## Description

The present invention is defined by the claims. Any subject-matter falling outside the scope of the claims is provided for information only.

Methods of treatment of the human body by therapy, referred to in this description, are not part of the present invention as such, but are described here in relation to compounds and pharmaceutical compositions for use in said methods for treatment of the human body by therapy, according to the present invention.

The present disclosure relates to methods of treating a brain tumour or a neuroblastoma in a patient in need thereof, the method comprising administering to the patient a therapeutically effective amount of a poly(ADP-ribose) polymerase (PARP) inhibitor which are substituted azaquinolone compounds. The brain tumours and neuroblastomas may comprise an alpha thalassemia/mental retardation syndrome X-linked (ATRX) deficient phenotype. The PARP inhibitor may be administered to the patient in combination with an alkylating chemotherapeutic and/or ionizing radiation.

Current therapeutic strategies for treating brain tumours such as gliomas include surgical resection, ionizing radiation and the use of alkylating chemotherapeutics such as temozolomide (TMZ). Unfortunately, many of these brain tumours such as glioblastoma (GBM) are associated with an extremely poor clinical prognosis. Surgical, instrument technology, diagnostic and radio/chemotherapeutic strategies have slowly evolved over time, but this has not translated into significant increases in patient survival. Guidelines for the treatment of brain cancers and neuroblastomas have been published by the National Comprehensive Cancer Network and by the American Cancer Society (Nabors et al., 2020; American Cancer Society, 2021).

Accordingly, there is a need for new treatment strategies for brain tumours such as GBM.

Several molecular markers have been used to classify gliomas. Gliomas can be classified on the basis of whether they contain mutations in the isocitrate dehydrogenase (IDH) 1 and/or 2 gene (Hartmann et al. 2009), and/or whether they contain a mutation in the alpha thalassemia/mental retardation syndrome X-linked (ATRX) gene (Haase et al. 2018). Genetic alterations in the ATRX gene are also associated with a poor outcome patient subgroup for neuroblastoma patients (George et al. 2020). Furthermore, MGMT promoter methylation has been used as a marker to predict favourable outcome in patients with GBM who are exposed to alkylating agent chemotherapy (Weller et al. 2010).

Temozolomide (TMZ) is the standard of care (SOC) chemotherapy in GBM (Stupp et al., 2014; Stuppet al., 2010). Mechanistically, TMZ methylates DNA at specific position leading to DNA adducts which unless repaired lead to single and double stranded DNA breaks, cell cycle arrest and apoptosis (Singh et al., 2021). Importantly, PARPs (especially PARP1) are key enzymes involved in the early stages of the DNA damage and repair (DDR) process as a response to single and double stranded breaks. In fact, one of the key functions of PARP1 is during base excision repair (BER) which is required to remove the DNA adducts generated by TMZ and recruit downstream factors to repair this damage. Various studies have investigated the combination benefit of PARP inhibitors (PARPi) and TMZ (Gupta et al., 2018; Higuchi et al., 2020; Murai et al., 2014).

Ionizing radiation (IR) induces single and double stranded DNA breaks which result in cell cycle arrest and cell death (Reisz et al., 2014). Important, PARP1 is involved in the repair these types of DNA damage. Various studies have investigated the combination benefit of PARPi and IR (Jannetti et al., 2020). As well as sensitising gliomas to radiation, it has also been described that PARPi sensitises ependymoma to radiation (van Vuurden et al., 2011).

Examples of PARP inhibitors and their mechanism of action are taught in e.g. WO2004/080976.

PARP1 and PARP2 are the most extensively studied PARPs for their role in DNA damage repair. PARP1 is activated by DNA damage breaks and functions to catalyse the addition of poly (ADP-ribose) (PAR) chains to target proteins. This post-translational modification, known as PARylation, mediates the recruitment of additional DNA repair factors to DNA lesions.

Following completion of this recruitment role, PARP auto-PARylation triggers the release of bound PARP from DNA to allow access to other DNA repair proteins to complete repair. Thus, the binding of PARP to damaged sites, its catalytic activity, and its eventual release from DNA are all important steps for a cancer cell to respond to DNA damage caused by chemotherapeutic agents and radiation therapy (Bai 2015).

It is believed that PARP inhibitors having improved selectivity for PARP1 may result in improved efficacy and reduced toxicity compared to other clinical PARP1/2 inhibitors. It is believed also that selective strong inhibition of PARP1 would lead to trapping of PARP1 on DNA, resulting in DNA double-strand breaks (DSBs) through collapse of replication forks in S-phase.

Thus, there is a need in the art to develop PARP inhibitors that can be used for the safe and effective treatment of brain tumours and neuroblastoma, both as monotherapies and in combination with alkylating chemotherapeutic and ionizing radiation. These PARP inhibitors may exhibit selectivity for PARP1.

The applicant has discovered that the azaquinolones described herein surprisingly have PARP inhibitory activity, and therefore may be useful for the treatment of diseases and conditions in which PARP function has pharmacological significance. Furthermore, azaquinolones described herein have surprisingly high selectivity for PARP1 over other PARP family members such as PARP2, PARP3, PARP5a, and PARP6.

The applicant has further discovered that the azaquinolones described herein surprisingly are capable of penetrating the blood brain barrier (BBB), making them useful for the treatment of brain tumours such as gliomas.

The applicant has demonstrated that a PARP inhibitor with these properties can be used in the treatment of gliomas, using both *in vitro* and *in vivo* models. As described herein, a PARP inhibitor was demonstrated to have potent activity as a monotherapy in an ATRX mutant cell line, both in an IDH1 mutant and IDH1 wild-type background.

In a first aspect, the applicant makes available a method of treating a brain tumour or a neuroblastoma in a patient in need thereof, the method comprising administering to the patient a therapeutically effective amount of a poly(ADP-ribose) polymerase (PARP) inhibitor,

wherein the brain tumour or neuroblastoma comprises an alpha thalassemia/mental retardation syndrome X-linked (ATRX) deficient phenotype,

wherein the PARP inhibitor is a compound of Formula (I):

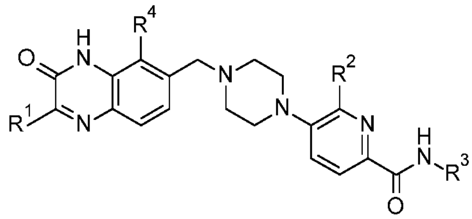

(I)

wherein:

$R^1$ is independently selected from H, $C_{1-4}$ alkyl, $C_{3-6}$ cycloalkyl, $C_{1-4}$ fluoroalkyl, and $C_{1-4}$ alkyloxy;

$R^2$ is independently selected from H, halo, $C_{1-4}$ alkyl, and $C_{1-4}$ fluoroalkyl; and

$R^3$ is H or $C_{1-4}$ alkyl;

$R^4$ is halo or $C_{1-4}$ alkyl,

or a pharmaceutically acceptable salt thereof.

In some embodiments, the brain tumour or neuroblastoma further comprises an isocitrate dehydrogenase 1 or 2 (IDH1 or IDH2) deficient phenotype (e.g. it is classed as IDH1 mutant, and/or IDH2 mutant). In other embodiments, the brain tumour or neuroblastoma does not comprise an IDH1 or IDH2 phenotype (e.g. it is classed as IDH1 wild type, and/or IDH1 wild type).

In some embodiments, the brain tumour or neuroblastoma comprises O6-methylguanine-DNA methyltransferase (MGMT) promoter methylation.

In some embodiments, the method comprises a further step of diagnosing the patient as having the brain tumour or neuroblastoma comprising the ATRX deficient phenotype prior to administering the PARP inhibitor. In other embodiments, the patient being treated has been previously diagnosed as a patient having the brain tumour or neuroblastoma comprising the ATRX deficient phenotype. Methods for diagnosing the patient are described herein and may, for example, involve assaying cells obtained from the brain tumour or neuroblastoma, or from the cerebrospinal fluid (CSF) from the patient.

The applicant has also demonstrated that the PARP inhibitors disclosed herein exhibit a synergistic anti-tumour activity when used in combination with alkylating chemotherapeutic agents, including temozolomide (TMZ) and VAL-083, and/or with ionizing radiation.

Thus, in some embodiments, the PARP inhibitor is administered to the patient in combination with

i) an alkylating chemotherapeutic agent; and/or

ii) a radiotherapeutic agent.

In a second aspect, the applicant makes available a method of treating a brain tumour or a neuroblastoma in a patient in need thereof, the method comprising administering to the patient a therapeutically effective amount of a

poly(ADP-ribose) polymerase (PARP) inhibitor, and

i) an alkylating chemotherapeutic agent; and/or
ii) a radiotherapeutic agent administered at a dose of 10 Gy or above,

wherein the PARP inhibitor is a compound of Formula I as defined in the first aspect.

In some embodiments, the brain tumour or neuroblastoma comprises:

(i) an alpha thalassemia/mental retardation syndrome X-linked (ATRX) deficient phenotype;

(ii) an isocitrate dehydrogenase 1 or 2 (IDH1 or IDH2) deficient phenotype; and/or

(iii) $O^6$-methylguanine-DNA methyltransferase (MGMT) promoter methylation.

In some embodiments, the alkylating chemotherapeutic agent is temozolomide (TMZ) or dianhydrogalactitol (VAL-083).

As described herein, a combination of a PARP inhibitor of the invention and TMZ was demonstrated to deliver anti-tumour benefit without requiring high doses of TMZ. This is beneficial, as it suggests that a combination therapy involving the PARP inhibitor can be used to treat brain tumours and neuroblastomas with reduced doses of the alkylating chemotherapeutic agent, which may advantageously allow for effective cancer treatment with reduced toxic side effects associated with chemotherapy. Similarly, using a PARP inhibitor of the invention was demonstrated to effectively treat gliomas in combination with a reduced clinically relevant dose of ionizing radiation, suggesting that that effective cancer treatment can be achieved with reduced toxic side effects associated with radiotherapy.

Thus, in some embodiments, TMZ is administered at:

(i) a dose of less than about 200 $mg/m^2$, a dose of less than about 150 $mg/m^2$, less than about 125 $mg/m^2$, or less than about 100 $mg/m^2$;

(ii) a dose of between about 50 and 200 $mg/m^2$, a dose of between about 75 and 150 $mg/m^2$, or a dose of between about 75 $mg/m^2$ and 125 $mg/m^2$.

In some embodiments, VAL-083 is administered at:

(i) a dose of less than about 50 $mg/m^2$, a dose of less than about 40 $mg/m^2$, less than about 30 $mg/m^2$, or less than about 20 $mg/m^2$;

(ii) a dose of between about 10 and 50 $mg/m^2$, a dose of between about 10 and 40 $mg/m^2$, or a dose of between about 10 $mg/m^2$ and 30 $mg/m^2$.

As described in more detail herein, the recited dosages of TMZ and VAL-083 are typically daily doses.

In some embodiments, the radiotherapeutic agent is administered at administered at:

(i) a dose of less than about 60 Gy, less than about 55 Gy, less than about 50 Gy, less than about 45 Gy, or less than about 40 Gy; or

(ii) a dose of between about 20 Gy and about 60 Gy, between about 20 Gy and about 55 Gy, between about 20 Gy and about 50 Gy, between about 20 and about 45 Gy, or between about 20 and about 40 Gy, between about 30 Gy and about 60 Gy, between about 30 Gy and about 55 Gy, between about 30 Gy and about 50 Gy, between about 30 Gy and about 45 Gy, between about 30 Gy and about 40 Gy.

As described in more detail herein, the ionizing radiation is typically administered as fractionated radiotherapy.

In some embodiments, the brain tumour is a glioma or an ependymoma. In some embodiments, the brain tumour is a glioma. The glioma can be an adult glioma or a paediatric glioma and can be a high grade glioma or a low grade glioma. In some embodiments, the glioma is a paediatric glioma. In some embodiments, the glioma is a high grade glioma, such as a glioma selected from the list consisting of: an oligodendroglioma, an anaplastic astrocytoma, a glioblastoma, and a diffuse midline glioma. In some embodiments, the glioma is a glioblastoma.

As further demonstrated herein, a PARP inhibitor of the invention in combination with ionizing radiation was demonstrated to treat a cell line model of a H3K27M-mutant diffuse midline glioma. H3K27M-mutant diffuse midline

gliomas are high-grade paediatric brain tumours associated with a poor prognosis and are challenging to treat with current therapeutic strategies. The data demonstrated herein supports the potential for the PARP inhibitors of the invention to be used in methods for treating these challenging gliomas.

Thus, in some embodiments, the glioma is a H3K27M mutant glioma.

Also made available is a PARP inhibitor for use in a method of treating a brain tumour or a neuroblastoma in a patient, the method comprising administering to the patient a therapeutically effective amount of the PARP inhibitor, wherein the brain tumour or neuroblastoma comprises an alpha thalassemia/mental retardation syndrome X-linked (ATRX) deficient phenotype, and wherein the PARP inhibitor is a compound of Formula I as defined in the first aspect.

Also made available is a PARP inhibitor for use in a method of treating a brain tumour or a neuroblastoma in a patient, the method comprising (e.g. separate, sequential or simultaneous) administration of a therapeutically effective amount of the PARP inhibitor, and

i) an alkylating chemotherapeutic agent; and/or
ii) ionizing radiation administered at a dose of 10 Gy or above,

to the patient,
wherein the PARP inhibitor is a compound of Formula I as defined in the first aspect.

Also made available is an alkylating chemotherapeutic agent for use in a method of treating a brain tumour or a neuroblastoma in a patient, the method comprising (e.g. separate, sequential or simultaneous) administration of the alkylating chemotherapeutic agent and a therapeutically effective amount of a PARP inhibitor to the subject, wherein the PARP inhibitor is a compound of Formula I as defined in the first aspect.

In an aspect of the above embodiments, the compound of Formula (I) is of Formula (Ia):

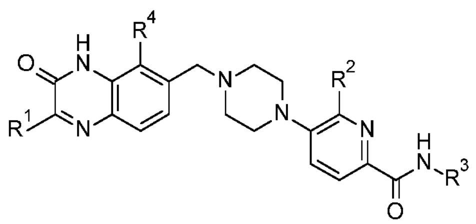

(Ia)

wherein:

$R^1$ is independently selected from H, $C_{1-4}$ alkyl, $C_{1-4}$ fluoroalkyl, and $C_{1-4}$ alkyloxy;

$R^2$ is independently selected from H, halo, $C_{1-4}$ alkyl, and $C_{1-4}$ fluoroalkyl; and

$R^3$ is H or $C_{1-4}$ alkyl;

$R^4$ is halo or $C_{1-4}$ alkyl,

or a pharmaceutically acceptable salt thereof.

In an aspect of the above embodiments, $R^1$ of formula (I) or formula (Ia) is selected from any one of methyl, ethyl, isopropyl, cyclopropyl, 1,1-difluoroethyl, 1-fluoroethyl, trifluoromethyl, difluoromethyl, and methoxy. In an particular aspect, $R^1$ is methyl or ethyl.

In an aspect of the above embodiments, $R^2$ of formula (I) or formula (Ia) is selected from any one of H, chloro, fluoro, methyl, and difluoromethyl. In an aspect, $R^2$ is fluoro or methyl.

In an aspect of the above embodiments, $R^3$ of formula (I) or formula (Ia) is methyl or ethyl.

In an aspect of the above embodiments, $R^4$ of formula (I) or formula (Ia) is selected from any one of chloro, fluoro and methyl. In a particular aspect, $R^4$ is fluoro.

In an aspect of the above embodiments, there is provided a compound of formula (I) or formula (Ia), wherein $R^1$ is $C_{1-4}$ alkyl, $R^2$ is halo, $R^3$ is $C_{1-4}$ alkyl, $R^4$ is halo or $C_{1-4}$ alkyl, or a pharmaceutically acceptable salt thereof.

In a further aspect, the compound of Formula (I) or Formula (Ia) is in the free base form.

In an aspect, there is provided a compound of Formula (I) which is 5-[4-[(2-ethyl-5-fluoro-3-oxo-4H-quinoxalin-6-yl)methyl]piperazin-1-yl]-N,6-dimethyl-pyridine-2-carboxamide or a pharmaceutically acceptable salt thereof.

In an aspect, there is provided a compound of Formula (I) which is 6-fluoro-5-[4-[(5-fluoro-2-methyl-3-oxo-4H-quinoxalin-6-yl)methyl]piperazin-1-yl]-N-methyl-pyridine-2-carboxamide or a pharmaceutically acceptable salt thereof.

In an aspect, there is provided a compound of Formula (I) which is 6-fluoro-5-[4-[(5-fluoro-2-methyl-3-oxo-4H-quinoxalin-6-yl)methyl]piperazin-1-yl]-N-methyl-pyridine-2-carboxamide crystalline form B or a pharmaceutically acceptable salt thereof.

In an aspect, there is provided a compound of Formula (I) which is 6-fluoro-5-[4-[(5-fluoro-2-methyl-3-oxo-4H-quinoxalin-6-yl)methyl]piperazin-1-yl]-N-methyl-pyridine-2-carboxamide crystalline form D or a pharmaceutically acceptable salt thereof.

In an aspect, there is provided a compound of Formula (I) which is 6-fluoro-5-[4-[(5-fluoro-2-methyl-3-oxo-4H-quinoxalin-6-yl)methyl]piperazin-1-yl]-N-methyl-pyridine-2-carboxamide mesylate, optionally as crystalline form C.

Further aspects will be apparent to one skilled in the art from reading this specification.

It is well known that blockade of the cardiac ion channel coded by human ether-à-gogo-related gene (hERG) is a risk factor in drug discovery and development. Blockage of hERG can cause safety problems such as cardiac arrhythmia. Advantageously, the compounds of Formula (I) have low hERG activity. In an aspect, the compound of Formula (I) has an IC50 >10 $\mu$M. In an aspect, the compound of Formula I has an IC50 >20 $\mu$M.

To minimize the risks of off-target effects, it is desirable for drug molecules to possess selectivity for a specific target. The compounds of Formula I advantageously possess selectivity for PARP1 over other members of the PARP family including PARP2, PARP3, PARP5a, and PARP6. Advantageously, the compounds of Formula (I) possess selectivity for PARP1 over PARP2. In an aspect, the compound of Formula (I) has 10-fold selectivity for PARP1 over PARP2. In an aspect, the compound of Formula (I) has 100-fold selectivity for PARP1 over PARP2.

In an aspect, the patient is heterozygous for one or more variations, such as mutations and polymorphisms, in BRCA1 and/or BRCA2 or a regulator thereof. The detection of variation in BRCA1 and BRCA2 is well-known in the art and is described, for example in EP 699 754, EP 705 903, Neuhausen and Ostrander 1997; Chappnis and Foulkes 2002; Janatova et al., 2003; Jancarkova 2003). Determination of amplification of the BRCA2 binding factor EMSY is described in Hughes-Davies, et al. (Hughes-Davies, et al 2003).

Mutations and polymorphisms associated with cancer may be detected at the nucleic acid level by detecting the presence of a variant nucleic acid sequence or at the protein level by detecting the presence of a variant (i.e. a mutant or allelic variant) polypeptide.

## Definitions

Alkyl groups and moieties are straight or branched chain, e.g. $C_{1-8}$ alkyl, $C_{1-6}$ alkyl, $C_{1-4}$ alkyl or $C_{5-6}$ alkyl. Examples of alkyl groups are methyl, ethyl, n-propyl, i-propyl, n-butyl, t-butyl, n-pentyl, n-hexyl, n-heptyl and n-octyl, such as methyl or n-hexyl.

Cycloalky groups are saturated cyclic alkyl groups. $C_{3-6}$ cycloalkyl is a saturated cyclic alkyl group having from 3 to 6 carbon atoms. Examples of $C_{3-6}$ cycloalkyl include cyclopropyl, cyclobutyl, cyclopentyl, and cyclohexyl. $C_{3-6}$ cycloalkyl includes $C_{3-5}$ cycloalkyl and $C_{3-4}$ cycloalkyl.

Fluoroalkyl groups are alkyl groups in which one or more H atoms is replaced with one or more fluoro atoms, e.g. $C_{1-8}$ fluoroalkyl, $C_{1-6}$ fluoroalkyl, $C_{1-4}$ fluoroalkyl or $C_{5-6}$ fluoroalkyl. Examples include fluoromethyl ($CH_2F$-), difluromethyl ($CHF_2$-), trifluoromethyl ($CF_3$-), 2,2,2-trifluoroethyl ($CF_3CH_2$-), 1,1-difluoroethyl ($CH_3CHF_2$-), 2,2-difluoroethyl ($CHF_2CH_2$-), 1-fluoroethyl ($CH_3CHF$-), and 2-fluoroethyl ($CH_2FCH_2$-).

Halo means fluoro, chloro, bromo, and iodo. In an aspect, halo is fluoro or chloro.

Alkyloxy groups are alkyl groups which are connected to the rest of the molecule via an oxygen atom. Examples of suitable $C_{1-4}$ alkyloxy groups include methoxy, ethoxy, n-propoxy, i-propoxy, n-butoxy, sec-butoxy and t-butoxy.

In this specification, unless otherwise stated, the term "pharmaceutically acceptable" as used herein refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

In this specification, unless otherwise stated, the phrase "effective amount" means an amount of a compound or composition which is sufficient enough to significantly and positively modify the symptoms and/or conditions to be treated (e.g., provide a positive clinical response). The effective amount of an active ingredient for use in a pharmaceutical composition will vary with the particular condition being treated, the severity of the condition, the duration of the treatment, the nature of concurrent therapy, the particular active ingredient(s) being employed, the particular pharmaceutically-acceptable excipient(s)/carrier(s) utilized, and like factors within the knowledge and expertise of the attending physician.

The term "treating", as used herein, unless otherwise indicated, means reversing, alleviating, inhibiting the progress of, delaying the progression of, delaying the onset of, or preventing the disorder or condition to which such term applies, or one or more symptoms of such disorder or condition. The term "treatment", as used herein, unless otherwise indicated, refers to the act of treating as "treating" is defined immediately above. The term "treating" also includes adjuvant and neo-adjuvant treatment of a subject. For the avoidance of doubt, reference herein to "treatment" includes reference to

curative, palliative and prophylactic treatment, and to the administration of a medicament for use in such treatment.

The compounds of Formula (I) or Formula (Ia) may form stable pharmaceutically acceptable acid or base salts, and in such cases administration of a compound as a salt may be appropriate. Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate (mesylate), meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate. Non-toxic physiologically-acceptable salts are preferred, although other salts may be useful, such as in isolating or purifying the product.

The salts may be formed by conventional means, such as by reacting the free base form of the product with one or more equivalents of the appropriate acid in a solvent or medium in which the salt is insoluble, or in a solvent such as water, which is removed *in vacuo* or by freeze drying or by exchanging the anions of an existing salt for another anion on a suitable ion-exchange resin.

The compounds of Formula (I) or Formula (Ia) may have more than one chiral center, and it is to be understood that the application encompasses all individual stereoisomers, enantiomers and diastereoisomers and mixtures thereof. Thus, it is to be understood that, insofar as the compounds of Formula I can exist in optically active or racemic forms by virtue of one or more asymmetric carbon atoms, the application includes in its definition any such optically active or racemic form which possesses the above-mentioned activity. The present application encompasses all such stereoisomers having activity as herein defined.

Thus, throughout the specification, where reference is made to the compound of Formula (I) or formula (Ia) it is to be understood that the term compound includes diastereoisomers, mixtures of diastereoisomers, and enantiomers that are PARP1 inhibitors.

It is also to be understood that certain compounds of Formula (I) or Formula (Ia), and pharmaceutically salts thereof, can exist in solvated as well as unsolvated forms such as, for example, hydrated and anhydrous forms. It is to be understood that the compounds herein encompass all such solvated forms. For the sake of clarity, this includes both solvated (e.g., hydrated) forms of the free form of the compound, as well as solvated (e.g., hydrated) forms of the salt of the compound.

Formula (I) or Formula (Ia) as described herein is intended to encompass all isotopes of its constituent atoms. For example, H (or hydrogen) includes any isotopic form of hydrogen including $^{1}H$, $^{2}H$ (D), and $^{3}H$ (T); C includes any isotopic form of carbon including $^{12}C$, $^{13}C$, and $^{14}C$; O includes any isotopic form of oxygen including $^{16}O$, $^{17}O$ and $^{18}O$; N includes any isotopic form of nitrogen including $^{13}N$, $^{14}N$ and $^{15}N$; F includes any isotopic form of fluorine including $^{19}F$ and $^{18}F$; and the like. In one aspect, the compounds of Formula I include isotopes of the atoms covered therein in amounts corresponding to their naturally occurring abundance. However, in certain instances, it may be desirable to enrich one or more atom in a particular isotope which would normally be present in a lower abundance. For example, $^{1}H$ would normally be present in greater than 99.98% abundance; however, in one aspect, a compound of any formula presented herein may be enriched in $^{2}H$ or $^{3}H$ at one or more positions where H is present. In another aspect, when a compound of any formula presented herein is enriched in a radioactive isotope, for example $^{3}H$ and $^{14}C$, the compound may be useful in drug and/or substrate tissue distribution assays. It is to be understood that the present application encompasses all such isotopic forms.

The compounds of Formula (I) or Formula (Ia), or pharmaceutically acceptable salts thereof, will normally be administered via the oral route in the form of pharmaceutical preparations comprising the active ingredient or a pharmaceutically acceptable salt or solvate thereof, or a solvate of such a salt, in a pharmaceutically acceptable dosage form. Depending upon the disorder and patient to be treated, the compositions may be administered at varying doses.

The pharmaceutical formulations of the compound of Formula (I) or Formula (Ia) described above may be prepared for oral administration, particularly in the form of tablets or capsules, and especially involving technologies aimed at furnishing colon-targeted drug release (Patel, 2011).

The pharmaceutical formulations of the compound of Formula (I) or Formula (Ia) described above may conveniently be administered in unit dosage form and may be prepared by any of the methods well-known in the pharmaceutical art, for example as described in Remington's Pharmaceutical Sciences, 17th ed., Mack Publishing Company, Easton, PA., (1985).

Pharmaceutical formulations suitable for oral administration may comprise one or more physiologically compatible carriers and/or excipients and may be in solid or liquid form. Tablets and capsules may be prepared with binding agents, fillers, lubricants and/or surfactants, such as sodium lauryl sulfate. Liquid compositions may contain conventional additives such as suspending agents, emulsifying agents and/or preservatives. Liquid compositions may be encapsulated in, for example, gelatin to provide a unit dosage form. Solid oral dosage forms include tablets, two-piece hard shell capsules and soft elastic gelatin (SEG) capsules. Such two-piece hard shell capsules may be made for example by filling a

compound of Formula (I) into a gelatin or hydroxypropyl methylcellulose (HPMC) shell.

A dry shell formulation typically comprises of about 40% to 60% w/w concentration of gelatin, about a 20% to 30% concentration of plasticizer (such as glycerin, sorbitol or propylene glycol) and about a 30% to 40% concentration of water. Other materials such as preservatives, dyes, opacifiers and flavours also may be present. The liquid fill material comprises a solid drug that has been dissolved, solubilized or dispersed (with suspending agents such as beeswax, hydrogenated castor oil or polyethylene glycol 4000) or a liquid drug in vehicles or combinations of vehicles such as mineral oil, vegetable oils, triglycerides, glycols, polyols and surface-active agents.

Suitable daily doses of the compounds of Formula (I) or Formula (Ia), or a pharmaceutically acceptable salt thereof, in therapeutic treatment of humans are about 0.0001-100 mg/kg body weight.

Oral formulations are preferred, particularly tablets or capsules which may be formulated by methods known to those skilled in the art to provide doses of the active compound in the range of 0.1 mg to 1000 mg.

**Molecular markers**

Brain tumours such as gliomas, and neuroblastomas may be characterised on the basis of their genetic and molecular classification.

In some embodiments, the brain tumour or neuroblastoma comprises an alpha thalassemia/mental retardation syndrome X-linked (ATRX) deficient phenotype.

The ATRX gene in humans spans almost 300 kbp of the X chromosome long-arm q21.1 band. The gene, containing 36 exons, encodes a 2492 amino acids protein with a molecular weight of 282,586 kDa, although alternatively spliced transcript variants have been identified. The NCBI Entrez Gene ID for human ATRX is Gene ID: 546. The protein contains an ATPase/helicase domain and a domain termed ADD (ATRX-DNMT3-DNMT3L, ADDATRX), which binds to histone H3. The amino acid sequence for the longest isoform of human ATRX is provided as NCBI Reference Sequence: NP_000480.3.

Inactivating mutations in the ATRX gene in glioma have been reported to be distributed evenly across the gene and reported as occurring in 7% of adult glioblastomas (GBMs) and 14-31% of paediatric GBMs (Jiao et al. 2012). ATRX loss has been reported to promote tumour growth and impair nonhomologous end joining DNA repair in glioma (Koschmann et al. 2017).

As used herein, a brain tumour or neuroblastoma comprising an ATRX deficient phenotype means that ATRX activity is reduced or abolished in the cancer cells. ATRX activity is typically removed or abolished by means of a mutation in the ATRX gene. Thus, in some embodiments a brain tumour or neuroblastoma comprising an ATRX deficient phenotype comprises cancer cells with a mutation in the ATRX gene. Such mutations in cancer cells are typically heterozygous, with cancer cells comprising one mutant copy of the ATRX gene and one wild-type copy.

In some embodiments, the brain tumour or neuroblastoma comprises an isocitrate dehydrogenase (IDH) 1 and/or 2 deficient phenotype. In some embodiments, the brain tumour or neuroblastoma comprises an IDH1 deficient phenotype. In some embodiments, the brain tumour or neuroblastoma comprises an IDH2 deficient phenotype. In some embodiments, the brain tumour or neuroblastoma comprises an IDH1 and IDH2 deficient phenotype. In some embodiments, the brain tumour or neuroblastoma does not comprise an IDH1 deficient phenotype (i.e. it is IDH1 wild type). In some embodiments, the brain tumour or neuroblastoma does not comprise an IDH2 deficient phenotype (i.e. it is IDH2 wild type). In some embodiments, the brain tumour or neuroblastoma does not comprise an IDH1 or an IDH2 deficient phenotype (i.e. it is IDH1 and IDH2 wild type).

Mutations in IDH1 and IDH2 are prevalent in human malignancies. In glioma, IDH1 and IDH2 mutations are recognised in >80% of World Health Organisation (WHO) grade II/III cases. IDH1 and IDH2 mutations that are associated with cancer tend to localise to the arginine residue that is crucial for the recognition of isocitrate and the enzymes' catalytic activity (R132 for IDH1, R140 or R172 for IDH2). Reducing or abolishing the catalytic activity of these enzymes affects the ability of cells to combat reactive oxygen species (Cohen et al. 2013). The NCBI Entrez Gene ID for human IDH1 is Gene ID: 3417 and the NCBI Entrez Gene ID for human IDH2 is Gene ID: 3418. The amino acid sequence for human IDH1 is provided as NCBI Reference Sequence: NP_001269315.1 and the amino acid sequence for human IDH2 is provided as NCBI Reference Sequence: NP_001276839.1.

As used herein, a brain tumour or neuroblastoma comprising an IDH1 and/or IDH2 deficient phenotype means that IDH1 and/or IDH2 activity is reduced or abolished in the cancer cells. IDH1 or IDH2 activity is typically removed or abolished by means of a mutation in the IDH1 or IDH2 gene, respectively. Thus, in some embodiments a brain tumour or neuroblastoma comprising an IDH1 and/or IDH2 deficient phenotype comprises cancer cells with a mutation in the IDH1 and/or IDH2 gene(s). Such mutations in cancer cells are typically heterozygous, with cancer cells comprising one mutant copy of the IDH1 gene and one wild-type copy. The mutation in the IDH1 gene may be a nonsynonymous mutation at a nucleotide encoding amino acid residue R132 of IDH1, while the mutation in the IDH2 gene may be a nonsynonymous mutation at a nucleotide encoding amino acid residue R140 or R174 of IDH2.

In some embodiments, the brain tumour or neuroblastoma comprises $O^6$-methylguanine-DNA methyltransfer-

ase (MGMT) promoter methylation.

The MGMT gene in humans is located on chromosome 10q26.3 and encodes a highly evolutionarily conserved and ubiquitously expressed enzyme involved in DNA repair. Methylation of the MGMT gene promoter induces a reduction in levels of MGMT protein and has been observed in approximately 50% of GBMs.

In some embodiments, the method comprises a further step of diagnosing the patient as having the brain tumour or neuroblastoma comprising one or more of the above molecular markers (ATRX mutation, IDH mutations and MGMT methylation). Various methods can be used for carrying out the genomic characterisation of these tumours and performing the diagnosis. For example, the diagnosis step may involve surgically isolating cells from the tumour and assaying cells obtained from the brain tumour or neuroblastoma (i.e. a tumour biopsy) from the patient. Alternatively, non-invasive methods can be used. For example, there have been reports of obtaining cell-free circulating tumour DNA from the cerebrospinal fluid (CSF) of patients with brain tumours and carrying assaying the CSF samples (Martinez-Ricarte et al. 2018).

A sample from a patient (e.g. cells from the tumour and/or a CSF sample) can be evaluated for an ATRX mutation, IDH1 and/or IDH2 mutation using any methods known to one skilled in the art. By way of non-limiting example such methods include: magnetic resonance, chemical assays (e.g., High performance liquid chromatography (HPLC)), sequencing and PCR.

Various assays are known and are available for assessing MGMT gene promoter methylation. Suitable assays include those described in Mansouri et al. 2019 and include, for example, methylationspecification PCR, pyrosequencing, quantitative real-time PCR high-resolution melt, Methylationspecific multiplex ligation-dependent probe amplification and immunohistochemistry.

It is also possible to characterise a brain tumour or neuroblastoma based on its level of genomic instability. Studies have demonstrated that DNA damage checkpoint signalling is aberrantly and constitutively active in gliomas, which can be measured using markers of ongoing DNA replication stress such as ygH2AX, phosphorylation of Chk2, Chk1 and Rad17 (Bartkova et al. 2010). In some embodiments, the brain tumour or neuroblastoma is characterised as having a high level of genomic instability, which may be demonstrated by comprising high (e.g. higher than non-tumour cells) levels of $\gamma$gH2AX, phosphorylation of Chk2, Chk1 and Rad17.

**Therapeutic application**

The term "treating", as used herein, unless otherwise indicated, means reversing, alleviating, inhibiting the progress of, delaying the progression of, delaying the onset of, or preventing the disorder or condition to which such term applies, or one or more symptoms of such disorder or condition. The term "treatment", as used herein, unless otherwise indicated, refers to the act of treating as "treating" is defined immediately above. The term "treating" also includes adjuvant and neo-adjuvant treatment of a subject (patient). For the avoidance of doubt, reference herein to "treatment" includes reference to curative, palliative and prophylactic treatment, and to the administration of a medicament for use in such treatment.

In this specification, unless otherwise stated, the phrase "effective amount" means an amount of a compound or composition which is sufficient enough to significantly and positively modify the symptoms and/or conditions to be treated (e.g., provide a positive clinical response). The effective amount of an active ingredient for use in a pharmaceutical composition will vary with the particular condition being treated, the severity of the condition, the duration of the treatment, the nature of concurrent therapy, the particular active ingredient(s) being employed, the particular pharmaceutically-acceptable excipient(s)/carrier(s) utilized, and like factors within the knowledge and expertise of the attending physician.

The PARP inhibitors of the invention may be formulated as pharmaceutical compositions for clinical use and may comprise a pharmaceutically acceptable carrier, diluent or adjuvant. The composition may be formulated for topical, parenteral, intravenous, intramuscular, intrathecal, intraocular, subcutaneous, oral, inhalational or transdermal routes of administration which may include injection. Injectable formulations may comprise the selected compound in a sterile or isotonic medium.

The PARP inhibitors of the invention will normally be administered via the oral route in the form of pharmaceutical preparations comprising the active ingredient or a pharmaceutically acceptable salt or solvate thereof, or a solvate of such a salt, in a pharmaceutically acceptable dosage form. Depending upon the disorder and patient to be treated, the compositions may be administered at varying doses.

The pharmaceutical formulations of the PARP inhibitors described herein may be prepared for oral administration, particularly in the form of tablets or capsules, and especially involving technologies aimed at furnishing colon-targeted drug release (Patel, 2011).

Suitable daily doses of the PARP inhibitors described herein in therapeutic treatment of humans are about 0.0001-100 mg/kg body weight.

Oral formulations are preferred, particularly tablets or capsules which may be formulated by methods known to those skilled in the art to provide doses of the active compound in the range of 0.1 mg to 1000 mg. In some embodiments,

the amount of PARP inhibitor administered to the patient is between 10 mg/day to 500 mg/day, between 20 mg/day to 500 mg/day, or between 40 mg/day to 500 mg/day, or between 10 mg/day to 400 mg/day, between 20 mg/day to 400 mg/day, or between 40 mg/day to 400 mg/day.

## Combinations

In some embodiments, the methods comprise administering the PARP inhibitor in combination with an alkylating chemotherapeutic agent and/or ionizing radiation. Such combination treatment may comprise the separate, sequential or simultaneous administration of the PARP inhibitor and the alkylating chemotherapeutic agent and/or ionizing radiation.

Also provided is a pharmaceutical product comprising i) a PARP inhibitor as defined herein, and ii) an alkylating chemotherapeutic agent. The PARP inhibitor and alkylating chemotherapeutic agent are typically present in separate dosage forms.

The alkylating chemotherapeutic agent may be temozolomide (TMZ).

TMZ is the standard of care (SOC) chemotherapy in GBM (Stupp et al., 2014; Stupp et al, 2010). Treatment may involve administering doses of 150-200 mg/m$^2$/day for 5 days every 28-day cycle. Temozolomide has the structure:

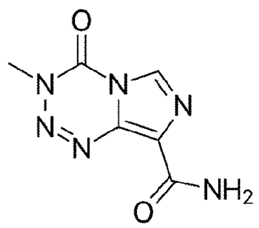

Thus, in some embodiments the method may comprise administering a therapeutically effective amount of a PARP inhibitor as described herein (e.g. between 10 mg/day to 500 mg/day) and administering TMZ at 150-200 mg/m$^2$/day. This treatment may take place across a 28-day cycle, where the TMZ is administered daily for the first 5 days and then stopped for the remaining days of the cycle. The PARP inhibitor may be administered every day of the 28-day cycle, or may only be administered when TMZ is being administered (e.g.. only during the first 5 days of the 28-day cycle).

The data provided herein also demonstrates that a PARP inhibitor described herein can deliver anti-tumour benefit in *in vitro* and *in vivo* models at relatively low doses of TMZ. Without wishing to be bound by theory, it is believed that the PARP inhibitors described herein may be able to achieve a clinical benefit (e.g. tumour reduction) at a lower dose of TMZ (e.g. lower than the SOC dose of TMZ for treating the disease in question).

Thus, in some embodiments, the TMZ is administered at a (e.g. daily) dose of: less than about 200 mg/m$^2$, less than about 175 mg/m$^2$, less than about 150 mg/m$^2$, less than about 125 mg/m$^2$, less than about 100 mg/m$^2$, less than about 75 mg/m$^2$, or less than about 50 mg/m$^2$.

In some embodiments, TMZ is administered at a (e.g. daily) dose of: between about 50 and 200 mg/m$^2$, between about 50 and 175 mg/m$^2$, between about 50 and 150 mg/m$^2$, between about 50 and 125 mg/m$^2$, between about 50 and 100 mg/m$^2$ , between about 50 and 75 mg/m$^2$,between about 75 and 200 mg/m$^2$, between about 75 and 175 mg/m$^2$, between about 75 and 150 mg/m$^2$, between about 75 and 125 mg/m$^2$, between about 75 and 100 mg/m$^2$ , between about 100 and 200 mg/m$^2$, between about 125 and 175 mg/m$^2$, or between about 125 and 150 mg/m$^2$.

The alkylating chemotherapeutic agent may be dianhydrogalactitol (VAL-083). VAL-083 is a blood brain barrier penetrant alkylating agent that induces DNA interstrand cross-links and leads to double stranded DNA breaks (Jimenez-Alcazar et al., 2021).

VAL-083 has been granted FDA and EMA orphan drug designation for the treatment of GBM which has progressed on a previous TMZ treatment regimen. Currently, VAL-083 is in clinical trials for both MGMT methylated and un-methylated GBM patients (NCT03050736, NCT02717962 and NCT03138629), and has the structure:

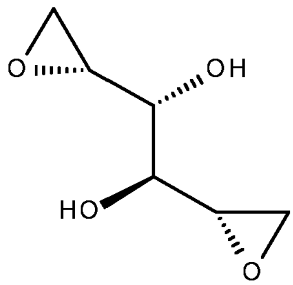
.

VAL-083 at 30 mg/m$^2$ per day, in combination with radiation therapy, was described as generally safe and well tolerated. VAL-083 was administered on days 1, 2 and 3 every 21 days.

Thus, in some embodiments the method may comprise administering a therapeutically effective amount of a PARP inhibitor as described herein (e.g. between 10 mg/day to 500 mg/day) and administering VAL-083 at 10-50 mg/m$^2$/day (e.g. 30 mg/m$^2$/day). This treatment may take place across a 21-day cycle, where the VAL-083 is administered daily for the first 3 days and then stopped for the remaining days of the cycle. The PARP inhibitor may be administered every day of the 21-day cycle, or may only be administered when VAL-083 is being administered (e.g. only during the first 3 days of the 21-day cycle).

In some embodiments, VAL-083 is administered at a (e.g. daily) dose of: less than about 50 mg/m$^2$, less than about 40 mg/m$^2$, less than about 30 mg/m$^2$, or less than about 20 mg/m$^2$.

In some embodiments, VAL-083 is administered at a (e.g. daily) dose of: between about 10 and 50 mg/m$^2$, between about 10 and 40 mg/m$^2$, or between about 10 mg/m$^2$ and 30 mg/m$^2$.

In some embodiments, the PARP inhibitor described herein may be administered in combination with ionizing radiation (e.g. for the treatment of H3K27M glioma). Ionizing radiation may be administered in addition to, or instead of, the alkylating chemotherapeutic agent.

In certain embodiments, the dose of ionizing radiation about 10 Gy, about 15 Gy, about 20 Gy, about 25 Gy, about 30 Gy, about 35 Gy, about 40 Gy, about 45 Gy, about 50 Gy, about 55 Gy, about 60 Gy, about 65 Gy, about 70 Gy, about 75 Gy, about 80 Gy, about 90 Gy, or about 100 Gy. In some embodiments, the dose of ionizing radiation is less than about 100 Gy, less than about 90 Gy, less than about 80 Gy, less than about 70 Gy, less than about 60 Gy, less than about 50 Gy, less than about 45 Gy, less than about 40 Gy, less than about 35 Gy, less than about 30 Gy, less than about 25 Gy, less than about 20 Gy. In certain embodiments, the dose of a radiation therapy is 10 Gy or above, more than about 15 Gy, more than about 20 Gy, more than about 25 Gy, more than about 30 Gy, more than about 35 Gy, more than about 40 Gy, more than about 45 Gy, more than about 50 Gy. In some embodiments, the dose of a radiation therapy is between about 10 Gy and about 100 Gy. In some embodiments, the dose of a radiation therapy is between about 20 Gy and about 80 Gy. In some embodiments, the dose of a radiation therapy is between about 20 Gy and about 60 Gy.

The dose of ionizing radiation may be administered as fractionated radiotherapy (i.e. a fraction of the total dose administered daily over several days / weeks). For example, the doses described above may be administered in 20-30 fractions over 4-6 weeks.

The dose of ionizing radiation administered may depend on the tumour being treated, and/or the age of the patient (e.g. adult or paediatric). For example, in the treatment of primary GBM (IDH wild type and ATRX wild type) a dose of 60 Gy may be used, whereas in the treatment of recurrent GBM (which can be IDH mutant / ATRX mutant), a dose of 35 Gy may be used.

The data provided herein also demonstrates that a PARP inhibitor described herein can deliver anti-tumour benefit in *in vitro* models at relatively low ionizing radiation. Without wishing to be bound by theory, it is believed that the PARP inhibitors described herein may be able to achieve a clinical benefit (e.g. tumour reduction) at a lower dose of ionizing radiation (e.g. lower than the SOC dose of ionizing radiation for treating the disease in question).

Thus, in some embodiments, the ionizing radiation is administered at a dose of less than about 60 Gy, less than about 55 Gy, less than about 50 Gy, less than about 45 Gy, or less than about 40 Gy. In some embodiments, the ionizing radiation is administered at a lose of between about 20 Gy and about 60 Gy, between about 20 Gy and about 55 Gy, between about 20 Gy and about 50 Gy, between about 20 and about 45 Gy, or between about 20 and about 40 Gy, between about 30 Gy and about 60 Gy, between about 30 Gy and about 55 Gy, between about 30 Gy and about 50 Gy, between about 30 Gy and about 45 Gy, between about 30 Gy and about 40 Gy.

**Diseases**

The methods described herein are for the treatment of brain tumours and neuroblastomas.

Examples of brain tumours that can be treated by these methods include gliomas and ependymomas. In some

embodiments, the brain tumour being treated is a glioma.

Gliomas suitable for treatment include adult and paediatric gliomas, as well as high grade and low grade gliomas. Examples of high grade gliomas include oligodendroglioma, anaplastic astrocytoma, glioblastoma, and diffuse midline glioma. Examples of low grade gliomas include pilocytic astrocytoma, optic pathway glioma, tectal glioma, oligodendroglioma, ganglioglioma, and a pleomorphic xanthoastrocytoma. In some embodiments, the brain tumour being treated is a high grade glioma.

In some embodiments, the brain tumour being treated is a glioblastoma. In some embodiments, the brain tumour being treated is a glioblastoma, e.g. an adult glioblastoma. In some embodiments, the brain tumour being treated is a diffuse midline glioma, e.g. a paediatric diffuse midline glioma. In some embodiments, the brain tumour being treated is a H3K27M mutant glioma.

## Brief Description of the Figures

FIG. 1 shows an X-ray powder diffractogram of 6-fluoro-5-[4-[(5-fluoro-2-methyl-3-oxo-4H-quinoxalin-6-yl)methyl]piperazin-1-yl]-N-methyl-pyridine-2-carboxamide Form B

FIG. 2 shows a DSC trace of 6-fluoro-5-[4-[(5-fluoro-2-methyl-3-oxo-4H-quinoxalin-6-yl)methyl]piperazin-1-yl]-N-methyl-pyridine-2-carboxamide Form B

FIG. 3 shows an X-ray powder diffractogram of 6-fluoro-5-[4-[(5-fluoro-2-methyl-3-oxo-4H-quinoxalin-6-yl)methyl]piperazin-1-yl]-N-methyl-pyridine-2-carboxamide Form D

FIG. 4 shows a single crystal structure of 6-fluoro-5-[4-[(5-fluoro-2-methyl-3-oxo-4H-quinoxalin-6-yl)methyl]piperazin-1-yl]-N-methyl-pyridine-2-carboxamide Form D (ORTEP50)

FIG. 5 shows an X-ray powder diffractogram of 6-fluoro-5-[4-[(5-fluoro-2-methyl-3-oxo-4H-quinoxalin-6-yl)methyl]piperazin-1-yl]-N-methyl-pyridine-2-carboxamide MSA salt Form C

FIG. 6 shows a DSC trace of 6-fluoro-5-[4-[(5-fluoro-2-methyl-3-oxo-4H-quinoxalin-6-yl)methyl]piperazin-1-yl]-N-methyl-pyridine-2-carboxamide MSA salt Form C

FIG. 7 shows cell confluence graphs representing the potent PARP inhibitor monotherapy activity in glioma cells with ATRX mutation (SJG2IDH1wt and SJG2IDH1mt) but not wild type ATRX (U87 IDH1wt and U87 IDH1mt). Representative graphs are shown, error bars represent +/- SD

FIG. 8 shows cell confluence graphs representing PARP inhibitor potentiation of TMZ efficacy in U87 IDH1wt (A) and U87 IDH1mt R132H (B) isogenic pairs. Representative graphs are shown from 3 biological replicates, error bars represent +/- SD

FIG. 9 shows cell viability graphs representing PARP inhibitor potentiation of TMZ efficacy in SJ-G2 IDH1wt (A) and SJ-G2 IDH1mt R132H (B) isogenic pairs. Representative graphs are shown, error bars represent +/- SD

FIG. 10 shows anti-tumour efficacy (A) and change in body weight (B) of a U87MG xenograft model dosed with: i) vehicle; ii) Compound 20 alone (3 mg/kg QD); iii) TMZ at a lower dose (6.25 mg/kg QD on days 1-5 and 29-33); iv) TMZ at a higher dose (25 mg/kg QD on days 1-5 and 29-33); and v) the combination of Compound 20 and TMZ at the lower dose.

FIG. 11 shows cell confluence graphs representing PARP inhibitor potentiation of VAL-083 efficacy in U87 IDH1wt (A) and U87 IDH1mt R132H (B) isogenic pairs. Representative graphs are shown, error bars represent +/- SD

FIG. 12 shows cell confluence graphs representing PARP inhibitor potentiation of VAL-083 efficacy in SJ-G2 IDH1wt (A) and SJ-G2 IDH1mt R132H (B) isogenic pairs. Representative graphs are shown, error bars represent +/- SD

FIG. 13 shows cell confluence graphs representing PARP inhibitor potentiation of IR efficacy in BT245 H3K27M paediatric gliomas. Representative graphs are shown, error bars represent +/- SD

## Examples

The compounds of the application will now be further explained by reference to the following non-limiting examples.

## General Experimental Conditions

$^1H$ NMR spectra were obtained using a Bruker 300 MHz, 400 MHz or 500 MHz spectrometer at 27 °C unless otherwise noted; chemical shifts are expressed in parts per million (ppm, $\delta$ units) and are referenced to the residual mono-$^1H$ isotopologue of the solvent ($CHCl_3$: 7.24 ppm; $CHDCl_2$: 5.32 ppm; $CD_3S(=O)CD_2H$: 2.49 ppm). Coupling constants are given in units of hertz (Hz). Splitting patterns describe apparent multiplicities and are designated as s (singlet), d (doublet), t (triplet), q (quartet), m (multiplet) and br s (broad singlet). LC-MS was carried out using a Waters UPLC fitted with a Waters SQD mass spectrometer or Shimadzu LC-20AD LC-20XR LC-30AD with a Shimadzu 2020 mass spectrometer. Reported molecular ions correspond to [M+H]+ unless otherwise noted; for molecules with multiple isotopic patterns (Br, Cl, etc.) the reported value is the one obtained for the lowest isotope mass unless otherwise specified.

Flash chromatography was performed using straight phase flash chromatography on a SP1™ Purification system from Biotage™, CombiFlash®Rf from ISCO or on Gilson system from Thermo Fisher using normal phase silica FLASH+™ (40M, 25M or 12 M) or SNAP™ KP-Sil Cartridges (340, 100, 50 or 10), Flash Column silica-CS columns from Agela, with C18-flash columns or standard flash chromatography. In general, all solvents used were commercially available and of analytical grade. Anhydrous solvents were routinely used for reactions. Phase Separators used in the examples are ISOLUTE® Phase Separator columns. The intermediates and examples named below were named using ACD/Name 12.01 from Advanced Chemistry Development, Inc. (ACD/Labs). The starting materials were obtained from commercial sources or made via literature routes.

### *X-Ray Powder Diffraction (XRPD) Analysis*

XRPD analysis was performed using a Bruker D8 diffractometer, which is commercially available from Bruker AXS Inc™ (Madison, Wisconsin). The XRPD spectra were obtained by mounting a sample (approximately 10 mg) of the material for analysis on a single silicon crystal wafer mount (e.g., a Bruker silicon zero background X-ray diffraction sample holder) and spreading out the sample into a thin layer with the aid of a microscope slide. The sample was spun at 30 revolutions per minute (to improve counting statistics) and irradiated with X-rays generated by a copper long-fine focus tube operated at 40 kV and 40 mA with a wavelength of 1.5406 angstroms (i.e., about 1.54 angstroms). The sample was exposed for 1 second per 0.02 degree 2-theta increment (continuous scan mode) over the range 5 degrees to 40 degrees 2-theta in theta-theta mode. The running time was ~15 min for D8.

XRPD $2\theta$ values may vary with a reasonable range, e.g., in the range $\pm$ 0.2° and that XRPD intensities may vary when measured for essentially the same crystalline form for a variety of reasons including, for example, preferred orientation. Principles of XRPD are described in publications, such as, for example, Giacovazzo, C. et al. (1995), Fundamentals of Crystallography, Oxford University Press; Jenkins, R. and Snyder, R. L. (1996), Introduction to X-Ray Powder Diffractometry, John Wiley & Sons, New York; and Klug, H. P. & Alexander, L. E. (1974), X-ray Diffraction Procedures, John Wiley and Sons, New York.

### *DSC Analysis*

DSC analysis was performed on samples prepared according to standard methods using a Q SERIES™ Q1000 DSC calorimeter available from TA INSTRUMENTS® (New Castle, Delaware). A sample (approximately 2 mg) was weighed into an aluminum sample pan and transferred to the DSC. The instrument was purged with nitrogen at 50 mL/min and data collected between 22 °C and 300 °C, using a dynamic heating rate of 10 °C/minute. Thermal data was analyzed using standard software, *e.g.,* Universal v.4.5A from TA INSTRUMENTS®.

The following abbreviations are used: AcOH = acetic acid; aq = aqueous; BAST = Bis(2-methoxyethyl) aminosulfur Trifluoride ; $Boc_2O$ = di-tert-butyl decarbonate; Boc = t-butyloxycarbonyl; $CDCl_3$ = deuterated chloroform; $CD_3OD$ = deuterated methanol; $CH_3NO_2$ = nitromethane; DAST = Diethylaminosulfur trifluoride; DCE = 1,2-dichloroethane; DCM = dichloromethane; DDQ = 2,3-Dichloro-5,6-dicyano-1,4-benzoquinone; DEA = diethylamine; DEAD = diethyl azodicarboxylate; Dess-martin periodinane = 1,1,1-Tris(acetyloxy)-1,1-dihydro-1,2-benziodoxol-3-(1*H*)-one; DIPEA = *N,N*-diisopropylethylamine; DMAP = 2,6-dimethylaminopyridine; DMF = N,N-dimethylformamide; DMSO = dimethylsulfoxide; DMSO-$d_6$ = deuterated dimethylsulfoxide; DPPA = diphenyl phosphorazidate; dppf = 1,1'-bis(diphenylphosphino)ferrocene; DIAD = Di-isopropyl (E)-diazene-1,2-dicarboxylate; DSC = differential scanning calorimetry; DTAD = Di-tert-butyl (E)-diazene-1,2-dicarboxylate; ee = enantiomeric excess; eq. = equivalent; ESI or ES = electrospray ionization; $Et_2O$ = diethyl ether; EtOAc or EA =ethylacetate; EtOH =ethanol; FA = formic acid; Grubbs catalyst (1,3-Dimesitylimidazolin-2-ylidene)(tricyclohexylphosphine)ruthenium dichloride; h = hour(s); HATU = (dimethylamino)-N,N-dimethyl(3-oxido-1*H*-[1,2,3]triazolo[4,5-*b*]pyridinyl)methaniminium hexafluorophosphate; HCl = hydrochloric acid; $H_2O_2$ = hydrogen peroxide; HP = high pressure; IPA = isopropylalcohol; KF = potassium fluoride; LC = liquid chromatography; $LiClO_4$ = lithium perchlorate; mmol = millimole; mCPBA = meta-chloroperoxybenzoic acid; MeOH = methanol; min = minute(s); MeCN or CH3CN or ACN = acetonitrile; $MeNO_2$ = nitromethane; MS = mass = spectrometery; NBS = N-Bromosuccinimide; NH4Cl = ammonium chloride; NMP = *N*-methyl-2-pyrrolidone; NMR = nuclear magnetic resonance; Pd/C = Palladium on carbon; $Pd_2dba_3$ = Tris(dibenzylideneacetone)dipalladium (0); $PdCl_2$(dppf) = 1,1'-bis(di-tert-butylphosphino)ferrocene palladium dichloride; PE = Petroleum ether; $PPh_3$ =Triphenylphosphine; rt =room temperature; Rt or RT = retention time; Ruphos Pd G3 = (2-Dicyclohexylphosphino-2' ,6' -diisopropoxy-1,1' -biphenyl)[2-(2' -amino-1,1' biphenyl)]palladium(II) methanesulfonate; Pd-PEPPSI™-IPent = Dichloro[1,3-bis(2,6-Di-3-pentylphenyl)imidazol-2-ylidene](3-chloropyridyl)palladium(II), [1,3-Bis(2,6-Di-3-pentylphenyl)imidazol-2-ylidene](3-chloropyridyl)dichloropalladium(II), [1,3-Bis(2,6-Di-3-pentylphenyl)imidazol-2-ylidene](3-chloropyridyl)palladium(II) dichloride; Xphos Pd G2 = Chloro(2-dicyclohexylphosphino-2' ,4' ,6' - triisopropyl-1,1' -biphenyl)[2-(2' -amino-1,1' -biphenyl)]palladium(II), X-Phos aminobiphenyl palladium chloride; CataCXium A-Pd-G2 = Chloro[(di(1-adamantyl)-N-butylphosphine)-2-(2-aminobiphenyl)]palladium(II); sat = saturated; SFC = supercritical fluid chromatography; T3P = 2,4,6-tripropyl-1,3,5,2,4,6-trioxatriphosphinane 2,4,6-trioxide; PPh3O = triphenylphosphine oxide; TBTU = 2-(1H-benzo[d][1,2,3]triazol-1-yl)-1,1,3,3-tetramethylisouronium tetrafluoroborate; TFA = trifluoroacetic acid; THF = tetrahydrofuran; TLC = thin layer chromatography; TMS = trimethylsilyl; Xantphos = 4,5-bis(diphenylphosphino)-9,9-dimethylxanthene; CBr4 = Carbon tetrabromide; HBr = hydrobromic acid; Cs2CO3 = Cesium carbonate; MgSO4 = Magnesium sulfate; NaHCO3 = Sodium bicarbonate; DDQ = 2,3-Dichloro-5,6-dicyano-1,4-benzoquinone; SOCl2 = Thionyl chloride; DIBAL-H = Diisobutylaluminium hydride; NH4HCO3 = Ammonium bicarbonate; BINAP = 2,2'-bis(diphenylphosphino)-1,1'-binaphthyl; SM = starting material; CH2Cl2 = dichloromethane; Et3N = triethylamine; HCO2H = formic acid; LCMS = liquid chromatography-mass spectrometry; N2 = dinitrogen; Na2SO4 = sodium sulfate; NH4CO3 = ammonium carbonate; UV = ultraviolet; XPhos Pd G2 = Chloro(2-dicyclohexylphosphino-2',4',6'-triisopropyl-1,1'-biphenyl)[2-(2'-amino-1,1'-biphenyl)]palladium(II); Pd(OAc)2 = palladium(II) acetate, ppt = precipitate.

**Preparation of Examples**

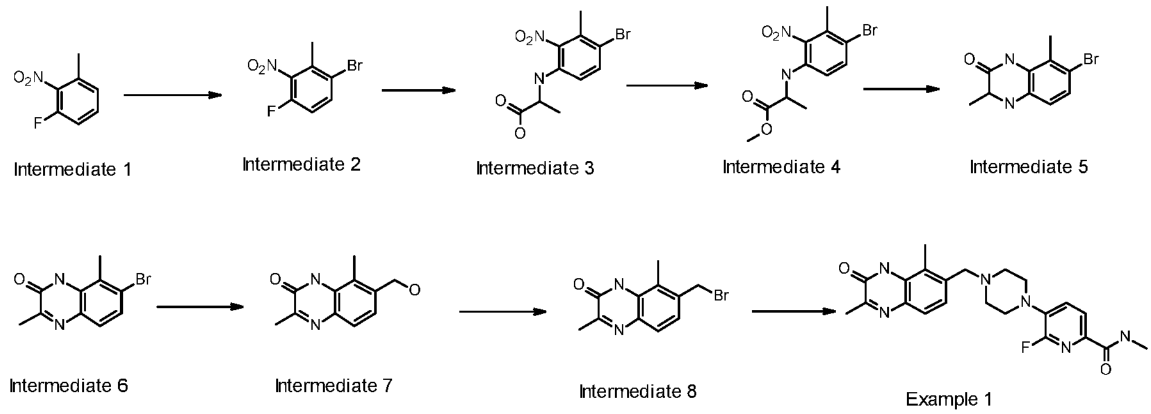

Intermediate 1
Intermediate 2
Intermediate 3
Intermediate 4
Intermediate 5
Intermediate 6
Intermediate 7
Intermediate 8
Example 1

***Intermediate 2:*** *1-bromo-4-fluoro-2-methyl-3-nitro-benzene*

To a solution of 1-fluoro-3-methyl-2-nitro-benzene (10.7 g, 68.98 mmol) **(*intermediate 1*)** in TFA (50 mL) was added conc. $H_2SO_4$ (20 mL) at 0°C slowly, followed by addition of NBS (13.50 g, 75.87 mmol) in portions. After addition, the mixture was stirred at room temperature for 4h. The resulting mixture was poured onto ice and the precipitate that formed collected by filtration, washed with water and dried under vacuum to give 1-bromo-4-fluoro-2-methyl-3-nitro-benzene **(*intermediate 2*)** as a white solid (14.80 g, 92 %). 1H NMR (500 MHz, CHLOROFORM-d) 2.43 (3H, s), 7.03 (1H, t), 7.68 (1H, dd).

***Intermediate 3:*** *2-(4-bromo-3-methyl-2-nitro-anilino)propanoic acid*

A mixture of 1-bromo-4-fluoro-2-methyl-3-nitro-benzene (13.8 g, 58.97 mmol) **(*intermediate 2*),** alanine (6.30 g, 70.76 mmol) and potassium carbonate (24.45 g, 176.90 mmol) in DMF (15 mL) was stirred at 100 °C for 5 h, then the

temperature was raised to 110 °C and stirred for 5 h. The mixture was poured onto ice, quenched slowly with 1M HCl aq. solution (~ 300 ml) at 0°C gave a yellow suspension. The solid was collected by filtration, washed with water and dried in a vacuum oven for 2 days at 50°C to give 2-(4-bromo-3-methyl-2-nitro-anilino)propanoic acid (14.03 g, 78 %) (***intermediate 3***) as a yellow solid (some impurities present). 1H NMR (500 MHz, DMSO-d6) 1.39 (3H, d), 2.28 (3H, s), 4.20 (1H, quin), 6.12 (1H, br d), 6.68 (1H, d), 7.58 (1H, d), 12.98 (1H, br s); m/z ($ES^+$) $[M+H]^+$ = 303.

***Intermediate 4:*** *methyl 2-(4-bromo-3-methyl-2-nitro-anilino)propanoate*

To a solution of 2-(4-bromo-3-methyl-2-nitro-anilino)propanoic acid (14.9 g, 49.16 mmol) ***(intermediate* 3)** in MeOH (150 mL) was added thionyl chloride (10.76 mL, 147.47 mmol) dropwise at 0°C and the mixture was stirred at rt for overnight. LCMS indicated full conversion. The reaction mixture was quenched slowly with aq. sat. $NaHCO_3$ solution at 0°C (~ 300 ml) to give an orange suspension. The solid was collected by filtration, washed with water and dried to yield the crude product (14.6g). The solid was purified on silica gel column (eluted with 0 to 25% ethyl acetate in hexanes), to give methyl 2-(4-bromo-3-methyl-2-nitro-anilino)propanoate **(*intermediate 4*)** as a bright orange solid (12.74g, 82 %). 1H NMR (500 MHz, CHLOROFORM-d) 1.52 (3H, d), 2.43 (3H, s), 3.76 (3H, s), 4.14 (1H, quin), 5.83 (1H, br d), 6.45 (1H, d), 7.48 (1H, d); m/z ($ES^+$) $[M+H]^+$ = 317.

***Intermediate 5:*** *7-bromo-3,8-dimethyl-3,4-dihydro-1H-quinoxalin-2-one*

To a stirred mixture of methyl 2-(4-bromo-3-methyl-2-nitro-anilino)propanoate (11.6 g, 36.58 mmol) ***(intermediate 4),*** zinc (23.91 g, 365.77 mmol) and ammonium chloride (19.56 g, 365.77 mmol) in MeOH (100 mL) was added small ice piece at 0 °C (exothermic). The reaction mixture was then stirred for 15 min at 0 °C (ice bath). Water (2 mL) was added and the resulting mixture was stirred at r.t for 15min. The bright orange color disappeared. The mixture was filtered through filter paper, washed with methanol and the filtrate was concentrated under vacuum. The residue was diluted with ethyl acetate and washed with water followed by brine. Organic layer was dried (anhydrous $Na_2SO_4$), filtered and concentrated to give a mixture of methyl 2-(2-amino-4-bromo-3-methyl-anilino)propanoate and 7-bromo-3,8-dimethyl-3,4-dihydro-1H-quinoxalin-2-one (9.8 g).

To the solution of above solid in MeOH (100 mL) was added 2ml of 4 M HCl in dioxanes at r.t and the mixture was stirred at r.t for 10min. Another 100 ml methanol was added (to make free suspension) and the resulting suspension was stirred at r.t for 1hr. The mixture was diluted with ether (~ 200 ml), the solid was collected by filtration and washed with ether. The filtrate was concentrated until a solid precipitate and the solid was collected by filtration. This procedure was repeated couple of times to yield first portion of the product 7.2g. The filtrate was concentrated and purified on silica gel column (eluted with 0 to 100% ethyl acetate in hexanes), and the product fraction were concentrated and resulting material was combined with above material to give 7-bromo-3,8-dimethyl-3,4-dihydro-1H-quinoxalin-2-one (9.10 g, 98 %) ***(intermediate 5*)** as an off white solid. 1H NMR (500 MHz, DMSO-d6) 1.23 (3H, d), 2.24 (3H, s), 3.68 (1H, q), 3.75 (br, 1H), (6.54 (1H; d), 7.00 (1H, d), 9.77 (1H, s); m/z ($ES^+$) $[M+H]^+$ = 255.

***Intermediate 6:*** *7-bromo-3,8-dimethyl-1H-quinoxalin-2-one*

DDQ (8.91 g, 39.24 mmol) was added to a suspension of 7-bromo-3,8-dimethyl-3,4-dihydro-1H-quinoxalin-2-one (9.1 g, 35.67 mmol) (***intermediate 5***) in $CH_2Cl_2$ (400 mL) at room temperature and the mixture was stirred for overnight. LCMS indicated clean conversion. Solvent was removed under reduced pressure, sat. $NaHCO_3$ (~ 300 ml) solution was added and the yellow suspension was stirred at rt for 4 h. The solid was collected by filtration and washed with water. The solid was slurry in sat. $NaHCO_3$ (100 ml) and stirred at rt for 1h. The solid was filtered, washed with water followed ether and dried to yield 7-bromo-3,8-dimethyl-1H-quinoxalin-2-one (7.29 g, 81 *%)* ***(intermediate* 6)** as an off white solid. 1H NMR (500 MHz, DMSO-d6) 2.40 (3H, s), 2.50 (3H, s) (overlapped with DMSO-d6 peak), 7.32 - 7.65 (2H, m), 11.76 (1H, br s); m/z ($ES^+$) $[M+H]^+$ = 253.

***Intermediate 7:*** *7-(hydroxymethyl)-3,8-dimethyl-1H-quinoxalin-2-one*

A mixture of (tributylstannyl)methanol (1142 mg, 3.56 mmol), 7-bromo-3,8-dimethyl-1H-quinoxalin-2-one (600 mg, 2.37 mmol) ***(intermediate* 6)** and Xphos Pd G2 (280 mg, 0.36 mmol) in 1,4-dioxane (40 mL) was stirred at 80 °C for 18 h. The solvent was removed under reduced pressure and the residue was purified on silica gel column (eluted with 0 to 15% methanol in DCM) to yield 7-(hydroxymethyl)-3,8-dimethyl-1H-quinoxalin-2-one (225 mg, 46 %) (***intermediate 7***) as an off white solid. 1H NMR (500 MHz, DMSO-d6) 2.31 (3H, s), 2.40 (3H, s), 4.58 (2H, d), 5.22 (1H, t), 7.33 (1H, d), 7.52 (1H, d), 11.53 (1H, br s); m/z ($ES^+$) $[M+H]^+$ = 205.

***Intermediate 8:*** *7-(bromomethyl)-3,8-dimethyl-1H-quinoxalin-2-one*

7-(hydroxymethyl)-3,8-dimethyl-1H-quinoxalin-2-one (223 mg, 1.09 mmol) ***(intermediate 7)*** in HBr (15 ml, 132.59 mmol) (48w% in water) was stirred at 80 °C for 3.5h. Solvent was removed under reduced pressure, diethyl ether was added to the residue, mixture was sonicated and the solid was collected to yield 7-(bromomethyl)-3,8-dimethyl-1H-quinoxalin-2-one (408 mg, 107 %) **(*intermediate 8*)** as a yellow solid. 1H NMR (500 MHz, DMSO-d6) 2.36 - 2.45 (6H, m), 4.83 (2H, s), 7.34 (1H, d), 7.53 (1H, d), 11.63 (1H, br s); m/z ($ES^+$) $[M+H]^+$ = 267, 269.

***Example 1:*** *5-[4-[(2,5-dimethyl-3-oxo-4H-quinoxalin-6-yl)methyllpiperazin-1-ylf-6-fluoro-N-methyl-pyridine-2-carboxamide*

To the suspension of 7-(bromomethyl)-3,8-dimethyl-1H-quinoxalin-2-one, HBr (37 mg, 0.10 mmol) **(*intermediate 8*)** was added ACN (5 ml), 6-fluoro-N-methyl-5-(piperazin-1-yl)picolinamide, 2HCl (31.5 mg, 0.10 mmol) **(*intermediate 32*)** and DIPEA (79 μl, 0.45 mmol) and the reaction mixture was stirred at 70°C for 1h to give a light yellow suspension. The suspension was cooled to rt, 1 drop of water was added, the solid was collected by filtration, washed with acetonitrile three time and dried to yield 5-[4-[(2,5-dimethyl-3-oxo-4H-quinoxalin-6-yl)methyl]piperazin-1-yl]-6-fluoro-N-methyl-pyridine-2-carboxamide (0.016 g, 33 %) **(*example 1*)** as a yellow solid. 1H NMR (500 MHz, DMSO-d6) 2.07 (3H, br s), 2.42 (3H, br d), 2.56 (4H, br s), 2.76 (3H, br s), 3.14 (4H, br s), 3.61 (2H, br s), 7.23 (1H, br d), 7.42 - 7.67 (2H, m), 7.83 (1H, brd), 8.38 (1H, br s), 11.13 - 11.97 (1H, m); m/z ($ES^+$) $[M+H]^+$ = 425.

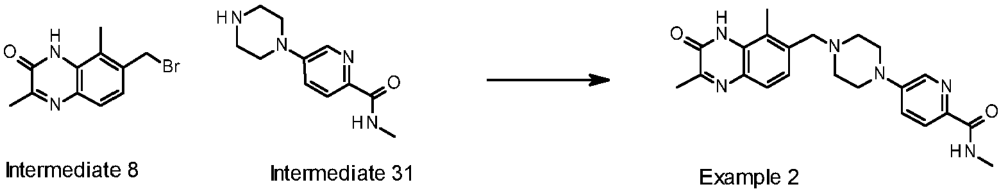

Intermediate 8 Intermediate 31 Example 2

***Example 2:*** *5-[4-[(2,5-dimethyl-3-oxo-4H-quinoxalin-6-yl)methyl]piperazin-1-yl]-N-methyl-pyridine-2-carboxamide*

To a suspension of 7-(bromomethyl)-3,8-dimethylquinoxalin-2(1H)-one, HBr (240 mg, 0.69 mmol) **(*intermediate 8*),** N-methyl-5-piperazin-1-yl-pyridine-2-carboxamide, 2HCl (202 mg, 0.69 mmol) **(*intermediate 31*)** in acetonitrile (13 mL) was added DIPEA (0.723 mL, 4.14 mmol) and the resulting mixture was stirred at 70 °C for 3 h. The mixture was concentrated and purified on reverse phase (C18 column, eluted with 0 to 100% ACN/water (0.2% ammonium hydroxide)) to yield the product as a brown solid. The solid was suspended in a mixture was DCM and MeOH (2:1), concentrated to remove DCM and the solid was filtered and washed with methanol. The solid was suspended in ACN (3 ml), 0.8 ml of 1M HCl in water was added, diluted with water (~ 3ml) and lyophilized to dryness to yield the HCl salt of the product 5-[4-[(2,5-dimethyl-3-oxo-4H-quinoxalin-6-yl)methyl]piperazin-1-yl]-N-methyl-pyridine-2-carboxamide (0.033g, 11%) **(*example 2*)**. 1HNMR (500 MHz, DMSO-d6) 2.45 (3H, s), 2.54 (3H, s), 2.81 (3H, br d), 3.24 - 3.56 (6H, m), 3.88 - 4.02 (2H, m), 4.54 (2H, br s), 7.48 - 7.71 (3H, m), 7.97 (1H, br d), 8.33 (1H, br d), 8.59 (1H, br s), 11.28 (1H, br s), 11.48 - 11.95 (1H, m); m/z ($ES^+$) $[M+H]^+$ = 407.

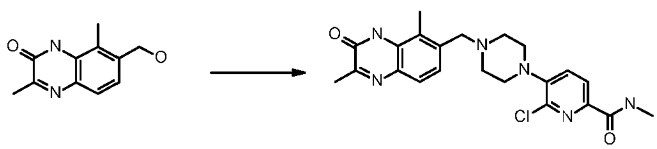

Intermediate 7 Example 3

***Example 3:*** *6-chloro-5-[4-[(2,5-dimethyl-3-oxo-4H-quinoxalin-6-yl)methyl]piperazin-1-yl]-N-methyl-pyridine-2-carboxamide*

HBr in AcOH (2 mL, 0.18 mmol) (33 wt%) was added to the solution 7-(hydroxymethyl)-3,8-dimethyl-1H-quinoxalin-2-one (36 mg, 0.18 mmol) **(*intermediate 7*)** in NMP (2 mL). The resulting mixture was stirred at 100 °C for 1 hour. The solvent was removed under reduced pressure. DIPEA (0.25 mL, 1.43 mmol) was added to a solution of 6-chloro-N-methyl-5-(piperazin-1-yl)picolinamide (48 mg, 0.19 mmol) **(*intermediate 30*)** in NMP (2 mL). The resulting mixture was stirred at 100 °C for 18 hours. The crude product was purified by preparative HPLC (column: YMC-Actus Triart C18, 30*250,5μm; Mobile Phase A: Water (0.05%$NH_3H_2O$), Mobile Phase B: ACN; Flow rate: 60 mL/min; Gradient: 41 B

to 61 B in 7 min; 254; 220 nm. Fractions containing the desired compound were evaporated to dryness to yield 6-chloro-5-[4-[(2,5-dimethyl-3-oxo-4H-quinoxalin-6-yl) methyl]piperazin-1-yl]-N-methyl-pyridine-2-carboxamide (33.0 mg, 42 %) **(*example 3*)** as a white solid. 1H NMR (400 MHz, DMSO-d6) 2.41 (3H, s), 2.43 (3H, s), 2.54 - 2.62 (4H, m), 2.78 (3H, d), 3.05 - 3.11 (4H, m), 3.62 (2H, s), 7.24 (1H, d), 7.51 (1H, d), 7.65 (1H, d), 7.92 (1H, d), 8.41 - 8.45 (1H, m), 11.56 (1H, s); m/z (ES$^+$) [M+H]$^+$ = 441.

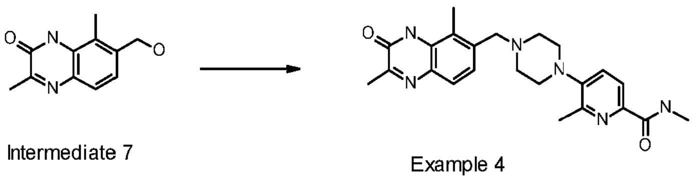

Intermediate 7

Example 4

***Example 4:*** *5-[4-f(2,5-dimethyl-3-oxo-4H-quinoxalin-6-yl)methyllpiperazin-1-yl]-N,6-dimethyl-pyridine-2-carboxamide*

HBr in AcOH (2 mL, 12.15 mmol) (33 wt %) was added to the solution of 7-(hydroxymethyl)-3,8-dimethyl-1H-quinoxalin-2-one (43 mg, 0.21 mmol) **(intermediate 7)** in NMP (2 mL). The resulting mixture was stirred at 80 °C for 1 hour. The solvent was removed under reduced pressure. To the solution of the resulting solid in NMP (3 mL) was added DIPEA (0.25 mL, 1.43 mmol) and N,6-dimethyl-5-(piperazin-1-yl)picolinamide (42 mg, 0.18 mmol) **(*intermediate 33*)**. The resulting mixture was stirred at 100 °C for 18 hours. The crude product was purified by preparative HPLC (Column: YMC-Actus Triart C18, 30*250,5μm; using water in acetonitrile (0.05%$NH_4OH$. Fractions containing the desired compound were evaporated to dryness to afford 5-[4-[(2,5-dimethyl-3-oxo-4H-quinoxalin-6-yl)methyl]piperazin-1-yl]-N,6-dimethyl-pyridine-2-carboxamide **(*example 4*)** (18.40 mg, 24 %) as a white solid. 1H NMR (400 MHz, DMSO-d6) 2.40 (3H, s), 2.43 (3H, s), 2.48 (3H, s), 2.55 - 2.63 (4H, m), 2.79 (3H, d), 2.87 - 2.94 (4H, m), 3.62 (2H, s), 7.24 (1H, d), 7.46 (1H, d), 7.51 (1H, d), 7.78 (1H, d), 8.39 - 8.44 (1H, m), 11.56 (1H, s); m/z (ES$^+$) [M+H]$^+$ = 421.

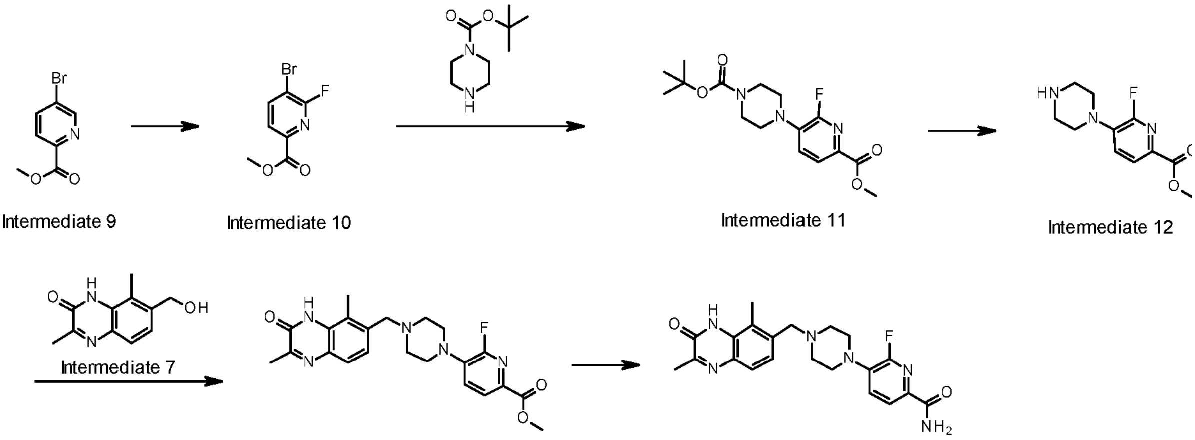

Intermediate 9

Intermediate 10

Intermediate 11

Intermediate 12

Intermediate 7

Intermediate 13

Example 5

***Intermediate 10:*** *methyl 5-bromo-6-fluoro-pyridine-2-carboxylate*

A dried flask was charged with methyl 5-bromopicolinate **(*intermediate 9*)** (24 g, 111.09 mmol) in acetonitrile (300 ml), silver(II) fluoride (50 g, 342.78 mmol) was added, the mixture was stirred at r.t for 1 day under $N_2$. LCMS indicated about 70% conversion. Another batch of AgF2 (16 g) was added and the resulting mixture was continued to stir at rt for overnight. The mixture was filtered through ceilite, washed with acetonitrile followed by DCM and the filtrate was concentrated to yield a light brown solid. The residue was partitioned between DCM and sat. $NH_4Cl$ solution which gave a white suspension. The solid was filtered off and discarded. The filtrate was transferred to separating funnel, organic layer was separated, and the aqueous layer was extracted with ethyl acetate (150 ml x 3). The organics were combined, dried (anhydrous $Na_2SO_4$), filtered and concentrated until solid precipitates out. The solid was collected by filtration. washed with ether, dried to yield a flaky off white solid. The combined filtrate was concentrated again and the solid was collected by filtration to give combined 19.96 g of product. The rest of the filtrate was concentrated and purified on silica gel column (eluted with 0 to 25% ethyl acetate in hexanes) to yield a second portion of the desired product as a white flaky solid 3.5 g. All the material was combined to yield methyl 5-bromo-6-fluoro-pyridine-2-carboxylate (23.46 g, 90%) **(*intermediate 10).*** 1H NMR (500 MHz, DMSO-d6) 3.89 (3H, s), 7.93 (1H, d), 8.51 (1H, t); m/z (ES$^+$) [M+H]$^+$ = 234.

***Intermediate 11:*** *tert-butyl 4-(2-fluoro-6-methoxycarbonyl-3-pyridyl)piperazine-1-carboxylate*

A mixture of tert-butyl piperazine-1-carboxylate (28.0 g, 150.37 mmol), methyl 5-bromo-6-fluoro-pyridine-2-carboxylate **(*intermediate 10*)** (23.46 g, 100.25 mmol), RuPhos Pd G3 (5.45 g, 6.52 mmol) and $Cs_2CO_3$ (82 g, 250.61 mmol) in 1,4-dioxane (400 mL) was stirred at 80 °C for overnight under $N_2$. The reaction mixture was diluted with water (250 ml) and extracted with ethyl acetate (250 ml). The organic layer was washed with brine, the water layer was extracted with ethyl acetate (100 ml x 1), the organics were dried (anhydrous $Na_2SO_4$), filtered and concentrated, the residue was purified on silica gel column (eluted with 0 to 50% ethyl acetate in hexanes) to yield the product as a yellow solid, the solid was recrystallized from ethyl acetate/hexanes, filtered, washed with hexanes, dried to yield the product as a crystalline white solid (24.8 g); The filtrate was concentrated and repurified on silica gel column to yield more of the product 1.9 g. In total yield tert-butyl 4-(2-fluoro-6-methoxycarbonyl-3-pyridyl)piperazine-1-carboxylate **(*intermediate 11*)** (26.7 g, 78 %). 1H NMR (500 MHz, CHLOROFORM-d) 1.51 (9H, s), 3.12 - 3.28 (4H, m), 3.48 - 3.67 (4H, m), 3.98 (3H, s), 7.27 (1H, d), 7.99 (1H, dd); m/z ($ES^+$) $[M+H]^+$ = 340.

***Intermediate 12:*** *methyl 6-fluoro-5-piperazin-1-yl-pyridine-2-carboxylate*

To a mixture of tert-butyl 4-(2-fluoro-6-methoxycarbonyl-3-pyridyl)piperazine-1-carboxylate (1.9 g, 5.60 mmol) **(intermediate 11)** in MeOH (10 mL) was added HCl 4 M in dioxane (10 ml, 40.00 mmol) at rt and the resulting mixture was stirred at r.t for 1 h. The mixture was diluted with ether, the solid was collected by filtration, washed with ether and dried under vacuum to yield methyl 6-fluoro-5-piperazin-1-yl-pyridine-2-carboxylate (1.360 g, 78 %) (**intermediate 12**) as a white solid. 1H NMR (500 MHz, DMSO-d6) 3.24 (4H, br s), 3.46 (4H, br s), 3.84 (3H, s), 7.65 (1H, br t), 7.94 (1H, br d), 9.43 (2H, br s); m/z ($ES^+$) $[M+H]^+$ = 240.

***Intermediate 13:*** *methyl 5-[4-[(2,5-dimethyl-3-oxo-4H-quinoxalin-6-yl)methyllpiperazin-1-yl]-6-fluoro-pyridine-2-carboxylate*

7-(hydroxymethyl)-3,8-dimethylquinoxalin-2(1H)-one (223 mg, 1.09 mmol) (**intermediate 7**) in HBr (15 ml, 132.59 mmol) (48w% in water) was stirred at 80 °C for 3.5 h. The solvent was removed under reduced pressure, DCM was added to the residue and concentrated to yield 7-(bromomethyl)-3,8-dimethylquinoxalin-2(1H)-one as a yellow solid.

To the solution of above solid in acetonitrile (20 ml) was added methyl6-fluoro-5-piperazin-1-yl-pyridine-2-carboxylate, 2HCl (260 mg, 0.83 mmol) (***intermediate 12***), and DIPEA (1.907 ml, 10.92 mmol) at rt and the reaction mixture was stirred at 70°C for 2 h. The mixture was cooled to rt, 0.5 ml of water was added and the solid was collected by filtration and washed with acetonitrile. The solid was dried to yield an off white solid as methyl 5-[4-[(2,5-dimethyl-3-oxo-4H-quinoxalin-6-yl)methyl]piperazin-1-yl]-6-fluoro-pyridine-2-carboxylate (0.371 g, 80 %) (***intermediate 13***). 1H NMR (500 MHz, DMSO-d6) 2.42 (6H, m), 2.52 - 2.59 (4H, m), 3.20 (4H, br d), 3.61 (2H, s), 3.82 (3H, s), 7.23 (1H, d), 7.41 - 7.59 (2H, m), 7.91 (1H, d), 11.55 (1H, s); m/z ($ES^+$) $[M+H]^+$ = 426.

***Example 5:*** *5-[4-[(2,5-dimethyl-3-oxo-4H-quinoxalin-6-yl)methyl]piperazin-1-yl]-6-fluoro-pyridine-2-carboxamide*

A sealed 40 ml vial was charged with methyl 5-[4-[(2,5-dimethyl-3-oxo-4H-quinoxalin-6-yl)methyl]piperazin-1-yl]-6-fluoro-pyridine-2-carboxylate (360 mg, 0.85 mmol) **(*intermediate 13*)** and ammonia (15 ml, 105.00 mmol, 7 N in methanol) and the mixture was stirred at 50 °C for overnight. LCMS indicated there was still some starting material left. The mixture was concentrated, 10 ml of 7 N ammonia in methanol was added to the solid and the mixture was stirred at 50 °C for 4 h gave a white suspension. The solid was collected by filtration, washed with hexanes and dried to yield 5-[4-[(2,5-dimethyl-3-oxo-4H-quinoxalin-6-yl)methyl]piperazin-1-yl]-6-fluoro-pyridine-2-carboxamide ***(example 5)***(340 mg, 98 %) as a white solid. 1H NMR (500 MHz, DMSO-d6) 2.40 (3H, s), 2.43 (3H, s), 2.56 (4H, br s), 3.14 (4H, br s), 3.61 (2H, s), 7.23 (1H, d), 7.46 (1H, br s), 7.48 - 7.58 (2H, m), 7.76 (1H, br s), 7.84 (1H, br d), 11.11 - 11.70 (1H, m); m/z ($ES^+$) $[M+H]^+$ = 411.

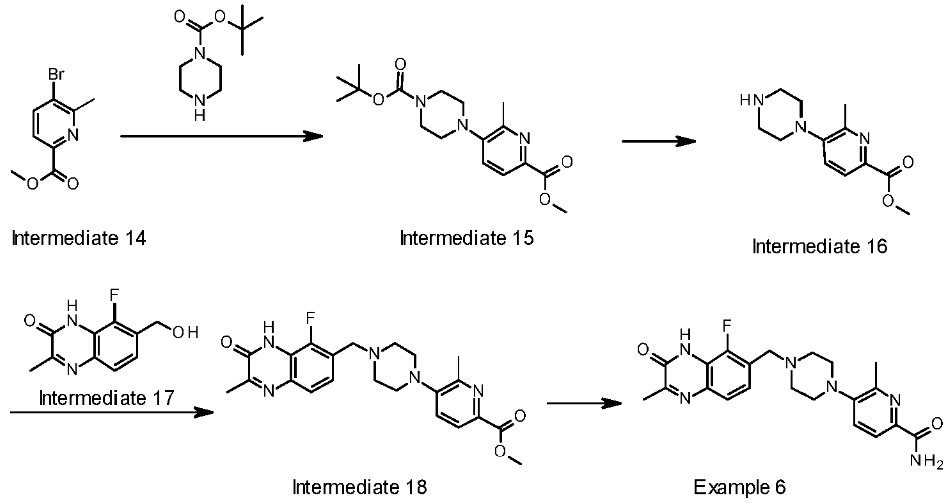


Intermediate 14 Intermediate 15 Intermediate 16

Intermediate 18 Example 6

***Intermediate 15:*** *tert-butyl 4-(6-methoxycarbonyl-2-methyl-3-pyridyl)piperazine-1-carboxylate*

A 40 mL vial fitted with septa cap was charged with methyl 5-bromo-6-methylpicolinate (**intermediate 14**) (2 g, 8.69 mmol), tert-butyl piperazine-1-carboxylate (3.24 g, 17.39 mmol), $Cs_2CO_3$ (5.66 g, 17.39 mmol) and Ruphos Pd G3 (0.727 g, 0.87 mmol). The reaction vial was evacuated under vacuum and filled with nitrogen. 1,4-dioxane (20 mL) was added and the reaction vial was placed in a heating block pre-heated to 80 °C and stirred for 16 h. The reaction mixture was cooled, diluted with water and extracted with ethyl acetate, the organic layer was washed with brine, dried over anhydrous $Na_2SO_4$ and concentrated. The residue was purified on silica gel column (eluted with 0 to 50% ethyl acetate in hexanes) to yield tert-butyl 4-(6-methoxycarbonyl-2-methyl-3-pyridyl)piperazine-1-carboxylate (***intermediate 15***)(2.090 g, 72 %) as a light-yellow solid. 1H NMR (500 MHz, DICHLOROMETHANE-d2) 1.49 (9H, s), 2.59 (3H, s), 2.88 - 3.00 (4H, m), 3.55 - 3.65 (4H, m), 3.92 (3H, s), 7.32 (1H, d), 7.92 (1H, d); m/z (ES$^+$) [M+H]$^+$ = 336.

***Intermediate 16:*** *methyl 6-methyl-5-piperazin-1-yl-pyridine-2-carboxylate*

4 M solution of hydrogen chloride in 1,4-dioxane (31.2 ml, 124.63 mmol) was added to a stirred solution of tert-butyl 4-(6-(methoxycarbonyl)-2-methylpyridin-3-yl)piperazine-1-carboxylate (***intermediate 15***) (4.18 g, 12.46 mmol) in DCM (30 mL) and the resulting solution was stirred at rt for 18 h. Solvent was removed under vacuum, the resulting solid was slurried in diethyl ether and solid was collected by filtration to give methyl 6-methyl-5-piperazin-1-yl-pyridine-2-carboxylate (***intermediate 16***) (3.80 g, 99 %) as a light-yellow solid; m/z (ES$^+$) [M+H]$^+$ = 236.

***Intermediate 18:*** *methyl 5-[4-[(5-fluoro-2-methyl-3-oxo-4H-quinoxalin-6-yl)methyl]piperazin-1-yl]-6-methyl-pyridine-2-carboxylate*

Triphenylphosphine (1.584 g, 6.04 mmol) (4.4 g added, calculated based on PPh3 loading of 1.6 mmol/g) was added to a stirred slurry of 8-fluoro-7-(hydroxymethyl)-3-methylquinoxalin-2(1H)-one (419 mg, 2.01 mmol) (***intermediate 17***) and perbromomethane (1.335 g, 4.03 mmol) in DCM (40 mL) at rt. The resulting mixture was stirred for 1 h. Reaction mixture was filtered, washed with DCM and THF and the filtrate was concentrated under vacuum to yield 7-(bromomethyl)-8-fluoro-3-methylquinoxalin-2(1H)-one as a light yellow solid.

To the slurry of above freshly prepared 7-(bromomethyl)-8-fluoro-3-methylquinoxalin-2(1H)-one in acetonitrile (25 mL) was added methyl 6-methyl-5-piperazin-1-yl-pyridine-2-carboxylate, 2HCl (590 mg, 1.91 mmol) **(intermediate 16),** and N-ethyl-N-isopropylpropan-2-amine (1754 μl, 10.07 mmol) and the reaction was heated to 70°C for 1h. The reaction mixture was cooled to rt, concentrated, and quenched with sat. aqueous $NaHCO_3$ solution and stirred for 1 h. Solid was isolated by filtration and washed with water. The crude solid was purified on silica column chromatography using 0-10% MeOH in DCM to yield methyl 5-[4-[(5-fluoro-2-methyl-3-oxo-4H-quinoxalin-6-yl)methyl]piperazin-1-yl]-6-methyl-pyridine-2-carboxylate **(*intermediate* 18)** (0.263 g, 31 %). 1H NMR (500 MHz, DMSO-d6) 2.40 - 2.49 (6H, m), 2.62 (4H, br s), 2.97 (4H, br s), 3.72 (2H, s), 3.83 (3H, s), 7.30 (1H, t), 7.44 (1H, d), 7.52 (1H, d), 7.85 (1H, d), 12.45 (1H, br s); 19F NMR (471 MHz, DMSO-d6) -135.54 (1F, s); m/z (ES$^+$) [M+H]$^+$ = 426.

***Example 6:*** *5-[4-[(5-fluoro-2-methyl-3-oxo-4H-quinoxalin-6-yl)methyllpiperazin-1-yl]-6-methyl-pyridine-2-carboxamide*

7 N ammonia in methanol (16.47 ml, 115.26 mmol) was added to methyl 5-[4-[(5-fluoro-2-methyl-3-oxo-4H-quinoxalin-6-yl)methyl]piperazin-1-yl]-6-methyl-pyridine-2-carboxylate **(intermediate 18)** (0.2452 g, 0.58 mmol) in a 40 mL scintillation vial, sealed and stirred at rt for 18 h. Additional 7 N $NH_3$ solution (15 mL) was added to the reaction mixture and stirred at 50°C for overnight. The reaction was concentrated under vacuum, slurry in 5 mL MeOH. Solid was filtered off, washed with methanol and dried to yield 5-[4-[(5-fluoro-2-methyl-3-oxo-4H-quinoxalin-6-yl)methyl]piperazin-1-yl]-6-methyl-pyridine-2-carboxamide **(Example 6)**(0.151 g, 64 %) as an off-white solid. 1H NMR (500 MHz, DMSO-d6) 2.42 (3H, s), 2.45 - 2.49 (3H, m), 2.52 - 2.69 (4H, m), 2.94 (4H, br s), 3.71 (2H, s), 7.29 (1H, t), 7.40 - 7.54 (3H, m), 7.77 - 7.84 (2H, m), 12.43 (1H, br s); 19F NMR (471 MHz, DMSO-d6) -135.53 (1F, s); m/z ($ES^+$) $[M+H]^+$ = 411.

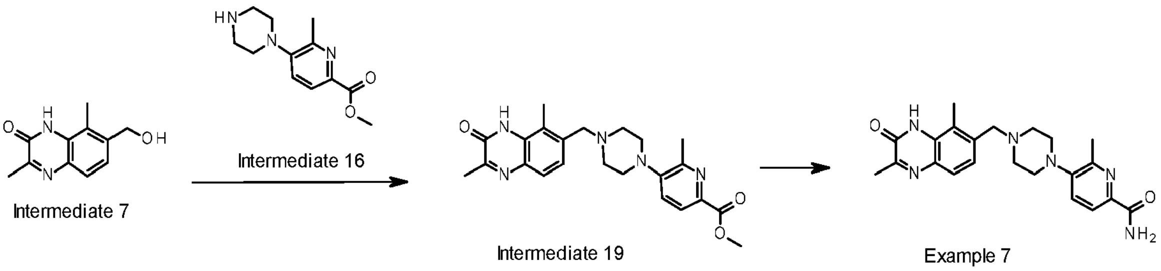

Intermediate 7
Intermediate 19
Example 7

***Intermediate 19:** methyl 5-[4-[(2,5-dimethyl-3-oxo-4H-quinoxalin-6-yl)methyl]piperazin-1-yl]-6-methyl-pyridine-2-carboxylate*

7-(hydroxymethyl)-3,8-dimethylquinoxalin-2(1H)-one (***intermediate 7***) (223 mg, 1.09 mmol) in HBr (15 ml, 132.59 mmol) (48w% in water) was stirred at 80 °C for 4 h. Solvent was removed under reduced pressure, DCM was added to the residue, mix was sonicated and concentrated to yield 7-(bromomethyl)-3,8-dimethylquinoxalin-2(1H)-one as a yellow solid.

To the slurry of above in acetonitrile (20 ml) was added methyl 6-methyl-5-piperazin-1-yl-pyridine-2-carboxylate, 2HCl (***intermediate 16***) (337 mg, 1.09 mmol), and DIPEA (1.907 ml, 10.92 mmol). The reaction mixture was stirred at 70 °C for 2 h gave clear solution. The resulting mixture was cooled to r.t, half of the solvent was removed, 0.5 ml of water was added. The solid was collected by filtration, washed with acetonitrile and dried to yield a yellow solid. This solid was purified on silica gel column (eluted with 0 to 20% methanol in DCM) to Yield methyl 5-[4-[(2,5-dimethyl-3-oxo-4H-quinoxalin-6-yl)methyl]piperazin-1-yl]-6-methyl-pyridine-2-carboxylate (***intermediate 19***) (213 mg, 46 %) as an off white solid. 1H NMR (500 MHz, DMSO-d6) 2.41 (3H, s), 2.43 (3H, s), 2.47 (3H, s), 2.58 (4H, br s), 2.95 (4H, br s), 3.63 (2H, s), 3.82 (3H, s), 7.24 (1H, d), 7.43 (1H, d), 7.51 (1H, d), 7.84 (1H, d), 11.55 (1H, s). m/z ($ES^+$) $[M+H]^+$ = 422.

***Example 7:** 5-[4-[(2,5-dimethyl-3-oxo-4H-quinoxalin-6-yl)methyl]piperazin-1-yl]-6-methyl-pyridine-2-carboxamide*

A sealed 40 ml vial was charged with methyl 5-[4-[(2,5-dimethyl-3-oxo-4H-quinoxalin-6-yl)methyl]piperazin-1-yl]-6-methyl-pyridine-2-carboxylate (***intermediate 19***) (210 mg, 0.50 mmol) and ammonia (15 ml, 105.00 mmol, 7 N in methanol) and the reaction was stirred at 50 °C for overnight. Reaction was not complete. The mixture was concentrated, 10 ml of 7 N ammonia in methanol was added to this solid. Vial was capped and stirred at 50 °C for 4 h to give a white suspension. The mixture was cooled to rt, the solid was collected by filtration, washed with hexanes and dried to yield 5-[4-[(2,5-dimethyl-3-oxo-4H-quinoxalin-6-yl)methyl]piperazin-1-yl]-6-methyl-pyridine-2-carboxamide *(example* 7)(191 mg, 94 %) as a white solid. 1H NMR (500 MHz, DMSO-d6) 2.41 (3H, s), 2.44 (3H, s), 2.49 (3H, s), 2.58 (4H, br s), 2.92 (4H, br s), 3.63 (2H, br s), 7.24 (1H, brd), 7.42 (1H, br s), 7.46 (1H, br d), 7.51 (1H, br d), 7.79 (2H, br d), 10.53 - 11.23 (1H, m); m/z ($ES^+$) $[M+H]^+$ = 407.

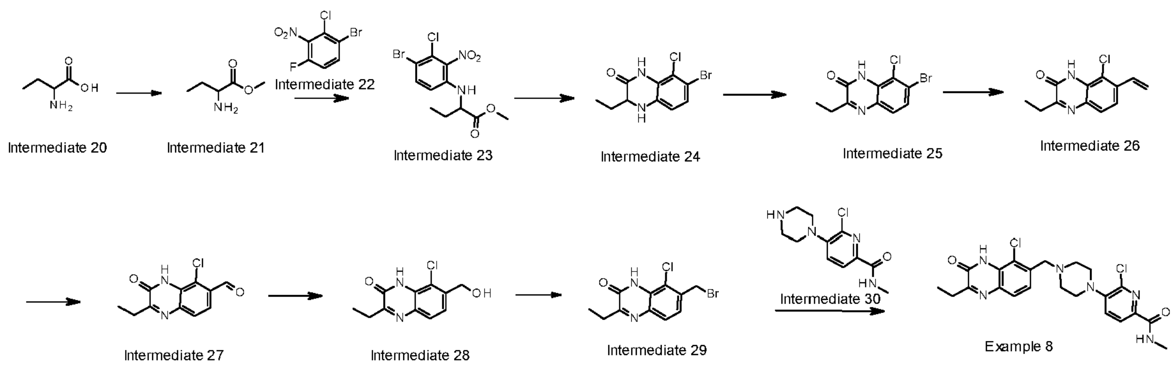

Intermediate 20
Intermediate 21
Intermediate 23
Intermediate 24
Intermediate 25
Intermediate 26
Intermediate 27
Intermediate 28
Intermediate 29
Example 8

***Intermediate 21:*** *methyl 2-aminobutanoate*

A slurry of 2-aminobutanoic acid (***intermediate 20***) (5 g, 48.49 mmol) in methanol (35 mL) was cooled in an ice bath. Thionyl chloride (11 mL, 150.72 mmol) was added dropwise to the above mixture at 0°C. The reaction was allowed to warm to rt and stirred overnight. The clear solution was concentrated to dryness to obtain a residue. The resulting solid was suspended into ether, filtered, washed with ether and dried to yield methyl 2-aminobutanoate.HCl (***intermediate 21***) (7.35 g, 99 %) as a white solid as HCl salt. 1H NMR (500 MHz, DMSO-d6) 0.92 (3H, t), 1.76 - 1.93 (2H, m), 3.75 (3H, s), 3.95 - 4.05 (1H, m), 8.53 (3H, br s).

***Intermediate 23:*** *methyl 2-(4-bromo-3-chloro-2-nitro-anilino)butanoate*

A flask was charged with methyl 2-aminobutanoate, HCl (***intermediate 21***)(1.811 g, 11.79 mmol), 1-bromo-2-chloro-4-fluoro-3-nitrobenzene (***intermediate 22***) (2.0 g, 7.86 mmol) in 1,4-dioxane (30 mL), DIPEA (8.24 mL, 47.16 mmol) was added and the mixture was stirred at 105°C for 24h. The mixture was concentrated, and the residue was purified on silica gel column (eluted with 0 to 50% ethyl acetate in hexanes) to yield methyl 2-(4-bromo-3-chloro-2-nitro-anilino)butanoate (***intermediate 23***) (2.100 g, 76 %) as a bright yellow oil, turned into yellow solid after standing. 1H NMR (500 MHz, CHLOROFORM-d) 0.99 (3H, t), 1.78 - 1.89 (1H, m), 1.91 - 2.02 (1H, m), 3.77 (3H, s), 4.04 (1H, q), 5.63 (1H, br d), 6.55 (1H, d), 7.52 (1H, d); m/z ($ES^+$) $[M+H]^+$ = 351.

***Intermediate 24:*** *7-bromo-8-chloro-3-ethyl-3,4-dihydro-1H-quinoxalin-2-one*

Sodium dithionite (3.05 g, 17.49 mmol) was added to a stirred mixture of methyl 2-(4-bromo-3-chloro-2-nitro-anilino)butanoate (***intermediate 23***) (2.05 g, 5.83 mmol) in DMSO (50 mL) and the mixture was stirred at 120 °C for 3 h. The mixture was quenched with water and extracted with ethyl acetate (50 ml x 2). The organic layer was dried (anhydrous $Na_2SO_4$), filtered, concentrated and the residue was purified on silica gel column (0 to 55% ethyl acetate in hexanes) to yield peak 1 as 7-bromo-8-chloro-3-ethyl-1H-quinoxalin-2-one (***intermediate 25***) (0.319 g, 19 %) as a light yellow solid. 1H NMR (500 MHz, METHANOL-d4) 1.31 (3H, t), 2.89 (2H, q), 7.56 - 7.67 (2H, m); m/z ($ES^+$) $[M+H]^+$ = 287, 289 and peak 2 as 7-bromo-8-chloro-3-ethyl-3,4-dihydro-1H-quinoxalin-2-one (***intermediate 24***) (0.895 g, 53%) as a yellow oil which turned into a yellow solid upon standing. 1H NMR (500 MHz, CHLOROFORM-d) 1.04 (3H, t), 1.75 - 1.84 (1H, m), 1.85 - 1.93 (1H, m), 3.89 (1H, dd), 6.51 (1H, d), 7.12 (1H, d), 7.82 (1H, br s). m/z ($ES^+$) $[M+H]^+$ = 289, 291.

***Intermediate 25:*** *7-bromo-8-chloro-3-ethyl-1H-quinoxalin-2-one*

DDQ (772 mg, 3.40 mmol) was added to a mixture of 7-bromo-8-chloro-3-ethyl-3,4-dihydro-1H-quinoxalin-2-one (***intermediate 24***)(895 mg, 3.09 mmol) in 1,4-dioxane (20 mL) at rt and the resulting suspension was stirred at rt for 3 h. LCMS indicated full conversion. The solvent was removed under reduced pressure and the residue was treated with sat. $NaHCO_3$ solution, stirred at rt for 2h. Solid was collected by filtration, washed with sat. $NaHCO_3$ solution, water and dried to yield 7-bromo-8-chloro-3-ethyl-1H-quinoxalin-2-one (***intermediate 25***) (780 mg, 88 %) as an off white solid. 1H NMR (500 MHz, METHANOL-d4) 1.31 (3H, t), 2.89 (2H, q), 7.56 - 7.67 (2H, m); m/z ($ES^+$) $[M+H]^+$ = 287, 289.

***Intermediate 26:*** *8-chloro-3-ethyl-7-vinyl-1H-quinoxalin-2-one*

A mixture of 7-bromo-8-chloro-3-ethyl-1H-quinoxalin-2-one (***intermediate 25***) (1.05 g, 3.65 mmol), tributyl(vinyl) stannane (1.737 g, 5.48 mmol) and $Pd(PPh_3)_4$ (0.422 g, 0.37 mmol) in toluene (50 mL) was stirred at 110 °C under $N_2$ for 2h. LCMS indicated about 44% starting material left. The mixture was continued to stir at this temperature for 4.5 h and then at 80°C for overnight. The mixture was concentrated, purified on silica gel column (eluted with 0 to 100% ethyl acetate in hexanes) to yield low soluble desired product 8-chloro-3-ethyl-7-vinyl-1H-quinoxalin-2-one (***intermediate 26***) (0.850 g, 99 %) as a light yellow solid. m/z ($ES^+$) $[M+H]^+$ = 235 (the product was contaminated by $PPh_3O$).

***Intermediate 27:*** *5-chloro-2-ethyl-3-oxo-4H-quinoxaline-6-carbaldehyde*

Osmium tetroxide in $H_2O$ (0.568 mL, 0.07 mmol) was added to a solution of 8-chloro-3-ethyl-7-vinyl-1H-quinoxalin-2-one (***intermediate 26***) (850 mg, 3.62 mmol), 2,6-lutidine (0.844 mL, 7.24 mmol) and sodium periodate (3099 mg, 14.49 mmol) in THF (50 mL)/water (10 mL)/ tert-butanol (3.46 mL, 36.22 mmol) and stirred at rt for overnight gave a yellow suspension. Reaction was concentrated, partitioned between water, sat. $NH_4Cl$ solution and DCM and the layers were separated. The aqueous layer was extracted with DCM and the combined organic layer was dried (anhydrous $Na_2SO_4$), filtered and concentrated. The residue was purified on silica gel column (eluted with 0 to 50% ethyl acetate in hexanes) to yield 5-chloro-2-ethyl-3-oxo-4H-quinoxaline-6-carbaldehyde (***intermediate 27***) (808 mg, 94 %) as a light

yellow solid. 1H NMR (500 MHz, CHLOROFORM-*d*) 1.34 - 1.42 (3H, m), 3.03 (2H, q), 7.88 (2H, d), 8.99 - 9.38 (1H, m), 10.54 (1H, s); m/z (ES$^+$) [M+H]$^+$ = 237.

***Intermediate 28:** 8-chloro-3-ethyl-7-(hydroxymethyl)-1H-quinoxalin-2-one*

5-chloro-2-ethyl-3-oxo-4H-quinoxaline-6-carbaldehyde (***intermediate 27***) (808 mg, 3.41 mmol) in MeOH (30 mL) was cooled to 0 °C and sodium borohydride (1292 mg, 3.41 mmol) (10 wt% on basic alumina) was added in one portion. The reaction mixture was continued to stir at 0 °C for 40 min. LCMS indicated some starting material remaining. Another 213 mgs of the $NaBH_4$ (10 wt%) was added to this mixture and stirring was continued at 0 °C for 10 min. To the mixture was added 1 ml of water, concentrated and the residue was purified on silica gel column (eluted with 0 to 25% methanol in DCM) to yield 8-chloro-3-ethyl-7-(hydroxymethyl)-1H-quinoxalin-2-one (***intermediate 28***) (625 mg, 77 %) (contaminated by 26% of over reduced side product). 1H NMR (500 MHz, METHANOL-d4) 1.27 - 1.34 (3H, m), 2.91 (2H, q), 4.79 - 4.82 (2H, m), 7.54 (1H, d), 7.75 (1H, d); m/z (ES$^+$) [M+H]$^+$ = 239.

***Intermediate 29:** 7-(bromomethyl)-8-chloro-3-ethyl-1H-quinoxalin-2-one*

carbon tetrabromide (1612 mg, 4.86 mmol) was added in one portion to a solution of 8-chloro-3-ethyl-7-(hydroxymethyl)-1H-quinoxalin-2-one (***intermediate 28***) (580 mg, 2.43 mmol) and triphenylphosphine (1275 mg, 4.86 mmol) in $CH_2Cl_2$ (40 mL) at 0 °C and the mixture was stirred at 0 °C for 1 h. LCMS indicated full conversion. The solvent was removed under reduced pressure and the residue was purified on silica gel column (eluted with 0 to 50% ethyl acetate in hexanes) to yield pure 7-(bromomethyl)-8-chloro-3-ethyl-1H-quinoxalin-2-one (***intermediate 29***)(200mgs, 27 %) as a white solid. 1H NMR (500 MHz, DMSO-d6) 1.22 (3H, t), 2.83 (2H, q), 4.85 (2H, s), 7.51 (1H, d), 7.71 (1H, d), 11.89 (1H, br s); m/z (ES$^+$) [M+H]$^+$ = 301, 303.

***Example 8:** 6-chloro-5-[4-[(5-chloro-2-ethyl-3-oxo-4H-quinoxalin-6-yl)methyl]piperazin-1-yl]-N-methyl-pyridine-2-carboxamide*

DIPEA (0.058 mL, 0.33 mmol) was added to a stirred suspension of 7-(bromomethyl)-8-chloro-3-ethyl-1H-quinoxalin-2-one (***intermediate 29***) (25 mg, 0.08 mmol) and 6-chloro-N-methyl-5-piperazin-1-yl-pyridine-2-carboxamide, 2HCl (***intermediate 30***) (27.2 mg, 0.08 mmol) in acetonitrile (4 mL) and the resulting mixture was stirred at 70 °C for 1.5 h gave a suspension,. LCMS indicated full conversion. The solvent was removed under reduced pressure and submitted to analytical purification group which after purification gave 6-chloro-5-[4-[(5-chloro-2-ethyl-3-oxo-4H-quinoxalin-6-yl)methyl]piperazin-1-yl]-N-methyl-pyridine-2-carboxamide (***example 8***) (24.00 mg, 61 %) as a yellow solid. Purification conditions (achiral): column (Xbridge C18 19mm x 100mm 5μm, mobile phase A: $H_2O$ with 0.2% $NH_4OH$ PH 10, mobile phase B: Acetonitrile; Gradient B%: 13-95%B over 8 min; Flow rate: 20ml/min; Concentration: 35 mg/ml in DMSO; Loading (mg/injection): 15; Column temperature: room temperature. 1H NMR (500 MHz, DMSO-d6) 1.23 (3H, t), 2.66 (4H, br s), 2.76 - 2.92 (5H, m), 3.13 (4H, br s), 3.77 (2H, s), 7.44 (1H, d), 7.69 (2H, dd), 7.94 (1H, d), 8.43 (1H, q), 10.75 - 11.45 (1H, m); m/z (ES$^+$) [M+H]$^+$ = 475.

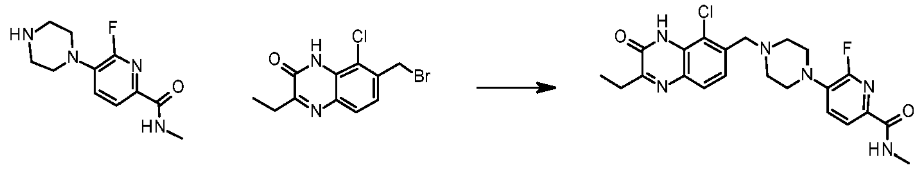

Intermediate 32 Intermediate 29 Example 9

***Example 9:** 5-[4-[(5-chloro-2-ethyl-3-oxo-4H-quinoxalin-6-yl)methyllpiperazin-1-yl]-6-fluoro-N-methylpyridine-2-carboxamide*

DIPEA (0.116 mL, 0.66 mmol) was added to a stirred suspension of 6-fluoro-N-methyl-5-piperazin-1-yl-pyridine-2-carboxamide, 2HCl (***intermediate 32***) (51.6 mg, 0.17 mmol) and 7-(bromomethyl)-8-chloro-3-ethylquinoxalin-2(1H)-one (***intermediate 29***) (50 mg, 0.17 mmol) in acetonitrile (4 mL) and the resulting mixture was stirred at 70 °C for 1.5h. LCMS indicated full conversion. The solvent was removed under reduced pressure and the residue was purified on silica gel column (eluted with 0 to 20% methanol in DCM) to give mixture of product and PPh3O. Material was submitted for analytical group for purification which after purification yield 5-[4-[(5-chloro-2-ethyl-3-oxo-4H-quinoxalin-6-yl)methyl]piperazin-1-yl]-6-fluoro-N-methyl-pyridine-2-carboxamide (***example 9***) (47.0 mg, 62 %) as a yellow solid. Purification conditions (achiral): column (Xbridge C18 19mm x 100mm 5μm, mobile phase A: $H_2O$ with 0.2% $NH_4OH$

PH 10, mobile phase B: Acetonitrile; Gradient B%: 13-95%B over 8 min; Flow rate: 20ml/min; Concentration: 35 mg/ml in DMSO; Loading (mg/injection): 15; Column temperature: room temperature.

1H NMR (500 MHz, DMSO-d6) 1.22 (3H, t), 2.63 (4H, br s), 2.76 (3H, d), 2.82 (2H, q), 3.19 (4H, br s), 3.75 (2H, s), 7.43 (1H, d), 7.52 - 7.64 (1H, m), 7.70 (1H, d), 7.84 (1H, d), 8.39 (1H, q), 11.17 - 11.55 (1H, m); m/z ($ES^+$) $[M+H]^+$ = 459.

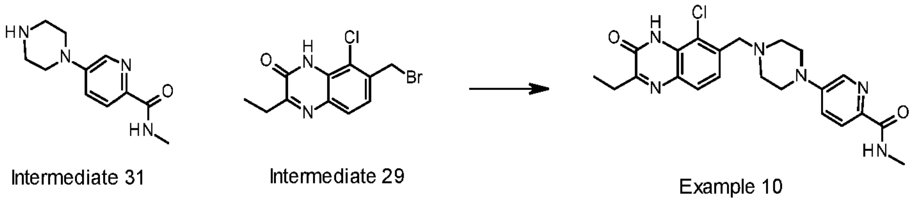

Intermediate 31 Intermediate 29 Example 10

***Example 10:*** *5-[4-[(5-chloro-2-ethyl-3-oxo-4H-quinoxalin-6-yl)methyl]piperazin-1-yl]-N-methylpyridine-2-carboxamide*

DIPEA (0.081 mL, 0.46 mmol) was added to a stirred suspension of N-methyl-5-(piperazin-1-yl)picolinamide, 2HCl (***intermediate 31***) (34.0 mg, 0.12 mmol) and 7-(bromomethyl)-8-chloro-3-ethylquinoxalin-2(1H)-one (***intermediate 29***) (35 mg, 0.12 mmol) in acetonitrile (4 mL) and the resulting mixture was stirred at 70 °C for 1.5 h. LCMS indicated full conversion. The solvent was removed under reduced pressure and the resulting residue was submitted to analytical group for purification which after purification gave 5-[4-[(5-chloro-2-ethyl-3-oxo-4H-quinoxalin-6-yl)methyl]piperazin-1-yl]-N-methylpyridine-2-carboxamide (***example 10***) (13.00 mg, 20 %) as a white solid. Purification conditions (achiral) column (Xbridge C18 19mm x 100mm 5μm, mobile phase A: $H_2O$ with 0.2% $NH_4OH$ PH 10, mobile phase B: Acetonitrile; Gradient B% 13-95%B over 8 min; Flow rate: 20ml/min; Concentration: 35 mg/ml in DMSO; Loading (mg/injection): 15; Column temperature: room temperature. 1H NMR (500 MHz, DMSO-d6) 1.24 (3H, t), 2.79 (3H, d), 2.86 (2H, q), 3.22 - 3.37 (8H, m, merged into water peak), 4.39 - 4.65 (2H, m), 7.46 (1H, dd), 7.57 (1H, br d), 7.79 - 7.90 (2H, m), 8.32 (1H, d), 8.43 (1H, br d), 11.87 - 12.21 (1H, m). m/z ($ES^+$) $[M+H]^+$ = 441.

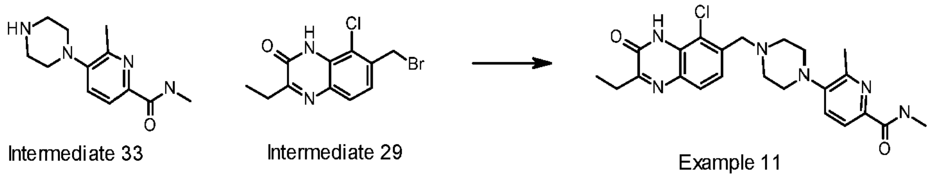

Intermediate 33 Intermediate 29 Example 11

***Example 11:*** *5-[4-[(5-chloro-2-ethyl-3-oxo-4H-quinoxalin-6-yl)methyl]piperazin-1-yl]-N,6-dimethylpyridine-2-carboxamide*

DIPEA (0.111 mL, 0.64 mmol) was added to a stirred suspension of N,6-dimethyl-5-piperazin-1-yl-pyridine-2-carboxamide, 2HCl (***intermediate 33***) (48.9 mg, 0.16 mmol) and 7-(bromomethyl)-8-chloro-3-ethyl-1H-quinoxalin-2-one (***intermediate 29***)(48 mg, 0.16 mmol) in acetonitrile (10 mL) and the resulting mixture was stirred at 70 °C for 2h gave a suspension. LCMS indicated full conversion. The mixture was cooled to rt, solid was collected by filtration, washed with water and dried to yield 5-[4-[(5-chloro-2-ethyl-3-oxo-4H-quinoxalin-6-yl)methyl)piperazin-1-yl]-N,6-dimethyl-pyridine-2-carboxamide (***example 11***)(42.0 mg, 58 %) as a white solid. 1H NMR (500 MHz, DMSO-d6) 1.22 (3H, t), 2.65 (4H, br s), 2.78 - 2.88 (5H, m), 2.95 (4H, br s), 3.38 (3H, s, overlapped water peak), 3.76 (2H, s), 7.47 (2H, dd), 7.71 (1H, d), 7.79 (1H, d), 8.42 (1H, brd), 11.59 - 11.99 (1H, m); m/z ($ES^+$) $[M+H]^+$ = 455.

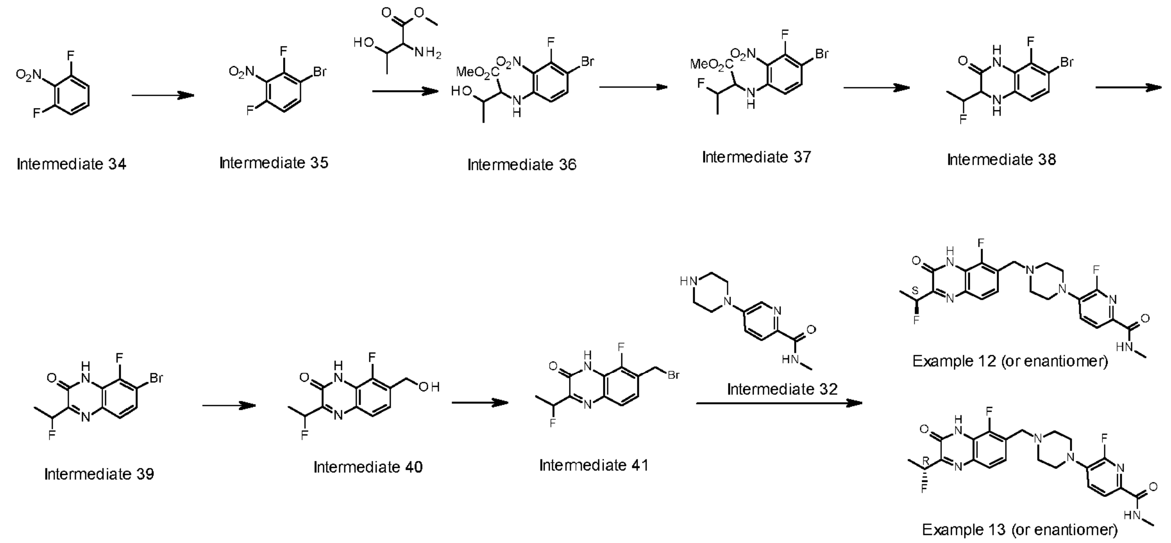

Intermediate 34 Intermediate 35 Intermediate 36 Intermediate 37 Intermediate 38

Intermediate 32

Example 12 (or enantiomer)

Intermediate 39 Intermediate 40 Intermediate 41

Example 13 (or enantiomer)

***Intermediate* 35:** *1-bromo-2,4-difluoro-3-nitro-benzene*

A mixture of 1,3-difluoro-2-nitrobenzene (***intermediate 34***) (19.5 g, 122.57 mmol) and NBS (26.2 g, 147.08 mmol) in sulfuric acid (150 mL) was stirred at 80 °C for overnight. LCMS indicated full conversion. The mixture was cooled to rt and slowly poured onto ice. This mixture was extracted with ethyl acetate (200 ml), the organic layer was washed with water (50 ml x 2), sat. $NaHCO_3$ solution (50ml x 2), brine, dried (anhydrous $Na_2SO_4$), filtered and concentrated. The residue was purified on silica gel column (eluted with 0 to 20% ethyl acetate in hexanes) to yield 1-bromo-2,4-difluoro-3-nitro-benzene ***(intermediate 35)*** (26.8 g, 92 %) as a light-yellow oil. 1H NMR (500 MHz, DMSO-d6) 7.42 - 7.73 (1H, m), 8.06 - 8.26 (1H, m); m/z ($ES^+$) $[M+H]^+$ = 238.

***Intermediate* 36:** *methyl 2-(4-bromo-3-fluoro-2-nitro-anilino)-3-hydroxy-butanoate*

DIPEA (8.56 mL, 48.99 mmol) was added slowly to a stirred solution of 1-bromo-2,4-difluoro-3-nitrobenzene ***(intermediate 35)*** (5.3 g, 22.27 mmol) and methyl 2-amino-3-hydroxybutanoate, HCl (4.53 g, 26.72 mmol) in 1,4-dioxane (50 mL) at r.t and the resulting mixture was stirred at 40 °C for 3 h. LCMS indicated some starting material remaining. To the mixture was added 800 mgs of the DL-threonine methyl ester HCl salt and the mixture was then continued to stir at 40 °C for overnight. The solvent was removed under reduced pressure and the residue was purified on silica gel column (eluted with 0 to 30% ethyl acetate in hexanes) to yield methyl 2-(4-bromo-3-fluoro-2-nitro-anilino)-3-hydroxy-butanoate (***intermediate 36***) (4.69 g, 60 %) as a bright orange solid. (HNMR indicated it was a mixture of diastereomers). 1H NMR (500 MHz, CHLOROFORM-d) 1.30 - 1.44 (3H, m), 3.81 (3H, s), 4.00 - 4.22 (1H, m), 4.22 - 4.48 (1H, m), 6.32 - 6.68 (1H, m), 7.40 - 7.66 (2H, m); m/z ($ES^+$) $[M+H]^+$ = 351, 353.

***Intermediate 37:*** *methyl 2-(4-bromo-3-fluoro-2-nitro-anilino)-3-fluoro-butanoate*

DAST (0.919 mL, 6.95 mmol) was added slowly over 10 min to a mixture of methyl 2-(4-bromo-3-fluoro-2-nitro-anilino)-3-hydroxy-butanoate (***intermediate 36***) (2.22 g, 6.32 mmol) in $CH_2Cl_2$ (40 mL) at 0 °C, the mixture was stirred at 0 °C for 20 min. LCMS and TLC showed starting material remaining. To the mixture was added 0.4 ml of DAST and reaction was stirred another 10 min. The mixture was quenched with sat. $NaHCO_3$ solution and extracted with DCM. The organic layer was dried (anhydrous $Na_2SO_4$), filtered and concentrated to yield a yellow oil. Resulting residue was purified on silica gel column (eluted with 0 to 30 % ethyl acetate in hexanes) to yield peak 2 as methyl 2-(4-bromo-3-fluoro-2-nitro-anilino)-3-fluoro-butanoate (1.110 g, 50 %) **(intermediate 37)** as a yellow oil and peak 4 as starting material methyl 2-((4-bromo-3-fluoro-2-nitrophenyl)amino)-3-hydroxybutanoate (0.300 g, 13 %) along with other byproduct. 1H NMR (500 MHz, CHLOROFORM-d) 1.37 - 1.51 (3H, m), 2.80 - 2.93 (0.5H, m), 3.00 (0.5H, br d), 3.75 (1.5H, s), 3.85 (1.5H, s), 4.40 - 4.56 (0.5H, m), 5.01 - 5.23 (0.5H, m), 6.43 - 6.58 (0.5H, m), 6.71 - 6.74 (0.5H, m), 7.38 - 7.69 (2H, m). m/z ($ES^+$) $[M+H]^+$ = 353.

***Intermediate 38:*** *7-bromo-8-fluoro-3-(1-fluoroethyl)-3,4-dihydro-1H-quinoxalin-2-one*

To a mixture of methyl 2-(4-bromo-3-fluoro-2-nitro-anilino)-3-fluoro-butanoate **(intermediate 37)**(1.11 g, 3.14

mmol), Zinc (2.466 g, 37.72 mmol) and ammonium chloride (3.36 g, 62.87 mmol) in MeOH (20 mL) was added water (2 mL) and the mixture was stirred at rt for 10 min. The orange color disappeared (exothermic) and LCMS showed full conversion. The mixture was filtered, the solid washed with methanol and the filtrate was concentrated. The resulting residue was dissolved DCM and the organic was washed with water, dried (anhydrous $Na_2SO_4$), filtered and concentrated to yield crude product. The residue was purified on silica gel column (0 to 30% ethyl acetate in hexanes) to yield methyl 2-((2-amino-4-bromo-3-fluorophenyl)amino)-3-fluorobutanoate (0.533 g, 52 %) a light yellow solid.

Above yellow solid was dissolved in 15 ml of methanol, 0.5 1M HCl in methanol was added and the reaction was stirred at rt for 4 h. LCMS indicated full conversion. The solvent was removed, and the residue was diluted with DCM/methanol (5:1). The organic layer was washed once with 50% $NaHCO_3$ solution, dried (anhydrous $Na_2SO_4$) and concentrated. The residue was purified on silica gel column (eluted with 0 to 31% ethyl acetate in hexanes) to yield 7-bromo-8-fluoro-3-(1-fluoroethyl)-3,4-dihydro-1H-quinoxalin-2-one ***(intermediate* 38)** (0.337 g, 37 %) as a white solid. 1H NMR (500 MHz, METHANOL-d4) 1.27 - 1.46 (3H, m), 4.26 (1H, dd), 4.90 - 5.12 (1H, m), 6.50 (1H, dd), 6.97 (1H, dd). m/z ($ES^+$) $[M+H]^+$ = 291, 293.

***Intermediate* 39:** *7-bromo-8-fluoro-3-(1-fluoroethyl)-1H-quinoxalin-2-one*

DDQ (289 mg, 1.27 mmol) was added to a slurry of 7-bromo-8-fluoro-3-(1-fluoroethyl)-3,4-dihydro-1H-quinoxalin-2-one **(*intermediate* 38)** (337 mg, 1.16 mmol) in $CH_2Cl_2$ (10 mL) and the mixture was stirred at rt for 2 h. The solvent was removed under reduced pressure, the residue was diluted with sat. $NaHCO_3$ solution (~ 20 ml) and the suspension was stirred at r.t for 3h. The solid was collected by filtration, washed with water and dried to yield 7-bromo-8-fluoro-3-(1-fluoroethyl)-1H-quinoxalin-2-one **(*intermediate 39*)** (333 mg, 100 %) as a white solid. 1H NMR (500 MHz, METHANOL-d4) 1.65 - 1.84 (3H, m), 5.87 - 6.18 (1H, m), 7.50 - 7.60 (1H, m), 7.61 - 7.78 (1H, m). m/z ($ES^+$) $[M+H]^+$ = 289, 291.

***Intermediate 40:*** *8-fluoro-3-(1-fluoroethyl)-7-(hydroxymethyl)-1H-quinoxalin-2-one*

A mixture of Pd-PEPPSI™-IPent catalyst (26 mg, 0.03 mmol), (tributylstannyl)methanol (1638 mg, 5.10 mmol) and 7-bromo-8-fluoro-3-(1-fluoroethyl)-1H-quinoxalin-2-one **(*intermediate 39*)** (590 mg, 2.04 mmol) in 1,4-dioxane (25 mL) was degassed, back filled with $N_2$ and the mixture was stirred at 80 °C for 17 h. The mixture was concentrated, and the residue was purified on silica gel column (eluted with 0 to 50% ethyl acetate in hexanes (to recover the remaining SM) followed by 0 to 20% methanol in DCM) to yield 8-fluoro-3-(1-fluoroethyl)-7-(hydroxymethyl)-1H-quinoxalin-2-one ***(intermediate 40)*** (282 mg, 57%). 1H NMR (500 MHz, DMSO-d6) 1.58 - 1.81 (3H, m), 4.67 (2H, br d), 5.46 (1H, br t), 5.87 - 6.24 (1H, m), 7.33 - 7.48 (1H, m), 7.65 (1H, br d), 12.65 - 12.83 (1H, m); m/z ($ES^+$) $[M+H]^+$ = 241.

***Intermediate 41:*** *7-(bromomethyl)-8-fluoro-3-(1-fluoroethyl)-1H-quinoxalin-2-one*

A suspension of triphenylphosphine (1223 mg, 4.66 mmol) and 8-fluoro-3-(1-fluoroethyl)-7-(hydroxymethyl)-1H-quinoxalin-2-one (280 mg, 1.17 mmol) ***(intermediate 40)*** in $CH_2Cl_2$ (15 mL) was cooled to 0 °C, carbon tetrabromide (1546 mg, 4.66 mmol) was added, the mixture turned clear and purple color instantly, the mixture was continued to stir at this temperature for 10 min, the mixture turned into a yellow solution, checked by LCMS which indicated full conversion (not very clean), the mixture was concentrated, purified on silica gel column (eluted with 0 to 20% methanol in DCM) to yield a major peak as 7-(bromomethyl)-8-fluoro-3-(1-fluoroethyl)-1H-quinoxalin-2-one **(*intermediate 41*)** which was contaminated by some impurities. Concentrated to yield 2.5 g of solid (theory mass was 353 mgs, assumed 100% yield to carry over to next step). m/z ($ES^+$) $[M+H]^+$ = 303, 305.

***Example 12 and Example 13:*** *6-fluoro-5-[4-[[5-fluoro-2-[(1S and 1R)-1-fluoroethyl]-3-oxo-4H-guinoxalin-6-yl]methyl]piperazin-1-yl]-N-methyl-pyridine-2-carboxamide*

DIPEA (0.265 mL, 1.52 mmol) was added to a mixture of 7-(bromomethyl)-8-fluoro-3-(1-fluoroethyl)-1H-quinoxalin-2-one ***(intermediate 41)*** (115 mg, 0.38 mmol) and 6-fluoro-N-methyl-5-(piperazin-1-yl)picolinamide, 2HCl (94 mg, 0.30 mmol) (***intermediate 32***) in acetonitrile (20 mL) and the resulting mixture was stirred at 70 °C for 1h. LCMS indicated full conversion. The mixture was concentrated, the residue was dissolved in DMSO (~4ml) and purified on C18 reverse phase column (eluted with 0 to 100% ACN/water/0.1%TFA). The product containing fractions were combined and lyophilized to dryness. Material was repurified on Gilson (eluted with 0 to 80% ACN/water/0.1%TFA) to yield the product 6-fluoro-5-[4-[[5-fluoro-2-[(1S and 1R)-1-fluoroethyl]-3-oxo-4H-quinoxalin-6-yl]methyl]piperazin-1-yl]-N-methyl-pyridine-2-carboxamide. The enantiomers were separated by chiral column. Chiral purification conditions: Column info: chiralpak OD 4.6mm x 100mm 5μm, mobile phase A: $CO_2$ (100%), mobile phase B: methanol with 0.2% $NH_4OH$, isocratic 25% B over 6min, flow rate: 4.0ml/min, diluent: methanol, column temperature: room temperature, Outlet pressure (SFC): N/A.

**Peak 1:** The white solid was diluted with water and ACN 0.1 mL of aq. 0.5M HCl was added and the mixture was

lyophilized to dryness to yield isomer 1, 6-fluoro-5-[4-[[5-fluoro-2-[(1S OR 1R)-1-fluoroethyl]-3-oxo-4H-quinoxalin-6-yl] methyl]piperazin-1-yl]-N-methyl-pyridine-2-carboxamide as a HCl salt (***Example 12,*** absolute stereochemistry not determined) (5.42 mg, 10.90 μmol, 3 %) as HCl salt as a yellow solid. 1H NMR (500 MHz, DMSO-d6) 1.54 - 1.73 (3H, m), 2.70 - 2.87 (3H, m), 3.53 - 3.90 (8H, m), 4.57 (2H, br s), 5.79 - 6.21 (1H, m), 7.59 - 7.81 (3H, m), 7.87 (1H, d), 8.43 (1H, br d), 11.40 - 11.85 (1H, m), 12.78 - 13.14 (1H, m); m/z ($ES^+$) $[M+H]^+$ = 461, >95%ee.

**Peak 2:** The white solid was diluted with water and ACN, 0.1 mL of aq. 0.5M HCl was added and the mixture was lyophilized to dryness to yield isomer 2, 6-fluoro-5-[4-[[5-fluoro-2-[(1S OR 1R)-1-fluoroethyl]-3-oxo-4H-quinoxalin-6-yl] methyl]piperazin-1-yl]-N-methyl-pyridine-2-carboxamide as a HCl salt (***Example 13,*** absolute stereochemistry not determined) . 1H NMR (500 MHz, DMSO-d6) 1.49 - 1.83 (3H, m), 2.77 (3H, br d), 3.46 - 3.91 (8H, m), 4.56 (2H, br s), 5.80 - 6.26 (1H, m), 7.52 - 7.80 (3H, m), 7.87 (1H, br d), 8.43 (1H, br d), 11.44 - 11.82 (1H, m), 12.77 - 13.24 (1H, m); m/z ($ES^+$) $[M+H]^+$ = 461, >95% ee.

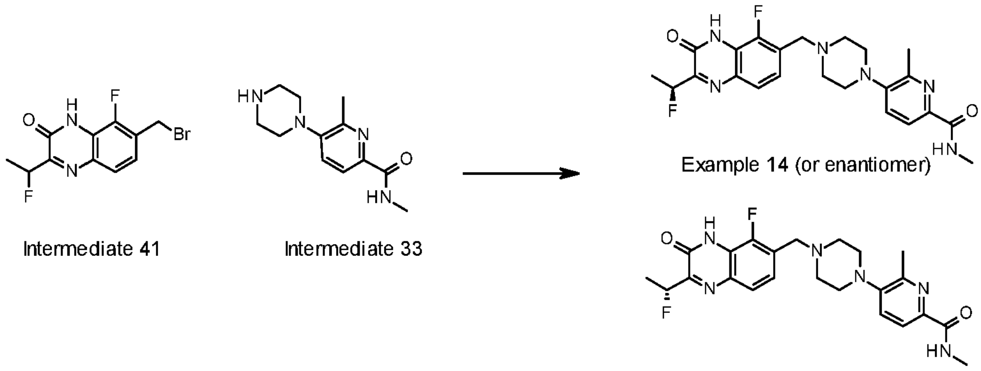
Intermediate 41
Intermediate 33
Example 14 (or enantiomer)
Example 15 (or enantiomer)

***Example 14 and Example 15:*** *5-[4-[[5-fluoro-2-[(1S and 1R)-1-fluoroethyl]-3-oxo-4H-quinoxalin-6-vl]methyl]piperazin-1-yl]-N,6-dimethyl-pyridine-2-carboxamide*

To a suspension of 7-(bromomethyl)-8-fluoro-3-(1-fluoroethyl)quinoxalin-2(1H)-one ***(intermediate 41)*** (138 mg, 0.41 mmol) and N,6-dimethyl-5-(piperazin-1-yl)picolinamide, 2HCl (126 mg, 0.41 mmol) ***(intermediate 33)*** in acetonitrile (13 mL) was added DIPEA (429 μl, 2.46 mmol) and the resulting mixture was stirred at 70 °C for 3h. LCMS indicated full conversion. Reaction was concentrated and the residue was purified on silica gel column (eluted with 0 to 20% methanol in DCM) to yield the racemic product 5-[4-[[5-fluoro-2-[(1S/1R)-1-fluoroethyl]-3-oxo-4H-quinoxalin-6-yl]methyl]piperazin-1-yl]-N,6-dimethyl-pyridine-2-carboxamide as a light yellow solid (169 mgs). Enantiomers were separated by chiral separation. Chiral purification conditions: Column info: chiralpak OD 21.2mm x 250mm 5μm, mobile phase A: $CO_2$ (100%), mobile phase B: methanol with 0.2% $NH_4OH$, isocratic: 25% B over 12 min, flow rate: 70.0ml/min, concentration: 8.45 mg/ml in methanol, loading: 4.23 mg/injection, column temperature: room temperature, Outlet pressure (SFC): N/A.

After chiral separation, each isomer was repurified on reverse phase column (eluted with 0 to 60% ACN/-water/0.2% ammonium hydroxide) to yield;

**Peak 1:** 5-[4-[[5-fluoro-2-[(1S OR 1R)-1-fluoroethyl]-3-oxo-4H-quinoxalin-6-yl]methyl]piperazin-1-yl]-N,6-dimethyl-pyridine-2-carboxamide, isomer 1 (***example 14,*** absolute stereochemistry not determined) (30.7 mg, 0.067 mmol, 16 %) as a white solid. 1H NMR (500 MHz, DMSO-d6) 1.40 - 1.70 (3H, m), 2.48 (3H, s), 2.54 - 2.69 (4H, m), 2.79 (3H, d), 2.94 (4H, br s), 3.74 (2H, s), 5.83 - 6.22 (1H, m), 7.17 - 7.41 (1H, m), 7.47 (1H, d), 7.65 (1H, d), 7.78 (1H, d), 8.40 (1H, br d), 11.65 - 12.88 (1H, m); m/z ($ES^+$) $[M+H]^+$ = 457; >98% ee.

**Peak 2:** 5-[4-[[5-fluoro-2-[(1S OR 1R)-1-fluoroethyl]-3-oxo-4H-quinoxalin-6-yl]methyl]piperazin-1-yl]-N,6-dimethyl-pyridine-2-carboxamide, isomer 2 ***(example 15,*** absolute stereochemistry not determined) (37 mg, 0.081 mmol, 20 %) as a white solid. 1H NMR (500 MHz, CHLOROFORM-d) 1.68 - 1.88 (3H, m), 2.51 (3H, s), 2.71 (4H, br s), 2.86 - 3.12 (7H, m), 3.81 (2H, s), 5.92 - 6.29 (1H, m), 7.34 (1H, d), 7.44 (1H, t), 7.76 (1H, d), 7.95 (1H, br d), 7.99 (1H, d), 10.24 (1H, br s); m/z ($ES^+$) $[M+H]^+$ = 457; 94.5% ee.

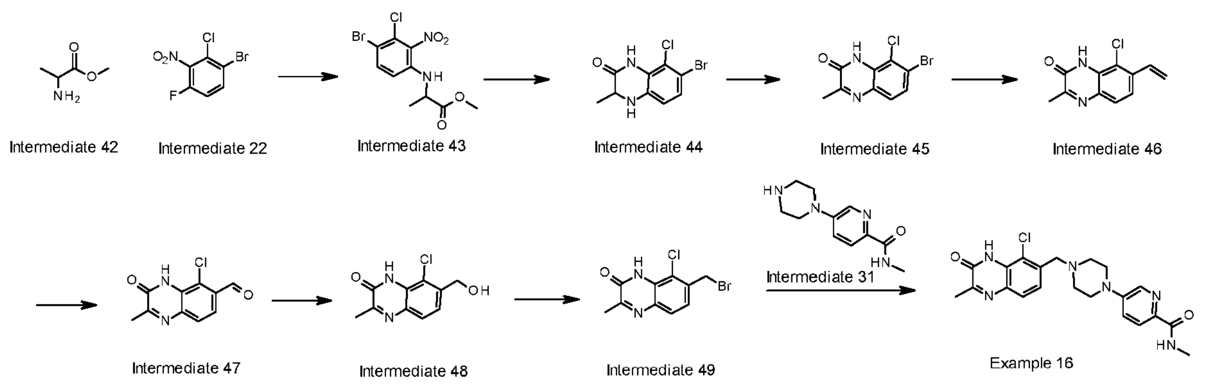


Intermediate 42 Intermediate 22 Intermediate 43 Intermediate 44 Intermediate 45 Intermediate 46

Intermediate 47 Intermediate 48 Intermediate 49 Example 16

***Intermediate 43:** methyl 2-(4-bromo-3-chloro-2-nitro-anilino)propanoate*

A flask was charged with methyl alaninate, HCl ***(intermediate 42)*** (1.851 g, 13.26 mmol) and 1-bromo-2-chloro-4-fluoro-3-nitrobenzene ***(intermediate 22)*** (2.25 g, 8.84 mmol) in 1,4-dioxane (70 mL). DIPEA (9.27 mL, 53.06 mmol) was added and the mixture was stirred at 105°C for 24 h gave a brown solution. LCMS showed reaction was complete. The mixture was concentrated, and the residue was purified on silica gel column (eluted with 0 to 30% ethyl acetate in hexanes) to yield methyl 2-(4-bromo-3-chloro-2-nitro-anilino)propanoate ***(intermediate 43)*** (2.120 g, 71 %) as a bright yellow oil, turned into yellow solid after standing. 1H NMR (500 MHz, CHLOROFORM-d) 1.52 (3H, d), 3.77 (3H, s), 6.53 (1H, d), 7.15 (1H, t), 7.53 (1H, d), 7.78 (1H, dd); m/z ($ES^+$) $[M+H]^+$ = 337.

***Intermediate 44:** 7-bromo-8-chloro-3-methyl-3,4-dihydro-1H-quinoxalin-2-one*

Sodium dithionite (3.28 g, 18.84 mmol) was added to a stirred solution of methyl 2-(4-bromo-3-chloro-2-nitro-anilino)propanoate ***(intermediate 43)*** (2.12 g, 6.28 mmol) in DMSO (50 mL) and the mixture was stirred at 120 °C for 5 h. LCMS and TLC indicated full conversion. The mixture was quenched with water and extracted with ethyl acetate (50 ml x 2). The organic layer was dried (anhydrous $Na_2SO_4$), filtered, concentrated and the residue was purified on silica gel column (0 to 55% ethyl acetate in hexanes) to yield 7-bromo-8-chloro-3-methyl-1H-quinoxalin-2-one ***(intermediate 45)*** (0.055 g, 3 %). m/z (ES+) $[M+H]^+$ 273, 275 and 7-bromo-8-chloro-3-methyl-3,4-dihydro-1H-quinoxalin-2-one ***(intermediate 44)*** (0.190 g, 11%). m/z ($ES^+$) $[M+H]^+$ = 275, 277.

***Intermediate 45:** 7-bromo-8-chloro-3-methyl-1H-quinoxalin-2-one*

DDQ (157 mg, 0.69 mmol) was added to a mixture of 7-bromo-8-chloro-3-methyl-3,4-dihydro-1H-quinoxalin-2-one ***(intermediate 44)*** (190 mg, 0.69 mmol) in 1,4-dioxane (10 mL) and the resulting mixture was stirred at rt for overnight, LCMS indicated full conversion. The mixture was concentrated and the residue was treated with sat. $NaHCO_3$ solution. Mixture was stirred at rt for 4, solid was isolated by filtration, washed with sat. $NaHCO_3$ solution and water. The solid was then purified on silica gel column (eluted with 0 to 20% methanol in DCM) to yield 7-bromo-8-chloro-3-methyl-1H-quinoxalin-2-one ***(intermediate 45)*** (122 mg, 65 %) as a yellow solid. m/z ($ES^+$) $[M+H]^+$ = 273, 275.

***Intermediate 46:** 8-chloro-3-methyl-7-vinyl-1H-quinoxalin-2-one*

A mixture of 7-bromo-8-chloro-3-methyl-1H-quinoxalin-2-one (***intermediate 45***) (122 mg, 0.45 mmol), tetrakis(triphenylphosphine)palladium(0) (51.5 mgs, 0.04 mmol) and tributyl(vinyl)stannane (212 mg, 0.67 mmol) in toluene (15 ml) was stirred at 110 °C under $N_2$ for 16h. LCMS indicated reaction completion. The mixture was concentrated, and residue was purified on silica gel column (eluted with 0 to 16% methanol in DCM) to yield 8-chloro-3-methyl-7-vinyl-1H-quinoxalin-2-one (***intermediate 46***) (98 mg, 100 %) as a brown solid. (contaminated by $PPh_3O$). m/z ($ES^+$) $[M+H]^+$ = 221.

***Intermediate 47:** 5-chloro-2-methyl-3-oxo-4H-quinoxaline-6-carbaldehyde*

Osmium tetroxide in $H_2O$ (0.1 mL, 0.01 mmol) was added to a solution of 8-chloro-3-methyl-7-vinyl-1H-quinoxalin-2-one (140 mg, 0.63 mmol) (***intermediate 46*)**, 2,6-lutidine (0.148 ml, 1.27 mmol) and sodium periodate (543 mg, 2.54 mmol) in THF (10 mL)/water (2 mL)/tert-butanol (0.607 mL, 6.34 mmol) and stirred at rt for overnight gave a yellow suspension. LCMS and TLC indicated there was still starting material remained. To the mixture was added THF (10 ml)/water (2.000 ml), 200 mgs of sodium periodate, 0.3 ml of osmium tetroxide and the mixture was continued to stir at rt for 5h. LCMS indicated full conversion. Reaction was diluted with water, sat. $NH_4Cl$ solution was added and extracted with

DCM. The combined organic layer was dried (anhydrous $Na_2SO_4$), filtered and concentrated. The residue was purified on silica gel column (eluted with 0 to 20% methanol in DCM) to yield 5-chloro-2-methyl-3-oxo-4H-quinoxaline-6-carbaldehyde (***intermediate 47***) (141 mg, 100 %) as a yellow solid. (not very pure, carried over to the next step). m/z (ES+) [M+H]+ = 223.

***Intermediate 48:*** *8-chloro-7-(hydroxymethyl)-3-methyl-1H-quinoxalin-2-one*

Sodium borohydride (23.96 mg, 0.63 mmol) was added to a cooled solution of 5-chloro-2-methyl-3-oxo-4H-quinoxaline-6-carbaldehyde (***intermediate 47***) (141 mg, 0.63 mmol) in a mixture of MeOH (16 mL) and DCM (8.00 mL) was cooled to 0 °C and the mixture was continued to stir at 0 °C for 1h. LCMS indicated full conversion. To the mixture was added 1 ml of water, concentrated and the residue was purified on silica gel column (eluted with 40 to 100% ethyl acetate in hexanes; then 0 to 20% methanol in DCM) to yield 8-chloro-7-(hydroxymethyl)-3-methyl-1H-quinoxalin-2-one (***intermediate 48***) (142 mg, 100 %) as a yellow solid; 1H NMR (500 MHz, DMSO-d6) 2.42 (3H, s), 4.65 (2H, br d), 5.53 (1H, br t), 7.46 (1H, br d), 7.69 (1H, br d), 11.77 (1H, br s); m/z (ES+) [M+H]+ = 225.

***Intermediate 49:*** *7-(bromomethyl)-8-chloro-3-methyl-1H-quinoxalin-2-one*

8-chloro-7-(hydroxymethyl)-3-methyl-1H-quinoxalin-2-one **(*intermediate 48*)** (142 mg, 0.63 mmol) and triphenylphosphine (332 mg, 1.26 mmol) in $CH_2Cl_2$ (20 ml) was cooled to 0 °C. Perbromomethane (419 mg, 1.26 mmol) was added in one portion and the mixture was stirred at 0 °C for 1h and at rt for 2 h. LCMS indicated no progress. To the mixture was added a second portion of triphenylphosphine (332

mg, 1.26 mmol) and perbromomethane (419 mg, 1.26 mmol) at rt and the mixture was stirred for 1 h. LCMS indicated full conversion. The solvent was removed under reduced pressure and the residue was purified on silica gel column (eluted with 0 to 100% ethyl acetate in hexanes) to yield the product 7-(bromomethyl)-8-chloro-3-methyl-1H-quinoxalin-2-one as a yellow solid **(*intermediate 49*)** (32 mgs, 18%). Further elution with 20% methanol in DCM gave the second portion 100 mgs (55%) of the product as a brown solid (47% purity). m/z (ES+) [M+H]+ = 287, 289.

***Example 16:*** *5-[4-[(5-chloro-2-methyl-3-oxo-4H-quinoxalin-6-yl)methyl]piperazin-1-yl]-N-methylpyridine-2-carboxamide*

DIPEA (0.053 mL, 0.31 mmol) was added to a mixture of N-methyl-5-(piperazin-1-yl)picolinamide, 2HCl (22.43 mg, 0.08 mmol) ***(intermediate 31)*** and 7-(bromomethyl)-8-chloro-3-methyl-1H-quinoxalin-2-one (***intermediate* 49**)(22 mg, 0.08 mmol) in acetonitrile (4 mL) and the resulting suspension was stirred at 70 °C for 1h. LCMS indicated full conversion. The mixture was concentrated, the residue was dissolved in DMSO and purified on reverse phase C18 column (eluted with 0 to 100% ACN/water/0.1%TFA). The fractions containing pure product were lyophilized to dryness to yield 5-[4-[(5-chloro-2-methyl-3-oxo-4H-quinoxalin-6-yl)methyl]piperazin-1-yl]-N-methyl-pyridine-2-carboxamide (20 mg, 56 %). 1 mL of 2M HCl in ether was added and the solvent was removed under vacuum to give the corresponding HCl salt as a yellow solid **(*example 16*).** 1H NMR (500 MHz, METHANOL-d4) 2.96 - 3.03 (3H, m), 3.48 - 3.86 (6H, m), 4.04 - 4.41 (2H, m), 4.78 (2H, s), 4.86 (3H, d, merged into water peak), 7.74 (1H, d), 7.84 (1H, d), 8.14 (1H, dd), 8.31 (1H, d), 8.47 (1H, d); m/z (ES+) [M+H]+ = 427.

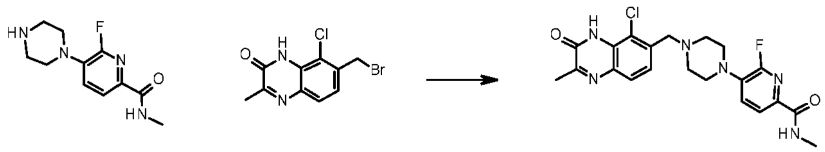

Intermediate 32 Intermediate 49 Example 17

***Example* 17:** *5-[4-[(5-chloro-2-methyl-3-oxo-4H-quinoxalin-6-yl)methyl]piperazin-1-yl]-6-fluoro-N-methyl-pyridine-2-carboxamide*

DIPEA (0.061 mL, 0.35 mmol) was added to a mixture of 6-fluoro-N-methyl-5-(piperazin-1-yl)picolinamide, 2HCl **(*intermediate 32*)** (27.1 mg, 0.09 mmol) and 7-(bromomethyl)-8-chloro-3-methyl-1H-quinoxalin-2-one **(*intermediate 49*)** (50 mg, 0.09 mmol) (around 50% purity) in acetonitrile (5 mL) and the resulting solution was stirred at 70 °C for 1h. LCMS indicated full conversion. The mixture was concentrated, and the residue was dissolved in DMSO and purified on a reverse phase C18 column (eluted with 0 to 100% ACN/water/0.1%TFA), then purified a second time on a reverse phase C18 column (eluted with 0 to 100% ACN/water/0.1%TFA). Material was finally purified a third time on reverse phase column (eluted with 0 to 100% ACN/water/ammonium hydroxide, PH~10) to yield 5-[4-[(5-chloro-2-methyl-3-oxo-4H-quinoxalin-6-yl)methyl]piperazin-1-yl]-6-fluoro-N-methyl-pyridine-2-carboxamide **(example 17)** (16.50 mg, 43 %) as a white solid. 1H NMR (500 MHz, DMSO-d6) 2.37 - 2.47 (3H, m), 2.63 (4H, br s), 2.76 (3H, d), 3.13 - 3.23 (4H, m), 3.74 (2H, s), 7.45 (1H, d), 7.57 (1H, dd), 7.68 (1H, d), 7.84 (1H, d), 8.39 (1H, br d), 10.71 - 12.11 (1H, m); m/z (ES$^+$) [M+H]$^+$ = 445.

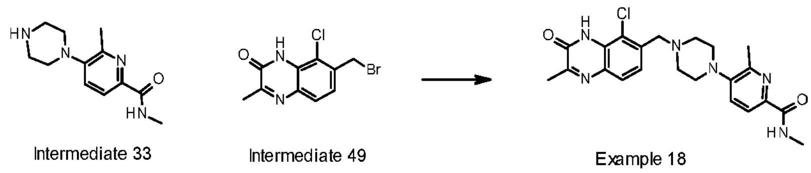


***Example 18:** 5-[4-[(5-chloro-2-methyl-3-oxo-4H-quinoxalin-6-yl)methyl]piperazin-1-yl]-N,6-dimethyi-pyridine-2-carboxamide*

DIPEA (0.061 mL, 0.35 mmol) was added to a mixture of N,6-dimethyl-5-(piperazin-1-yl)picolinamide, 2HCl **(intermediate 33)** (26.7 mg, 0.09 mmol) and 7-(bromomethyl)-8-chloro-3-methyl-1H-quinoxalin-2-one (50 mg, 0.09 mmol) **(*intermediate 49,*** ~50% purity) in acetonitrile (5 mL) and the resulting solution was stirred at 70 °C for 1 hr. LCMS indicated full conversion. The mixture was concentrated, the residue was dissolved in DMSO and the residue was purified on reverse phase C18 column (eluted with 0 to 100% ACN/water/0.1%TFA). After concentration of the fraction, the residue was repurified on reverse phase C18 column (eluted with 0 to 100% ACN/water/ammonium hydroxide, PH~10). The pure fractions were lyophilized to dryness to yield 5-[4-[(5-chloro-2-methyl-3-oxo-4H-quinoxalin-6-yl)methyl]piperazin-1-yl]-N,6-dimethyl-pyridine-2-carboxamide **(*example 18*)** (18.4 mg, 48 %) as free base. 1H NMR (500 MHz, METHANOL-d4) 2.53 (6H, d), 2.76 (4H, br s), 2.94 (3H, s), 3.04 (4H, br t), 3.86 (2H, s), 7.48 (1H, d), 7.51 - 7.60 (1H, m), 7.69 (1H, d), 7.86 (1H, d); m/z (ES$^+$) [M+H]$^+$ = 441.

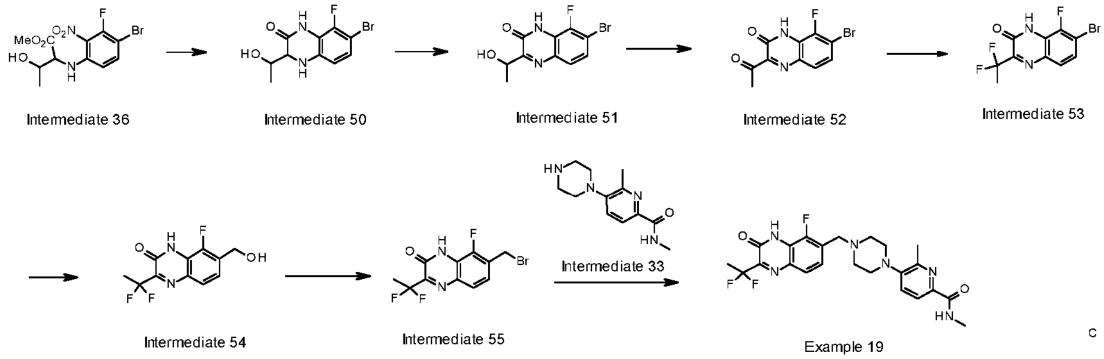


***Intermediate 50:** 7-bromo-8-fluoro-3-(1-hydroxyethyl)-3,4-dihydro-1H-quinoxalin-2-one*

Ammonium chloride (6.70 g, 125.31 mmol) was added to a suspension of methyl 2-((4-bromo-3-fluoro-2-nitrophenyl)amino)-3-hydroxybutanoate **(*intermediate 36*)** (4.4 g, 12.53 mmol) and zinc (8.19 g, 125.31 mmol) in MeOH (65 mL) at 0 °C. To this was added water (2 mL) and the mixture was stirred at 0 °C for 60 min. The orange color disappeared which indicated full conversion, LCMS indicated the reaction was complete. The mixture was filtered, washed with methanol and the filtrate was concentrated. The residue was diluted with ethyl acetate/methanol (10/1), the organic was washed e with water (~ 20 ml), brine, dried (anhydrous $Na_2SO_4$), concentrated to yield the intermediate methyl 2-((2-amino-4-bromo-3-fluorophenyl)amino)-3-hydroxybutanoate.

The above solid was slurried in methanol (~ 30 ml), 4 M HCl in dioxanes (~ 1 ml) was added and the mixture was stirred at rt for 2 h. LCMS indicated full conversion. The mixture was concentrated, and the residue was purified on silica gel column (eluted with 0 to 100% ethyl acetate in hexanes) to yield 7-bromo-8-fluoro-3-(1-hydroxyethyl)-3,4-dihydro-1H-quinoxalin-2-one **(*intermediate 50*)** (3.20 g, 88 %) as a yellow solid. (a mixture of diastereomers). m/z (ES$^+$) [M+H]$^+$ = 289, 291.

***Intermediate 51:** 7-bromo-8-fluoro-3-(1-hydroxyethyl)-1H-quinoxalin-2-one*

DDQ (432 mg, 1.90 mmol) was added to a suspension of 7-bromo-8-fluoro-3-(1-hydroxyethyl)-3,4-dihydro-1H-quinoxalin-2-one **(*intermediate 50*)** (500 mg, 1.73 mmol) in $CH_2Cl_2$ (30 mL) and the mixture was stirred at rt for overnight. LCMS indicated clean conversion. The solvent was removed under reduced pressure, sat. $NaHCO_3$ (~ 100 ml) solution

was added and the mixture was stirred at rt for 3 h. The solid was collected by filtration, washed with water and dried to yield 7-bromo-8-fluoro-3-(1-hydroxyethyl)-1H-quinoxalin-2-one **(*intermediate 51*)** (439 mg, 88 %). 1H NMR (500 MHz, DMSO-d6) 1.39 (3H, d), 4.78 - 5.31 (2H, m), 7.39 - 7.71 (2H, m), 12.72 (1H, br s); m/z ($ES^+$) $[M+H]^+$ = 287, 289.

***Intermediate 52:*** *3-acetyl-7-bromo-8-fluoro-1H-quinoxalin-2-one*

A solution of DMSO (0.651 mL, 9.17 mmol) in DCM was added dropwise to a stirred solution of oxalyl chloride (3.06 mL, 6.12 mmol) (2 M in DCM) in dichloromethane (20 ml) at -78 °C. The solution of 7-bromo-8-fluoro-3-(1-hydroxyethyl)-1H-quinoxalin-2-one **(*intermediate 51*)** (439 mg, 1.53 mmol) was added slowly to the above reaction mixture and the resultant slurry was stirred for 15 min at -78 °C. Triethylamine (1.279 mL, 9.17 mmol) was added dropwise and the resultant slurry was stirred an additional 30 min at 0 °C. LCMS indicated the formation of desired product. Water (30 ml) was added and the mixture was extracted with dichloromethane/MeOH (5:1) (2 x 50 ml). The organic phases were combined and dried over magnesium sulfate. The solvent was removed under vacuum and the residue was purified by reverse phase C18 column (eluted with 0 to 100% ACN/water/0.1%TFA) to yield 3-acetyl-7-bromo-8-fluoro-1H-quinoxalin-2-one (***intermediate 52***) (85 mg, 19 %) as a yellow solid. 1H NMR (500 MHz, DMSO-d6) 2.52 - 2.66 (3H, m), 7.42 - 7.76 (2H, m), 13.03 (1H, br s). m/z ($ES^+$) $[M+H]^+$ = 285, 287.

***Intermediate* 53:** *7-bromo-3-(1,1-difluoroethyl)-8-fluoro-1H-quinoxalin-2-one*

DAST (0.148 mL, 1.12 mmol) was added to a suspension of 3-acetyl-7-bromo-8-fluoro-1H-quinoxalin-2-one **(intermediate 52)** (80 mg, 0.28 mmol) in $CH_2Cl_2$ (20 mL) at rt and the resulting suspension was stirred at rt for 24 h. LCMS indicated 42% of the product formation. The mixture was continued to stir for over the weekend. To the mixture was added water and extracted with DCM. The organic layer was dried (anhydrous $Na_2SO_4$), filtered and concentrated. The residue was purified on silica gel column (eluted with 0 to 20% methanol in DCM) to yield 7-bromo-3-(1,1-difluoroethyl)-8-fluoro-1H-quinoxalin-2-one (***intermediate 53***) (65.0 mg, 75 %) as a light yellow solid. m/z ($ES^+$) $[M+H]^+$ = 307, 309. (the material was not very pure, carried onto next step).

***Intermediate 54:*** *3-(1,1-difluoroethyl)-8-fluoro-7-(hydroxymethyl)-1H-quinoxalin-2-one*

A mixture of (tributylstannyl)methanol (102 mg, 0.32 mmol), Xphos Pd G2 (24.98 mg, 0.03 mmol) and 7-bromo-3-(1,1-difluoroethyl)-8-fluoro-1H-quinoxalin-2-one (***intermediate 53***) (65 mg, 0.21 mmol) in 1,4-dioxane (10 mL) was stirred at 80 °C for 6h under $N_2$ atmosphere. LCMS indicated full conversion. Solvent was removed under vacuum and the residue was purified on silica gel column (eluted with 0 to 20% methanol in DCM) to yield 3-(1,1-difluoroethyl)-8-fluoro-7-(hydroxymethyl)-1H-quinoxalin-2-one (***intermediate 54***) (55.0 mg, 100 %) as a brown solid. m/z ($ES^+$) $[M+H]^+$ = 259.

***Intermediate 55:*** *7-(bromomethyl)-3-(1,1-difluoroethyl)-8-fluoro-1H-quinoxalin-2-one*

$CBr_4$ (129 mg, 0.39 mmol) was added to a mixture of 3-(1,1-difluoroethyl)-8-fluoro-7-(hydroxymethyl)-1H-quinoxalin-2-one (***intermediate 54***) (67 mg, 0.26 mmol) and triphenylphosphine (102 mg, 0.39 mmol) in $CH_2Cl_2$ (6 mL) at 0°C and the resulting mixture was stirred at rt for overnight. LCMS indicated full conversion. The solvent was removed under vacuum and the residue was purified on silica gel column (eluted with 0 to 100% ethyl acetate in hexanes) to yield 7-(bromomethyl)-3-(1,1-difluoroethyl)-8-fluoro-1H-quinoxalin-2-one (***intermediate 55***) (56.0 mg, 67 %) as a white solid. m/z ($ES^+$) $[M+H]^+$ = 321, 323.

***Example 19:*** *5-[4-[[2-(1,1-difluocroethyl)-5-fluoro-3-oxo-4H-quinoxalin-6-yllmethylipiperazin-1-yl]-N,6-dimethyl-pyridine-2-carboxamide*

To a suspension of 7-(bromomethyl)-3-(1,1-difluoroethyl)-8-fluoro-1H-quinoxalin-2-one (***intermediate 55***) (56 mg, 0.16 mmol) and N,6-dimethyl-5-(piperazin-1-yl)picolinamide, 2HCl (***intermediate 33***) (48.2 mg, 0.16 mmol) in acetonitrile (4 mL) was added DIPEA (0.164 mL, 0.94 mmol) and the resulting mixture was stirred at 70 °C for 1.5h. LCMS indicated full conversion. The mixture was concentrated, and the residue was purified on reverse phase Gilson column (eluted with 0 to 70% ACN/water/0.1%TFA). The pure fractions were combined, 0.5 ml of aq. 1M HCl was added to the combined fractions and lyophilized to dryness to yield 5-[4-[[2-(1,1-difluoroethyl)-5-fluoro-3-oxo-4H-quinoxalin-6-yl]methyl]piperazin-1-yl]-N,6-dimethyl-pyridine-2-carboxamide as HCl salt (***example 19***) (35.0 mg, 44 %) as a yellow solid. 1H NMR (500 MHz, DMSO-d6) 2.08 (3H, br t), 2.52 (3H, s), 2.80 (3H, br d), 3.02 - 3.54 (8H, m), 4.61 (2H, br s), 7.57 (1H, br d), 7.67 - 8.04 (3H, m), 8.52 (1H, brd), 11.74 (1H, br s), 12.77 - 13.55 (1H, m); m/z ($ES^+$) $[M+H]^+$ = 475.

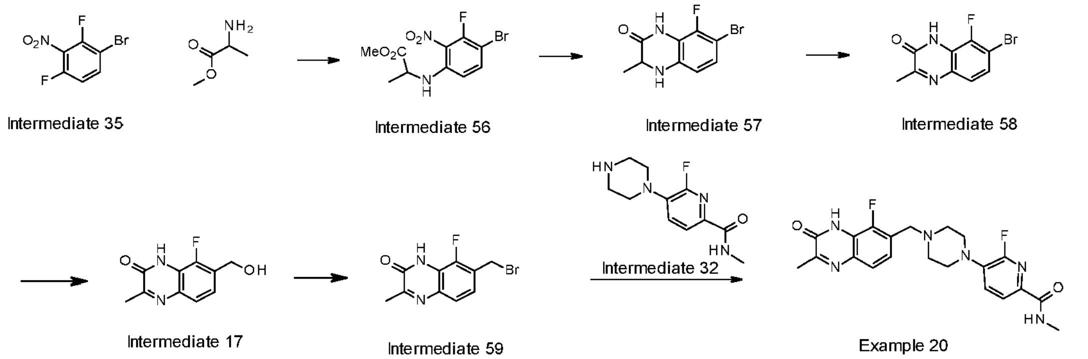


Intermediate 35 Intermediate 56 Intermediate 57 Intermediate 58 Intermediate 17 Intermediate 59 Example 20

***Intermediate 56:*** *methyl 2-(4-bromo-3-fluoro-2-nitro-anilino)propanoate*

DIPEA (151 ml, 867.27 mmol) was added slowly to a stirred solution of 1-bromo-2,4-difluoro-3-nitrobenzene **(*intermediate 35*)** (68.8 g, 289.09 mmol) and methyl alaninate, HCl (40.4 g, 289.09 mmol) in DMF (300 mL). The resulting solution was stirred at rt for 18 hours (Complete conversion to desired product by LCMS). Reaction mixture was concentrated using rocket evaporation system, diluted with water and extracted with ethyl acetate. Organic layer was washed throughly with water, dried over sodium sulphate, filtered and concentrated under vacuum. 100 mL DCM was added to the above orange solid, the suspension was stirred at r.t for 30 min, and the solid was filtered to yield methyl 2-(4-bromo-3-fluoro-2-nitro-anilino)propanoate (24.00 g, 26 %) **(*intermediate 56*)** as a bright orange solid. 1H NMR (500 MHz, DICHLOROMETHANE-d2) 1.52 - 1.62 (3H, m), 3.80 (3H, s), 4.28 (1H, quin), 6.49 (1H, dd), 7.19 - 7.39 (1H, m), 7.54 (1H, dd); 19F NMR (471 MHz, DICHLOROMETHANE-d2) -109.49 (1F, s) ; m/z ($ES^+$) $[M+H]^+$ = 321, 323.

***Intermediate 57:*** *7-bromo-8-fluoro-3-methyl-3,4-dihydro-1H-quinoxalin-2-one*

Zinc (78 g, 1195.88 mmol) was added portion wise to a mixture of methyl 2-(4-bromo-3-fluoro-2-nitro-anilino) propanoate ***(intermediate 56)*** (48 g, 149.49 mmol) and ammonium chloride (64.0 g, 1195.88 mmol) in MeOH (720 ml) and water (16 ml) at 0° C (exothermic reaction) and the mixture was stirred at rt for 2 h (Complete disappearance of orange coloration is indicative of reaction completion). Solid was filtered off and the solid cake was washed with 20% MeOH in DCM. The filtrate was concentrated, water was added to the crude product and the product was extracted by ethyl acetate. The organic layer was dried and concentrated under vacuum to furnish an oil. m/z ($ES^+$) $[M+H]^+$ = 291, 293.

This material was slurry in ethyl acetate (50 mL) and methanol (50 mL), 2 mL 4N HCl in dioxane was added and the mixture was stirred for 1 h. The reaction mixture was concentrated to yield the crude product 7-bromo-8-fluoro-3-methyl-3,4-dihydro-1H-quinoxalin-2-one (***intermediate 57***) (38.7 g) as a grey solid. The crude product (38.7 g) was subjected to the next step without any further purification assuming the yield of this reaction to be 100%. m/z ($ES^+$) $[M+H]^+$ = 259.

***Intermediate 58:*** *7-bromo-8-fluoro-3-methyl-1H-quinoxalin-2-one*

DDQ (21.55 g, 94.95 mmol) was added in one portion to a stirred solution of 7-bromo-8-fluoro-3-methyl-3,4-dihydro-1H-quinoxalin-2-one **(intermediate 57)** (20.5 g, 79.13 mmol) in DCM (200 mL), resulted in a very thick off-white slurry, added additional dichloromethane (800 mL). The resulting slurry was stirred at rt for 2 hours (Complete conversion to desired product by LCMS). The reaction mixture was concentrated under vacuum and quenched with saturated aq. sodium bicarbonate solution (about 500 mL, quenching leads to intense frothing). The above slurry was stirred at rt for overnight and the solid was filtered off, washed thoroughly with water and solid dried on the filter for overnight. This solid was washed with diethyl ether and dried for 30 mins to give 7-bromo-8-fluoro-3-methyl-1H-quinoxalin-2-one **(intermediate 58)** (16.28 g, 80 %) as an off-white solid. 19F NMR (471 MHz, DMSO-d6) -124.18 (1F, s); 1H NMR (500 MHz, DMSO-d6) 2.41 (3H, s), 7.45 - 7.54 (2H, m), 12.60 (1H, br s); m/z ($ES^+$) $[M+H]^+$ = 257.

***Intermediate 17:*** *8-fluoro-7-(hydroxymethyl)-3-methyl-1H-quinoxalin-2-one*

A mixture of (tributylstannyl)methanol (15.39 g, 47.93 mmol), 7-bromo-8-fluoro-3-methyl-1H-quinoxalin-2-one **(intermediate 58)** (11.2 g, 43.57 mmol) and Xphos Pd G2 (1.714 g, 2.18 mmol) in 1,4-dioxane (200 mL) was stirred at 80 °C for 7 h. LCMS indicated full conversion. The solvent was removed under reduced pressure, the residue was purified on silica gel column (eluted with 0 to 15% methanol in DCM).

The fractions were concentrated to a slurry, diluted with ether, the solid was collected by filtration and dried to yield

8-fluoro-7-(hydroxymethyl)-3-methyl-1H-quinoxalin-2-one **(intermediate 17)** (8.10 g, 89 %) as a white solid. 1H NMR (500 MHz, DMSO-d6) 2.41 (3H, s), 4.63 (2H, br d), 5.39 (1H, t), 7.31 (1H, br t), 7.51 (1H, d), 12.41 (1H, br s). m/z ($ES^+$) $[M+H]^+$ = 209.

***Example 20:*** *6-fluoro-5-[4-[(5-fluoro-2-methyl-3-oxo-4H-quinoxalin-6-yl)methyl]piperazin-1-yl]-N-methyl-pyridine-2-carboxamide*

Triethylphosphane (20.90 ml, 145.06 mmol) was added dropwise with an addition funnel to a stirred suspension of 8-fluoro-7-(hydroxymethyl)-3-methyl-1H-quinoxalin-2-one **(intermediate 17)** (15.1 g, 72.53 mmol) and 1,2-dibromo-1,1,2,2-tetrachloroethane (52.0 g, 159.56 mmol) in DCM (400 mL) at 0°C under nitrogen. The mixture was stirred at r.t for 3 h gave a light-yellow suspension. Crude LCMS indicated full conversion. DCM was removed under vacuum; the residue was slurry in 300 mL diethyl ether at rt and the light yellow ppt was filtered and washed with 200 ml ether. The solid was taken into 300 ml of water, stirred at rt for 10 min, the solid was collected by filtration, thorough wash (200 ml) with water to remove the salts. The solid was dried under vacuum for overnight (no heat). The solid was washed with hexanes and dried in vacuum in a bushel funnel to give 7-(bromomethyl)-8-fluoro-3-methylquinoxalin-2(1H)-one **(intermediate 59)** (22.76 g, 116 %, likely contains some inorganic salts) as an off white solid. Used as such for next reaction. 1H NMR (500 MHz, DMSO-d6) 2.42 (3H, s), 4.65 - 4.93 (2H, m), 7.28 - 7.42 (1H, m), 7.51 (1H, d), 12.53 (1H, br s); m/z ($ES^+$) $[M+H]^+$ = 271, 273.

To a flask charged with 7-(bromomethyl)-8-fluoro-3-methylquinoxalin-2(1H)-one **(intermediate 59)** (22.76 g) and 6-fluoro-N-methyl-5-(piperazin-1-yl)picolinamide, 2HCl **(*intermediate 32*)** (24.24 g, 77.9 mmol) in acetonitrile (350 ml), was added DIPEA (38.0 ml, 217.59 mmol) at rt and the resulting mixture was stirred at 70°C for 4 h. Reaction was not complete. To the mixture was added 5 g of KI and 2 g of NaI and the mixture was stirred at 50°C for 20 h. More 540 mgs (~0.03eq) of 6-fluoro-N-methyl-5-(piperazin-1-yl)picolinamide, 2HCl **(intermediate 32)** was added to the mixture and the stirring continued at 50°C for 2 h. The solid from the reaction suspension was collected by filtration, washed with acetonitrile and dried. The resulting material was then suspended in water (~400 ml), slurred at rt for 20 min, filtered and dried (97% purity by LCMS). The solid was then dissolved into a mixture of DCM/MeOH (3/1) (about 1.5 L) at reflux, filtered through a pad of silica gel, removed most of the DCM until solid precipitate out and the mixture was kept at rt for 20 min. The solid was collected by filtration and repeated the procedure for the filtrate, and the solids were combined to yield the product 6-fluoro-5-[4-[(5-fluoro-2-methyl-3-oxo-4H-quinoxalin-6-yl)methyl]piperazin-1-yl]-N-methyl-pyridine-2-carboxamide **(example 20)** (26 g, 84%) as a light yellow solid. 1H NMR (500 MHz, DMSO-d6) 2.41 (3H, s), 2.57 - 2.69 (4H, m), 2.76 (3H, d), 3.16 (4H, br s), 3.70 (2H, s), 7.29 (1H, br t), 7.40 - 7.60 (2H, m), 7.83 (1H, d), 8.38 (1H, br d), 12.44 (1H, br s); m/z ($ES^+$) $[M+H]^+$ = 429.

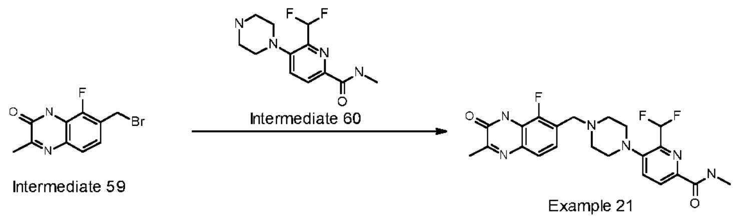


**Example 21:** *6-(difluoromethyl)-5-[4-[(5-fluoro-2-methyl-3-oxo-4H-quinoxalin-6-yl)methyl]piperazin-1-yl]-N-methyl-pyridine-2-carboxamide*

DIPEA (0.052 mL, 0.30 mmol) was added to a stirred mixture of 7-(bromomethyl)-8-fluoro-3-methylquinoxalin-2(1H)-one **(intermediate 59)**(40 mg, 0.15 mmol) and 6-(difluoromethyl)-N-methyl-5-(piperazin-1-yl)picolinamide, 2HCl **(intermediate 60)** (50.6 mg, 0.15 mmol) in acetonitrile (mL) and The resulting mixture was stirred at 70 °C for 2 h. Reaction was concentrated and submitted to analytical group for purification (Purification conditions: the residue was purified by reverse phase C18 column (eluted with 0 to 100% ACN/water/0.1%NH4OH) which yield 6-(difluoromethyl)-5-[4-[(5-fluoro-2-methyl-3-oxo-4H-quinoxalin-6-yl)methyl]piperazin-1-yl]-N-methyl-pyridine-2-carboxamide **(example 21)** (15 mg, 22%) as white solid.1H NMR (500 MHz, DMSO-d6) 2.37 (3H, s), 2.64 (4H, br s), 2.84 (3H, d), 3.01 (4H, br d), 3.70 (2H, s), 7.00 - 7.28 (2H, m), 7.42 (1H, br d), 7.86 (1H, d), 8.09 (1H, d), 8.39 (1H, q), 12.24 - 12.63 (1H, m); m/z ($ES^+$) $[M+H]^+$ = 461.

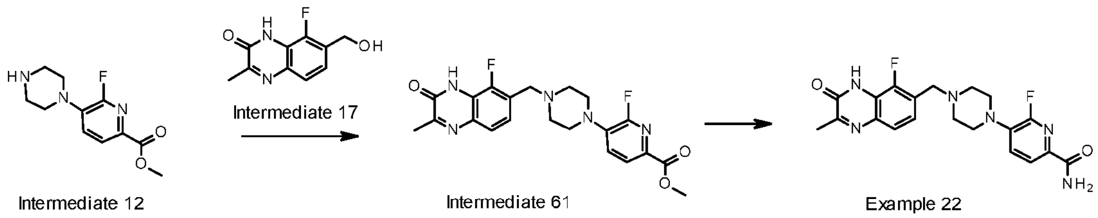


Intermediate 12 Intermediate 61 Example 22

***Intermediate 61:** methyl 6-fluoro-5-[4-[(5-fluoro-2-methyl-3-oxo-4H-quinoxalin-6-yl)methyl]piperazin-1-yl]pyridine-2-carboxylate*

Polymer supported triphenylphosphine (1.512 g, 5.76 mmol) (3.4 g added, calculated based on $PPh_3$ loading of 1.6 mmol/g) was added to a stirred slurry of 8-fluoro-7-(hydroxymethyl)-3-methylquinoxalin-2(1H)-one **(intermediate 17)** (400 mg, 1.92 mmol) and perbromomethane (1.274 g, 3.84 mmol) in DCM (40 mL) at rt. The resulting mixture was stirred at 23 °C for 1 h. Reaction was not complete. Additional polymer bound-$PPh_3$ (1 g) was added to get the reaction to complete. Reaction mixture was filtered, washed with DCM, THF and the filtrate was concentrated under vacuum to yield 7-(bromomethyl)-8-fluoro-3-methylquinoxalin-2(1H)-one as a light-yellow solid.

To the above freshly prepared 7-(bromomethyl)-8-fluoro-3-methylquinoxalin-2(1H)-one, was added methyl 6-fluoro-5-(piperazin-1-yl)picolinate, 2HCl (**intermediate 12**) (600 mg, 1.92 mmol), acetonitrile (25 mL) and N-ethyl-N-isopropylpropan-2-amine (1674 µl, 9.61 mmol) and the reaction mixture was heated to 70°C 1 h. The reaction mixture was cooled to rt, concentrated, and the crude solid was purified via normal phase chromatography using 0-10% MeOH in DCM to yield methyl 6-fluoro-5-[4-[(5-fluoro-2-methyl-3-oxo-4H-quinoxalin-6-yl)methyl]piperazin-1-yl]pyridine-2-carboxylate **(intermediate 61)** (0.484 g, 59 %) as an off-white solid. 1H NMR (500 MHz, DMSO-d6) 2.34 - 2.49 (3H, m), 2.52 - 2.62 (4H, m), 3.08 - 3.28 (4H, m), 3.70 (2H, s), 3.83 (3H, s), 7.29 (1H, t), 7.44 - 7.54 (2H, m), 7.91 (1H, dd), 12.45 (1H, s); 19F NMR (471 MHz, DMSO-d6) -135.50 (1F, s), -70.49 (1F, s). m/z ($ES^+$) $[M+H]^+$ = 430.

***Example 22:** 6-fluoro-5-[4-[(5-fluoro-2-methyl-3-oxo-4H-quinoxalin-6-yl)methyl]piperazin-1-yl]pyridine-2-carboxamide*

Ammonia (7 N Ammonia in MeOH) (31.3 ml, 218.90 mmol) was added to methyl 6-fluoro-5-[4-[(5-fluoro-2-methyl-3-oxo-4H-quinoxalin-6-yl)methyl]piperazin-1-yl]pyridine-2-carboxylate ***(intermediate 61)***(0.470 g, 1.09 mmol) in a 40 mL scintillation vial, sealed and stirred at rt for 18 h. Complete conversion to desired product by LCMS. The white solid was filtered to give103 mg pure product. The filtrate was concentrated under vacuum, the resulting off-white solid was slurry in about 5 mL methanol and filtered to obtain additional pure product 298 mg. Both batches were combined to obtain 6-fluoro-5-[4-[(5-fluoro-2-methyl-3-oxo-4H-quinoxalin-6-yl)methyl]piperazin-1-yl]pyridine-2-carboxamide **(example 22)** (0.401 g, 88 %). 1H NMR (500 MHz, DMSO-d6) 2.42 (3H, s), 2.59 (4H, br s), 3.09 - 3.27 (4H, m), 3.70 (2H, s), 7.29 (1H, br t), 7.46 (1H, br s), 7.49 - 7.58 (2H, m), 7.76 (1H, br s), 7.85 (1H, br d), 12.35 (1H, br s); 19F NMR (471 MHz, DMSO-d6) -135.49 (1F, s), -72.40 (1F, s); m/z ($ES^+$) $[M+H]^+$ = 415.

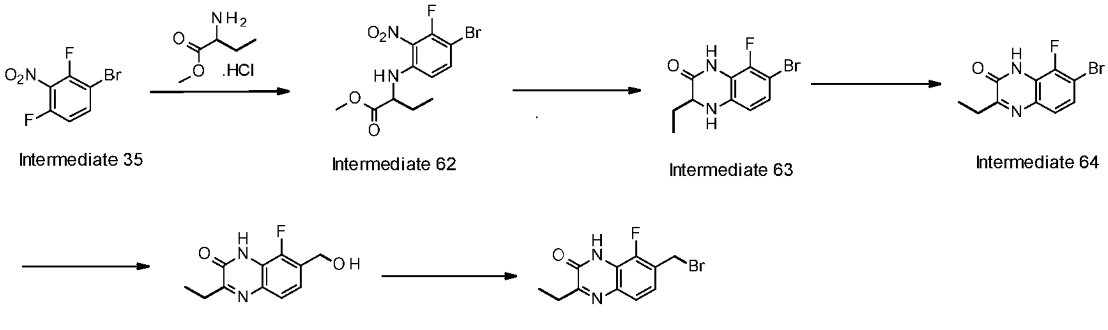


Intermediate 35 Intermediate 62 Intermediate 63 Intermediate 64

Intermediate 65 Intermediate 66

***Intermediate 62:** methyl 2-(4-bromo-3-fluoro-2-nitro-anilino)butanoate*

DIPEA (165 ml, 942.91 mmol) was added slowly to a stirred solution of 1-bromo-2,4-difluoro-3-nitrobenzene **(intermediate 35)** (74.8 g, 314.30 mmol) and methyl 2-aminobutanoate, HCl (48.3 g, 314.30 mmol) in DMF (733 mL) and the resulting solution was stirred at rt for 18 hours. DMF was removed on rocket evaporator, diluted with water and extracted with ethyl acetate. After concentration the crude material was purified by flash silica chromatography, elution gradient 0 to 70% EtOAc in hexanes. Product fractions were concentrated under reduced pressure to afford methyl 2-(4-bromo-3-fluoro-2-nitro-anilino)butanoate **(intermediate 62)** (49.4 g, 47 %) as a red solid. 1H NMR (500 MHz, DMSO-d6) 0.82 - 0.98 (3H, m), 1.77 - 1.93 (2H, m), 3.70 (3H, d), 4.38 - 4.54 (1H, m), 6.77 (1H, br d), 7.28 (1H, br d), 7.64 - 7.80 (1H, t); m/z (ES$^+$) [M+H]$^+$ = 335.

***Intermediate 63:*** *7-bromo-3-ethyl-8-fluoro-3,4-dihydro-1H-quinoxalin-2-one*

Zinc (44.1 g, 674.07 mmol) was added portion wise (exothermic reaction) to a mixture of methyl 2-(4-bromo-3-fluoro-2-nitro-anilino)butanoate (intermediate 62) (50.2 g, 149.79 mmol) and ammonium chloride (64.1 g, 1198.34 mmol) in MeOH (468 mL). The mixture was stirred at rt for 1 h. Solid was filtered off and washed with 20% MeOH in DCM. This material was dissolved in methanol (120 mL) and 4N HCl in dioxane (10 mL) was added and reaction was stirred for 30 min. Solvent was removed under vacuum, diluted with ethyl acetate and basified with sat $NaHCO_3$ solution. Organic layer was separated, washed with water, dried over sodium sulphate and concentrated to give the crude product. The solid was triturated with 100 mL methanol, stirred for 10 min and the light brown solid was filtered off to give 7-bromo-3-ethyl-8-fluoro-3,4-dihydro-1H-quinoxalin-2-one (**intermediate 63**) (39.8 g, 97 %) product. 1H NMR (500 MHz, DMSO-d6) 0.92 (3H, t), 1.49 - 1.77 (2H, m), 3.57 - 3.87 (1H, m), 6.24 - 6.62 (2H, m), 6.99 (1H, dd), 10.44 (1H, s); m/z (ES$^+$) [M+H]$^+$ = 273.

***Intermediate 64:*** *7-bromo-3-ethyl-8-fluoro-1H-quinoxalin-2-one*

DDQ (29.7 g, 130.94 mmol) was added in one portion to a stirred solution of 7-bromo-3-ethyl-8-fluoro-3,4-dihydro-1H-quinoxalin-2-one (**intermediate 63**) (29.8 g, 109.12 mmol) in DCM (546 mL) and the resulting solution was stirred at rt for 2 h. Solvent was removed under vacuum, and the solid was slurry with 150 mL of methanol and stirred for 30 min. Solid was filtered off and washed with 30 mL methanol. Solid was transferred to a 2 L round bottom flask; 200 mL water was added follow by slow addition of 300 mL of sodium bicarbonate. After complete addition the mixture was stirred for overnight at rt to give light yellow slurry. Stirring was stopped and the aq layer was decanned. Solid was collected by filtration and washed thoroughly with water to give 7-bromo-3-ethyl-8-fluoro-1H-quinoxalin-2-one (**intermediate 64**) (24.45 g, 83 %) as yellow colored solid. 1H NMR (500 MHz, DMSO-d6) 1.22 (3H, t), 2.81 (2H, q), 7.27 - 7.69 (2H, m), 12.59 (1H, br s); m/z (ES$^+$) [M+H]$^+$ = 271.

***Intermediate* 65:** *3-ethyl-8-fluoro-7-(hydroxymethyl)-1H-quinoxalin-2-one*

Xphos Pd G2 (1.121 g, 1.43 mmol) was added to a stirred degassed solution of 7-bromo-3-ethyl-8-fluoro-1H-quinoxalin-2-one (**intermediate 64**) (7.727 g, 28.50 mmol) and (tributylstannyl)methanol (10.98 g, 34.20 mmol) in 1,4-dioxane (143 mL). The resulting solution was stirred at 80 °C for 6 hours. Solvent was removed under vacuum, 100 mL diethyl ether was added, and the slurry was stirred for 30 min. Solid was filtered off and washed with 50 mL of diethyl ether to give 3-ethyl-8-fluoro-7-(hydroxymethyl)-1H-quinoxalin-2-one (5.88 g, 93 %) (**intermediate 65**) as off white solid. 1H NMR (500 MHz, DMSO-*d6*) 1.22 (3H, t), 2.82 (2H, q), 4.64 (2H, br d), 5.40 (1H, t), 7.32 (1H, br t), 7.55 (1H, d), 12.40 (1H, br s); m/z (ES$^+$) [M+H]$^+$ = 223.

***Intermediate 66:*** *7-(bromomethyl)-3-ethyl-8-fluoro-1H-quinoxalin-2-one*

Triethylphosphane (19.94 ml, 135.00 mmol) was added dropwise to a stirred solution of 3-ethyl-8-fluoro-7-(hydroxymethyl)-1H-quinoxalin-2-one (**intermediate 65**) (10 g, 45.00 mmol) and $CBr_4$ (49.2 g, 148.50 mmol) in DCM (355 mL) at 0°C over a period of 5 minutes under nitrogen. Reaction was stirred at rt for 1 h. DCM was removed under vacuum and residue was slurry in 150 mL diethyl ether. The white ppt was filtered off and washed with 50 mL diethyl ether. This solid was slurry with water (200 mL) and stirred for 30 min. Solid was filtered off and washed thoroughly with water. The solid was dried under vacuum for overnight to give 7-(bromomethyl)-3-ethyl-8-fluoroquinoxalin-2(1H)-one (**intermediate 66**) (11.38 g, 89 %) as light brown solid. 1H NMR (500 MH z, DMSO-d6) 1.22 (3H, t), 2.83 (2H, q), 4.81 (2H, s), 7.37 (1H, brt), 7.55 (1H, d), 12.53 (1H, br s); m/z (ES$^+$) [M+H]$^+$ = 285.

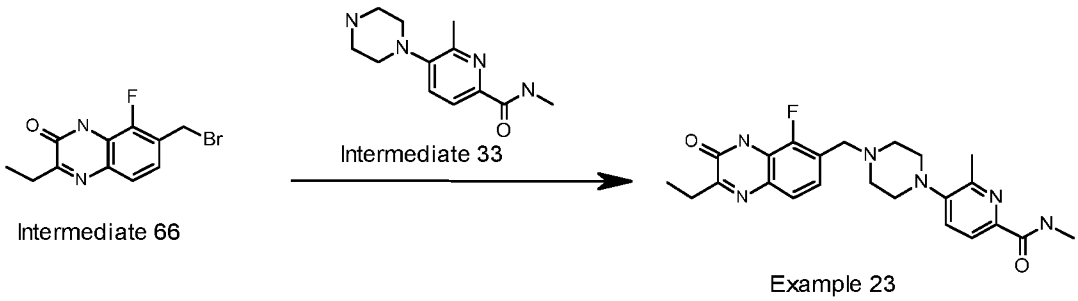


***Example 23:** 5-[4-[(2-ethyl-5-fluoro-3-oxo-4H-quinoxalin-6-yl)methyl]piperazin-1-yl]-N,6-dimethylpyridine-2-carboxamide*

DIPEA (20.90 ml, 119.64 mmol) was added to a stirred slurry of 7-(bromomethyl)-3-ethyl-8-fluoroquinoxalin-2(1H)-one (**intermediate 66**) (11.37 g, 39.88 mmol) and N,6-dimethyl-5-piperazin-1-yl-pyridine-2-carboxamide, 2HCl (**intermediate 33**) (14.09 g, 45.86 mmol) in acetonitrile (178 mL). The resulting solution was stirred at 50 °C for 2 h. Reaction was complete. Half of the solvent was removed by evaporation, 10 mL sat sodium bicarbonate was added and mixture was stirred for 15 min. Solid was filtered off, washed with water followed by 50 mL acetonitrile. Solid was dissolved in DCM/Methanol (-9/1) and filtered through silica bed. Filtrate was concentrated to give the light yellow solid. This material was triturated with ~120 mL methanol, solid was filtered off and dried. LCMS still shows ~2.1% impurity (likely from the reagent). Material was again triturated with acetonitrile and then with 3% methanol in acetonitrile to give ~14 g white solid. Methanol (40 mL) was added and stirred for 3 h. Solid was filtered off to give pure product 5-[4-[(2-ethyl-5-fluoro-3-oxo-4H-quinoxalin-6-yl)methyl]piperazin-1-yl]-N,6-dimethyl-pyridine-2-carboxamide (**example 23**) (12.26 g, 70 %). 1HNMR (500 MHz, DMSO-d6) 1.23 (3H, t), 2.48 (3H, s), 2.62 (4H, br s), 2.76 - 2.88 (5H, m), 2.95 (4H, br s), 3.73 (2H, s), 7.31 (1H, t), 7.47 (1H, d), 7.56 (1H, d), 7.79 (1H, d), 8.41 (1H, q), 12.44 (1H, s); m/z ($ES^+$) $[M+H]^+$ = 439.

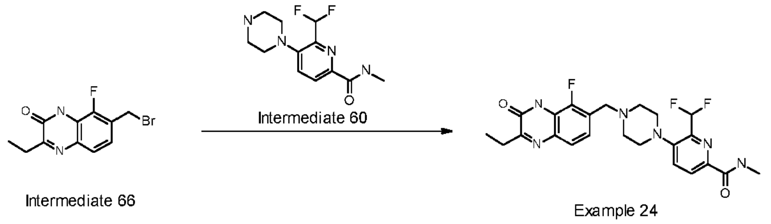


***Example 24:** 6-(difluoromethyl)-5-[4-[(2-ethyl-5-fluoro-3-oxo-4H-quinoxalin-6-yl)methyl]piperazin-1-yl]-N-methyl-pyridine-2-carboxamide*

DIPEA (0.049 mL, 0.28 mmol) was added to a stirred mixture of 7-(bromomethyl)-3-ethyl-8-fluoroquinoxalin-2(1H)-one (**intermediate 66**) (40 mg, 0.14 mmol) and 6-(difluoromethyl)-N-methyl-5-(piperazin-1-yl)picolinamide, 2HCl (**intermediate 60**) (48.1 mg, 0.14 mmol) in acetonitrile (2 mL) and The resulting mixture was stirred at 70 °C for 2 hours. Reaction was concentrated and submitted to analytical group for purification (Purification conditions: the residue was purified by reverse phase C18 column (eluted with 0 to 100% ACN/water/0.1%NH4OH) which yield 6-(difluoromethyl)-5-[4-[(2-ethyl-5-fluoro-3-oxo-4H-quinoxalin-6-yl)methyl]piperazin-1-yl]-N-methyl-pyridine-2-carboxamide (**example 24**) (56.0 mg, 84 %).1H NMR (500 MHz, DMSO-d6) 1.23 (3H, t), 2.65 (4H, br d), 2.77 - 2.86 (5H, m), 2.97 - 3.06 (4H, m), 3.73 (2H, s), 7.00 - 7.26 (1H, t), 7.30 (1H, br d), 7.55 (1H, br d), 7.86 (1H, d), 8.09 (1H, d), 8.39 (1H, q), 12.45 (1H, br d); m/z ($ES^+$) $[M+H]^+$ = 475.

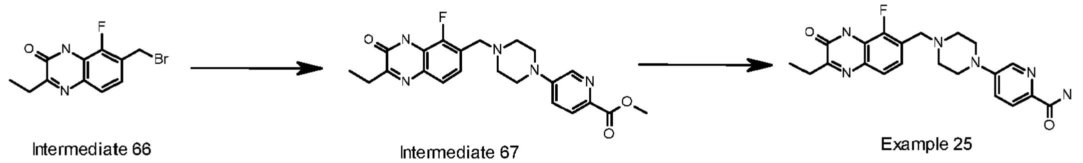


***Intermediate 67:** methyl 5-[4-[(2-ethyl-5-fluoro-3-oxo-4H-quinoxalin-6-yl)methyl]piperazin-1-yllpyridine-2-carboxylate*

DIPEA (246 µl, 1.41 mmol) was added to a stirred slurry of 7-(bromomethyl)-3-ethyl-8-fluoro-1H-quinoxalin-2-one (**intermediate 66**) (134 mg, 0.47 mmol) and methyl 5-(piperazin-1-yl)picolinate, 2HCl (**intermediate 119**)(138 mg, 0.47 mmol) in acetonitrile (2 mL). The resulting solution was stirred at 50 °C for 2 h. Solvent was removed under vacuum and the resulting residue was purified by flash silica chromatography, elution gradient 0 to 20% MeOH in DCM. Product fractions were concentrated under reduced pressure to afford methyl 5-[4-[(2-ethyl-5-fluoro-3-oxo-4H-quinoxalin-6-yl)methyl]piperazin-1-yl]pyridine-2-carboxylate (**intermediate 67**) (0.142 g, 71.0 %) as a white solid; m/z (ES$^+$) $[M+H]^+$ = 426.

***Example 25:** 5-[4-[(2-ethyl-5-fluoro-3-oxo-4H-quinoxalin-6-yl)methyl]piperazin-1-yl]pyridine-2-carboxamide*

Ammonia (7 N) in methanol (3 mL, 6.00 mmol) was added to methyl methyl 5-[4-[(2-ethyl-5-fluoro-3-oxo-4H-quinoxalin-6-yl)methyl]piperazin-1-yl]pyridine-2-carboxylate (**intermediate 67**) (130 mg, 0.31 mmol). The resulting suspension was stirred at 50 °C for 24 hours (sealed tube). Solvent was removed and the resulting residue was purified by flash silica chromatography, elution gradient 0 to 35% MeOH in DCM. Product fractions were concentrated under reduced pressure to afford 5-[4-[(2-ethyl-5-fluoro-3-oxo-4H-quinoxalin-6-yl)methyl]piperazin-1-yl]pyridine-2-carboxamide (**example 25**) (0.079 g, 63 %) as a pale yellow solid. 1H NMR (500 MHz, DMSO-*d6*) 1.23 (3H, t), 2.54 - 2.61 (4H, m), 2.83 (2H, q), 3.32 - 3.40 (4H, m), 3.70 (2H, s), 7.24 - 7.34 (2H, m), 7.38 (1H, dd), 7.56 (1H, d), 7.76 (1H, br d), 7.84 (1H, d), 8.27 (1H, d), 12.44 (1H, br s); m/z (ES$^+$) $[M+H]^+$ = 411.

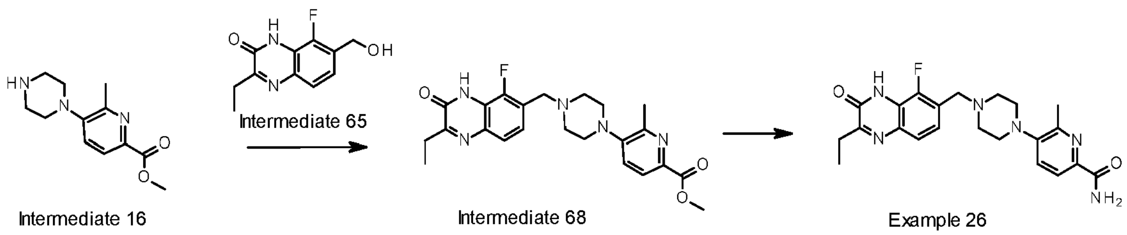
Intermediate 65
Intermediate 16
Intermediate 68
Example 26

***Intermediate 68:** methyl 5-[4-[(2-ethyl-5-fluoro-3-oxo-4H-quinoxalin-6-yl)methyl]piperazin-1-yl]-6-methyl-pyridine-2-carboxylate*

Triethylphosphine (0.399 ml, 2.70 mmol) was added dropwise to a stirred solution of 3-ethyl-8-fluoro-7-(hydroxymethyl)quinoxalin-2(1H)-one (**intermediate 65**) (0.2 g, 0.90 mmol) and $CBr_4$ (0.985 g, 2.97 mmol) in DCM (7.10 mL) at 0°C over a period of 5 minutes under nitrogen. Reaction was stirred at rt for 1 h. DCM was removed under vacuum and the resulting solid was slurry in diethyl ether. The white ppt was filtered under vacuum, washed with water followed by ether. The solid was dried under vacuum for overnight (no heat) to give 7-(bromomethyl)-3-ethyl-8-fluoroquinoxalin-2(1H)-one as a light brown solid.

To the above crude product was added methyl 6-methyl-5-(piperazin-1-yl)picolinate, 2HCl (**intermediate 16**) (278 mg, 0.90 mmol), acetonitrile (10 mL) and N-ethyl-N-isopropylpropan-2-amine (785 μl, 4.51 mmol) and heated to 70 °C for1 h. The reaction mixture was cooled, concentrated, quenched with aq. NaHCO3 solution (1 mL) and stirred for 1 h at rt. Water (3 mL) was added to the above mixture and stirred for 10 mins. The precipitate was filtered and washed with water (50 mL). The solid was purified via normal phase chromatography using 0-10% MeOH in DCM. The isolated product was 89% pure by LCMS. The above solid was further purified using mass directed Prep HPLC using 20-40% acetonitrile in water with NH4OH modifier to yield methyl 5-[4-[(2-ethyl-5-fluoro-3-oxo-4H-quinoxalin-6-yl)methyl]piperazin-1-yl]-6-methyl-pyridine-2-carboxylate (**intermediate 68**) (115 mg, 0.262 mmol, 29%) as white solid with 93% LCMS purity. 1H NMR (500 MHz, DMSO-d6) 1.23 (3H, t), 2.45 - 2.49 (3H, m), 2.53 - 2.69 (4H, m), 2.83 (2H, q), 2.98 (4H, br s), 3.73 (2H, s), 3.83 (3H, s), 7.31 (1H, t), 7.45 (1H, d), 7.56 (1H, d), 7.86 (1H, d), 12.44 (1H, br s); 19F NMR (471 MHz, DMSO-d6) -135.54 (1F, s). m/z (ES$^+$) $[M+H]^+$ = 440.

***Example 26:** 5-[4-[(2-ethyl-5-fluoro-3-oxo-4H-quinoxalin-6-yl)methyl]piperazin-1-yl]-6-methyl-pyridine-2-carboxamide*

7 N ammonia in methanol (6.40 ml, 44.78 mmol) was added to methyl 5-[4-[(2-ethyl-5-fluoro-3-oxo-4H-quinoxalin-6-yl)methyl]piperazin-1-yl]-6-methyl-pyridine-2-carboxylate (**intermediate 68**) (0.0984 g, 0.22 mmol) in a 40 mL scintillation vial, sealed and stirred at rt for 18 h. The reaction was concentrated under vacuum, added additional ammonia (6.40 ml, 44.78 mmol) solution and stirred at 50 °C for 16 h. The reaction was concentrated under vacuum and additional NH3 in methanol was added and stirred at rt for the weekend. Reaction was complete by LCMS. The reaction mixture was concentrated under vacuum, the resulting solid was slurry in diethyl ether. Solid was filtered and washed with additional ether and methanol to yield 5-[4-[(2-ethyl-5-fluoro-3-oxo-4H-quinoxalin-6-yl)methyl]piperazin-1-yl]-6-methyl-pyridine-2-carboxamide (**example 26**) (0.095 g, 100 %) as a white solid; 1H NMR (500 MHz, DMSO-d6) 1.22 (3H, br t),

2.45 - 2.49 (3H, m), 2.52 - 2.68 (4H, m), 2.82 (2H, q), 2.94 (4H, br s), 3.72 (2H, br s), 7.30 (1H, br t), 7.38 - 7.51 (2H, m), 7.55 (1H, br d), 7.80 (2H, br d), 12.41 (1H, br s); 19F NMR (471 MHz, DMSO-d6) -135.53 (1F, s); m/z ($ES^+$) $[M+H]^+$ = 425.

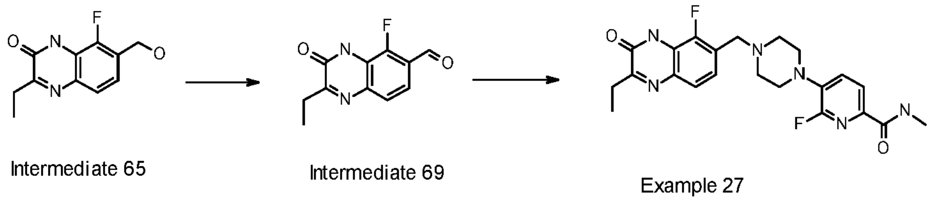

Intermediate 65 Intermediate 69 Example 27

***Intermediate 69:*** *2-ethyl-5-fluoro-3-oxo-4H-quinoxaline-6-carbaldehyde*

Dess-Martin periodinane (458 mg, 1.08 mmol) was added to 3-ethyl-8-fluoro-7-(hydroxymethyl)-1H-quinoxalin-2-one (**intermediate 65**) (contaminated by its regio isomer 3-ethyl-8-fluoro-5-(hydroxymethyl)-1H-quinoxalin-2-one) (160 mg, 0.72 mmol) in DCM (5 mL). The resulting mixture was stirred at room temperature for 4 h. The solvent was evaporated to afford crude product which was purified by flash C18-flash chromatography, elution gradient 5 to 30% MeCN in water (0.4% FA). Pure fractions were evaporated to dryness to afford 2-ethyl-5-fluoro-3-oxo-4H-quinoxaline-6-carbaldehyde (contaminated by its regio isomer 3-ethyl-8-fluoro-2-oxo-1H-quinoxaline-5-carbaldehyde) (**intermediate 69**) (110 mg, 69 %) as a yellow solid. m/z ($ES^+$) $[M+H]^+$ = 221.

***Example 27:*** *5-[4-[(2-ethyl-5-fluoro-3-oxo-4H-quinoxalin-6-yl)methyl]piperazin-1-yl]-6-fluoro-N-methylpyridine-2-carboxamide*

Titanium isopropoxide (64.5 mg, 0.23 mmol) was added to 2-ethyl-5-fluoro-3-oxo-4H-quinoxaline-6-carbaldehyde (**intermediate 69**) (contaminated by its regio isomer 3-ethyl-8-fluoro-2-oxo-1H-quinoxaline-5-carbaldehyde) (50 mg, 0.23 mmol) and 6-fluoro-N-methyl-5-(piperazin-1-yl)picolinamide (54.1 mg, 0.23 mmol) in THF (3 mL). The resulting mixture was stirred at room temperature for 2 mins. Sodium triacetoxyborohydride (**intermediate 32**)(192 mg, 0.91 mmol) was added. The resulting mixture was stirred at room temperature for 16 h. The reaction mixture was quenched with MeOH (0.1 mL). The solvent was evaporated to afford crude product. The crude residue was purified by preparative HPLC (Column: Xselect CSH OBD Column 30*150mm 5um; Mobile Phase A: Water(0.05% TFA), Mobile Phase B: ACN; Flow rate:60 mL/min; Gradient:10% B to 20% B in 10 min; 254; 220 nm) and (Column: XBridge Shield RP18 OBD Column, 19*250mm,10um; Mobile Phase A:Water(10MMOL/L $NH_4HCO_3$+0.1%$NH_3.H_2O$), Mobile Phase B:ACN; Flow rate:20 mL/min; Gradient:21 B to 95 B in 7 min; 254/220 nm. Fractions containing the desired compound were evaporated to dryness to afford 5-[4-[(2-ethyl-5-fluoro-3-oxo-4H-quinoxalin-6-yl)methyl]piperazin-1-yl]-6-fluoro-N-methyl-pyridine-2-carboxamide (**example 27**) (6 mg, 6 %) as a white solid. 1H NMR (400 MHz, DMSO-d6) 1.22 (3H, t), 2.56 - 2.64 (4H, m), 2.76 (3H, d), 2.82 (2H, q), 3.14 - 3.20 (4H, m), 3.71 (2H, s), 7.27 - 7.33 (1H, m), 7.53 - 7.59 (2H, m), 7.82 - 7.86 (1H, m), 8.38 - 8.45 (1H, m), 12.46 (1H, s); 19F NMR (376 MHz, DMSO-d6) -72.58, -135.51; m/z ($ES^+$) $[M+H]^+$ = 443.

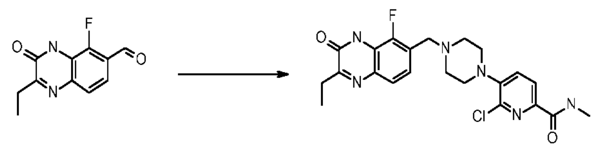

Intermediate 69 Example 28

***Example 28:*** *6-chloro-5-[4-[(2-ethyl-5-fluoro-3-oxo-4H-quinoxalin-6-yl)methyl]piperazin-1-yl]-N-methylpyridine-2-carboxamide*

Titanium isopropoxide (51.6 mg, 0.18 mmol) was added to 2-ethyl-5-fluoro-3-oxo-4H-quinoxaline-6-carbaldehyde (**intermediate 69**) (contaminated by its regio isomer 3-ethyl-8-fluoro-2-oxo-1H-quinoxaline-5-carbaldehyde) (40 mg, 0.18 mmol) and 6-chloro-N-methyl-5-(piperazin-1-yl)picolinamide (**intermediate 30**)(50 mg, 0.20 mmol) in THF (3 mL). The resulting mixture was stirred at room temperature for 2 minutes. Sodium triacetoxyborohydride (154 mg, 0.73 mmol) was added. The resulting mixture was stirred at room temperature for 16 hours. The reaction mixture was quenched with MeOH (0.1 mL) and evaporated to afford crude product. The crude product was purified by preparative HPLC (Column: XBridge Shield RP18 OBD Column, 30*150mm,5um ; Mobile Phase A:Water (0.05% $NH_3H_2O$), Mobile Phase B:ACN; Flow rate:60 mL/min; Gradient:21 B to 41 B in 7 min; 254/220 nm. Fractions containing the desired compound were evaporated to dryness to afford 6-chloro-5-[4-[(2-ethyl-5-fluoro-3-oxo-4H-quinoxalin-6-yl)methyl]piperazin-1-yl]-N-methyl-pyridine-2-carboxamide (**example 28**) (37.5 mg, 45 %) as a white solid. 1H NMR (400 MHz, DMSO-d6) 1.22 (3H, t), 2.57 - 2.65 (4H, m), 2.76 - 2.86 (5H, m), 3.05 - 3.15 (4H, m), 3.72 (2H, s), 7.29 (1H, t), 7.55 (1H, d), 7.65 (1H, d), 7.93 (1H, d), 8.40 - 8.45 (1H, m), 12.45 (1H, s); 19F NMR (376 MHz, DMSO-d6) -135.46; m/z (ES+) $[M+H]^+$ = 459.

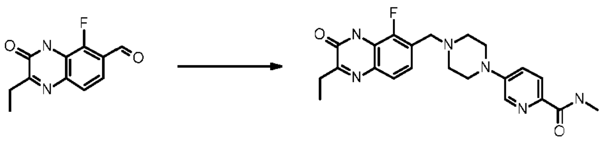

Intermediate 69 Example 29

***Example 29:*** *5-[4-[(2-ethyl-5-fluoro-3-oxo-4H-quinoxalin-6-yl)methyl]piperazin-1-yl]-N-methyl-pyridine-2-carboxamide*

Titanium isopropoxide (51.6 mg, 0.18 mmol) was added to 2-ethyl-5-fluoro-3-oxo-4H-quinoxaline-6-carbaldehyde (**intermediate 69**) (contaminated by its regio isomer 3-ethyl-8-fluoro-2-oxo-1H-quinoxaline-5-carbaldehyde) (40 mg, 0.18 mmol) and N-methyl-5-(piperazin-1-yl)picolinamide (**intermediate 31**)(50 mg, 0.23 mmol) in THF (3 mL). The resulting mixture was stirred at room temperature for 2 minutes. Sodium triacetoxyborohydride (154 mg, 0.73 mmol) was added. The resulting mixture was stirred at room temperature for 16 hours. The reaction mixture was quenched with MeOH (0.1 mL) and concentrated to afford crude product. The crude product was purified by preparative HPLC (Column: Xselect CSH OBD Column 30*150mm 5um, n; Mobile Phase A:Water (0.1% FA), Mobile Phase B:ACN; Flow rate:60 mL/min; Gradient:6 B to 17 B in 7 min; 254;220 nm. Fractions containing the desired compound were evaporated to dryness to afford 5-[4-[(2-ethyl-5-fluoro-3-oxo-4H-quinoxalin-6-yl)methyl]piperazin-1-yl]-N-methyl-pyridine-2-carboxamide (**example 29**) (17.29 mg, 22 %) as a white solid. 1H NMR (400 MHz, DMSO-d6) 1.22 (3H, t), 2.53 - 2.63 (4H, m), 2.74 - 2.87 (5H, m), 3.05 - 3.15 (4H, m, merged into water peak), 3.69 (2H, s), 7.29 (1H, t), 7.37 (1H, dd), 7.54 (1H, d), 7.81 (1H, d), 8.25 (1H, d), 8.35 - 8.42 (1H, m), 12.44 (1H, s); 19F NMR (376 MHz, DMSO-*d6*) -135.49; m/z (ES+) $[M+H]^+$ = 425.

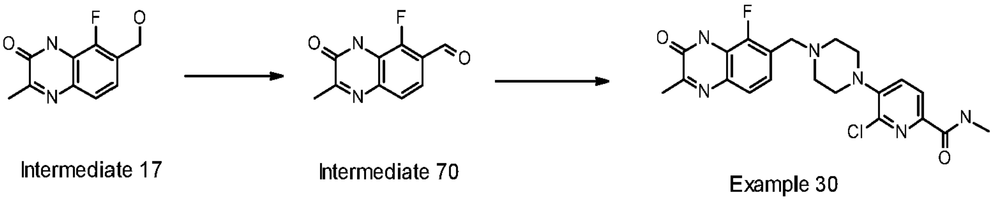

Intermediate 17 Intermediate 70 Example 30

***Intermediate 70:*** *5-fluoro-2-methyl-3-oxo-4H-quinoxaline-6-carbaldehyde*

Dess-Martin periodinane (1.34 g, 3.16 mmol) was added to 8-fluoro-7-(hydroxymethyl)-3-methyl-1H-quinoxalin-2-one (**intermediate 17**) (contaminated with 8-fluoro-5-(hydroxymethyl)-3-methyl-1H-quinoxalin-2-one) (0.33 g, 0.79 mmol) in DCM (20 ml). The resulting mixture was stirred at room temperature for 6 hours. The reaction mixture was evaporated to afford crude product. The crude product was purified by flash C18-flash chromatography, elution gradient 5 to 30% MeCN in water (0.4% FA). Pure fractions were evaporated to dryness to afford 5-fluoro-2-methyl-3-oxo-4H-quinoxaline-6-carbaldehyde (contaminated with 8-fluoro-3-methyl-2-oxo-1H-quinoxaline-5-carbaldehyde) **(intermediate 70)** (0.300 g, 92 %) as an off-white solid. m/z (ES+) $[M+H]^+$ = 207.

***Example 30:*** *6-chloro-5-[4-[(5-fluoro-2-methyl-3-oxo-4H-quinoxalin-6-yl)methyl]piperazin-1-yl]-N-methyl-pyridine-2-carboxamide*

Titanium isopropoxide (89 mg, 0.31 mmol) was added to 5-fluoro-2-methyl-3-oxo-4H-quinoxaline-6-carbaldehyde (contaminated with 8-fluoro-3-methyl-2-oxo-1H-quinoxaline-5-carbaldehyde) (**intermediate 70**) (150 mg, 0.36 mmol) and 6-chloro-N-methyl-5-piperazin-1-yl-pyridine-2-carboxamide (**intermediate 30**)(80 mg, 0.31 mmol) in THF (3 mL). The resulting mixture was stirred at room temperature for 20 minutes. Sodium triacetoxyborohydride (266 mg, 1.26 mmol) was added. The resulting mixture was stirred at room temperature for 16 hours. The reaction mixture was quenched with MeOH (0.1 mL) and concentrated to afford crude product. The crude product was purified by preparative HPLC (Column: XBridge Shield RP18 OBD Column, 19*250mm, 10 um; Mobile Phase A: Water (10mmol/L $NH_4HCO_3$+0.1% $NH_3.H_2O$), Mobile Phase B: MeOH-Preparative; Flow rate: 20 mL/min; Gradient: 57 B to 80 B in 7 min; 254/220 nm. Fractions containing the desired compound were evaporated to dryness to afford 6-chloro-5-[4-[(5-fluoro-2-methyl-3-oxo-4H-quinoxalin-6-yl)methyl]piperazin-1-yl]-N-methyl-pyridine-2-carboxamide (**example 30**) (35.6 mg, 25 %) as a white solid. 1H NMR (400 MHz, DMSO-*d6*) 2.42 (3H, s), 2.58 - 2.66 (4H, m), 2.79 (3H, d), 3.06 - 3.16 (4H, m), 3.72 (2H, s),

7.30 (1H, t), 7.53 (1H, d), 7.65 (1H, d), 7.93 (1H, d), 8.41 - 8.48 (1H, m), 12.47 (1H, s); 19F NMR (376 MHz, DMSO-d6) -135.45; m/z (ES+) $[M+H]^+$ = 445.

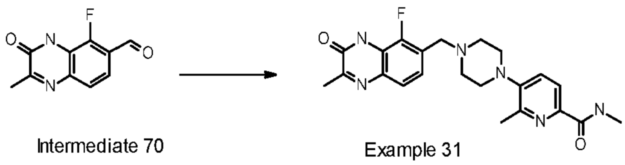
Intermediate 70 Example 31

***Example 31:*** *5-[4-[(5-fluoro-2-methyl-3-oxo-4H-quinoxalin-6-yl)methyl]piperazin-1-yl]-N,6-dimethylpyridine-2-carboxamide*

Titanium isopropoxide (105 mg, 0.37 mmol) was added to 5-fluoro-2-methyl-3-oxo-4H-quinoxaline-6-carbaldehyde (contaminated with 8-fluoro-3-methyl-2-oxo-1H-quinoxaline-5-carbaldehyde) (**intermediate 70**) (150 mg, 0.36 mmol) and N,6-dimethyl-5-piperazin-1-yl-pyridine-2-carboxamide, HCl (**intermediate 33**)(100 mg, 0.37 mmol) in THF (3 mL). The resulting mixture was stirred at rt for 2 minutes. Sodium triacetoxyborohydride (313 mg, 1.48 mmol) was added. The resulting mixture was stirred at room temperature for 16 hours. The reaction mixture was quenched with MeOH (0.1 mL) and evaporated to afford crude product. The crude product was purified by preparative HPLC (Column: XBridge Shield RP18 OBD Column, 30*150mm,5um ; Mobile Phase A:Water(0.05% $NH_3H_2O$), Mobile Phase B:ACN; Flow rate:60 mL/min; Gradient:19 B to 39 B in 7 min; 254/220 nm. Fractions containing the desired compound were evaporated to dryness to afford 5-[4-[(5-fluoro-2-methyl-3-oxo-4H-quinoxalin-6-yl)methyl]piperazin-1-yl]-N,6-dimethyl-pyridine-2-carboxamide (**example 31**) (12.39 mg, 8 %) as an off- white solid. 1H NMR (400 MHz, DMSO-*d6*) 2.42 (3H, s), 2.48 (3H, s), 2.57 - 2.67 (4H, m), 2.80 (3H, d), 2.90 - 2.98 (4H, m), 3.72 (2H, s), 7.30 (1H, t), 7.47 (1H, d), 7.52 (1H, d), 7.79 (1H, d), 8.39 - 8.46 (1H, m), 12.46 (1H, s); 19F NMR (376 MHz, DMSO-d6) -135.52; m/z (ES+) $[M+H]^+$ = 425.

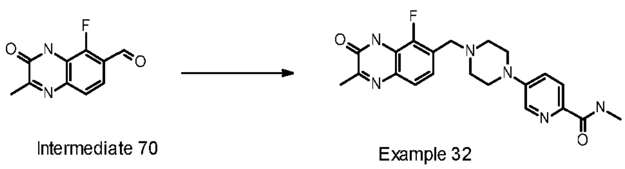
Intermediate 70 Example 32

***Example 32:*** *5-[4-[(5-fluoro-2-methyl-3-oxo-4H-quinoxalin-6-yl)methyl]piperazin-1-yl]-N-methylpyridine-2-carboxamide*

Titanium isopropoxide (103 mg, 0.36 mmol) was added to 5-fluoro-2-methyl-3-oxo-4H-quinoxaline-6-carbaldehyde (contaminated with 8-fluoro-3-methyl-2-oxo-1H-quinoxaline-5-carbaldehyde) (150 mg, 0.36 mmol) and N-methyl-5-(piperazin-1-yl)picolinamide (**intermediate 31**)(80 mg, 0.36 mmol) in THF (3 mL). The resulting mixture was stirred at room temperature for 2 minutes. Sodium triacetoxyborohydride (308 mg, 1.45 mmol) was added. The resulting mixture was stirred at room temperature for 16 hours. The reaction mixture was quenched with MeOH (0.1 mL). The reaction mixture was evaporated to afford crude product. The crude product was purified by preparative HPLC (Column: Xbridge Phenyl OBD Column,, 5um,19*150mm; Mobile Phase A:Water(0.05%TFA ), Mobile Phase B:MeOH-Preparative; Flow rate:20 mL/min; Gradient:24 B to 32 B in 12 min; 254/220 nm and (Column: XBridge Shield RP18 OBD Column, 19*250mm,10um; Mobile Phase A:Water(10mmol/L $NH_4HCO_3$+0.1%$NH_3.H_2O$), Mobile Phase B:ACN; Flow rate:20 mL/min; Gradient:15 B to 30 B in 10 min; 254/220 nm. Fractions containing the desired compound were evaporated to dryness to afford 5-[4-[(5-fluoro-2-methyl-3-oxo-4H-quinoxalin-6-yl)methyl]piperazin-1-yl]-N-methyl-pyridine-2-carboxamide (12.4 mg, 8 %) as a white solid. 1H NMR (400 MHz, DMSO-d6) 2.42 (3H, s), 2.55 - 2.60 (4H, m), 2.78 (3H, d), 2.90 - 2.98 (4H, m, merged into water peak), 3.70 (2H, s), 7.30 (1H, t), 7.38 (1H, dd), 7.52 (1H, d), 7.82 (1H, d), 8.26 (1H, d), 8.38 - 8.43 (1H, m), 12.47 (1H, s); 19F NMR (376 MHz, DMSO-*d6*) - 135.48; m/z (ES+) $[M+H]^+$ = 411.

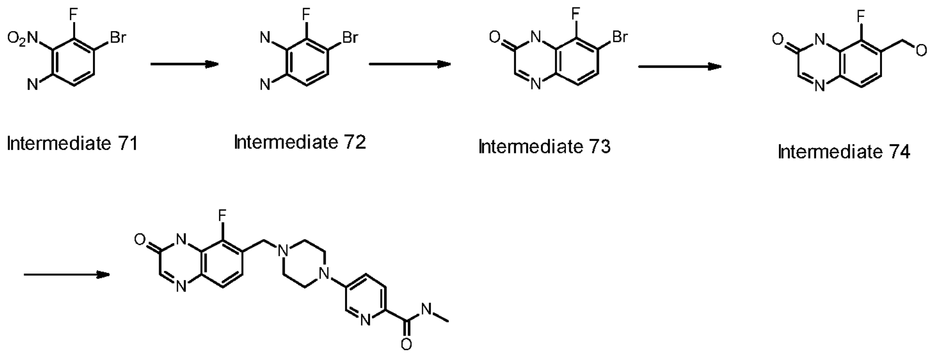

Intermediate 71 Intermediate 72 Intermediate 73 Intermediate 74

Example 33

***Intermediate 72:*** *4-bromo-3-fluoro-benzene-1,2-diamine*

Iron powder (5.2 g, 93.11 mmol) was added to 4-bromo-3-fluoro-2-nitro-aniline (**intermediate 71**) (7.3 g, 31.06 mmol) and HCl (10 mL, 100.00 mmol)(10 M) in MeOH (30 mL). The resulting mixture was stirred at room temperature for 18 hours. The solvent was removed under reduced pressure. The reaction mixture was basified with saturated $Na_2CO_3$ solution (100 mL). The aqueous layer was extracted with EtOAc (2 x 100 mL). The organic layer was dried over $Na_2SO_4$, filtered and evaporated to afford 4-bromo-3-fluoro-benzene-1,2-diamine (**intermediate 72**) (6.05 g, 95 %) as a dark solid. 1H NMR (400 MHz,DMSO-*d6*) 4.66 (2H, s), 4.94 (2H, s), 6.30 (1H, dd), 6.56 (1H, dd); m/z ($ES^+$) $[M+H]^+$ = 205, 207.

***Intermediate 73:*** *7-bromo-8-fluoro-1H-quinoxalin-2-one*

Ethyl 2-oxoacetate in toluene (6.41 g, 31.39 mmol) was added to 4-bromo-3-fluoro-benzene-1,2-diamine (**intermediate 72**) (4.46 g, 21.75 mmol) in toluene (30 mL). The resulting mixture was stirred at 100 °C for 30 minutes. The solvent was removed under reduced pressure. The reaction mixture was diluted with (PE: 10 mL and EA: 2 mL). The precipitate was collected by filtration, washed with EtOAc (5 mL) and dried under vacuum to afford 7-bromo-8-fluoro-1H-quinoxalin-2-one (**intermediate 73**) (contaminated by 6-bromo-5-fluoro-1H-quinoxalin-2-one) (2.75 g, 52 %) as an off-white solid. m/z ($ES^+$) $[M+H]^+$ = 243.

***Intermediate 74:*** *8-fluoro-7-(hydroxymethyl)-1H-quinoxalin-2-one*

CataCXium A-Pd-G2 (0.12 g, 0.18 mmol) was added to (tributylstannyl)methanol (1.25 g, 3.89 mmol) and 7-bromo-8-fluoro-1H-quinoxalin-2-one (**intermediate 73**) (1 g, 2.06 mmol) (contaminated by 6-bromo-5-fluoro-1H-quinoxalin-2-one) in 1,4-dioxane (30 mL). The resulting mixture was stirred at 100 °C for 18 hours under nitrogen. The reaction mixture was quenched with saturated KF (10 mL), filtered and evaporated to afford crude product. The crude product was purified by flash C18-flash chromatography, elution gradient 3 to 30% MeCN in water (0.1% formic acid). Pure fractions were evaporated to dryness to afford 8-fluoro-7-(hydroxymethyl)-1H-quinoxalin-2-one (**intermediate 74**) (260 mg, 69 %) (contaminated by 5-fluoro-6-(hydroxymethyl)-1H-quinoxalin-2-one) as an off-white solid. m/z ($ES^+$) $[M+H]^+$ = 195.

***Example 33:*** *5-[4-[(5-fluoro-3-oxo-4H-quinoxalin-6-yl)methyl]piperazin-1-yl]-N-methyl-pyridine-2-carboxamide*

$SOCl_2$ (0.3 mL, 4.11 mmol) was added to 8-fluoro-7-(hydroxymethyl)-1H-quinoxalin-2-one (**intermediate 74**) (158 mg, 0.41 mmol) (contaminated by 5-fluoro-6-(hydroxymethyl)-1H-quinoxalin-2-one) in DCM (3 mL). The resulting mixture was stirred at room temperature for 1 h. The solvent was removed under reduced pressure. DIPEA (0.25 mL, 1.43 mmol) and N-methyl-5-piperazin-1-yl-pyridine-2-carboxamide (**intermediate 31**)(141 mg, 0.64 mmol) were added to the mixture in NMP (3.00 mL). The resulting mixture was stirred at 80 °C for 1 h. The crude product was purified by preparative HPLC (Column: XBridge Prep OBD C18 Column, 19*250mm,5um; Mobile Phase A:Water(10 mmol/L $NH_4HCO_3$+0.1% $NH_3.H_2O$), Mobile Phase B:ACN; Flow rate:20 mL/min; Gradient:18 B to 24 B in 9 min; 254;220 nm). Fractions containing the desired compound were evaporated to dryness to afford 5-[4-[(5-fluoro-3-oxo-4H-quinoxalin-6-yl)methyl]piperazin-1-yl]-N-methyl-pyridine-2-carboxamide (**example 33**) (13.00 mg, 8 %) as a white solid. 1H NMR (400 MHz, DMSO-*d6*) 2.55 - 2.60 (4H, m), 2.77 (3H, d), 3.28 - 3.33 (4H, m), 3.71 (2H, s), 7.30 - 7.42 (2H, m), 7.61 (1H, d), 7.82 (1H, d), 8.20 (1H, s), 8.25 (1H, d), 8.37 - 8.42 (1H, m); 19F NMR (376 MHz, DMSO-*d6*) -129.26; m/z ($ES^+$) $[M+H]^+$ = 397.

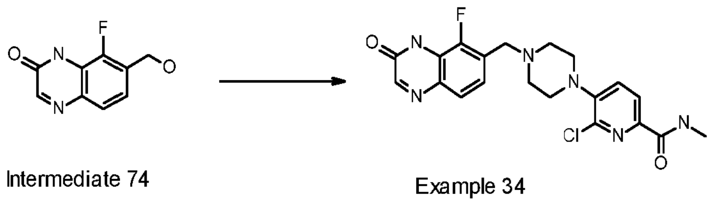

Intermediate 74 Example 34

***Example 34:*** *6-chloro-5-[4-[(5-fluoro-3-oxo-4H-quinoxalin-6-yl)methyl]piperazin-1-yl]-N-methylpyridine-2-carboxamide*

$SOCl_2$ (0.3 mL, 4.11 mmol) was added to 8-fluoro-7-(hydroxymethyl)-1H-quinoxalin-2-one (**intermediate 74**) (contaminated by 5-fluoro-6-(hydroxymethyl)-1H-quinoxalin-2-one) (143 mg, 0.37 mmol) in DCM (3 mL). The resulting mixture was stirred at room temperature for 1 h. The solvent was removed under reduced pressure. DIPEA (0.25 mL, 1.43 mmol) and 6-chloro-N-methyl-5-piperazin-1-yl-pyridine-2-carboxamide (**intermediate 30**)(101 mg, 0.40 mmol) were added to the mixture in NMP (3.00 mL). The resulting mixture was stirred at 80 °C for 1 h. The crude product was purified by preparative HPLC (Column: XBridge Prep OBD C18 Column, 19*250mm,5um; Mobile Phase A:Water(10MMOL/L $NH_4HCO_3$+0.1%$NH_3.H_2O$), Mobile Phase B:ACN; Flow rate:20 mL/min; Gradient:25 B to 28 B in 9 min; 254/220 nm. Fractions containing the desired compound were evaporated to dryness to afford 6-chloro-5-[4-[(5-fluoro-3-oxo-4H-quinoxalin-6-yl)methyl]piperazin-1-yl]-N-methyl-pyridine-2-carboxamide (**example 34**) (23.0 mg, 14 %) as a white solid. 1H NMR (400 MHz, DMSO-*d6*) 2.58 - 2.65 (4H, m), 2.78 (3H, d), 3.07 - 3.14 (4H, m), 3.73 (2H, s), 7.35 (1H, dd), 7.61 (1H, d), 7.65 (1H, d), 7.93 (1H, d), 8.20 (1H, s), 8.41 - 8.45 (1H, m), 12.58 (1H, s); 19F NMR (376 MHz, DMSO-*d6*) -135.18; m/z ($ES^+$) $[M+H]^+$ = 431.

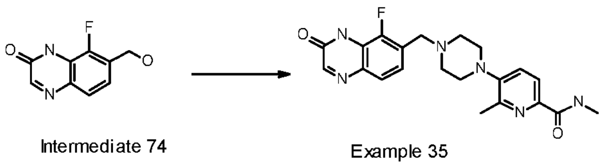

Intermediate 74 Example 35

***Example 35:*** *5-[4-[(5-fluoro-3-oxo-4H-quinoxalin-6-yl)methyl]piperazin-1-yl]-N,6-dimethyl-pyridine-2-carboxamide*

$SOCl_2$ (0.3 mL, 4.11 mmol) was added to 8-fluoro-7-(hydroxymethyl)-1H-quinoxalin-2-one (**intermediate 74**) (contaminated by 5-fluoro-6-(hydroxymethyl)-1H-quinoxalin-2-one) (143 mg, 0.37 mmol) (153 mg, 0.39 mmol) in DCM (3 mL). The resulting mixture was stirred at room temperature for 1 h. The solvent was removed under reduced pressure. DIPEA (0.25 mL, 1.43 mmol) and N,6-dimethyl-5-piperazin-1-yl-pyridine-2-carboxamide (**intermediate 33**)(137 mg, 0.58 mmol) in NMP (3 mL) were added to the mixture. The resulting mixture was stirred at 80 °C for 1 h. The crude product was purified by preparative HPLC (Column: XBridge Prep OBD C18 Column, 19*250mm,5um; Mobile Phase A:Water(10MMOL/L $NH_4HCO_3$+0.1%$NH_3.H_2O$), Mobile Phase B:ACN; Flow rate:20 mL/min; Gradient:24 B to 28 B in 9 min; 254/220 nm. Fractions containing the desired compound were evaporated to dryness to afford 5-[4-[(5-fluoro-3-oxo-4H-quinoxalin-6-yl)methyl]piperazin-1-yl]-N,6-dimethyl-pyridine-2-carboxamide (**example 35**) (13.0 mg, 8%) as a white solid. 1H NMR (400 MHz, DMSO-*d6*) 2.48 (3H, s), 2.56 - 2.65 (4H, s), 2.79 (3H, d), 2.92 - 2.97 (4H, m), 3.73 (2H, s), 7.31 - 7.39 (1H, m), 7.47 (1H, d), 7.61 (1H, d), 7.78 (1H, d), 8.19 (1H, s), 8.39 - 8.44 (1H, m), 12.55 (1H, s); 19F NMR (376 MHz, DMSO-*d6*) -135.25; m/z ($ES^+$) $[M+H]^+$ = 411.

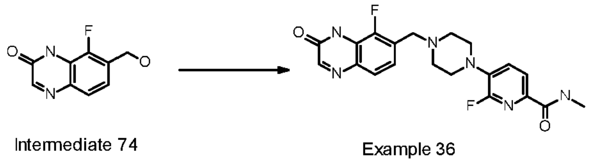

Intermediate 74 Example 36

***Example 36:*** *6-fluoro-5-[4-[(5-fluoro-3-oxo-4H-quinoxalin-6-yl)methyl]piperazin-1-yl]-N-methylpyridine-2-carboxamide*

$SOCl_2$ (0.3 mL, 4.11 mmol) was added to 8-fluoro-7-(hydroxymethyl)-1H-quinoxalin-2-one (contaminated by 5-fluoro-6-(hydroxymethyl)-1H-quinoxalin-2-one) (144 mg, 0.37 mmol) in DCM (3 mL). The resulting mixture was stirred at room temperature for 1 h. The solvent was removed under reduced pressure. DIPEA (0.25 mL, 1.43 mmol) and 6-fluoro-N-methyl-5-piperazin-1-yl-pyridine-2-carboxamide (**intermediate 32**)(94 mg, 0.39 mmol) were added to the mixture in NMP (3.00 mL). The resulting mixture was stirred at 80 °C for 1 h. The crude product was purified by preparative HPLC (Column:

XBridge Prep OBD C18 Column, 19*250mm,5um; Mobile Phase A:Water(10MMOL/L $NH_4HCO_3$+0.1%$NH_3.H_2O$), Mobile Phase B:ACN; Flow rate:20 mL/min; Gradient:23 B to 25 B in 9 min; 254/220 nm; RT1:6.9,8.46. Fractions containing the desired compound were evaporated to dryness to afford 6-fluoro-5-[4-[(5-fluoro-3-oxo-4H-quinoxalin-6-yl)methyl]piperazin-1-yl]-N-methyl-pyridine-2-carboxamide (**example 36**) (16.0 mg, 10 %) as a white solid. 1H NMR (400 MHz, DMSO-*d6*) 2.56 - 2.62 (4H, m), 2.76 (3H, d), 3.13 - 3.20 (4H, m), 3.71 (2H, d), 7.33 (1H, dd), 7.55 (1H, t), 7.61 (1H, d), 7.83 (1H, dd), 8.19 (1H, s), 8.37 - 8.43 (1H, m), 12.56 (1H, brs); 19F NMR (376 MHz, DMSO-*d6*) -72.57, -135.21; m/z ($ES^+$) $[M+H]^+$ = 415.

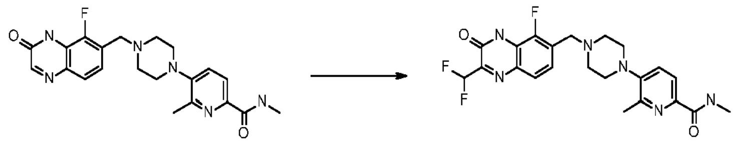

Example 35 Example 37

***Example 37:*** *5-[4-[[2-(difluoromethyl)-5-fluoro-3-oxo-4H-quinoxalin-6-yl]methyl]piperazin-1-yl]-N,6-dimethyl-pyridine-2-carboxamide*

A solution of iron(II) chloride (6.18 mg, 0.05 mmol) and zinc(II) difluoromethanesulfinate (86 mg, 0.29 mmol) in water (0.5 mL) was added portion wise to a stirred solution of 5-[4-[(5-fluoro-3-oxo-4H-quinoxalin-6-yl)methyl]piperazin-1-yl]-N,6-dimethyl-pyridine-2-carboxamide (**example 35**) (40.0 mg, 0.10 mmol) and TFA (7.51 μl, 0.10 mmol) in DMSO (3 mL) at room temperature. Followed by addition of tert-butyl hydroperoxide (9.44 μl, 0.10 mmol) and the resulting mixture was stirred at room temperature for 2 h. The crude product was purified by preparative HPLC (column, Column: XBridge Prep OBD C18 Column 30×150mm 5um; Mobile Phase A: Water (0.05% $NH_3H_2O$), Mobile Phase B: ACN; Flow rate: 60 mL/min; Gradient: 12% B to 32% B in 7 min; 254/220 nm; Rt: 6.07 min. Fractions containing the desired compound were evaporated to dryness to afford 5-[4-[[2-(difluoromethyl)-5-fluoro-3-oxo-4H-quinoxalin-6-yl]methyl]piperazin-1-yl]-N,6-dimethyl-pyridine-2-carboxamide (**example 37**) (2.2 mg, 5 %) as a pale yellow solid. 1H NMR (400 MHz, DMSO-*d6*) 2.49 (3H, s), 2.60 - 2.65 (4H, m), 2.80 (3H, d), 2.92 - 2.99 (4H, m), 3.76 (2H, s), 7.08 (1H, t), 7.39 (1H, t), 7.48 (1H, d), 7.70 (1H, d), 7.79 (1H, d), 8.42 (1H, q), 13.01 (1H, s); 19F NMR (376 MHz, DMSO-*d6*) -124.324, -134.183; m/z ($ES^+$) $[M+H]^+$ = 461.

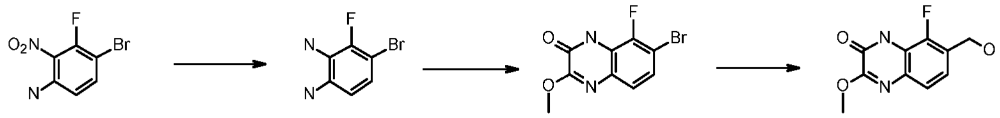

Intermediate 71 Intermediate 72 Intermediate 75 Intermediate 76

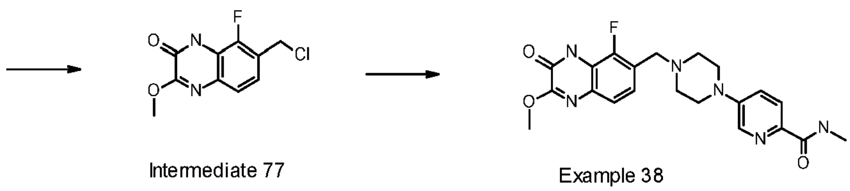

Intermediate 77 Example 38

***Intermediate 72:*** *4-bromo-3-fluoro-benzene-1,2-diamine*

Iron powder (3.56 g, 63.83 mmol) was added to 4-bromo-3-fluoro-2-nitro-aniline (**intermediate 71**) (3.00 g, 12.77 mmol), concentrated hydrogen chloride (10.64 ml, 127.65 mmol) in MeOH (30 mL) at rt. The resulting mixture was stirred at rt for 16 h. The reaction mixture was diluted with water (100 mL) and extracted with ethyl acetate (100 mL x 3). The organic layer was dried over $MgSO_4$, filtered and evaporated to afford 4-bromo-3-fluoro-benzene-1,2-diamine (**intermediate 72**) (2.5 g, 96%). 1H NMR (400 MHz, DMSO-d6) 4.66 (2H, s), 4.94 (2H, s), 6.30 (dd, 1H), 6.56 (dd, 1H); m/z ($ES^+$) $[M+H]^+$ = 205.

***Intermediate 75:*** *7-bromo-8-fluoro-3-methoxy-1H-quinoxalin-2-one*

Methyl 2,2,2-trimethoxyacetate (2.402 g, 14.63 mmol) was added to 4-bromo-3-fluorobenzene-1,2-diamine (**intermediate 72**) (1.500 g, 7.32 mmol), tris(((trifluoromethyl)sulfonyl)oxy)ytterbium (0.454 g, 0.73 mmol) in toluene (20 mL) at room temperature. The resulting mixture was stirred at 100 °C for 5 h. The solvent was removed under reduced pressure. The crude product was purified by reverse phase chromatography on C18 column, elution gradient 5 to 70% MeCN in water. Pure fractions were evaporated to dryness to afford 7-bromo-8-fluoro-3-methoxy-1H-quinoxalin-2-one

(**intermediate 75**) (0.650 g, 32 %) as a white solid. 1H NMR (300 MHz, DMSO-*d6*) 3.97 (3H, s), 7.31 (1H, dd), 7.45 (1H, dd); m/z ($ES^+$) $[M+H]^+$ = 273.

***Intermediate 76:** 8-fluoro-7-(hydroxymethyl)-3-methoxy-1H-quinoxalin-2-one*

(tributylstannyl)methanol (882 mg, 2.75 mmol) was added to 7-bromo-8-fluoro-3-methoxyquinoxalin-2(1H)-one (**intermediate 75**) (300.0 mg, 1.1 mmol), cataCXium A-Pd-G2 (73 mg, 0.11 mmol) in 1,4-dioxane (20 mL) at room temperature under nitrogen. The resulting mixture was stirred at 100 °C for 16 h. The reaction mixture was quenched with sat. KF (10 mL) and then filtered. The solvent was removed under reduced pressure. The crude product was purified by reverse phase chromatography on C18 column, elution gradient 5 to 100% MeOH in water. Pure fractions were evaporated to dryness to afford 8-fluoro-7-(hydroxymethyl)-3-methoxy-1H-quinoxalin-2-one (**intermediate 76**) (130 mg, 53 %) as a white solid. 1H NMR (300 MHz, DMSO-*d6*) 3.97 (3H, s), 4.88 (2H, d), 5.36 (1H, s), 7.27 - 7.32 (1H, m), 7.36 (1H, d), 12.45 (1H, s); m/z ($ES^+$) $[M+H]^+$ = 225.

***Intermediate 77:** 7-(chloromethyl)-8-fluoro-3-methoxy-1H-quinoxalin-2-one*

$SOCl_2$ (8 ml, 109.62 mmol) was added to 8-fluoro-7-(hydroxymethyl)-3-methoxy-1H-quinoxalin-2-one (**intermediate 76**) (50.0 mg, 0.22 mmol) in diethyl ether (50 mL) at room temperature. The resulting mixture was stirred at room temperature for 16 h. The solvent was removed under reduced pressure to afford 7-(chloromethyl)-8-fluoro-3-methoxy-1H-quinoxalin-2-one (**intermediate 77**) (66.7 mg, 122%, crude) as a yellow oil. The product was used in the next step directly without further purification. 1H NMR (300 MHz, DMSO-*d6*) 3.97 (3H, s), 4.88 (2H, s), 7.27 - 7.42 (2H, m), 12.60 (1H, s) m/z ($ES^+$) $[M+H]^+$ = 243.

***Example 38**: 5-[4-[(5-fluoro-2-methoxy-3-oxo-4H-quinoxalin-6-yl)methyl]piperazin-1-yl]-N-methylpyridine-2-carboxamide*

N-methyl-5-piperazin-1-yl-pyridine-2-carboxamide (**intermediate 31**)(150 mg, 0.68 mmol) was added to 7-(chloromethyl)-8-fluoro-3-methoxy-1H-quinoxalin-2-one (**intermediate 77**)(198 mg, 0.82 mmol), DIPEA (0.595 mL, 3.40 mmol) in MeCN (10 mL) at room temperature. The resulting mixture was stirred at 60 °C for 16 h. The solvent was removed under reduced pressure. The crude product was purified by flash C18-flash chromatography, elution gradient 5 to 70% MeCN in water. Pure fractions were evaporated to dryness to afford the product (103.0 mg) as a yellow solid (80% purity by UV). Repurified by flash C18-flash chromatography, elution gradient 5 to 70% MeCN in water. Pure fractions were evaporated to dryness to afford 5-[4-[(5-fluoro-2-methoxy-3-oxo-4H-quinoxalin-6-yl)methyl]piperazin-1-yl]-N-methylpyridine-2-carboxamide (**example 38**) (36.0 mg, 12 %) as a yellow solid. 1H NMR (300 MHz, DMSO-*d6*) 2.51 - 2.60 (4H, m), 2.77 (3H, d), 3.18 - 3.45 (4H, m), 3.66 (2H, s), 3.95 (3H, s), 7.10 - 7.27 (1H, m), 7.27 - 7.42 (2H, m), 7.81 (1H, d), 8.25 (1H, d), 8.39 (1H, q), 12.30 (1H, s); 19F NMR (282 MHz, DMSO-*d6*) -134.783; m/z ($ES^+$) $[M+H]^+$ = 427.

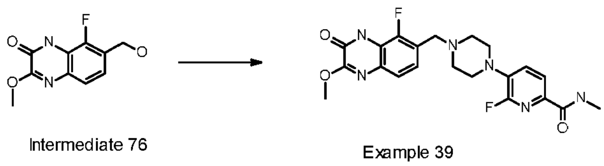

Intermediate 76 Example 39

***Example 39**: 6-fluoro-5-[4-[(5-fluoro-2-methoxy-3-oxo-4H-quinoxalin-6-yl)methyl]piperazin-1-yl]-N-methyl-pyridine-2-carboxamide*

$SOCl_2$ (0.065 mL, 0.89 mmol) was added to 8-fluoro-7-(hydroxymethyl)-3-methoxy-1H-quinoxalin-2-one (**intermediate 76**) (0.040 g, 0.18 mmol) in diethyl ether (10 mL) at room temperature. The resulting mixture was stirred at room temperature for 16 h. The solvent was removed under reduced pressure. 6-fluoro-N-methyl-5-piperazin-1-yl-pyridine-2-carboxamide (**intermediate 32**)(0.043 g, 0.18 mmol) and DIPEA (0.156 mL, 0.89 mmol) in MeCN (10.00 mL) were added to the above solid at room temperature. The resulting mixture was stirred at 60 °C for 16 h. The solvent was removed under reduced pressure. The crude product was purified by preparative HPLC column, Column: XBridge Shield RP18 OBD Column, 19*250mm, 10um; Mobile Phase A: Water (10 mmol/L $NH_4HCO_3$ + 0.1%$NH_3.H_2O$), Mobile Phase B: ACN; Flow rate: 20 mL/min; Gradient: 34 B to 48 B in 7 min; 254/220 nm; RT1:5.9. Fractions containing the desired compound were evaporated to dryness to afford 6-fluoro-5-[4-[(5-fluoro-2-methoxy-3-oxo-4H-quinoxalin-6-yl)methyl]piperazin-1-yl]-N-methyl-pyridine-2-carboxamide (**example 39**)(0.020 g, 25 %) as a white solid. 1H NMR (300 MHz, DMSO-*d6*) 2.55 - 2.61 (4H, m), 2.75 (3H, d), 3.11 - 3.19 (4H, m), 3.67 (2H, s), 3.96 (3H, s), 7.18 - 7.29 (1H, m), 7.35 (1H, d), 7.55 (1H, dd), 7.79 -

7.88 (1H, m), 8.41 (1H, d), 12.50 (1H, s); 19F NMR (282 MHz, DMSO-d6) -72.581, -134.799; m/z (ES+) [M+H]+ = 445.

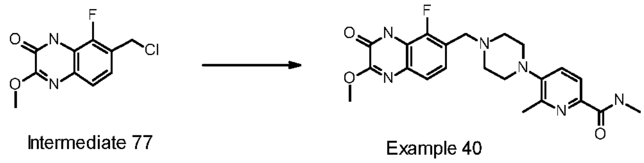

Intermediate 77 Example 40

***Example 40:** 5-[4-[(5-fluoro-2-methoxy-3-oxo-4H-quinoxalin-6-yl)methyl]piperazin-1-yl]-N,6-dimethylpyridine-2-carboxamide*

N,6-dimethyl-5-piperazin-1-yl-pyridine-2-carboxamide (**intermediate 33**)(0.097 g, 0.41 mmol) was added to 7-(chloromethyl)-8-fluoro-3-methoxyquinoxalin-2(1H)-one (**intermediate 77**) (0.100 g, 0.41 mmol), DIPEA (0.360 mL, 2.06 mmol) in MeCN (10 mL) at room temperature. The resulting mixture was stirred at 60 °C for 16 h. The solvent was removed under reduced pressure. The crude product was purified by preparative HPLC, Column: XBridge Prep OBD C18 Column, 30×150mm 5um; Mobile Phase A: Water (0.05% $NH_3H2_O$), Mobile Phase B: ACN; Flow rate: 60 mL/min; Gradient: 10B to 30B in 7 min; 254/220 nm. Fractions containing the desired compound were evaporated to dryness to afford 5-[4-[(5-fluoro-2-methoxy-3-oxo-4H-quinoxalin-6-yl)methyl]piperazin-1-yl]-N,6-dimethyl-pyridine-2-carboxamide (**example 40**) (0.063 g, 35%) as a white solid. 1H NMR (400 MHz, DMSO-*d6*) 2.48 (3H, s), 2.58 - 2.63 (4H, m), 2.80 (3H, d), 2.92 - 2.96 (4H, m), 3.69 (2H, s), 3.97 (3H, s), 7.25 (1H, t), 7.36 (1H, d), 7.47 (1H, d), 7.79 (1H, d), 8.43 (1H, q), 12.51 (1H, s); 19F NMR (376 MHz, DMSO-d6) -134.815; m/z (ES+) [M+H]+ = 441.

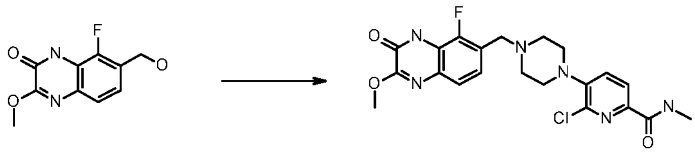

Intermediate 76 Example 41

***Example 41:** 6-chloro-5-[4-[(5-fluoro-2-methoxy-3-oxo-4H-quinoxalin-6-yl)methyl]piperazin-1-yl]-N-methyl-pyridine-2-carboxamide*

$SOCl_2$ (0.065 mL, 0.89 mmol) was added to 8-fluoro-7-(hydroxymethyl)-3-methoxy-1H-quinoxalin-2-one (**intermediate 76**) (0.040 g, 0.18 mmol) in diethyl ether (10 mL) at room temperature. The resulting mixture was stirred at room temperature for 16 h. The solvent was removed under reduced pressure to afford crude 7-(chloromethyl)-8-fluoro-3-methoxy-1H-quinoxalin-2-one (0.045 g, 0.18 mmol). MeCN (10.00 mL) was added to the above solid followed by addition of 6-chloro-N-methyl-5-piperazin-1-yl-pyridine-2-carboxamide (**intermediate 30**)(0.045g, 0.18 mmol) and DIPEA (0.156 mL, 0.89 mmol). The resulting mixture was stirred at 80 °C for 16 hours. The solvent was removed under reduced pressure. The crude product was purified by preparative HPLC column, Column: YMC-Actus Triart C18, 30*250,5um; Mobile Phase A: Water (0.05% $NH_3H_2O$), Mobile Phase B: ACN; Flow rate: 60 mL/min; Gradient: 21 B to 41 B in 7 min; 254, 220 nm; RT1:6.18. Fractions containing the desired compound were evaporated to dryness to afford 6-chloro-5-[4-[(5-fluoro-2-methoxy-3-oxo-4H-quinoxalin-6-yl)methyl]piperazin-1-yl]-N-methyl-pyridine-2-carboxamide (**example 41**) (0.041 g, 50 %) as a white solid. 1H NMR (300 MHz, DMSO-*d6*) 2.56 - 2.66 (4H, m), 2.79 (3H, d), 3.06 3.16 (4H, m), 3.70 (2H, s), 3.97 (3H, s), 7.19 - 7.30 (m, 1H), 7.36 (d, 1H), 7.66 (d, 1H), 7.93 (d, 1H), 8.44 (d, 1H), 12.47 (s, 1H); 19F NMR (282 MHz, DMSO-*d6*) -134.746; m/z (ES+) [M+H]+ = 461.

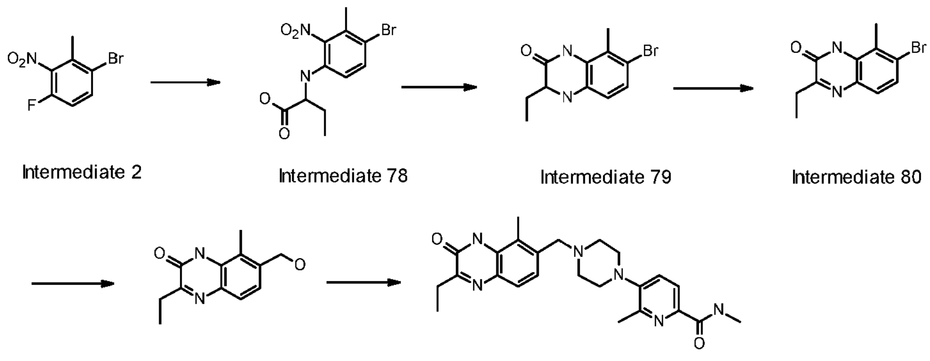

Intermediate 2 Intermediate 78 Intermediate 79 Intermediate 80

Intermediate 81 Example 42

***Intermediate 78:*** *2-(4-bromo-3-methyl-2-nitro-anilino)butanoic acid*

2-aminobutanoic acid (0.793 g, 7.69 mmol) was added to 1-bromo-4-fluoro-2-methyl-3-nitrobenzene (**intermediate 2**)(1.500 g, 6.41 mmol), $K_2CO_3$ (2.66 g, 19.23 mmol) in DMF (20 mL) at room temperature. The resulting mixture was stirred at 100 °C for 6 h. The reaction mixture was poured into ice water, quenched slowly with 1 M HCl (20 mL) at 0 °C to give a yellow suspension. The solid was collected by filtration, washed with water and dried to afford 2-(4-bromo-3-methyl-2-nitro-anilino)butanoic acid (**intermediate 78**) (1.4 g, 74%) as a yellow solid (not very pure, carried over to next step without further purification). m/z ($ES^+$) $[M+H]^+$ = 317.

***Intermediate 79:*** *7-bromo-3-ethyl-8-methyl-3,4-dihydro-1H-quinoxalin-2-one*

Iron powder (1.585 g, 28.38 mmol) was added slowly to 2-(4-bromo-3-methyl-2-nitro-anilino)butanoic acid (**intermediate 78**) (1.800 g, 5.68 mmol), concentrated hydrogen chloride (4.73 ml, 56.76 mmol) in MeOH (100 mL) at room temperature. The resulting mixture was stirred at room temperature for 7 h. The reaction mixture was filtered. The solvent was removed under reduced pressure. The reaction mixture was quenched with saturated $Na_2CO_3$ (40 mL) and extracted with EtOAc (3 x 50 mL). The organic layer was dried over $Na_2SO_4$, filtered and evaporated to afford brown solid. The crude product was purified by flash C18-flash chromatography, elution gradient 5 to 50% MeCN in water. Pure fractions were evaporated to dryness to afford 7-bromo-3-ethyl-8-methyl-3,4-dihydro-1H-quinoxalin-2-one (**intermediate 79**) (650 mg, 43 %) as a white solid. 1H NMR (300 MHz, DMSO-*d6*) 0.90 (3H, t), 1.43 - 1.72 (2H, m), 2.22 (3H, s), 3.56 (1H, ddd), 6.16 (1H, d), 6.56 (1H, d), 6.97 (1H, d), 9.76 (1H, s); m/z ($ES^+$) $[M+H]^+$ = 269.

***Intermediate 80:*** *7-bromo-3-ethyl-8-methyl-1H-quinoxalin-2-one*

DDQ (1.316 g, 5.80 mmol) was added to 7-bromo-3-ethyl-8-methyl-3,4-dihydro-1H-quinoxalin-2-one (**intermediate 79**) (1.300 g, 4.83 mmol) in 1,4-dioxane (150 mL) at room temperature. The resulting mixture was stirred at room temperature for 3 h. The solvent was removed under reduced pressure. The reaction mixture was quenched with saturated $NaHCO_3$ (150 mL). The precipitate was collected by filtration. The solid was washed with water (10 mL x 3) and dried under vacuum to afford the desired product 7-bromo-3-ethyl-8-methyl-1H-quinoxalin-2-one (**intermediate 80**) (1.2 g, 93%) as yellow solid. 1H NMR (300 MHz, DMSO-*d6*) 1.20 (3H, t), 2.44 - 2.53 (3H, m), 2.78 (2H, q), 7.48 (2H, s), 11.74 (1H, s); m/z ($ES^+$) $[M+H]^+$ = 267.

***Intermediate 81:*** *3-ethyl-7-(hydroxymethyl)-8-methyl-1H-quinoxalin-2-one*

(tributylstannyl)methanol (1202 mg, 3.74 mmol) was added to 7-bromo-3-ethyl-8-methylquinoxalin-2(1H)-one (**intermediate 80**) (400 mg, 1.50 mmol), $Pd(PPh_3)_4$ (173 mg, 0.15 mmol) in 1,4-dioxane (40 mL) at room temperature under nitrogen. The resulting mixture was stirred at 60 °C for 16 h. The reaction mixture was quenched with KF (10 mL) and the solid was filtered off. The solvent was removed under reduced pressure. The crude product was purified by flash C18-flash chromatography, elution gradient 5 to 100% MeOH in water. Pure fractions were evaporated to dryness to afford 3-ethyl-7-(hydroxymethyl)-8-methyl-1H-quinoxalin-2-one (**intermediate 81**) (100 mg, 31 %) as a white solid. 1H NMR (400 MHz, DMSO-*d6*) 1.22 (3H, t), 2.32 (3H, s), 2.81 (2H, q), 4.59 (2H, d), 5.25 (1H, s), 7.33 (1H, d), 7.55 (1H, d); m/z ($ES^+$) $[M+H]^+$ = 219.

***Example 42:** 5-[4-[(2-ethyl-5-methyl-3-oxo-4H-quinoxalin-6-yl)methyl]piperazin-1-yl]-N,6-dimethylpyridine-2-carboxamide*

HBr in AcOH (1 ml, 6.08 mmol) (33 w%) was added to 3-ethyl-7-(hydroxymethyl)-8-methylquinoxalin-2(1H)-one (**intermediate 81**) (65.0 mg, 0.30 mmol) at rt. The resulting mixture was stirred at 60 °C for 2 h. The solvent was removed under reduced pressure. N,6-dimethyl-5-piperazin-1-yl-pyridine-2-carboxamide (**intermediate 33**)(69.8 mg, 0.30 mmol) and DIPEA (0.156 ml, 0.89 mmol) in NMP (3 mL) were added to the above solid at room temperature. The resulting mixture was stirred at 60 °C for 2 h. The crude product was purified by preparative HPLC ( column, Column: Sunfire prep C18 column, 30*150, 5um; Mobile Phase A:Water(0.1%FA), Mobile Phase B:ACN; Flow rate:60 mL/min; Gradient:9 B to 20 B in 7 min; 254/220 nm; RT1:5.15; Fractions containing the desired compound were evaporated to dryness to afford 5-[4-[(2-ethyl-5-methyl-3-oxo-4H-quinoxalin-6-yl)methyl]piperazin-1-yl]-N,6-dimethyl-pyridine-2-carboxamide (**example 42**) (0.049 g, 38%) as a pale yellow solid. 1H NMR (300 MHz, DMSO-*d6*) 1.20 (3H, t), 2.42 (3H, s), 2.50 (3H, s), 2.53 - 2.59 (4H, m), 2.73 - 2.86 (5H, m), 2.87 - 2.93 (4H, m), 3.61 (2H, s), 7.23 (1H, d), 7.45 (1H, d), 7.52 (1H, d), 7.76 (1H, d), 8.39 (1H, d), 11.52 (1H, s); m/z (ES$^+$) $[M+H]^+$ = 435.

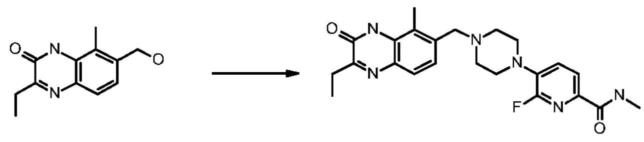

Intermediate 81 Example 43

***Example 43:** 5-[4-[(2-ethyl-5-methyl-3-oxo-4H-quinoxalin-6-yl)methyl]piperazin-1-yl]-6-fluoro-N-methyl-pyridine-2-carboxamide*

HBr in AcOH (1ml, 6.08 mmol)(33 w%) was added to 3-ethyl-7-(hydroxymethyl)-8-methyl-1H-quinoxalin-2-one (**intermediate 81**) (65.0 mg, 0.30 mmol) at room temperature. The resulting mixture was stirred at 60 °C for 2 hours. The solvent was removed under reduced pressure. 6-fluoro-N-methyl-5-(piperazin-1-yl)picolinamide (**intermediate 32**)(71.0 mg, 0.30 mmol) was added to the above solid, followed by addition of DIPEA (0.156 ml, 0.89 mmol) in NMP (3 mL) at room temperature. The resulting mixture was stirred at 60 °C for 2 hours. The crude product was purified by preparative HPLC ( column, Column: XBridge Prep OBD C18 Column, 30×150mm 5um; Mobile Phase A:Water(0.05%NH3H2O), Mobile Phase B:ACN; Flow rate:60 mL/min; Gradient:31 B to 51 B in 7 min; 254/220 nm; RT1:6.27; Fractions containing the desired compound were evaporated to dryness to afford 5-[4-[(2-ethyl-5-methyl-3-oxo-4H-qu inoxa lin-6-yl) methyl] piperazin-1-yl]-6- fluoro- N-methyl-pyrid ine-2-carboxa mide (**example 43**) (0.043 g, 33 %) as a pale yellow solid. 1H NMR (300 MHz, DMSO-*d6*) 1.20 (3H, t), 2.41 (3H, s), 2.49 - 2.59 (4H, m), 2.70 - 2.81 (5H, m), 3.08 - 3.16 (4H, m), 3.59 (2H, s), 7.22 (1H, d), 7.47 - 7.60 (2H, m), 7.82 (1H, dd), 8.37 (1H, d), 11.52 (1H, s); 19F NMR (282 MHz, DMSO-*d6*) -72.539; m/z (ES$^+$) $[M+H]^+$ = 439.

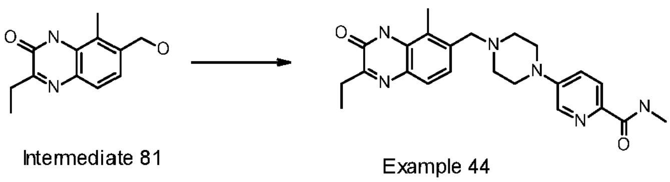

Intermediate 81 Example 44

***Example 44:** 5-[4-[(2-ethyl-5-methyl-3-oxo-4H-quinoxalin-6-yl)methyl]piperazin-1-yl]-N-methylpyridine-2-carboxamide*

HBr in AcOH (1ml, 18.42 mmol) (33 wt%) was added to 3-ethyl-7-(hydroxymethyl)-8-methyl-1H-quinoxalin-2-one (**intermediate 81**) (65.0 mg, 0.30 mmol) at room temperature. The resulting mixture was stirred at 60 °C for 2 hours. The solvent was removed under reduced pressure. N-methyl-5-(piperazin-1-yl)picolinamide (**intermediate 31**)(65.6 mg, 0.30 mmol) and DIPEA (0.156 ml, 0.89 mmol) was added to the above solid in NMP (3 mL) at room temperature. The resulting mixture was stirred at 60 °C for 2 h. The crude product was purified by preparative HPLC (Column: Sunfire prep C18 column, 30*150, 5um; Mobile Phase A:Water(0.1% FA), Mobile Phase B:ACN; Flow rate:60 mL/min; Gradient:9 B to 20 B in 7 min; 254/220 nm; RT1:5.15; Fractions containing the desired compound were evaporated to dryness to afford 5-[4-[(2-ethyl-5-methyl-3-oxo-4H-quinoxalin-6-yl)methyl]piperazin-1-yl]-N-methylpyridine-2-carboxamide (**example 44**) (0.014 g, 10%) as a pale yellow solid. 1H NMR (400 MHz, DMSO-*d6*) 1.16-1.26 (3H, m), 2.41 (3H, s), 2.49 - 2.59 (4H, m), 2.70 - 2.81 (5H, m), 3.30 - 3.35 (4H, m, merged into water peak), 3.61 (2H, s), 7.25 (1H, d), 7.38 (1H, d), 7.55 (1H, d), 7.82 (1H, d), 8.26 (1H, s), 8.38 (1H, s), 11.53 (1H, s); m/z (ES$^+$) $[M+H]^+$ = 421.

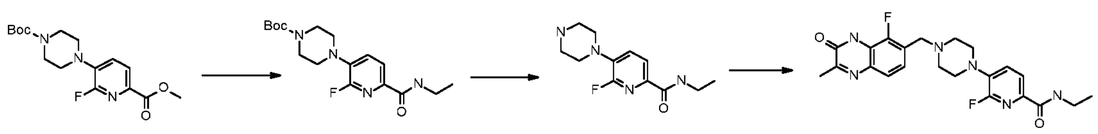

Intermediate 11 Intermediate 82 Intermediate 83 Example 45

***Intermediate 82:** tert-butyl 4-[6-(ethylcarbamoyl)-2-fluoro-3-pyridyl]piperazine-1-carboxylate*

tert-butyl 4-(2-fluoro-6-methoxycarbonyl-3-pyridyl)piperazine-1-carboxylate (**intermediate 11**) (500 mg, 1.47 mmol) was added to ethylamine in water (10 mL, 1.47 mmol) (65 wt%). The resulting mixture was stirred at room temperature for 2 h. The reaction went to completion. The precipitate was collected by filtration, washed with water (2 mL x 3) and dried under vacuum to afford tert-butyl 4-[6-(ethylcarbamoyl)-2-fluoro-3-pyridyl]piperazine-1-carboxylate (**intermediate 82**) (0.515 g, 99 %) as an off-white solid. 1H NMR (400 MHz, DMSO-*d6*) 1.09 (3H, t), 1.42 (9H, s), 3.11 (4H, t), 3.23 - 3.30 (2H, m), 3.49 (4H, t), 7.59 (1H, dd), 7.85 (1H, d), 8.45 (1H, t); m/z (ES$^+$) $[M+H]^+$ = 353.

***Intermediate 83**: N-ethyl-6-fluoro-5-piperazin-1-yl-pyridine-2-carboxamide*

tert-butyl 4-[6-(ethylcarbamoyl)-2-fluoro-3-pyridyl]piperazine-1-carboxylate (**intermediate 82**) (536 mg, 1.52 mmol) was added to HCl in 1,4-dioxane (5 mL, 20.00 mmol). The resulting mixture was stirred at room temperature for 1 h. DIPEA (5 mL) was added and the resulting mixture was stirred at room temperature for 15 min. The reaction mixture was evaporated to afford crude product. The crude product was purified by flash C18-flash chromatography, elution gradient 5 to 50% MeCN in water (0.1% $NH_4HCO_3$). Pure fractions were evaporated to dryness to afford N-ethyl-6-fluoro-5-piperazin-1-yl-pyridine-2-carboxamide (**intermediate 83**) (0.368 g, 96 %) as a yellow solid. The sample was not pure, carried over to next step without further purification; m/z (ES$^+$) $[M+H]^+$ = 253.

***Example 45:** N-ethyl-6-fluoro-5-[4-[(5-fluoro-2-methyl-3-oxo-4H-quinoxalin-6-yl)methyl]piperazin-1-yl]pyridine-2-carboxamide*

$Ph_3P$ (94 mg, 0.36 mmol) was added to $CBr_4$ (119 mg, 0.36 mmol), 8-fluoro-7-(hydroxymethyl)-3-methylquinoxalin-2(1H)-one (**intermediate 17**) (50 mg, 0.24 mmol) in $CH_2Cl_2$ (3 mL). The resulting mixture was stirred at room temperature for 1 h. The solvent was removed under reduced pressure. N-ethyl-6-fluoro-5-piperazin-1-yl-pyridine-2-carboxamide (**intermediate 83**) (60 mg, 0.24 mmol) and DIPEA (1.5 mL, 8.59 mmol) in NMP (3 mL) were added to the mixture. The resulting mixture was stirred at 80 °C for 2 h. The solvent was removed under reduced pressure. The crude product was purified by flash C18-flash chromatography, elution gradient 0 to 25% MeCN in water ($NH_4HCO_3$). Pure fractions were evaporated to dryness to afford N-ethyl-6-fluoro-5-[4-[(5-fluoro-2-methyl-3-oxo-4H-quinoxalin-6-yl)methyl] piperazin-1-yl]pyridine-2-carboxamide (**example 45**) (2.60 mg, 3 %) as a white solid. 1H NMR (300 MHz, DMSO-*d6*) 1.09 (3H, t), 2.40 (3H, s), 2.52 - 2.62 (4H, m), 3.17 - 3.27 (4H, m), 3.25 (2H, q), 3.68 (2H, s), 7.28 (1H, t), 7.48 - 7.59 (2H, m), 7.82 (1H, d), 8.41 (1H, t),12.48 (1H, s); 19F NMR (282 MHz, DMSO-*d6*) -72.58, -135.52; m/z (ES$^+$) $[M+H]^+$ = 443.

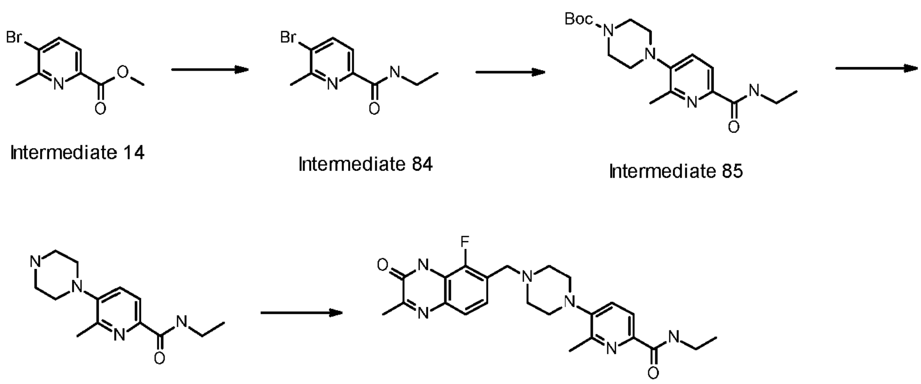

Intermediate 14 Intermediate 84 Intermediate 85

Intermdieate 86 Example 46

***Intermediate 84:** 5-bromo-N-ethyl-6-methyl-pyridine-2-carboxamide*

Ethanamine in $H_2O$ (3 mL, 2.20 mmol) (65 wt%) was added to methyl 5-bromo-6-methyl-pyridine-2-carboxylate (**intermediate 14**) (505 mg, 2.20 mmol). The resulting mixture was stirred at room temperature for 18 h. The solvent was

removed under reduced pressure to afford 5-bromo-N-ethyl-6-methyl-pyridine-2-carboxamide (**intermediate 84**) (0.500 g, 94 %) as a yellow solid. 1H NMR (300 MHz, DMSO-d6) 1.13 (3H, t), 2.66 (3H, s), 3.26 - 3.39 (2H, m), 7.76 (1H, d), 8.18 (1H, d), 8.67 - 8.72 (1H, m); m/z ($ES^+$) $[M+H]^+$ = 243.

***Intermediate* 85:** *tert-butyl 4-[6-(ethylcarbamoyl)-2-methyl-3-pyridyl]piperazine-1-carboxylate*

$Cs_2CO_3$ (1.340 g, 4.11 mmol) was added to 5-bromo-N-ethyl-6-methyl-pyridine-2-carboxamide (**intermediate 84**) (0.5 g, 2.06 mmol), tert-butyl piperazine-1-carboxylate (0.575 g, 3.09 mmol), BINAP (0.128 g, 0.21 mmol) and $Pd(OAc)_2$ (0.046 g, 0.21 mmol) in 1,4-dioxane (5 mL). The resulting mixture was stirred at 100 °C for 18 h under nitrogen. The reaction mixture was diluted with EtOAc (10 mL), and washed sequentially with water (10 mL x 2) followed by brine (10 mL x 1). The organic layer was dried over $Na_2SO_4$, filtered and evaporated to afford crude product. The crude product was purified by flash silica chromatography, elution gradient 0 to 40% EtOAc in petroleum ether. Pure fractions were evaporated to dryness to afford tert-butyl 4-[6-(ethylcarbamoyl)-2-methyl-3-pyridyl]piperazine-1-carboxylate (**intermediate 85**) (0.481 g, 67 %) as a yellow solid. 1H NMR (300 MHz, Chloroform-*d*) 1.26 (3H, t), 1.49 (9H, s), 2.54 (3H, s), 2.85 - 2.98 (4H, m), 3.49 (2H, qd), 3.56 - 3.65 (4H, m), 7.32 (1H, d), 7.91 - 8.01 (2H, m); m/z ($ES^+$) $[M+H]^+$ = 349.

***Intermediate 86:*** *N-ethyl-6-methyl-5-piperazin-1-yl-pyridine-2-carboxamide*

HCl in 1,4-dioxane (4 ml, 16.00 mmol, 4M) was added to tert-butyl 4-[6-(ethylcarbamoyl)-2-methyl-3-pyridyl] piperazine-1-carboxylate (**intermediate 85**) (0.481g, 1.38 mmol) in MeOH (10 mL). The resulting mixture was stirred at room temperature for 2 hours. The solvent was removed under reduced pressure. The reaction mixture was basified with DIPEA (1 mL) in MeOH (3 mL). The solvent was removed under reduced pressure. The crude product was purified by flash C18-flash chromatography, elution gradient 0 to 20% MeCN in water ($NH_4HCO_3$). Pure fractions were evaporated to dryness to afford N-ethyl-6-methyl-5-piperazin-1-yl-pyridine-2-carboxamide (**intermediate 86**) (0.189 g, 55 %) as a yellow oil. 1H NMR (400 MHz, DMSO-d6) 1.12 (3H, t), 2.81 - 2.92 (8H, m), 3.27 - 3.36 (5H, m), 7.46 (1H, d), 7.81 (1H, d), 8.43 (1H, t); m/z ($ES^+$) $[M+H]^+$ = 249.

***Example 46:*** *N-ethyl-5-[4-[(5-fluoro-2-methyl-3-oxo-4H-quinoxalin-6-yl)methyl]piperazin-1-yl]-6-methyl-pyridine-2-carboxamide*

$Ph_3P$ (299 mg, 1.14 mmol) was added to 8-fluoro-7-(hydroxymethyl)-3-methylquinoxalin-2(1H)-one (158 mg, 0.76 mmol) (**intermediate 17**), $CBr_4$ (378 mg, 1.14 mmol) in $CH_2Cl_2$ (3.00 mL). The resulting mixture was stirred at room temperature for 1 h. The solvent was removed under reduced pressure. N-ethyl-6-methyl-5-piperazin-1-yl-pyridine-2-carboxamide (**intermediate 86**) (188 mg, 0.76 mmol) and DIPEA (1.5 mL, 8.59 mmol) were added to the mixture in NMP (3 mL). The resulting mixture was stirred at 80 °C for 2 h. The solvent was removed under reduced pressure. The crude product was purified by flash C18-flash chromatography, elution gradient 0 to 25% MeCN in water ($NH_4HCO_3$). Pure fractions were evaporated to dryness to afford N-ethyl-5-[4-[(5-fluoro-2-methyl-3-oxo-4H-quinoxalin-6-yl)methyl]piperazin-1-yl]-6-methyl-pyridine-2-carboxamide (**example 46**) (7.40 mg, 2 %) as a white solid. 1H NMR (300 MHz, DMSO-d6) 1.10 (3H, t), 2.40 (3H, s), 2.50 (3H, s), 2.54 - 2.64 (4H, m), 2.87 - 2.97 (4H, m), 3.30 (2H, q), 3.70 (2H, s), 7.28 (1H, t), 7.47 (1H, d), 7.52 (1H, d), 7.77 (1H, d), 8.42 (1H, t), 12.44 (1H, s); 19F NMR (282 MHz, DMSO-*d6*) -135.54; m/z ($ES^+$) $[M+H]^+$ = 439.

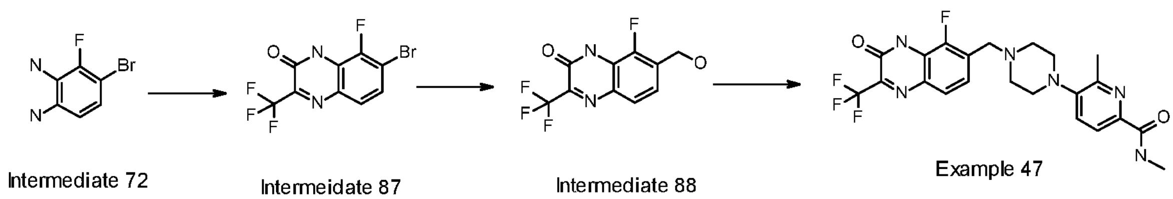

Intermediate 72 Intermeidate 87 Intermediate 88 Example 47

***Intermediate 87:*** *7-bromo-8-fluoro-3-(trifluoromethyl)-1H-quinoxalin-2-one*

Ethyl 3,3,3-trifluoro-2-oxopropanoate (2.30 g, 13.52 mmol) was added to 4-bromo-3-fluoro-benzene-1,2-diamine (**intermediate 72**) (2.20 g, 10.73 mmol) in toluene (10 mL). The resulting mixture was stirred at 100 °C for 18 h. The solvent was removed under reduced pressure. The crude product was purified by flash C18-flash chromatography, elution gradient 3 to 70% MeCN in water (0.1% $NH_4HCO_3$). Pure fractions were evaporated to dryness to afford 7-bromo-8-fluoro-3-(trifluoromethyl)-1H-quinoxalin-2-one (**intermediate 87**) (contaminated by 6-bromo-5-fluoro-3-(trifluoromethyl)-1H-quinoxalin-2-one) (3.40 g, 501 %) as an off-white solid. m/z ($ES^+$) $[M+H]^+$ = 311.

***Intermediate 88***: *8-fluoro-7-(hydroxymethyl)-3-(trifluoromethyl)-1H-quinoxalin-2-one*

CataCxium A Pd G2 (53 mg, 0.08 mmol) was added to 7-bromo-8-fluoro-3-(trifluoromethyl)-1H-quinoxalin-2-one (**intermediate 87**) (contaminated by 6-bromo-5-fluoro-3-(trifluoromethyl)-1H-quinoxalin-2-one) (0.5 g, 0.80 mmol) and (Tributylstannyl)methanol (0.5 mL, 0.80 mmol) in 1,4-dioxane (15 mL). The resulting mixture was stirred at 80 °C for 18 h under nitrogen. The reaction mixture was quenched with saturated KF (1.25 mL). The reaction solution was collected by filtration, washed with dioxane (2.5 mL). The solvent of the combined organic layers was removed under reduced pressure. The crude product was purified by flash C18-flash chromatography, elution gradient 3 to 40% MeCN in water (0.1%, TFA). Pure fractions were evaporated to dryness to afford 8-fluoro-7-(hydroxymethyl)-3-(trifluoromethyl)-1H-quinoxalin-2-one (**intermediate 88**) (contaminated by 5-fluoro-6-(hydroxymethyl)-3-(trifluoromethyl)-1H-quinoxalin-2-one) (0.217 g, 51 %) as an off-white solid. m/z ($ES^+$) $[M+H]^+$ = 263.

***Example 47:*** *5-[4-[[5-fluoro-3-oxo-2-[trifluoromethyl)-4H-quinoxalin-6-yl]methyl]piperazin-1-yl]-N,6-dimethyl-pyridine-2-carboxamide*

$SOCl_2$ (0.5 mL, 6.85 mmol) was added to 8-fluoro-7-(hydroxymethyl)-3-(trifluoromethyl)-1H-quinoxalin-2-one (**intermediate 88**) (contaminated by 5-fluoro-6-(hydroxymethyl)-3-(trifluoromethyl)-1H-quinoxalin-2-one) (160 mg, 0.31 mmol) in $Et_2O$ (5 mL). The resulting mixture was stirred at room temperature for 2 h. The solvent was removed under reduced pressure. DIPEA (4 mL, 22.90 mmol) and N,6-dimethyl-5-piperazin-1-yl-pyridine-2-carboxamide (**intermediate 33**)(134 mg, 0.57 mmol) were added to the mixture in MeCN (10 mL). The resulting mixture was stirred at room temperature for 24 h. The crude product was purified by preparative HPLC (Column: XBridge BEH C18 OBD Prep Column, 5 μm, 19 mm 250 mm; Mobile Phase A: Water (10 mmol/L $NH_4HCO_3$+0.1%$NH_3.H_2O$), Mobile Phase B: ACN; Flow rate: 20 mL/min; Gradient: 24 B to 33 B in 10 min; 254/220 nm; RT1:8.2/9.5) Fractions containing the desired compound were evaporated to dryness to afford 5-[4-[[5-fluoro-3-oxo-2-(trifluoromethyl)-4H-quinoxalin-6-yl]methyl]piperazin-1-yl]-N,6-dimethyl-pyridine-2-carboxamide (**example 47**) (8.8 mg, 6%) as a white solid. 1H NMR (400 MHz, DMSO-*d6*) 2.50 (3H, s), 2.58 - 2.66 (4H, m), 2.79 (3H, d), 2.90 - 2.99 (4H, m), 3.77 (2H, s), 7.41 (1H, t), 7.47 (1H, d), 7.72 (1H, d), 7.78 (1H, d), 8.39 - 8.44 (1H, m),13.21 (1H, brs); 19F NMR (376 MHz, DMSO-*d6*) -68.50, -133.81; m/z ($ES^+$) $[M+H]^+$ = 479.

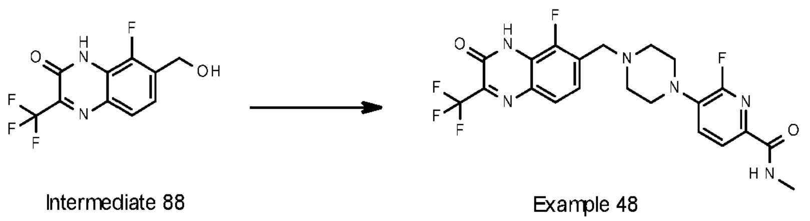

Intermediate 88 Example 48

***Example 48:*** *6-fluoro-5-[4-[[5-fluoro-3-oxo-2-(trifluoromethyl)-4H-quinoxalin-6-yl]methyl]piperazin-1-yl]-N-methyl-pyridine-2-carboxamide*

$SOCl_2$ (0.4 mL, 5.48 mmol) was added to 8-fluoro-7-(hydroxymethyl)-3-(trifluoromethyl)-1H-quinoxalin-2-one (**intermediate 88**) (contaminated by 5-fluoro-6-(hydroxymethyl)-3-(trifluoromethyl)-1H-quinoxalin-2-one) (120 mg, 0.23 mmol) in $Et_2O$ (5 mL). The resulting mixture was stirred at room temperature for 2 h. The solvent was removed under reduced pressure. DIPEA (2 mL, 11.45 mmol) and 6-fluoro-N-methyl-5-piperazin-1-yl-pyridine-2-carboxamide (**intermediate 32**)(156 mg, 0.65 mmol) were added to the mixture in MeCN (10 mL) . The resulting mixture was stirred at room temperature for 24 h. The crude product was purified by preparative HPLC (Column: XBridge BEH C18 OBD Prep Column, 5 μm, 19 mm 250 mm; Mobile Phase A: Water (10mmol/L $NH_4HCO_3$+0.1%$NH_3.H_2O$), Mobile Phase B: ACN; Flow rate: 20 mL/min; Gradient: 25 B to 37 B in 10 min; 254/220 nm; RT1:7.58/8.97). Fractions containing the desired compound were evaporated to dryness to afford 6-fluoro-5-[4-[[5-fluoro-3-oxo-2-(trifluoromethyl)-4H-quinoxalin-6-yl]methyl]piperazin-1-yl]-N-methyl-pyridine-2-carboxamide (**example 48**) (8.9 mg, 8%) as a white solid. 1H NMR (400 MHz, DMSO-*d6*) 2.58 - 2.65 (4H, m), 2.76 (3H, d), 3.14-3.21 (4H, m), 3.75 (2H, s), 7.39 (1H, t), 7.56 (1H, dd), 7.71 (1H, d), 7.84 (1H, dd), 8.37 - 8.43 (1H, m), 13.39 (1H, brs); 19F NMR (376 MHz, DMSO-*d6*) -68.48, -72.59, -133.78; m/z ($ES^+$) $[M+H]^+$ = 483.

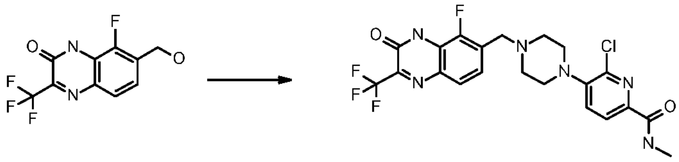

Intermediate 88

Example 49

*__Example 49:__ 6-chloro-5-[4-[[5-fluoro-3-oxo-2-(trifluoromethyl)-4H-quinoxalin-6-yl]methyl]piperazin-1-yl]-N-methyl-pyridine-2-carboxamide*

$SOCl_2$ (0.4 mL, 5.48 mmol) was added to 8-fluoro-7-(hydroxymethyl)-3-(trifluoromethyl)-1H-quinoxalin-2-one (**intermediate 88**) (contaminated by 5-fluoro-6-(hydroxymethyl)-3-(trifluoromethyl)-1H-quinoxalin-2-one) (120 mg, 0.23 mmol) in $Et_2O$ (5 mL). The resulting mixture was stirred at room temperature for 2 h. The solvent was removed under reduced pressure. DIPEA (2 mL, 11.45 mmol) and 6-chloro-N-methyl-5-piperazin-1-yl-pyridine-2-carboxamide (**intermediate 30**)(157 mg, 0.62 mmol) were added to the mixture in MeCN (10 mL). The resulting mixture was stirred at room temperature for 24 h. The crude product was purified by preparative HPLC (Column: XBridge Shield RP18 OBD Column, 19*250mm, 10 um; Mobile Phase A: Water (10MMOL/L $NH_4HCO_3$+0.1%$NH_3$.$H_2O$), Mobile Phase B: ACN; Flow rate: 20 mL/min; Gradient: 45 B to 57 B in 10 min; 254/220 nm). Fractions containing the desired compound were evaporated to dryness to afford 6-chloro-5-[4-[[5-fluoro-3-oxo-2-(trifluoromethyl)-4H-quinoxalin-6-yl]methyl]piperazin-1-yl]-N-methyl-pyridine-2-carboxamide (**example 49**) (18 mg, 16%) as a white solid. 1H NMR (400 MHz, DMSO-*d6*) 2.60 - 2.68 (4H, m), 2.78 (3H, d), 3.07 - 3.16 (4H, m), 3.77 (2H, s), 7.40 (1H, t), 7.66 (1H, d), 7.72 (1H, d), 7.93 (1H, d), 8.40 - 8.43 (1H, m), 13.25 (1H, brs); 19F NMR (376 MHz, DMSO-*d6*) -68.51, -133.73; m/z ($ES^+$) $[M+H]^+$ = 499.

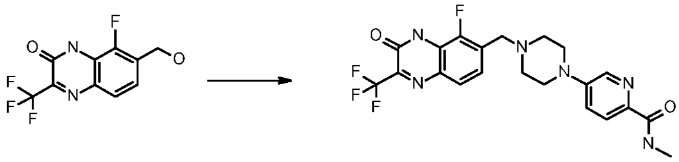

Intermediate 88

Example 50

*__Example 50:__ 5-[4-[[5-fluoro-3-oxo-2-(frifluoromethyl)-4H-quinoxalin-6-yl]methyl]piperazin-1-yl]-N-methyl-pyridine-2-carboxamide*

$SOCl_2$ (0.4 mL, 5.48 mmol) was added to 8-fluoro-7-(hydroxymethyl)-3-(trifluoromethyl)-1H-quinoxalin-2-one (**intermediate 88**) (contaminated by 5-fluoro-6-(hydroxymethyl)-3-(trifluoromethyl)-1H-quinoxalin-2-one) (120 mg, 0.23 mmol) in $Et_2O$ (5 mL). The resulting mixture was stirred at room temperature for 2. The solvent was removed under reduced pressure. DIPEA (2 mL, 11.45 mmol) and N-methyl-5-piperazin-1-yl-pyridine-2-carboxamide (**intermediate 31**) (259 mg, 1.18 mmol) were added to the mixture in MeCN (10 mL). The resulting mixture was stirred at room temperature for 24 h. The crude product was purified by preparative HPLC (Column: XBridge Shield RP18 OBD Column, 19*250mm, 10 um; Mobile Phase A: Water (10mmol/L $NH_4HCO_3$+0.1%$NH_3$.$H_2O$), Mobile Phase B: ACN; Flow rate: 20 mL/min; Gradient: 15 B to 35 B in 10 min; 254/220 nm; RT1:10.18/11.2). Fractions containing the desired compound were evaporated to dryness to afford 5-[4-[[5-fluoro-3-oxo-2-(trifluoromethyl)-4H-quinoxalin-6-yl]methyl]piperazin-1-yl]-N-methyl-pyridine-2-carboxamide (**example 50**) (6 mg, 6%) as a white solid. 1H NMR (400 MHz, DMSO-*d6*) 2.51 - 2.57(4H, m), 2.76 (3H, d), 3.25 - 3.34 (4H, m), 3.72 (2H, s), 7.30 (1H, t), 7.39 (1H, dd), 7.65 (1H, d), 7.83 (1H, d), 8.27 (1H, d), 8..36 - 8.41 (1H, m); 19F NMR (376 MHz, DMSO-*d6*) -68.34, -133.80; m/z ($ES^+$) $[M+H]^+$ = 465.

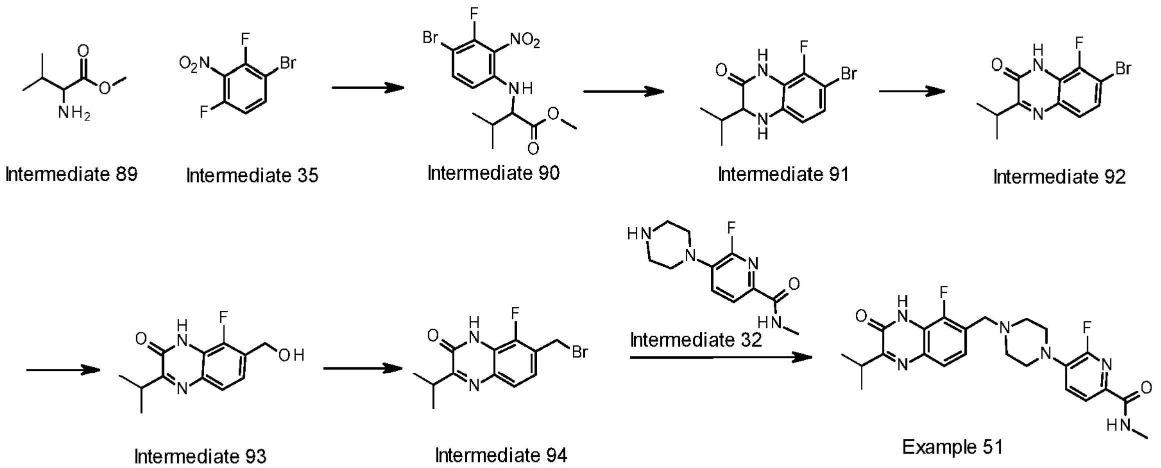

Intermediate 89 Intermediate 35 Intermediate 90 Intermediate 91 Intermediate 92

Intermediate 32

Intermediate 93 Intermediate 94 Example 51

***Intermediate 90:*** *Methyl 2-(4-bromo-3-fluoro-2-nitro-anilino)-3-methyl-butanoate*

DIPEA (2.202 mL, 12.61 mmol) was added slowly to a stirred solution of 1-bromo-2,4-difluoro-3-nitrobenzene (**intermediate 35**) (1 g, 4.20 mmol) and methyl valinate, HCl (**intermediate 89**) (0.704 g, 4.20 mmol) in DMF (6 mL). The resulting solution was stirred at rt for 18 hours (complete conversion to desired product by LCMS). Reaction mixture was concentrated, diluted with water and extracted with ethyl acetate, organic layer was dried over sodium sulphate, filtered and concentrated under vacuum. The crude product was purified via normal phase chromatography with hexane: Ethyl acetate to yield methyl 2-(4-bromo-3-fluoro-2-nitro-anilino)-3-methyl-butanoate (0.763 g, 52.0 %) ***(intermediate 90)*** as a bright orange solid. 1H NMR (500 MHz, DICHLOROMETHANE-d2) 1.00 - 1.14 (6H, m), 2.20 - 2.35 (1H, m), 3.78 (3H, s), 4.06 (1H, dd), 6.52 (1H, br d), 7.39 (1H, br d), 7.52 (1H, dd); 19F NMR (471 MHz, DICHLOROMETHANE-d2) -109.33 (1F, s); m/z ($ES^+$) $[M+H]^+$ = 349.

***Intermediate 91:*** *7-bromo-8-fluoro-3-isopropyl-3,4-dihydro-1H-quinoxalin-2-one*

Zinc powder (1.143 g, 17.48 mmol) was added to a mixture of methyl 2-(4-bromo-3-fluoro-2-nitro-anilino)-3-methyl-butanoate (0.763 g, 2.19 mmol) (***intermediate 90***) and ammonium chloride (0.935 g, 17.48 mmol) in MeOH (12 mL) and water (0.3 mL) at 0 °C portion-wise (exothermic reaction), the mixture was stirred at rt for 2 h (No SM remaining, complete disappearance of orange coloration is indicative of reaction completion). Zn was filtered off, the solid cake was washed with 20% MeOH in DCM and the filtrate was concentrated under vacuum. Water was added to the above crude product and the product was extracted into the ethyl acetate layer. The organic layer was dried and concentrated under vacuum to furnish a colorless oil. The crude product was slurried in 1:1 ethylacetate:methanol, 0.5 mL 4N HCl in dioxane was added and the reaction was stirred for 1 h (No uncyclized product remaining). The reaction mixture was concentrated to yield 7-bromo-8-fluoro-3-isopropyl-3,4-dihydro-1H-quinoxalin-2-one (***intermediate 91***). The crude product was subjected to reagents for the next step without any further purification assuming the yield of this reaction to be 100%. m/z ($ES^+$) $[M+H]^+$ = 287.

***Intermediate 92:*** *7-bromo-8-fluoro-3-isopropyl-1H-quinoxalin-2-one*

4,5-dichloro-3,6-dioxocyclohexa-1,4-diene-1,2-dicarbonitrile (595 mg, 2.62 mmol) was added in one portion to a stirred solution of 7-bromo-8-fluoro-3-isopropyl-3,4-dihydroquinoxalin-2(1H)-one (627 mg, 2.18 mmol) (***intermediate 91***) in DCM (20 mL). The resulting slurry was stirred at rt for 2 hours (complete conversion to desired product by LCMS). The reaction mixture was concentrated under vacuum and quenched with saturated aq. sodium bicarbonate solution. The above slurry was stirred at rt for overnight and the solid was filtered off. The filtered solid was washed thoroughly with water followed by diethyl ether and dried to give 7-bromo-8-fluoro-3-isopropyl-1H-quinoxalin-2-one (0.425 g, 68.3%) (***intermediate 92***) as an off-white solid. 1H NMR (500 MHz, DMSO-d6) 1.22 (6H, d), 3.36 - 3.52 (1H, m), 7.45 - 7.58 (2H, m), 12.62 (1H, br s); 19F NMR (471 MHz, DMSO-d6) -124.16 (1F, s).; m/z ($ES^+$) $[M+H]^+$ = 285.

***Intermediate* 93:** *8-fluoro-7-(hydroxymethyl)-3-isopropyl-1H-quinoxalin-2-one*

Xphos Pd G2 (103 mg, 0.13 mmol) was added to a stirred degassed solution of 7-bromo-8-fluoro-3-isopropylquinoxalin-2(1H)-one (375 mg, 1.32 mmol) (***intermediate 92***) and (tributylstannyl)methanol (507 mg, 1.58 mmol) in 1,4-dioxane (6.58 mL). The resulting solution was stirred at 80 °C for 16 hours. The reaction mixture was concentrated

under vacuum, and purified via normal phase chromatography using 0-10% MeOH in DCM to yield 8-fluoro-7-(hydroxymethyl)-3-isopropyl-1H-quinoxalin-2-one (0.255 g, 82 %) (***intermediate 93***) as a white solid. 1H NMR (500 MHz, DMSO-d6) 1.22 (6H, d), 3.39 - 3.52 (1H, m), 4.64 (2H, d), 5.41 (1H, t), 7.33 (1H, s), 7.55 (1H, d), 12.42 (1H, br s).; 19F NMR (471 MHz, DMSO-d6) -137.71 (1F, s).; m/z ($ES^+$) $[M+H]^+$ = 237.

***Intermediate 94:*** *7-(bromomethyl)-8-fluoro-3-isopropyl-1H-quinoxalin-2-one*

Triethylphosphane (0.477 ml, 3.23 mmol) was added dropwise to a stirred solution of 8-fluoro-7-(hydroxymethyl)-3-isopropylquinoxalin-2(1H)-one (0.2541 g, 1.08 mmol) (***intermediate 93***) and CBr4 (1.177 g, 3.55 mmol) in DCM (8.49 mL) at 0 °C over a period of 5 minutes under nitrogen. The reaction mixture was stirred at rt for 1 h, DCM was removed under vacuum and the resulting solid was slurried in diethyl ether. The white ppt was filtered under vacuum, washed with water followed by ether. The solid was dried under vacuum for overnight (no heat) to give 7-(bromomethyl)-8-fluoro-3-isopropyl-1H-quinoxalin-2-one (0.313 g, 97%) (***intermediate 94***) as light brown solid. m/z ($ES^+$) $[M+H]^+$ = 299.

***Example 51:*** *6-fluoro-5-[4-[(5-fluoro-2-isopropyl-3-oxo-4H-quinoxalin-6-yl)methyl]piperazin-1-yl]-N-methyl-pyridine-2-carboxamide*

To 7-(bromomethyl)-8-fluoro-3-isopropylquinoxalin-2(1H)-one (100 mg, 0.33 mmol) (***intermediate 94***) was added 6-fluoro-N-methyl-5-piperazin-1-yl-pyridine-2-carboxamide, 2HCl (104 mg, 0.33 mmol) (***intermediate 32***), acetonitrile (5 mL) and N-ethyl-N-isopropylpropan-2-amine (291 μL, 1.67 mmol) and heated to 70 °C. LCMS indicated complete disappearance of SM and formation of desired product after 1 h. The reaction mixture was cooled, concentrated, quenched with aq NaHCO3 solution (1 mL) and stirred for 1 h at rt. Water (3 mL) was added to the above mixture and stirred for 10 mins. The precipitate was filtered and washed with copious amounts of water (50 mL). The solid was purified via normal phase chromatography using 0-10% MeOH in DCM to yield 6-fluoro-5-[4-[(5-fluoro-2-isopropyl-3-oxo-4H-quinoxalin-6-yl)methyl]piperazin-1-yl]-N-methyl-pyridine-2-carboxamide (0.050 g, 32.8 %) (***Example 51***) as a white solid. 1H NMR (500 MHz, DMSO-d6) 1.22 (6H, d), 2.53 - 2.65 (4H, m), 2.77 (3H, d), 3.12 - 3.24 (4H, m), 3.36 - 3.52 (1H, m), 3.71 (2H, s), 7.30 (1H, t), 7.52 - 7.59 (2H, m), 7.84 (1H, d), 8.36 - 8.41 (1H, m), 12.46 (1H, br s).; 19F NMR (471 MHz, DMSO-d6) -135.53 (1F, s), -72.59 (1F, s).; m/z ($ES^+$) $[M+H]^+$ = 457.

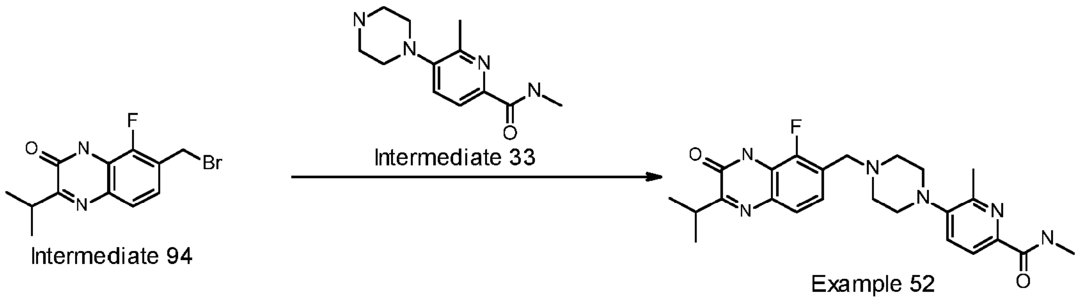


Intermediate 94

Example 52

***Example 52:*** *5-[4-[(5-fluoro-2-isopropyl-3-oxo-4H-quinoxalin-6-yl)methyl]piperazin-1-yl]-N,6-dimethylpyridine-2-carboxamide*

To 7-(bromomethyl)-8-fluoro-3-isopropylquinoxalin-2(1H)-one (109 mg, 0.36 mmol) (***intermediate 94***), was added N,6-dimethyl-5-(piperazin-1-yl)picolinamide, 2HCl (112 mg, 0.36 mmol) (***intermediate 33***), acetonitrile (5 mL) and N-ethyl-N-isopropylpropan-2-amine (317 μl, 1.82 mmol) and heated to 70 C. LCMS indicated complete disappearance of SM and formation of desired product after 1 h. The reaction mixture was cooled, concentrated, quenched with aq NaHCO3 solution (1 mL) and stirred for 1 h at rt. Water (3 mL) was added to the above mixture and stirred for 10 mins. The precipitate was filtered and washed with copious amounts of water (50 mL). The solid was purified via normal phase chromatography using 0-10% MeOH in DCM to yield 5-[4-[(5-fluoro-2-isopropyl-3-oxo-4H-quinoxalin-6-yl)methyl]piperazin-1-yl]-N,6-dimethyl-pyridine-2-carboxamide (0.057 g, 34.6 %) as a white solid (***Example 52***). 1H NMR (500 MHz, DMSO-d6) 1.22 (6H, d), 2.46 - 2.49 (3H, m), 2.52 - 2.68 (4H, m), 2.80 (3H, d), 2.94 (4H, br s), 3.36 - 3.52 (1H, m), 3.73 (2H, s), 7.30 (1H, t), 7.47 (1H, d), 7.56 (1H, d), 7.79 (1H, d), 8.37 - 8.44 (1H, m), 12.46 (1H, s).; 19F NMR (471 MHz, DMSO-d6) -135.55 (1F, s).; m/z ($ES^+$) $[M+H]^+$ = 453.

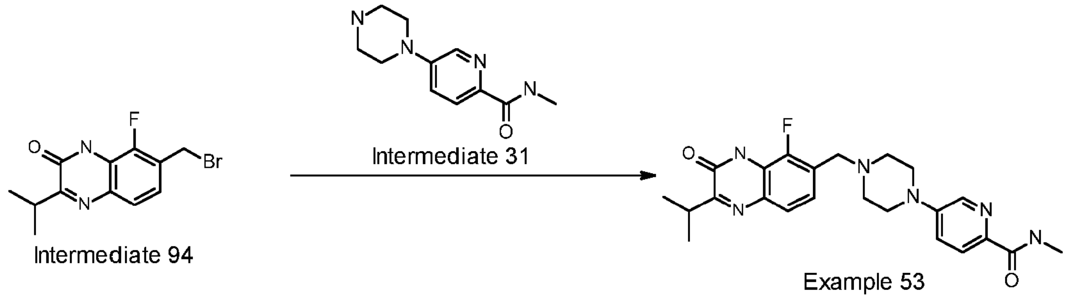


Intermediate 94

Example 53

***Example* 53**: *5-[4-[(5-fluoro-2-isopropyl-3-oxo-4H-quinoxalin-6-yl)methyl]piperazin-1-yl]-N-methylpyridine-2-carboxamide*

To 7-(bromomethyl)-8-fluoro-3-isopropylquinoxalin-2(1H)-one (100 mg, 0.33 mmol) (***intermediate 94***), was added N-methyl-5-(piperazin-1-yl)picolinamide, 2HCl (98 mg, 0.33 mmol) (***intermediate 31***), acetonitrile (5 mL) and N-ethyl-N-isopropylpropan-2-amine (291 μl, 1.67 mmol) and heated to 70 °C. LCMS indicated complete disappearance of SM and formation of desired product after 1 h. The reaction mixture was cooled, concentrated, quenched with aq NaHCO3 solution (1 mL) and stirred for 1 h at rt. Water (3 mL) was added to the above mixture and stirred for 10 mins. The precipitate was filtered and washed with copious amounts of water (50 mL). The solid was purified via normal phase chromatography using 0-10% MeOH in DCM to yield 5-[4-[(5-fluoro-2-isopropyl-3-oxo-4H-quinoxalin-6-yl)methyl]piperazin-1-yl]-N-methyl-pyridine-2-carboxamide (0.052 g, 35.5 %) (***Example* 53**) as a white solid. 1H NMR (500 MHz, DMSO-d6) 1.22 (6H, d), 2.52 - 2.61 (4H, m), 2.78 (3H, d), 3.26 - 3.30 (4H, m), 3.36 - 3.52 (1H, m), 3.70 (2H, s), 7.31 (1H, t), 7.38 (1H, dd), 7.56 (1H, d), 7.82 (1H, d), 8.26 (1H, d), 8.38 (1H, br d), 12.45 (1H, br s).; 19F NMR (471 MHz, DMSO-d6) -135.54 (1F, s).; m/z ($ES^+$) $[M+H]^+$ = 439.

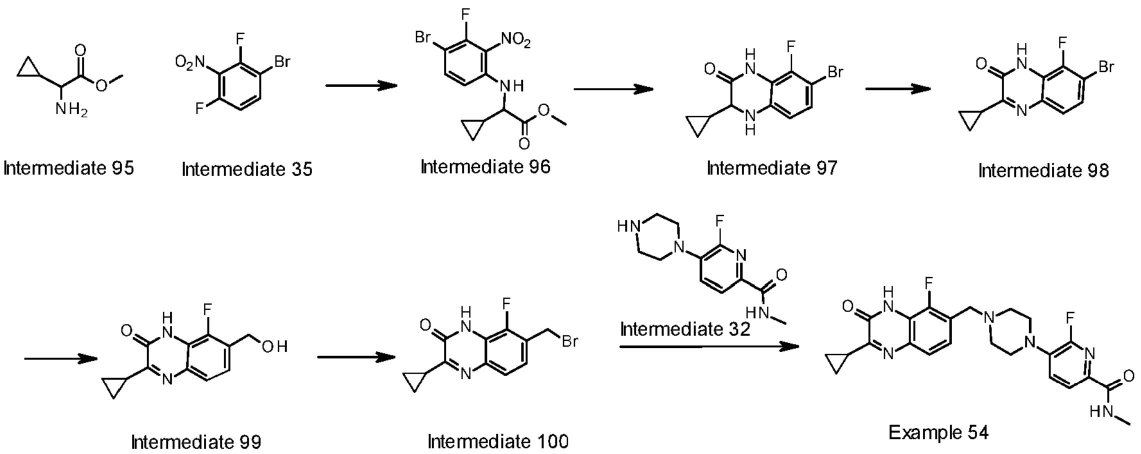


Intermediate 95 Intermediate 35 Intermediate 96 Intermediate 97 Intermediate 98

Intermediate 99 Intermediate 100 Example 54

***Intermediate 96:*** *methyl 2-(4-bromo-3-fluoro-2-nitro-anilino)-2-cyclopropyl-acetate*

DIPEA (2.202 mL, 12.61 mmol) was added slowly to a stirred solution of 1-bromo-2,4-difluoro-3-nitrobenzene (**intermediate 35**) (1 g, 4.20 mmol) and methyl 2-amino-2-cyclopropylacetate, HCl (**intermediate 95**) (0.696 g, 4.20 mmol) in DMF (6 mL). The resulting solution was stirred at rt for 18 hours (complete conversion to desired product by LCMS). The reaction mixture was concentrated, diluted with water and extracted with ethyl acetate, organic layer was dried over sodium sulphate, filtered and concentrated under vacuum. The crude product was purified via normal phase chromatography using hexane and ethyl acetate to yield methyl 2-(4-bromo-3-fluoro-2-nitro-anilino)-2-cyclopropyl-acetate (0.635 g, 43.5 %) (***intermediate 96)*** as a bright orange solid. 1H NMR (500 MHz, DICHLOROMETHANE-d2) 0.39 - 0.49 (1H, m), 0.54 (1H, td), 0.64 - 0.75 (2H, m), 1.25 - 1.39 (1H, m), 3.74 - 3.83 (4H, m), 6.45 (1H, dd), 7.34 (1H, br d), 7.52 (1H, dd). 19F NMR (471 MHz, DICHLOROMETHANE-d2) -109.53 (1F, s).; m/z ($ES^+$) $[M+H]^+$ = 347.

***Intermediate 97:*** *7-bromo-3-cyclopropyl-8-fluoro-3,4-dihydro-1H-quinoxalin-2-one*

Zinc powder (957 mg, 14.63 mmol) was added to a mixture of methyl 2-((4-bromo-3-fluoro-2-nitrophenyl) amino)-2-cyclopropylacetate (635 mg, 1.83 mmol) (***intermediate 96***) and ammonium chloride (783 mg, 14.63 mmol) in

MeOH (12 mL) and water (0.3 mL) at 0 °C portion-wise (exothermic reaction), the mixture was stirred at rt for 2 h (No SM remaining, Complete disappearance of orange coloration is indicative of reaction completion). Zn was filtered off and the solid cake was washed with 20% MeOH in DCM. The filtrate was concentrated, the crude material showed mostly uncyclized product. Water was added to the above crude product and the product was extracted into the ethyl acetate layer. The organic layer was dried and concentrated under vacuum to furnish an oil. This material was slurried in 1:1 ethylacetate:methanol, 0.5 mL 4 N HCl in dioxane was added and the reaction mixture was stirred for 1 h (No uncyclized product remaining). The reaction mixture was concentrated to yield 7-bromo-3-cyclopropyl-8-fluoro-3,4-dihydro-1H-quinoxalin-2-one (***intermediate 97***) as a grey solid. The crude product was subjected to reagents for the next step without any further purification assuming the yield of this reaction to be 100%. m/z ($ES^+$) $[M+H]^+$ = 285.

***Intermediate* 98:** *7-bromo-3-cyclopropyl-8-fluoro-1H-quinoxalin-2-one*

4,5-dichloro-3,6-dioxocyclohexa-1,4-diene-1,2-dicarbonitrile (499 mg, 2.20 mmol) was added in one portion to a stirred solution of 7-bromo-3-cyclopropyl-8-fluoro-3,4-dihydroquinoxalin-2(1H)-one (522 mg, 1.83 mmol) (***intermediate 97***) in DCM (20 mL). The resulting slurry was stirred at rt for 2 hours (complete conversion to desired product by LCMS). The reaction mixture was concentrated under vacuum and quenched with saturated aq. sodium bicarbonate solution. The above slurry was stirred at rt for overnight and the solid was filtered off. The solid was washed thoroughly with water followed by diethyl ether and dried to give 7-bromo-3-cyclopropyl-8-fluoro-1H-quinoxalin-2-one (0.382 g, 73.7 %) (***intermediate 98***) as an off-white solid. m/z ($ES^+$) $[M+H]^+$ = 283.

***Intermediate 99*:** *3-cyclopropyl-8-fluoro-7-(hydroxymethyl)-1H-quinoxalin-2-one*

Xphos Pd G2 (92 mg, 0.12 mmol) was added to a stirred degassed solution of 7-bromo-3-cyclopropyl-8-fluoroquinoxalin-2(1H)-one (332 mg, 1.17 mmol) (***intermediate 98***) and (tributylstannyl)methanol (452 mg, 1.41 mmol) in 1,4-dioxane (5.86 mL) and the resulting solution was stirred at 80 °C for 16 hours. The reaction mixture was concentrated under vacuum, and purified via normal phase chromatography using 0-10% MeOH in DCM to yield 3-cyclopropyl-8-fluoro-7-(hydroxymethyl)-1H-quinoxalin-2-one (0.224 g, 82 %) (***intermediate 99***) as a white solid. 1H NMR (500 MHz, DMSO-d6) 1.02 - 1.14 (4H, m), 2.52 - 2.73 (1H, m), 4.62 (2H, d), 5.38 (1H, t), 7.29 (1H, t), 7.43 (1H, d), 12.43 (1H, br s).; 19F NMR (471 MHz, DMSO-d6) -137.67 (1F, s).; m/z ($ES^+$) $[M+H]^+$ = 235.

***Intermediate 100:*** *7-(bromomethyl)-3-cyclopropyl-8-fluoro-1H-quinoxalin-2-one*

Triethylphosphane (0.422 ml, 2.86 mmol) was added dropwise to a mixture of 3-cyclopropyl-8-fluoro-7-(hydroxymethyl)quinoxalin-2(1H)-one (0.223 g, 0.95 mmol) (***intermediate 99***) and CBr4 (1.043 g, 3.14 mmol) in DCM (7.52 mL) at 0°C over a period of 5 minutes under nitrogen. Reaction was stirred at rt for 1 h. DCM was removed under vacuum and the resulting solid was slurried in diethyl ether. The light greenish white ppt was filtered under vacuum, washed with water followed by ether. The solid was dried under vacuum for overnight (no heat) to give 7-(bromomethyl)-3-cyclopropyl-8-fluoro-1H-quinoxalin-2-one (0.193 g, 68.2 %) (***intermediate 100***) as a light green solid. 1H NMR (500 MHz, DMSO-d6) 1.03 - 1.17 (4H, m), 2.63 - 2.76 (1H, m), 4.79 (2H, s), 7.33 (1H, t), 7.43 (1H, d), 12.55 (1H, br s).; 19F NMR (471 MHz, DMSO-d6) -133.65 (1F, s).; m/z ($ES^+$) $[M+H]^+$ = 297.

***Example 54:*** *5-[4-[(2-cyclopropyl-5-fluoro-3-oxo-4H-quinoxalin-6-yl)methyl]piperazin-1-yl]-6-fluoro-N-methyl-pyridine-2-carboxamide*

To 7-(bromomethyl)-3-cyclopropyl-8-fluoroquinoxalin-2(1H)-one (75 mg, 0.25 mmol) (***intermediate 100***), was added 6-fluoro-N-methyl-5-(piperazin-1-yl)picolinamide, 2HCl (79 mg, 0.25 mmol) (***intermediate 32***), acetonitrile (5 mL) and N-ethyl-N-isopropylpropan-2-amine (220 μl, 1.26 mmol) and heated to 70 C. LCMS indicated complete disappearance of SM and formation of desired product after 1 h. The reaction mixture was cooled, concentrated, quenched with aq NaHCO3 solution (1 mL) and stirred for 1 h at rt. Water (3 mL) was added to the above mixture and stirred for 10 mins. The precipitate was filtered and washed with copious amounts of water (50 mL). The solid was purified via normal phase chromatography using 0-10% MeOH in DCM to yield 5-[4-[(2-cyclopropyl-5-fluoro-3-oxo-4H-quinoxalin-6-yl)methyl]piperazin-1-yl]-6-fluoro-N-methyl-pyridine-2-carboxamide (0.048 g, 41.8 %) (***Example 54***) as a white solid. 1H NMR (500 MHz, DMSO-d6) 1.04 - 1.13 (4H, m), 2.52 - 2.63 (4H, m), 2.71 (1H, s), 2.77 (3H, d), 3.12 - 3.21 (4H, m), 3.69 (2H, s), 7.26 (1H, t), 7.43 (1H, d), 7.55 (1H, dd), 7.84 (1H, d), 8.39 (1H, br d), 12.46 (1H, br s).; 19F NMR (471 MHz, DMSO-d6) -135.52 (1F, s), -72.58 (1F, s).; m/z ($ES^+$) $[M+H]^+$ = 455.

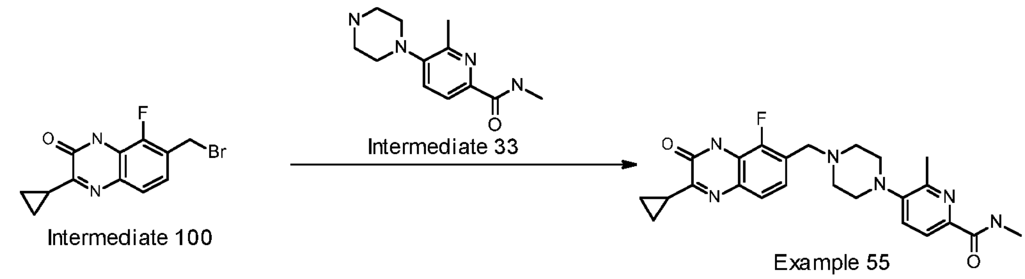

***Example 55:*** *5-[4-[(2-cyclopropyl-5-fluoro-3-oxo-4H-quinoxalin-6-yl)methyl]piperazin-1-yl]-N,6-dimethyl-pyridine-2-carboxamide*

To 7-(bromomethyl)-3-cyclopropyl-8-fluoroquinoxalin-2(1H)-one (75 mg, 0.25 mmol) (***intermediate 100***), was added N,6-dimethyl-5-(piperazin-1-yl)picolinamide, 2HCl (78 mg, 0.25 mmol) (***intermediate 33***), acetonitrile (5 mL) and N-ethyl-N-isopropylpropan-2-amine (220 μL, 1.26 mmol) and heated to 70 °C. LCMS indicated complete conversion to the desired product after 1 h. The reaction mixture was cooled, concentrated, quenched with aq NaHCO3 solution (1 mL) and stirred for 1 h at rt. Water (3 mL) was added to the above mixture and stirred for 10 mins. The precipitate was filtered and washed with copious amounts of water (50 mL). The solid was purified via normal phase chromatography using 0-10% MeOH in DCM to yield 5-[4-[(2-cyclopropyl-5-fluoro-3-oxo-4H-quinoxalin-6-yl)methyl]piperazin-1-yl]-N,6-dimethyl-pyridine-2-carboxamide (0.049 g, 43.1 *%)* **(*Example 55***) as a white solid. 1H NMR (500 MHz, DMSO-d6) 1.03 - 1.15 (4H, m), 2.46 - 2.49 (3H, m), 2.52 - 2.65 (4H, m), 2.65 - 2.75 (1H, m), 2.80 (3H, d), 2.94 (4H, br s), 3.71 (2H, s), 7.26 (1H, t), 7.40 - 7.50 (2H, m), 7.79 (1H, d), 8.37 - 8.44 (1H, m), 12.46 (1H, s).; 19F NMR (471 MHz, DMSO-d6) -135.54 (1F, s).; m/z ($ES^+$) $[M+H]^+$ = 451.

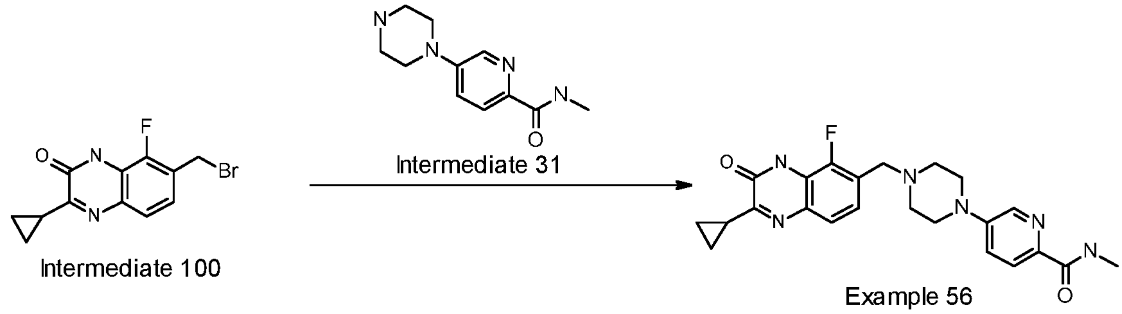

***Example* 56:** *5-[4-[(2-cyclopropyl-5-fluoro-3-oxo-4H-quinoxalin-6-yl)methyl]piperazin-1-yl]-N-methylpyridine-2-carboxamide*

To 7-(bromomethyl)-3-cyclopropyl-8-fluoroquinoxalin-2(1H)-one (43 mg, 0.14 mmol) (***intermediate 100***), was added N-methyl-5-(piperazin-1-yl)picolinamide, 2HCl (42.4 mg, 0.14 mmol) (***intermediate 31***), acetonitrile (5 mL) and N-ethyl-N-isopropylpropan-2-amine (126 μl, 0.72 mmol) and heated to 70 °C. LCMS indicated complete disappearance of SM and formation of desired product after 1 h. The reaction mixture was cooled, concentrated, quenched with aq NaHCO3 solution (1 mL) and stirred for 1 h at rt. Water (3 mL) was added to the above mixture and stirred for 10 mins. The precipitate was filtered and washed with copious amounts of water (25 mL). The solid was purified via normal phase chromatography using 0-10% MeOH in DCM to yield 5-[4-[(2-cyclopropyl-5-fluoro-3-oxo-4H-quinoxalin-6-yl)methyl]piperazin-1-yl]-N-methyl-pyridine-2-carboxamide (0.020 g, 31.7 %) (***Example 56***) as a white solid. 1H NMR (500 MHz, DMSO-d6) 1.03 - 1.16 (4H, m), 2.53 - 2.60 (4H, m), 2.65 - 2.80 (5H, m), 3.68 (2H, s), 7.25 (1H, br t), 7.38 (1H, dd), 7.42 (1H, d), 7.82 (1H, d), 8.25 (1H, d), 8.35 - 8.40 (1H, m), 12.38 - 12.51 (1H, m) (missing 3H likely overlaps with DMSO peak); 19F NMR (471 MHz, DMSO-d6) -135.52 (1F, s).; m/z ($ES^+$) $[M+H]^+$ = 437

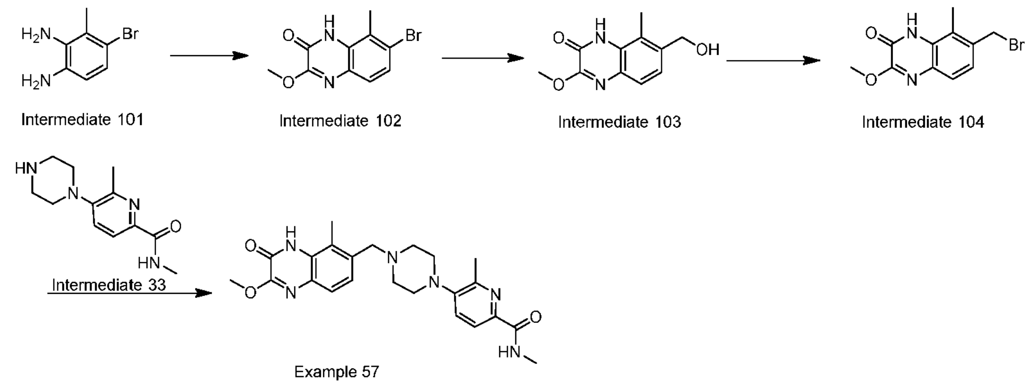


Intermediate 101 Intermediate 102 Intermediate 103 Intermediate 104

Example 57

***Intermediate 102:*** *7-bromo-3-methoxy-8-methyl-1H-quinoxalin-2-one*

A mixture of 4-bromo-3-methylbenzene-1,2-diamine (1.75 g, 8.70 mmol) **(Intermediate 101),** methyl 2,2,2-trimethoxyacetate (2.86 g, 17.41 mmol) and ytterbium(III) trifluoromethanesulfonate (0.540 g, 0.87 mmol) in toluene (10 mL) in a sealed tube was degassed, back filled with $N_2$, stirred at 100 °C for overnight gave a brown suspension, LCMS indicated the formation of desired product, the mixture was cooled to rt, the solid was collected by filtration, washed with methanol, dried to yield 7-bromo-3-methoxy-8-methyl-1H-quinoxalin-2-one (1.2 g, 51.2 %) **(Intermediate 102)** as a yellow solid (contaminated by about 8% of its regio-isomer 6-bromo-3-methoxy-5-methylquinoxalin-2(1H)-one). 1H NMR (500 MHz, DMSO-d6) 2.50 (3H, br s), 3.97 (3H, s), 7.32 (1H, d), 7.45 (1H, d), 11.79 (1H, br s); (m/z) ($ES^+$) $[M+H]^+$ = 269.

***Intermediate 103:*** *7-(hydroxymethyl)-3-methoxy-8-methyl-1H-quinoxalin-2-one*

A mixture of (tributylstannyl)methanol (1.844 g, 5.74 mmol), 7-bromo-3-methoxy-8-methyl-1H-quinoxalin-2-one (1.03 g, 3.83 mmol) **(Intermediate 102)** and Xphos Pd G2 (0.452 g, 0.57 mmol) in 1,4-dioxane (40 mL) was stirred at 80 °C for overnight under $N_2$ gave a dark mixture, LCMS indicated near full conversion. The solvent was removed under reduced pressure, the residue was purified on silica gel column (eluted with 0 to 20% methanol in DCM), the fractions were concentrated to a yellow solid, the solid was checked by LCMS which indicated it was not very pure, the product was then slurried in 20 mL of methanol, the solid was collected by filtration, dried to yield the product with 55% purity as a yellow solid (contaminated by 30% starting material and 9.5% de-brominated side product).

The solid obtained above was charged into a dry flask with 1,4-dioxane (40 mL), to the flask was added 900 mg of (tributylstanny)methanol and 300 mg of xphos Pd G2, the mixture was degassed, then stirred at 80 °C for overnight under $N_2$. The solvent was removed under reduced pressure, the mixture was purified on silica gel column (eluted with 0 to 20% methanol in DCM) to yield 7-(hydroxymethyl)-3-methoxy-8-methyl-1H-quinoxalin-2-one (800mg, 95%) (**Intermediate 103)** as a yellow solid with 80% purity by LCMS. 1H NMR (500 MHz, DMSO-d6) 2.32 (3H, s), 3.91 - 4.01 (3H, m), 4.56 (2H, d), 5.15 (1H, t), 7.27 (1H, d), 7.37 (1H, d), 11.57 (1H, br s); (m/z) ($ES^+$) $[M+H]^+$ = 221.

***Intermediate 104:*** *7-(bromomethyl)-3-methoxy-8-methyl-1H-quinoxalin-2-one*

Triethylphosphane (294 μl, 2.04 mmol) was added dropwise to a suspension of 7-(hydroxymethyl)-3-methoxy-8-methyl-1H-quinoxalin-2-one (300 mg, 1.36 mmol) **(Intermediate 103)** and 1,1,2,2-tetrabromo-1,2-dichloroethane (875 mg, 2.11 mmol) in $CH_2Cl_2$ (20 mL) at 0 °C under $N_2$, the resulting mixture was then stirred at rt for 3.5 h, the solvent was removed under reduced pressure, the residue was suspended in ether (10 mL), filtered, the solid was washed with ether (10 mL x 2), the solid was then suspended in water (20 mL), filtered, washed with water (5 mL x 3), dried to yield 7-(bromomethyl)-3-methoxy-8-methyl-1H-quinoxalin-2-one (0.250 g, 64.8 %) (**Intermediate 104)** as a light yellow solid. (m/z) ($ES^+$) $[M+H]^+$ = 285.

***Example 57:*** *5-[4-[(2-methoxy-5-methyl-3-oxo-4H-quinoxalin-6-yl)methyl]piperazin-1-yl]-N,6-dimethylpyridine-2-carboxamide*

To a suspension of N,6-dimethyl-5-(piperazin-1-yl)picolinamide, 2HCl (83 mg, 0.27 mmol) **(Intermediate 33)** and 7-(bromomethyl)-3-methoxy-8-methyl-1H-quinoxalin-2-one (85 mg, 0.27 mmol) **(Intermediate 104)** in acetonitrile (6 mL) was added DIPEA (236 μL, 1.35 mmol), the resulting mixture was stirred at 70 °C for 2 h gave a clear solution, the mixture was cooled to rt gave a suspension, the solid was collected by filtration, washed with water, acetonitrile, dried to yield 5-[4-[(2-methoxy-5-methyl-3-oxo-4H-quinoxalin-6-yl)methyl]piperazin-1-yl]-N,6-dimethyl-pyridine-2-carboxamide

(0.064 g, 54.3 %) **(Example 57)** as a white solid. 1H NMR (500 MHz, DMSO-d6) 2.43 (3H, s), 2.49 (3H, s), 2.57 (4H, br s), 2.80 (3H, d), 2.91 (4H, br s), 3.60 (2H, s), 3.95 (3H, s), 7.18 (1H, d), 7.35 (1H, d), 7.46 (1H, d), 7.78 (1H, d), 8.40 (1H, q), 11.58 (1H, br s); (m/z) (ES$^+$) [M+H]$^+$ = 437

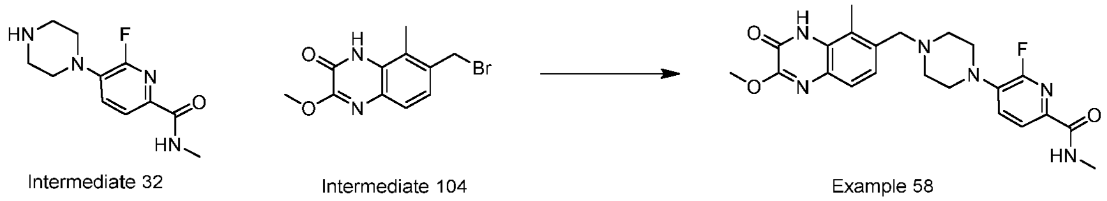

Intermediate 32 Intermediate 104 Example 58

***Example 58**: 6-fluoro-5-[4-[(2-methoxy-5-methyl-3-oxo-4H-quinoxalin-6-yl)methyl]piperazin-1-yl]-N-methyl-pyridine-2-carboxamide*

To a suspension of 6-fluoro-N-methyl-5-(piperazin-1-yl)picolinamide, 2HCl (84 mg, 0.27 mmol) **(Intermediate 32)** and 7-(bromomethyl)-3-methoxy-8-methyl-1H-quinoxalin-2-one (85 mg, 0.27 mmol) **(Intermediate 104)** in acetonitrile (6 mL) was added DIPEA (236 µL, 1.35 mmol), the resulting mixture was stirred at 70 °C for 2 h gave a suspension, the mixture was cooled to rt, the solid was collected by filtration, washed with water, acetonitrile, dried, the solid was suspended in acetonitrile, filtered and dried to yield 6-fluoro-5-[4-[(2-methoxy-5-methyl-3-oxo-4H-quinoxalin-6-yl)methyl] piperazin-1-yl]-N-methyl-pyridine-2-carboxamide (0.073 g, 61.3 %) **(Example 58)** as a beige colored solid. 1H NMR (500 MHz, DMSO-d6) 2.42 (3H, s), 2.55 (4H, br s), 2.76 (3H, d), 3.14 (4H, br s), 3.58 (2H, s), 3.95 (3H, s), 7.17 (1H, br d), 7.35 (1H, br d), 7.50 - 7.63 (1H, m), 7.83 (1H, br d), 8.38 (1H, br d), 11.58 (1H, s); (m/z) (ES$^+$) [M+H]$^+$ = 442.

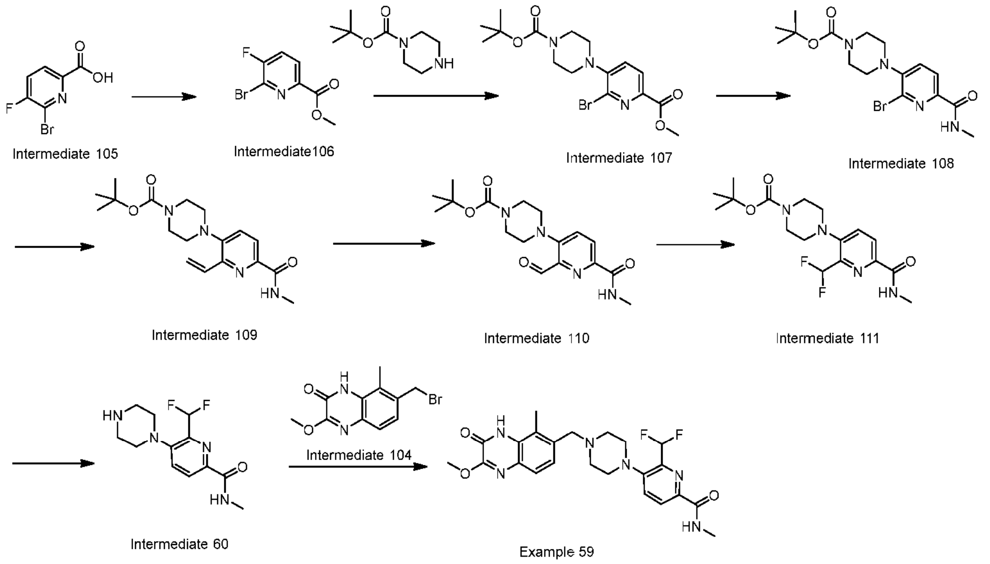


Intermediate 105 Intermediate106 Intermediate 107 Intermediate 108

Intermediate 109 Intermediate 110 Intermediate 111

Intermediate 60 Example 59

***Intermediate 106:** methyl 6-bromo-5-fluoro-pyridine-2-carboxylate*

Sulfuric acid (1.5 mL, 28.14 mmol) was added to a mixture of 6-bromo-5-fluoropicolinic acid (500 mg, 2.27 mmol) **(Intermediate 105)** in MeOH (8 mL) slowly. The mixture was continued to stir at rt for 3 h gave a white suspension. LCMS indicated full conversion, the mixture was poured into sat. aq $NaHCO_3$ solution, extracted with DCM (40 mL x 2), the organic layers were dried (anhydrous $Na_2SO_4$), filtered and concentrated to yield methyl 6-bromo-5-fluoro-pyridine-2-carboxylate (532 mg, 100 %) **(Intermediate 106)** as a white solid which was used for next step without further purification. 1H NMR (500 MHz, CHLOROFORM-d) 4.01 (3H, s), 7.55 (1H, t), 8.15 (1H, dd); (m/z) (ES$^+$) [M+H]$^+$ = 236.

***Intermediate 107:** tert-butyl 4-(2-bromo-6-methoxycarbonyl-3-pyridyl)piperazine-1-carboxylate*

A mixture of tert-butyl piperazine-1-carboxylate (8.21 g, 44.06 mmol), methyl 6-bromo-5-fluoro-pyridine-2-carboxylate (6.065 g, 25.92 mmol) **(Intermediate 106)** and potassium carbonate (4.66 g, 33.69 mmol) in DMF (60 mL) was stirred at 110 °C for 5 hours, LCMS indicated full conversion. The mixture was cooled to rt, diluted with DCM and water, the layers were separated, the water layer was extracted with DCM twice, the organic layers were combined, dried

(anhydrous $Na_2SO_4$), filtered and concentrated, the residue was purified on silica gel column (eluted with 0 to 50% ethyl acetate in hexanes, UV at 221, 310 nm) to yield desired product tert-butyl 4-(2-bromo-6-methoxycarbonyl-3-pyridyl) piperazine-1-carboxylate (7.68 g, 74.1 %) **(Intermediate 107)** as a white solid. 1H NMR (500 MHz, CHLOROFORM-d) 1.51 (9H, s), 3.14 (4H, br t), 3.60 - 3.71 (4H, m), 3.99 (3H, s), 7.32 (1H, d), 8.08 (1H, d); (m/z) ($ES^+$) $[M+H]^+$ = 402.

***Intemediate 108:** tert-butyl 4-[2-bromo-6-(methylcarbamoyl)-3-pyridyl]piperazine-1-carboxylate*

tert-Butyl 4-(2-bromo-6-methoxycarbonyl-3-pyridyl)piperazine-1-carboxylate (7.67 g, 19.16 mmol) **(Intermediate 107)** in methanamine (100 mL, 19.16 mmol) (33% in ethanol) in a sealed vessel was stirred at 60 °C for 4.5 h, LCMS indicated full conversion, the mixture was cooled to rt, concentrated, the residue was dissolved into DCM, washed with sat. $NH_4Cl$ solution, dried (anhydrous $Na_2SO_4$), filtered and concentrated to yield tert-butyl 4-[2-bromo-6-(methylcarbamoyl)-3-pyridyl]piperazine-1-carboxylate (7.48 g, 98 %) **(Intermediate 108)** as a white solid. 1H NMR (500 MHz, CHLOROFORM-d) 1.50 (9H, s), 3.02 (3H, d), 3.08 (4H, br t), 3.60 - 3.71 (4H, m), 7.36 (1H, d), 7.68 (1H, br d), 8.11 (1H, d); (m/z) ($ES^+$) $[M+H]^+$ = 401.

***Intermediate 109:** tert-butyl 4-[6-(methylcarbamoyl)-2-vinyl-3-pyridyl]piperazine-1-carboxylate*

A mixture of tert-butyl 4-[2-bromo-6-(methylcarbamoyl)-3-pyridyl]piperazine-1-carboxylate (1.344 g, 3.37 mmol) **(Intermediate 108),** tributyl(vinyl)stannane (1.174 g, 3.70 mmol) and Xphos Pd G2 (0.132 g, 0.17 mmol) in 1,4-dioxane (25 ml) was stirred at 100 °C under $N_2$ for 2.5 hr, LCMS indicated full conversion. The mixture was diluted with DCM, washed with sat. $NH_4Cl$, the organic layer was dried (anhydrous $Na_2SO_4$), filtered and concentrated, the residue was purified on silica gel column (eluted with 0 to 80% ethyl acetate in hexanes, UV at 226, 293 nm) to yield tert-butyl 4-[6-(methylcarbamoyl)-2-vinyl-3-pyridyl]piperazine-1-carboxylate (0.961 g, 82 %) **(Intermediate 109)** as a white solid. 1H NMR (500 MHz, CHLOROFORM-d) 1.50 (9H, s), 2.90 - 3.01 (4H, m), 3.05 (3H, d), 3.55 - 3.68 (4H, m), 5.54 (1H, dd), 6.42 (1H, dd), 7.10 (1H, dd), 7.39 (1H, d), 7.98 (1H, br d), 8.07 (1H, d); m/z ($ES^+$) $[M+H]^+$ = 346.6, 348.5.

***Intermediate 110:** tert-butyl 4-[2-formyl-6-(methylcarbamoyl)-3-pyridyl)piperazine-1-carboxylate*

Osmium tetroxide in $H_2O$ (0.0435 mL, 6.00 μmol) was added to a solution of tert-butyl 4-[6-(methylcarbamoyl)-2-vinyl-3-pyridyl]piperazine-1-carboxylate (960 mg, 2.77 mmol) **(Intermediate 109),** 2,6-lutidine (646 μl, 5.54 mmol) and sodium periodate (2371 mg, 11.08 mmol) in THF (25 mL)/water (5 mL)/ tert-butanol (2650 μL, 27.71 mmol) and stirred at rt for overnight gave a yellow suspension. LCMS and TLC indicated full conversion. Reaction was diluted with water and extracted with ethyl acetate. After concentration the crude material was purified through silica column (eluted with 0 to 100% ethyl acetate in hexanes, UV at 226, 310 nm) to yield tert-butyl 4-[2-formyl-6-(methylcarbamoyl)-3-pyridyl] piperazine-1-carboxylate (0.732 g, 76 %) **(Intermediate 110)** as a yellow solid. 1H NMR (500 MHz, CHLOROFORM-d) 1.50 (9H, s), 3.07 (3H, d), 3.15 - 3.30 (4H, m), 3.63 - 3.79 (4H, m), 7.48 (1H, d), 7.85 (1H, br d), 8.28 (1H, d), 10.10 (1H, s); (m/z) ($ES^+$) $[M+H]^+$ = 349.

***Intermediate 111:** tert-butyl 4-[2-(difluoromethyl)-6-(methylcarbamoyl)-3-pyridyl]piperazine-1-carboxylate*

tert-Butyl 4-[2-formyl-6-(methylcarbamoyl)-3-pyridyl]piperazine-1-carboxylate (730 mg, 2.10 mmol) **(Intermediate 110)** in $CH_2Cl_2$ (10 mL) was cooled to 0 °C, DAST (692 μL, 5.24 mmol) in DCM (5 mL) was added to the mixture, the resulting mixture was then stirred at rt for 4 h, TLC and LCMS indicated full conversion. The reaction mixture was quenched with sat. aq $NaHCO_3$ solution dropwise, extracted with DCM, the organics were dried (anhydrous $Na_2SO_4$), filtered and concentrated, the residue was purified on silica gel column (eluted with 0 to 100% ethyl acetate in hexanes, UV at 254, 293 nm) to yield tert-butyl 4-[2-(difluoromethyl)-6-(methylcarbamoyl)-3-pyridyl]piperazine-1-carboxylate (0.666 g, 86 %) **(Intermediate 111)** as a white solid. 1H NMR (500 MHz, CHLOROFORM-d) 1.50 (9H, s), 2.93 - 3.02 (4H, m), 3.05 (3H, d), 3.57 - 3.72 (4H, m), 6.99 (1H, t), 7.62 (1H, d), 7.92 (1H, br d), 8.27 (1H, d); (m/z) ($ES^+$) $[M+H]^+$ = 371.

***Intermediate 60:** 6-(difluoromethyl)-N-methyl-5-piperazin-1-yl-pyridine-2-carboxamide, 2HCl*

HCl 4M in Dioxane (7 mL, 28.00 mmol) was added to a flask charged with tert-butyl 4-[2-(difluoromethyl)-6-(methylcarbamoyl)-3-pyridyl]piperazine-1-carboxylate (665 mg, 1.80 mmol) **(Intermediate 111)** and a stir bar, the mixture was stirred at rt for 1 hr gave a yellow suspension. The solvent was removed, the residue was diluted with ether, the solid was collected by filtration, dried to yield 6-(difluoromethyl)-N-methyl-5-piperazin-1-yl-pyridine-2-carboxamide, 2HCl (0.617 g, 100 %) **(Intermediate 60)** as an orange solid. (m/z) ($ES^+$) $[M+H]^+$ = 272.

***Example 59**: 6-(difluoromethyl)-5-[4-[(2-methoxy-5-methyl-3-oxo-4H-quinoxalin-6-yl)methyl]piperazin-1-yl]-N-methylpyridine-2-carboxamide*

To a suspension of 6-(difluoromethyl)-N-methyl-5-piperazin-1-yl-pyridine-2-carboxamide, 2HCl (87 mg, 0.25 mmol) **(Intermediate 60)** and 7-(bromomethyl)-3-methoxy-8-methyl-1H-quinoxalin-2-one (80 mg, 0.25 mmol) **(Intermediate 104)** in acetonitrile (6 mL) was added DIPEA (222 μL, 1.27 mmol), the resulting mixture was stirred at 70 °C for 2 h gave a clear solution, the mixture was cooled to rt gave a suspension, the solid was collected by filtration, washed with acetonitrile, water, dried to yield 6-(difluoromethyl)-5-[4-[(2-methoxy-5-methyl-3-oxo-4H-quinoxalin-6-yl)methyl]piperazin-1-yl]-N-methylpyridine-2-carboxamide (0.070 g, 58.3 %) **(Example 59)** as a white solid. 1H NMR (500 MHz, DMSO-d6) 2.43 (3H, s), 2.59 (4H, br s), 2.83 (3H, br d), 2.98 (4H, br s), 3.60 (2H, s), 3.95 (3H, s), 6.92 - 7.29 (2H, m), 7.35 (1H, d), 7.85 (1H, br d), 8.08 (1H, d), 8.38 (1H, br d), 11.58 (1H, br s); ((m/z) ($ES^+$) $[M+H]^+$ = 473.

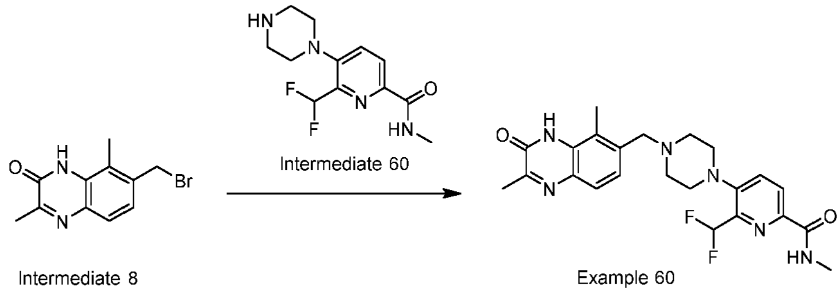


***Example 60:** 6-(difluoromethyl)-5-[4-[(2,5-dimethyl-3-oxo-4H-quinoxalin-6-yl)methyl]piperazin-1-yl]-N-methyl-pyridine-2-carboxamide*

A mixture of 7-(bromomethyl)-3,8-dimethyl-1H-quinoxalin-2-one (196 mg, 0.73 mmol) **(Intermediate 8),** 6-(difluoromethyl)-N-methyl-5-piperazin-1-yl-pyridine-2-carboxamide, 2HCl (252 mg, 0.73 mmol) **(Intermediate 60)** and $Et_3N$ (0.614 mL, 4.41 mmol) in acetonitrile (25 mL) was stirred at 70 °C for 2 h gave a clear solution, LCMS indicated full conversion. The mixture was cooled to rt overnight. A solid crystallized from the mixture, the solid was collected by filtration, washed with acetonitrile, water, dried to yield part 1 of the product 141 mg, the filtrate was concentrated, purified on reverse phase gilson (eluted with 5 to 80% ACN/water/0.1%TFA) to yield a second portion of the product 92 mg as TFA salt. In total: 6-(difluoromethyl)-5-[4-[(2,5-dimethyl-3-oxo-4H-quinoxalin-6-yl)methyl]piperazin-1-yl]-N-methyl-pyridine-2-carboxamide (0.233 g, 64.0 %) **(Example 60)** as an off white solid. 1H NMR (500 MHz, DMSO-d6) 2.40 (3H, s), 2.43 (3H, s), 2.60 (4H, br s), 2.83 (3H, d), 2.98 (4H, br d), 3.63 (2H, s), 6.95 - 7.22 (1H, m), 7.23 - 7.29 (1H, m), 7.51 (1H, d), 7.85 (1H, d), 8.08 (1H, d), 8.38 (1H, br d), 11.54 (1H, br s); (m/z) ($ES^+$) $[M+H]^+$ = 457.

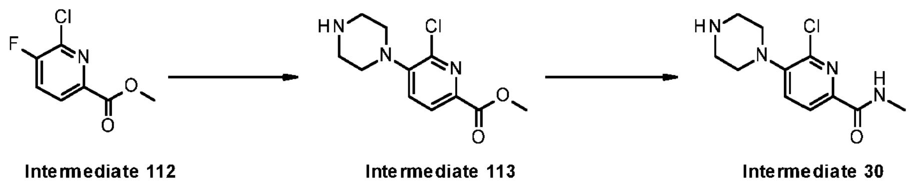


***Intermediate 113:** methyl 6-chloro-5-(piperazin-1-yl)picolinate*

Piperazine (1.0 g, 11.61 mmol) was added to methyl 6-chloro-5-fluoropicolinate **(Intermediate 112,** 1.0 g, 5.28 mmol) in MeCN (30 mL). The resulting mixture was stirred at 80 °C for 18 hours. The solvent was removed under reduced pressure. The crude product was purified by reverse phase chromatography, elution gradient 5 to 60% MeCN in water (0.1% $NH_4HCO_3$). Pure fractions were evaporated to dryness to afford methyl 6-chloro-5-(piperazin-1-yl)picolinate **(Intermediate 113,** 1.28 g, 95%) as a red oil. $^1H$ NMR (400 MHz, DMSO-$d_6$) δ 2.81 - 2.91 (4H, m), 3.04 - 3.08 (4H, m), 3.85 (3H, s), 7.61 (1H, d), 8.00 (1H, d) (NH proton is not shown); m/z ($ES^+$) $[M+H]^+$ = 256.

***Intermediate 30:** 6-chloro-N-methyl-5-(piperazin-1-yl)picolinamide*

A 2 M solution of methylamine in THF (40 mL, 80.00 mmol) was added to methyl 6-chloro-5-(piperazin-1-yl) picolinate **(Intermediate 113,** 1.26 g, 4.93 mmol). The resulting mixture was stirred at 80 °C for 18 hours. The solvent was removed under reduced pressure. The crude product was purified by reverse phase chromatography, elution gradient 5 to

60% MeCN in water (0.1% $NH_4HCO_3$). Pure fractions were evaporated to dryness to afford 6-chloro-N-methyl-5-(piperazin-1-yl)picolinamide **(Intermediate 30,** 1.12 g, 89%) as a pale yellow oil. $^1$H NMR (300 MHz, DMSO-$d_6$) δ 2.79 (3H, d), 2.85 - 2.89 (4H, m), 2.97 - 3.02 (4H, m), 7.63 (1H, d), 7.94 (1H, d), 8.45 (1H, q) (Piperazine-NH proton is not shown); m/z (ES$^+$) [M+H]$^+$ = 255.

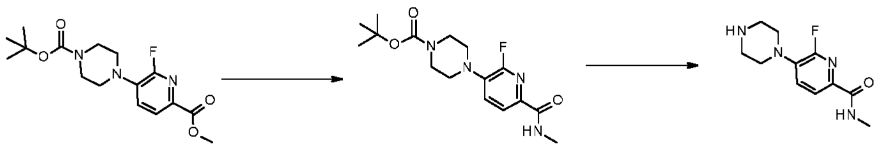

Intermediate 11 Intermediate 114 Intermediate 32

***Intermediate 114:** tert-butyl 4-[2-fluoro-6-(methylcarbamoyl)-3-pyridyl]piperazine-1-carboxylate*

tert-butyl 4-(2-fluoro-6-methoxycarbonyl-3-pyridyl)piperazine-1-carboxylate **(Intermediate 11,** 12.49 g, 36.80 mmol) in methylamine (120 mL, 36.80 mmol, 33 wt% in ethanol) was stirred at rt for 24 hrs. (sealed tube). The solvent was removed under reduced pressure. The residue was dissolved into DCM and filtered through silica gel bed and washed with ethyl acetate. The filtrate was concentrated and dried under vacuum to afford tert-butyl 4-[2-fluoro-6-(methylcarbamoyl)-3-pyridyl]piperazine-1-carboxylate (**Intermediate 114**,12.45 g, 100 %) as a yellow solid. 1H NMR (500 MHz, DMSO-d6) 1.42 (9H, s), 2.77 (3H, d), 3.04 - 3.16 (4H, m), 3.43 - 3.56 (4H, m), 7.59 (1H, dd), 7.80 - 7.93 (1H, m), 8.41 (1H, q); m/z (ES$^+$) [M+H]$^+$ = 340.

***Intermediate 32:** 6-fluoro-N-methyl-5-piperazin-1-yl-pyridine-2-carboxamide*

HCl (4M in dioxane, 100 ml, 400.00 mmol) was added to a solution of tert-butyl 4-[2-fluoro-6-(methylcarbamoyl)-3-pyridyl]piperazine-1-carboxylate (**Intermediate 114,** 12.5 g, 36.94 mmol) in 1,4-dioxane (50 mL) at 0 °C. the reaction was stirred for 5 h during which the temperature was warmed to room temperature to give a yellow suspension. The suspension was diluted with ether, solid was filtered off and washed with ether. This solid was dried under vacuum to afford 6-fluoro-N-methyl-5-piperazin-1-yl-pyridine-2-carboxamide, 2HCl (**Intermediate 32**,11.42 g, 99 %) as a light-yellow solid. 1H NMR (500 MHz, DMSO-d6) 5 ppm 2.8 (d, J=4.6 Hz, 3 H) 3.3 (br s, 4 H) 3.4 (br d, J=4.4 Hz, 4 H) 7.6 - 7.7 (m, 1 H) 7.9 (d, J=8.1 Hz, 1 H) 8.4 (br d, J=4.4 Hz, 1 H) 9.0 - 9.3 (m, 2 H); m/z (ES$^+$) [M+H]$^+$ = 239

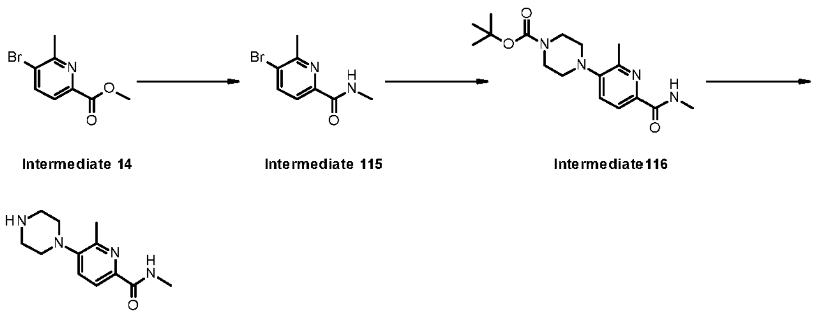

Intermediate 14 Intermediate 115 Intermediate116

Intermediate 33

***Intermediate 115:** 5-bromo-N,6-dimethylpicolinamide*

A 2 M solution of methylamine in THF (20 mL, 40.00 mmol) was added to methyl 5-bromo-6-methylpicolinate (**Intermediate 14**, 2.0 g, 8.69 mmol) and the resulting mixture was stirred at 80 °C for 18 hours. The solvent was removed under reduced pressure. The crude product was purified by reverse phase chromatography, elution gradient 5 to 80% MeOH in water (0.1% $NH_4HCO_3$). Pure fractions were evaporated to dryness to afford 5-bromo-N,6-dimethylpicolinamide (**Intermediate 115,** 1.5 g, 75%) as a pale yellow solid. $^1$H NMR (400 MHz, DMSO-$d_6$) δ 2.65 (3H, s), 2.82 (3H, d), 7.75 (1H, d), 8.17 (1H, d), 8.57 - 8.76 (1H, m); m/z (ES$^+$) [M+H]$^+$ = 229

***Intermediate 116:** tert-butyl 4-(2-methyl-6-(methylcarbamoyl)pyridin-3-yl)piperazine-1-carboxylate*

5-bromo-N,6-dimethylpicolinamide (**Intermediate 115,** 1.0 g, 4.37 mmol) was added to tert-butyl piperazine-1-carboxylate (0.894 g, 4.80 mmol), BINAP (0.272 g, 0.44 mmol), $Pd(OAc)_2$ (0.098 g, 0.44 mmol) and $Cs_2CO_3$ (3.56 g, 10.91

mmol) in toluene (20 mL) under nitrogen. The resulting mixture was stirred at 80 °C for 16 hours. The solvent was removed under reduced pressure. The crude product was purified by reverse phase chromatography, elution gradient 5 to 30% MeOH in water (0.4% $HCO_2H$). Pure fractions were evaporated to dryness to afford tert-butyl 4-(2-methyl-6-(methylcarbamoyl)pyridin-3-yl)piperazine-1-carboxylate (**Intermediate 116**, 1.2 g, 82%) as a brown solid. $^1H$ NMR (300 MHz, $CD_3OD$) δ 1.50 (9H, s), 2.58 (3H, s), 2.92 - 3.00 (7H, m), 3.62 (4H, m), 7.50 (1H, d), 7.88 (1H, d); m/z ($ES^+$) $[M+H]^+$ = 335.

***Intermediate 33:*** *N,6-dimethyl-5-(piperazin-1-yl)picolinamide*

tert-butyl 4-(2-methyl-6-(methylcarbamoyl)pyridin-3-yl)piperazine-1-carboxylate (**Intermediate 115**, 1.18 g, 3.53 mmol) was added to a 4 M solution of HCl in the 1,4-dioxane (10 mL, 329.15 mmol). The resulting mixture was stirred at room temperature for 1 hour. The precipitate was collected by filtration, washed with petroleum ether (5 mL x 2), $Et_2O$ (5 mL x 2), and dried under vacuum to afford N,6-dimethyl-5-(piperazin-1-yl)picolinamide (**Intermediate 33,** 0.77 g, 81%) as an yellow solid. $^1H$ NMR (300 MHz, $CD_3OD$) δ 2.86 (3H, s), 3.02 (3H, s), 3.42 - 3.54 (8H, m), 8.29 (2H, d); m/z ($ES^+$) $[M+H]^+$ = 235.

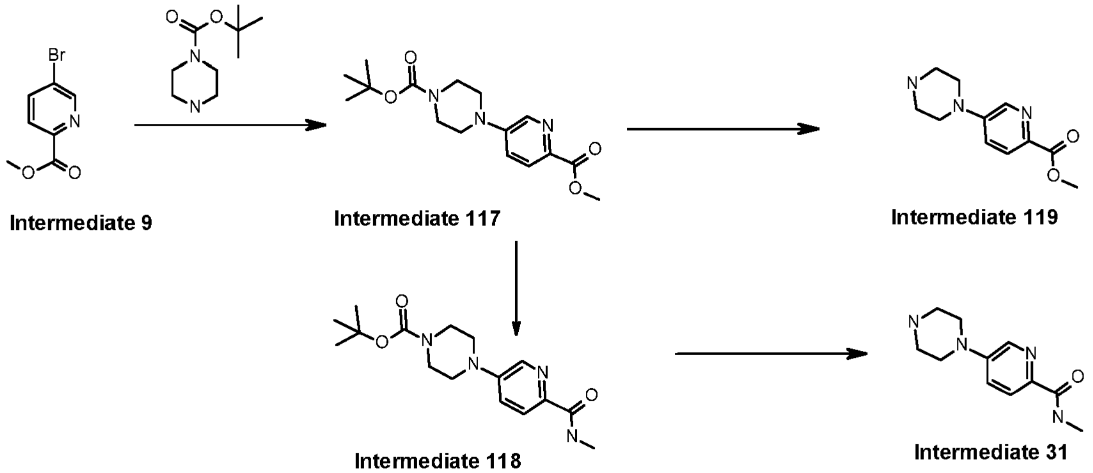

Intermediate 9

Intermediate 117

Intermediate 119

Intermediate 118

Intermediate 31

***Intermediate 117:*** *tert-butyl 4-(6-methoxycarbonyl-3-pyridyl)piperazine-1-carboxylate*

Ruphos Pd G3 (4.07 g, 4.86 mmol) was added to a degassed mixture of methyl 5-bromopyridine-2-carboxylate (**Intermediate 9,** 30 g, 138.87 mmol), tert-butyl piperazine-1-carboxylate (27.2 g, 145.81 mmol), $Cs_2CO_3$ (90 g, 277.73 mmol) in 1,4-dioxane (200 mL) and the mixture was stirred at 110 °C for 6 hrs under $N_2$ atmosphere. The mixture was then cooled to room temperature, diluted with water, extracted with ethyl acetate (150 ml x 3). Combined organic layers were dried over anhydrous $Na_2SO_4$ and filtered. To this filtrate was added 3-(Diethylenetriamino)propyl-functionalized silica gel (12 g, 1.3mmol/g loading) and the mixture was stirred at rt for 1 hr. The mixture was filtered, and the filtrate was concentrated to ~100 mL. The crystalline yellow solid was filtered off, washed with ether and dried under vacuum to afford tert-butyl 4-(6-methoxycarbonyl-3-pyridyl)piperazine-1-carboxylate (**Intermediate 117,** 26.36 g, 82 mmol, 59.1 %) as a yellow solid. 1H NMR (500 MHz, CHLOROFORM-*d*) 1.50 (9H, s), 3.31 - 3.42 (4H, m), 3.56 - 3.68 (4H, m), 3.98 (3H, s), 8.04 (1H, d), 8.37 (1H, d); m/z ($ES^+$) $[M+H]^+$ = 322.

***Intermediate 118:*** *tert-butyl 4-[6-(methylcarbamoyl)-3-pyridyl]piperazine-1-carboxylate*

Methylamine (100 mL, 1155.26 mmol, 40% in water) was added to a solution of tert-butyl 4-(6-methoxycarbonyl-3-pyridyl)piperazine-1-carboxylate (**Intermediate 117,** 36 g, 112.02 mmol) in MeOH (100 mL) and the reaction was stirred at room temperature for 4hs to give a white suspension. The mixture was concentrated, the residue was partitioned between sat. $NH_4Cl$ solution and DCM, the layers were separated. The aqueous layer was extracted with DCM, the organic layers were combined, washed with brine, dried over $Na_2SO_4$, filtered and concentrated to give tert-butyl 4-[6-(methylcarbamoyl)-3-pyridyl]piperazine-1-carboxylate (**Intermediate 118,** 35.9 g, 100 %) as a yellow solid. 1H NMR (500 MHz, CHLOROFORM-d) 1.49 (9H, s), 3.02 (3H, d), 3.26 - 3.35 (4H, m), 3.58 - 3.67 (4H, m), 7.23 (1H, dd), 7.81 (1H, br d), 8.07 (1H, d), 8.16 (1H, d); m/z ($ES^+$) $[M+H]^+$ = 321.

***Intermediate 119:** methyl 5-(piperazin-1-yl)picolinate*

HCl 4M in Dioxane (20 ml, 576.01 mmol) was added to a mixture of tert-butyl 4-(6-(methoxycarbonyl)pyridin-3-yl) piperazine-1-carboxylate (**Intermediate 117**, 1.55 g, 4.82 mmol) in MeOH (2 mL) at 0°C, the reaction was stirred at r.t for 2hr gave a suspension, LCMS indicated full conversion, the mixture was diluted with ether (~ 80 ml), the solid was collected by filtration, washed with ether, dried to yield methyl 5-(piperazin-1-yl)picolinate (**intermediate 119**)(1.384 g, 98 %) as a yellow solid. 1H NMR (500 MHz, DMSO-d6) 3.21 (4H, br s), 3.66 (4H, br d), 3.83 (3H, s), 7.43 - 7.55 (1H, m), 7.95 (1H, br d), 8.43 (1H, br s), 9.49 (2H, br s); (m/z) (ES+) [M+H]+ = 223.0.

***Intermediate 31:** carboxylate N-methyl-5-piperazin-1-yl-pyridine-2-carboxamide*

HCl (4M in dioxane, 150 mL, 600.00 mmol) was added to a suspension of tert-butyl 4-[6-(methylcarbamoyl)-3-pyridyl]piperazine-1-carboxylate (**Intermediate 118**, 35.9 g, 112.05 mmol) in MeOH (50 mL) and the resulting orange suspension was stirred at rt for 4hr. About 80 mL of solvent was removed under reduced pressure and the mixture was diluted with ether and hexanes (200 ml, 1/1). The solid was collected by filtration, washed with hexanes, dried and dried under vacuum to afford N-methyl-5-piperazin-1-yl-pyridine-2-carboxamide, 2HCl salt (**Intermediate 31**, 37.0 g, 100 %) as a yellow solid. 1H NMR (500 MHz, DMSO-d6) 2.79 (3H, d), 3.22 (4H, br s), 3.53 - 3.67 (4H, m), 7.51 (1H, dd), 7.91 (1H, d), 8.33 (1H, d), 8.50 (1H, br s), 9.19 - 9.49 (2H, m); m/z ($ES^+$) $[M+H]^+$ = 221.

**Example 61:** Preparation of 6-fluoro-5-[4-[(5-fluoro-2-methyl-3-oxo-4H-quinoxalin-6-yl)methyl]piperazin-1-yl]-N-methyl-pyridine-2-carboxamide Crystalline Form B (anhydrous form)

Method 1

43 mg (0.10 mmol) of 6-fluoro-5-[4-[(5-fluoro-2-methyl-3-oxo-4H-quinoxalin-6-yl)methyl]piperazin-1-yl]-N-methyl-pyridine-2-carboxamide (from e.g. Example 20) was suspended in 1.0 ml of MeOH and 0.11 ml of 1M methanesulfonic acid (MSA) aqueous solution was added to get a clear solution. To the solution, 0.11 ml of 1N NaOH aqueous solution was added. White solid started to precipitate after completion of adding the NaOH solution. The slurry was stirred at the room temperature for 1 day. 36 mg of the white solid was filtered and dried in air. XRPD shows that the solid is pure 6-fluoro-5-[4-[(5-fluoro-2-methyl-3-oxo-4H-quinoxalin-6-yl)methyl]piperazin-1-yl]-N-methyl-pyridine-2-carboxamide Form B.

Method 2

Pyridine (93.5 g) was added to pure 6-fluoro-5-[4-[(5-fluoro-2-methyl-3-oxo-4H-quinoxalin-6-yl)methyl]piperazin-1-yl]-N-methyl-pyridine-2-carboxamide mesylate (4.67 kg, prepared via method 2 of Example 63) solution in water (47.9 kg) and ethanol (38.0 kg) at 75±5°C, followed by 6-fluoro-5-[4-[(5-fluoro-2-methyl-3-oxo-4H-quinoxalin-6-yl)methyl] piperazin-1-yl]-N-methyl-pyridine-2-carboxamide Form B seed (4.7 g) prepared according to method 1. The slurry was stirred at 75±5°C for 40 min then pyridine (651 g) solution in 50:50 v:v water:ethanol (4.2 kg) was added gradually over 3 h 40 min. The slurry was stirred at 75±5°C for 50 min then 4-methylmorpholine (900 g) solution in 50:50 v:v water:ethanol (4.1 kg) was added gradually over 3 h 50 min. The slurry was stirred at 75±5°C for 1 h 10 min, cooled to 25±5°C over 4 h 50 min, stirred at 25±5°C for 15 h then filtered. The resulting solid was washed twice with 50:50 v:v water:ethanol (12.5 kg x 2) and then dried under vacuum at between 25°C and 50°C for 1 day to give pure 6-fluoro-5-[4-[(5-fluoro-2-methyl-3-oxo-4H-quinoxalin-6-yl)methyl]piperazin-1-yl]-N-methyl-pyridine-2-carboxamide Form B (3.54 kg) in 93% yield.

Form B from method 1 was analyzed by XRPD and the results are tabulated below (Table 1) and shown in **Figure 1.**

**Table 1.** XRPD Peaks for Form B

| Angle (2$\theta$±0.2°) | Intensity (%) |
|---|---|
| 18.2 | 100.0 |
| 9.6 | 86.7 |
| 9.1 | 80.7 |
| 18.7 | 55.8 |
| 12.7 | 24.1 |
| 8.5 | 23.9 |

(continued)

| Angle (2$\theta\pm$0.2°) | Intensity (%) |
|---|---|
| 10.0 | 15.6 |
| 20.1 | 14.8 |
| 21.7 | 13.9 |
| 23.2 | 12.3 |
| 12.4 | 12.2 |
| 16.1 | 9.6 |
| 14.3 | 9.2 |
| 6.2 | 8.9 |
| 15.6 | 7.7 |
| 27.4 | 6.9 |
| 26.4 | 6.6 |
| 29.7 | 6.2 |
| 27.1 | 6.0 |
| 25.0 | 5.0 |

Form B is characterized in providing at least one of the following 2$\theta$ values measured using CuK$\alpha$ radiation: 6.2, 14.3, and 15.6°.

Form B (from method 1) was analyzed by thermal techniques. DSC analysis indicated that Form B has a melting point with an onset at 275 °C and a peak at 276 °C. TGA indicated that Form B exhibits a mass loss of about 0.2 % upon heating from about 25 °C to about 100 °C. A representative DSC/TGA thermogram of Form B is shown in **Figure 2.**

**Example 62:** Preparation of 6-fluoro-5-[4-[(5-fluoro-2-methyl-3-oxo-4H-quinoxalin-6-yl)methyl]piperazin-1-yl]-N-methyl-pyridine-2-carboxamide Crystalline Form D (anhydrous form).

5-6 mg of 6-fluoro-5-[4-[(5-fluoro-2-methyl-3-oxo-4H-quinoxalin-6-yl)methyl]piperazin-1-yl]-N-methylpyridine-2-carboxamide (Example 20) was dissolved in mixed solvents of MeOH/DCM/$H_2O$ (0.50ml/0.50ml/0.20ml), the clear solution was slowly evaporated in the ambient condition to obtain a white solid. XRPD shows that the resulting white solid is 6-fluoro-5-[4-[(5-fluoro-2-methyl-3-oxo-4H-quinoxalin-6-yl)methyl]piperazin-1-yl]-N-methyl-pyridine-2-carboxamide Form D.

Form D was analyzed by XRPD and the results are tabulated below (Table 2) and shown in **Figure 3.**

**Table 2.** XRPD Peaks for Form D

| Angle (2$\theta\pm$0.2°) | Intensity (%) |
|---|---|
| 9.6 | 100.0 |
| 18.4 | 43.0 |
| 13.1 | 32.0 |
| 19.9 | 17.9 |
| 27.1 | 17.3 |
| 9.2 | 15.9 |
| 21.7 | 14.0 |
| 23.4 | 11.2 |
| 24.5 | 9.6 |
| 19.3 | 8.8 |
| 16.8 | 8.7 |
| 22.2 | 8.3 |

(continued)

| Angle ($2\theta \pm 0.2°$) | Intensity (%) |
|---|---|
| 16.3 | 8.0 |
| 18.8 | 7.6 |
| 25.4 | 6.9 |
| 10.2 | 6.6 |
| 33.0 | 6.3 |
| 22.5 | 5.8 |
| 12.6 | 5.7 |
| 7.9 | 4.5 |

Form D is characterized in providing at least one of the following $2\theta$ values measured using CuK$\alpha$ radiation: 7.9, 13.1 and 16.3°.

Single crystals of Form D were obtained from evaporation of the DMF solution (or DMF/$H_2O$) of 6-fluoro-5-[4-[(5-fluoro-2-methyl-3-oxo-4H-quinoxalin-6-yl)methyl]piperazin-1-yl]-N-methyl-pyridine-2-carboxamide. Single crystal structure analysis confirmed that Form D is an anhydrous form. The molecular structure of 6-fluoro-5-[4-[(5-fluoro-2-methyl-3-oxo-4H-quinoxalin-6-yl)methyl]piperazin-1-yl]-N-methyl-pyridine-2-carboxamide Form D is shown in **Figure 4.** Crystallographic data: Space group monoclinic $P2_1/c$, unit cell dimensions: $a$ = 17.4559(8) Å, $b$ = 5.0647(2) Å, $c$ = 22.564(1) Å, $\beta$ = 92.609(1)°, $V$ = 1992.8(2) Å$^3$.

**Example 63:** Preparation of 6-fluoro-5-[4-[(5-fluoro-2-methyl-3-oxo-4H-quinoxalin-6-yl)methyl]piperazin-1-yl]-N-methyl-pyridine-2-carboxamide MSA Crystalline Salt Form C (anhydrous form).

Method 1

427 mg of 6-fluoro-5-[4-[(5-fluoro-2-methyl-3-oxo-4H-quinoxalin-6-yl)methyl]piperazin-1-yl]-N-methylpyridine-2-carboxamide (Example 20) was suspended in 8.0 ml of MeOH. To the suspension, 1.1 ml of 1.0 M aqueous MSA solution (1.1 mmol) was added, a clear solution was obtained. The resulting solution was filtered, and the solvents of the clear solution was removed. The resulting solid was suspended in 1.0 ml of EtOH and 2.0 ml of THF, to obtain a slurry. The slurry was stirred at the room temperature for 1 day. The solid was collected by filtration and air-dried. 452 mg of off-white solid was obtained. XRD shows that 6-fluoro-5-[4-[(5-fluoro-2-methyl-3-oxo-4H-quinoxalin-6-yl)methyl]piperazin-1-yl]-N-methyl-pyridine-2-carboxamide MSA salt Form C was obtained.

Method 2

Methanesulfonic acid (16.8 g) was added to a stirring suspension of 6-fluoro-5-[4-[(5-fluoro-2-methyl-3-oxo-4H-quinoxalin-6-yl)methyl]piperazin-1-yl]-N-methyl-pyridine-2-carboxamide (80.8 g, 92.8% w/w) in 4:1 v:v THF:ethanol (750 mL) at 25°C. The resulting suspension was stirred at 25°C for 16 h and then filtered. The solid was washed with 4:1 v:v THF:ethanol (300 mL) and then dried at 35°C under vacuum to give 6-fluoro-5-[4-[(5-fluoro-2-methyl-3-oxo-4H-quinoxalin-6-yl)methyl]piperazin-1-yl]-N-methylpyridine-2-carboxamide mesylate Form C (90.3 g) in 98% yield.

MSA-Form C from method 1 was analyzed by XRPD and the results are tabulated below (Table 3) and shown in **Figure 5.**

**Table 3.** XRPD Peaks for MSA-Form C

| Angle ($2\theta \pm 0.2°$) | Intensity (%) |
|---|---|
| 17.5 | 100.0 |
| 24.6 | 95.7 |
| 25.4 | 75.8 |
| 16.0 | 73.8 |
| 22.5 | 72.6 |
| 19.4 | 72.5 |

(continued)

| Angle (2$\theta$±0.2°) | Intensity (%) |
|---|---|
| 24.3 | 71.6 |
| 13.6 | 65.6 |
| 19.0 | 61.3 |
| 20.4 | 53.3 |
| 21.5 | 43.4 |
| 15.5 | 43.2 |
| 22.0 | 40.2 |
| 28.8 | 35.8 |
| 14.6 | 34.2 |
| 26.9 | 29.7 |
| 21.0 | 29.4 |
| 18.3 | 29.1 |
| 31.4 | 26.8 |
| 16.8 | 26.0 |

MSA-Form C obtained from method 1 was analyzed by thermal techniques. DSC analysis indicated that MSA-Form C starts to melting and decomposition at the temperature with an onset at 254 °C and a peak at 258 °C. TGA indicated that MSA-Form C exhibits a mass loss of about 0.3 % upon heating from about 25 °C to about 100 °C. A representative DSC/TGA thermogram of MSA-Form C is shown in

**Figure 6.**

**Biological Assays**

The following test procedures may be employed to determine the inhibitory properties of the compounds described herein.

PARP Fluorescence Anisotropy binding assays

Recombinant full length 6HIS tagged PARP1 protein was diluted to 6 nM with 50 mM Tris pH 8, 0.001% Triton X100, 10 mM $MgCl_2$, 150 mM NaCl and incubated for four hours with an equivalent volume of 2 nM fluorescent probe diluted with 50 mM Tris pH 8, 0.001% Triton X100, 10 mM $MgCl_2$, 150 mM NaCl. The final DMSO concentration of the probe was kept below 1% (v/v).

Recombinant full length PARP2 protein was diluted to 6 nM with 50 mM Tris pH 8, 0.001% Triton X100, 10 mM $MgCl_2$, 150 mM NaCl and incubated for four hours with an equivalent volume of 2 nM fluorescent probe diluted with 50 mM Tris pH 8, 0.001% Triton X100, 10 mM $MgCl_2$, 150 mM NaCl. The final DMSO concentration of the probe was kept below 1% (v/v).

Recombinant full length PARP3 protein was diluted to 100 nM with 50 mM Tris pH 8, 0.001% Triton X100, 10 mM $MgCl_2$, 150 mM NaCl and incubated for four hours with an equivalent volume of 6 nM fluorescent probe diluted with 50 mM Tris pH 8, 0.001% Triton X100, 10 mM $MgCl_2$, 150 mM NaCl. The final DMSO concentration of the probe was kept below 1% (v/v).

Recombinant PARP5a binding domain was diluted to 160 nM with 50 mM Tris pH 8, 0.001% Triton X100, 10 mM $MgCl_2$, 150 mM NaCl and incubated for four hours with an equivalent volume of 6 nM fluorescent probe diluted with 50 mM Tris pH 8, 0.001% Triton X100, 10 mM $MgCl_2$, 150 mM NaCl. The final DMSO concentration of the probe was kept below 1% (v/v).

Recombinant full length GST tagged PARP6 protein was diluted to 160 nM with 50 mM Tris pH 8, 0.001% Triton X100, 10 mM $MgCl_2$, 150 mM NaCl and incubated for four hours with an equivalent volume of 6 nM fluorescent probe diluted with 50 mM Tris pH 8, 0.001% Triton X100, 10 mM $MgCl_2$, 150 mM NaCl. The final DMSO concentration of the probe was kept below 1% (v/v).

Fluorescence anisotropy of the probe when bound to the proteins was measured using a BMG Pherastar FSX® in the presence of test compounds or solvent control and the effect on anisotropy determined. % inhibition values for different test compound concentrations were calculated and fitted to a four parameter logistic plot in order to determine the $IC_{50}$ value. Where necessary, the compound $K_i$ can be determined from the $IC_{50}$ value using a Munson Rodbard equation defined in Anal Biochem. 1980 Sep 1;107(1):220-39 and is based on the known $K_D$ of the probe binding to the relevant PARP protein

PARP Proliferation Assay (7 day compound dosing)

DLD1 and BRCA2 (-/-) DLD1 cells were harvested to a density of 5000 cells/ml and 2.5E4 cells/ml respectively in complete media, 40 μL/well seeded into 384-well plates (Greiner, Kremsmunster, Austria; 781090) using a Multidrop Combi then incubated at 37°C, 5% $CO_2$ overnight. Next day (Day 1) using a Multidrop Combi add sytox green (5ul, 2uM) and saponin (10ul, 0.25% stock) to a day 0 plate, seal the plate using a black adhesive lid and incubate for >3 h at rt. Cells were imaged using Cell Insight (Thermo Fisher) fitted with a 4x objective. Test compounds are added using an Echo 555 and placed in incubator maintained at 37°C, 5% $CO_2$ and incubated for 7 days. On Day 8 add sytox green (5ul, 2uM) and then saponin (10ul, 0.25% stock) to plates, seal the plate using a black adhesive lid and incubate for >3 h at rt. Read all cells on the Cell Insight with 4x Objective. The rate of proliferation is determined in Genedata by assessing the total cell number output from the Cell Insight for Day 0 and Day 8 plates.

In vitro human transporter efflux

MDCKII cells expressing MDR1 and BCRP were seeded onto polyethylene membranes in 96-well Transwell insert systems at a density to form a confluent cell monolayer. Test and reference compounds were diluted with the transport buffer (HBSS HEPES pH7.4) to a concentration of 1 or 0.1 μM. The final percent volume of the organic solvent was less than 1%. Permeation of the test compounds from A to B direction and from B to A direction was determined over a 90-min incubation at 37°C and 5% CO2 with a relative humidity of 95%. At the end of the incubation, samples from the apical and basolateral side were taken and then precipitated with cold acetonitrile containing internal standard. After centrifugation at 4000 rpm, the supernatant was diluted with 0.1% formic acid aqueous solution and quantified by LC-MS/MS. The integrity of the cell monolayers was confirmed by using the marker Lucifer yellow.

The permeability coefficient (1 × 10-6 cm/s) was calculated using the following equation

$$Papp = (dCr/dt) \times Vr/(A \times C0)$$

(1)The efflux ratio was calculated using the following equation

$$\text{Efflux ratio} = Papp(B\ to\ A)/Papp(A\ to\ B)$$

(2)where dCr/dt is the cumulative concentration of the compound in the receiver chamber as a function of time (in μM/s); Vr is the solution volume in the receiver chamber (0.1 ml on the apical side and 0.3 ml on the basolateral side); A is the surface area for the transport, that is, 0.11 cm2 for the area of the monolayer; and C0 is the initial concentration in the donor chamber (in μM).

Determination of fraction unbound in plasma

The fraction unbound was determined using the RED Device.

Compounds were prepared as 10 mM solutions in DMSO. A 1 mM working stock was prepared by mixing up to 9 test compound (4 uL each) and 1 control (uL). If less than 9 test compounds were included then the addition volume of blank DMSO was added to make up volume to 40 uL.

Frozen plasma was thawed in a water bath at 37°C. Plasma was then centrifuged for 2 minutes at 4,000 rpm to remove clots and collect supernatant into a fresh tube. The pH of the plasma was checked and only used if within the range of pH 7 to pH 8. 3 μL of the working solution of each cassette was added to 597 μL of blank plasma and vortexed for 5 minutes at 1000 rpm. The final percent volume of organic solvent is 0.5% and the final concentration for test compound was 5 μM. Immediately transfer 50 μL of the spiked plasma suspension to a 96-well plate to act as T=0 control sample. The samples are treated the same as the samples after incubation. The remaining plasma is kept at 37°C prior to starting the dialysis.

Place inserts open end up into the wells of the base plate. Add 300 μL of spiked plasma sample into the sample

chamber, indicated by the red ring. Add 500 μL of phosphate buffer (pH 7.4) to the buffer chamber. Cover the unit with gas permeable lid and incubate for 18 hours at 37°C at 300 rpm with 5% CO2 on an orbital shaker in the CO2 incubator. At the end of incubation, remove lid and pipette 50 μL of post-dialysis samples from both buffer and plasma chambers into separated 96-well plate for analysis, respectively.

Samples were matrix matched by adding 50 μL of blank rat plasma to the buffer samples and an equal volume of PBS to the collected plasma samples and vortexed to mix. 400 μL of acetonitrile containing an appropriate internal standard (IS) was added to precipitate protein and release compound and mixed by vortexing the plate for 10 minutes followed by centrifugation for 30 minutes at 4,000 rpm. 250 μL of the supernatant was transferred to new 96-well plates and centrifuged again (4,000 rpm, 30 minutes). 100 μL of the supernatant was then transferred to new 96-well plates and mixed with 100 μL of distilled water to each sample by vortex for 5 minutes at 1,000 rpm. Samples were analysed by LC-MS/MS and drug concentrations were determined vs a calibration curve produced from spiked blank plasma with a typical range of 1-7500 nM.

The % unbound was determined as % Unbound = (Conc. buffer chamber / Conc. plasma chamber) × 100%. Fraction unbound = % unbound/100.

Determination of fraction unbound in brain slice

The principle for the method for the determination of volume unbound in brain slices has previously been published (Development of a High-Throughput Brain Slice Method for Studying Drug Distribution in the Central Nervous System; Fridén et al,; Drug Metabolism and Disposition, 2009, 37 (6) 1226-1233). In brief:
Compound stock solutions were prepared in DMSO at the concentration of 10 mM. A 1 mM working stock was prepared by mixing up to 9 test compound (4 uL each) and 1 control (4 uL). If less than 9 test compounds were included, then blank DMSO was added to make up volume to 40 uL. On the day of the experiment 4 ul was diluted in 40 mL ECF buffer to give a 100 nM solution of each test compounds which was then pre-warmed to 37°C before the start of the incubation.

To prepare brain slices, rats weighing approximately 300 g were terminally anesthetised with isoflurane by inhalation, the brain carefully removed and immersed in ice-cold oxygenated ECF buffer. The rat brain was transferred to a dish containing ice-cold, O2 supplied ECF buffer and trimmed with razor before gluing to the tray of a microslicer with the brain placed with the back section surface down centrally on the tray. Ice cold ECF buffer was added to harden the glue and wet the brain. The tray was placed in the microslicer and using a suitable cutting speed, 100-400 μm were cut slices until the striatum area appeared. Four to six for each brain, 300 μm thick, coronal slices of striatum areas were cut and placed in ice cold O2 supplied buffer until incubation. Six slices were transferred to the incubation tray containing 40 mL pre warmed (37°C) cassette mixture. The time from when the brain is removed until the slices are in the cocktail mixture was a maximum of 20 minutes. The incubation tray was covered with a gas permeable lid and placed in a water bath with O2 pumped into it at 37°C and incubated at a shaking frequency of 45 rpm for 5h.

Before incubation, 200 μL of the non-incubated cassette solution is saved as the T=0 sample. 200 μL was then mixed with 200 μL blank brain homogenate in ECF buffer (4 volumes (w/v). After incubation the pH in the cassette solution is measured and recorded, and the pH value must be above 7.3. 200 μL from the surface of the cassette solution was transferred in to a tube containing 200 μL blank brain homogenate in ECF buffer (4 volumes (w/v)). Each brain slice is dried on a filter paper and weighed in a 2 mL eppendorf tube. After addition of 9 volumes (w/v) of ECF buffer the slices are homogenized with a sonicator. The samples were precipitated and diluted as below.

50 μL aliquots from each sample and 3x50 μL from each cassette solution (mixed with blank homogenate) were transferred to 0.6 mL centrifuge tubes. The samples are precipitated with 200 μL ice cold acetonitrile containing internal standard and vortexed at 2,000 rpm for 3 minutes followed by centrifugation at 14000 rpm, for 15 minutes at 4°C. 100 μL of the supernatant was transferred to a new 96-well plate for analysis and 100 μL of distilled water to each sample and the plate shaken for 2 minutes at 1000 rpm for analysis by LC-MS/MS.

Then the mixed slice samples are further diluted in two steps, 10 and 100 times with double blank samples prepared with 150 μL of blank brain homogenate in ECF buffer (4 volumes (w/v)) are transferred to 1.5 mL centrifuge tubes containing 150 μL of ECF buffer, vortexed at 2,000 rpm for 2 minutes. The samples are precipitated with 1200 μL ice cold acetonitrile and vortexed at 2,000 rpm for 3 minutes followed by centrifugation at 14000 rpm, for 15 minutes at 4°C. Then transfer 100 μL of the supernatant to a new 96-well plate for analysis. Add 100 μL of distilled water to each sample to obtain the double blank samples.

The unbound volume brain ($V_{u,brain}$) was calculated as $V_u = (C_{slice} - V_0 * C_{ECF})/(1-V_0)*C_{ECF}$

Where $C_{slice}$, $C_{ECF}$ and $V_0$ are amount of drug in the slice, the drug concentration in the ECF (representing the drug concentration in the brain ECF, i.e. the free concentration), and the water adhesion of the brain slice (0.0931), respectively.

The fraction unbound in brain $f_{u,brain} = 1/V_{u,brain}$

Determination of Kpuu in rat

The ratio of total/unbound drug in plasma to total/unbound drug in brain (Kp/Kpuu) was determined as follows.

Compounds were formulated as a mixture at a concentration of 0.5 mM each in 1:1:1 tetraethyleneglycol:dimethylacetamide:waterand administered to Han Wistar rats via intravenous infusion at 2 □mols/kg/h in a volume of 4 mL/kg. After 4h the animals were sacrificed, and brain and blood samples collected. Plasma was prepared from blood and all samples were stored frozen at - 20°C until analysis. Following collection brain samples were homogenised in purified water at a ratio of 1:3 (w/v) and stored frozen at -20°C until analysis.

Plasma and brain samples were analysed by protein precipitation followed by LC-MS/MS and concentrations determined against a calibration curve generated by spiking blank rat plasma or brain homogenate with drug across an appropriate concentration range. The brain concentration was corrected for the residual blood by subtracting 0.8% of the plasma concentration from the total brain concentration.

The Kp was then calculated as: Kp = ((4 * [brain homogenate]) - (0.008 * [plasma]))/[plasma]

The Kpuu was then calculated as: Kpuu = Kp * (fraction unbound in brain slice/fraction unbound in plasma)

BIOLOGICAL DATA

**Table 4**

| Example Number | PARP1 IC50 (μM) | PARP2 IC50 (μM) | PARP3 IC50 (μM) | PARP5a IC50 (μM) | PARP6 IC50 (IJM) | hERG IC50 (μM) | BRCA2 -/-DLD-1 prolif. 7 d IC50 (μM) | WT DLD-1 prolif. 7 d IC50 (μM) | MDCK-MDR1-BCRP Efflux Ratio | Rat Kpuu |
|---|---|---|---|---|---|---|---|---|---|---|
| 1 | 0.004 | >70 | >100 | >100 | >100 | >40 | 0.014 | 24 | 0.7 | 0.07 |
| 2 | 0.011 | 15 | | | | >40 | 0.734 | | | |
| 3 | 0.003 | >100 | >100 | >100 | >100 | >40 | 0.008 | 25 | 1.3 | |
| 4 | 0.005 | >83 | 78 | >100 | >100 | >40 | 0.081 | >14 | 0.8 | 0.13 |
| 5 | 0.005 | >63 | | | | | 2.89 | | | |
| 6 | 0.004 | >100 | | | | | | | | |
| 7 | 0.006 | 58 | | | | | 8.43 | >30 | | |
| 8 | 0.004 | >66 | >100 | >100 | >100 | >40 | 0.012 | >7.9 | | |
| 9 | 0.004 | >100 | >100 | >100 | >100 | >40 | 0.01 | >10 | 0.4 | |
| 10 | 0.013 | >100 | >100 | >100 | >100 | >40 | >30.0 | >30 | | |
| 11 | 0.003 | >100 | >100 | >100 | >100 | >40 | 0.009 | >30 | | |
| 12 | 0.015 | | 85 | >100 | >100 | >40 | 0.008 | | 4.2 | |
| 13 | 0.009 | >100 | 62 | >100 | >100 | >40 | 0.019 | 22 | 3.0 | |
| 14 | 0.013 | >83 | 52 | >100 | >100 | >40 | 0.015 | | 3.7 | |
| 15 | 0.012 | >100 | 43 | >100 | >100 | >38 | 0.014 | | 3.7 | |
| 16 | 0.01 | >100 | >100 | >29 | >100 | >40 | 3.62 | >30 | | |
| 17 | 0.006 | >100 | >100 | >100 | >100 | >40 | 1.05 | >10 | | |
| 18 | 0.005 | >100 | >100 | >100 | >100 | 14 | 4.33 | 21 | | |
| 19 | 0.013 | 40 | | | | >40 | 0.006 | | 2.4 | |
| 20 | <0.005 | >93 | >100 | >100 | >100 | >40 | 0.008 | >30 | 1.1 | 0.27 |
| 21 | 0.007 | >100 | 5.8 | >100 | >100 | >40 | 1.42 | | 1.6 | |
| 22 | 0.006 | 87 | | | | | | | | |

(continued)

| Example Number | PARP1 IC50 (μM) | PARP2 IC50 (μM) | PARP3 IC50 (μM) | PARP5a IC50 (μM) | PARP6 IC50 (IJM) | hERG IC50 (μM) | BRCA2 -/-DLD-1 prolif. 7 d IC50 (μM) | WT DLD-1 prolif. 7 d IC50 (μM) | MDCK-MDR1-BCRP Efflux Ratio | Rat Kpuu |
|---|---|---|---|---|---|---|---|---|---|---|
| 23 | **0.005** | **>67** | **>100** | **>100** | **>100** | **>40** | **0.003** | **>30** | **0.8** | 0.62 |
| 24 | **0.006** | **>81** | **>100** | **>100** | **>100** | **>36** | **0.003** | **>30** | **1.1** | |
| 25 | **0.005** | **>100** | | | | | **0.297** | | | |
| 26 | **0.005** | **88** | | | | | | | | |
| 27 | **0.004** | **>64** | **>100** | **>100** | **>100** | **>40** | **0.006** | **6.3** | **0.8** | 0.43 |
| 28 | **0.005** | **>100** | **>100** | **>100** | **>100** | **>38** | **0.004** | | **0.8** | |
| 29 | **0.015** | **>100** | **>100** | **>100** | **>100** | **>40** | **0.005** | **>10** | **0.7** | 0.33 |
| 30 | **0.006** | **>100** | **>100** | **>100** | **>100** | **>40** | **0.003** | **>30** | **1.8** | |
| 31 | **0.007** | **>100** | **>100** | **>100** | **>100** | **>40** | **0.007** | **>30** | **1.6** | 0.19 |
| 32 | **0.007** | **>100** | **>100** | **>100** | **>100** | **>40** | **1.08** | **23** | | |
| 33 | **0.167** | **>100** | | | | | **>30.0** | | | |
| 34 | **0.026** | **>100** | | | | **29** | **8.44** | | | |
| 35 | **0.03** | **>100** | | | | | **10.3** | **>30** | | |
| 36 | **0.022** | **>100** | | | | | **16.2** | | | |
| 37 | **0.026** | **57** | | | | | **0.01** | | **5.0** | |
| 38 | **0.012** | **21** | **>100** | **>100** | **>100** | **>40** | **0.547** | | | |
| 39 | **0.006** | **>100** | **>100** | **>100** | **>100** | **>40** | **0.008** | | **1.9** | |
| 40 | **0.011** | **>100** | **>100** | **13** | **>100** | **>40** | **0.007** | | **2.0** | |
| 41 | **0.005** | **>100** | **>100** | **>100** | **>100** | **>40** | **0.007** | | **2.4** | |
| 42 | **0.005** | **>88** | | | | | **0.006** | | | |
| 43 | **0.005** | **>100** | | | | | **0.01** | | | |
| 44 | **0.128** | **>100** | | | | | **>30.0** | | | |
| 45 | **0.006** | **>100** | | | | | **0.006** | | **1.5** | |
| 46 | **0.01** | **>100** | | | | | **0.013** | | | |
| 47 | **0.076** | **>100** | **>100** | **>100** | **>100** | **>40** | **0.058** | | | |
| 48 | **0.039** | **26** | **>100** | **>100** | **>100** | **>40** | **0.018** | **27** | **8.8** | |
| 49 | **0.043** | **11** | **>29** | **>100** | **>100** | **>40** | **0.041** | | | |
| 50 | **0.060** | **35** | | | | **>40** | **0.136** | | | |
| 51 | **0.006** | **1.2** | | | | | | | | |
| 52 | **0.085** | **3.8** | | | | | | | | |
| 53 | **0.026** | **>100** | | | | | | | | |
| 54 | **0.111** | **>100** | | | | | | | | |
| 55 | **0.005** | **>100** | | | | | | | | |
| 56 | **0.008** | **>100** | | | | | | | | |
| 57 | **0.011** | **>100** | | | | | | | | |
| 58 | **0.008** | **>100** | | | | | | | | |

(continued)

| Example Number | PARP1 IC50 (μM) | PARP2 IC50 (μM) | PARP3 IC50 (μM) | PARP5a IC50 (μM) | PARP6 IC50 (IJM) | hERG IC50 (μM) | BRCA2 -/-DLD-1 prolif. 7 d IC50 (μM) | WT DLD-1 prolif. 7 d IC50 (μM) | MDCK-MDR1-BCRP Efflux Ratio | Rat Kpuu |
|---|---|---|---|---|---|---|---|---|---|---|
| 59 | **0.006** | **2.1** | | | | | | | | |
| 60 | **0.023** | **>100** | | | | | | | | |

*Further Materials and methods*

**Cell lines**

To evaluate the activity and potency of the PARP inhibitors described herein in combination with the standard of care chemotherapy Temozolamide (TMZ), ionizing radiation (IR) and the emerging chemotherapy VAL-083, U87, SJG2 and BT245 glioblastoma cells were assayed. Parental U87 cell lines were obtained from the AstraZeneca Global Cell Bank in Alderley Park, UK and the isocistrate dehydrogenase 1 mutant cell line (IDH1mt R132H) was licensed in from ATCC. The SJ-G2 (ATRX mutant) and BT245 (H3K27M mutant) cell lines were obtained from The Research Institute of the McGill University Health Centre, Montreal, QC H4A 3J1, Canada. IDH1R132 mutation was induced in SJG2 cell line to generated isogenic pair (SJG2 IDH1mt and SJG2 IDH1wt).

**Combination assay - PARP inhibitor with Temozolamide (TMZ) or VAL-083**

To evaluate the combination activity of the PARP inhibitor produced in Example 20 (6-fluoro-5-[4-[(5-fluoro-2-methyl-3-oxo-4H-quinoxalin-6-yl)methyl]piperazin-1-yl]-N-methyl-pyridine-2-carboxamide; hereafter Compound **20**) with TMZ or VAL-083, a nuclear count end point assay was employed in the U87 isogenic pair and a CellTiter-Glo (CTG) endpoint was used for the SJ-G2 isogenic pairs. Here, cells were seeded at low density (500 cells per well) in a 96-well plate and exposed to Compound **20** for a time corresponding to more than 4-5 replication cell cycles (7-8) days. After cell attachment, the plates were pre-treated with the PARP inhibitor with an automated digital D300 HP dispenser (Tecan) for an hour, after which TMZ or VAL-083 were automatically dispensed. Drugs were added from compound stocks dissolved in DMSO (PARP inhibitor 10 mM stock, TMZ 100 mM stock and VAL-083 100 mM stock), at the titration dilutions indicated, where each concentration was tested in duplicate in each plate. DMSO served as a vehicle control. The concentration ranges tested were chosen to obtain optimal PARP inhibitor, TMZ or VAL-083 activity in the isogenic pairs and assess combination at multiple doses. Plates were incubated at 37°C, 5% $CO_2$ for indicated times. Cell growth and absence of contamination was regularly checked at microscope.

For the nuclear count end point assay, cells were fixed for ~ 15 minutes (min) by the addition of 50 μl perwell of Formaldehyde (Sigma-Aldrich F8775). The fixing solution was then removed and plates were washed with PBS once prior to the addition of hoechst (Thermo Scientific 33342) at 1:10000 dilution of the stock in PBS for 30 min at room temperature (RT). The plates were washed again and PBS was added. The images were acquired using CellInsight CX5 (Thermo Fisher) and analysed by measuring the total number of hoechst stained nuclei. The number of nuclei (% confluence) of the drug treated wells were normalised to the DMSO control and the growth inhibition was calculated using the combenefit software (Di Veroli et al., 2016). Similarly, for the CTG endpoint assay, the combination plates for the SJ-G2 isogenic pairs were stopped by the addition of the reagents following manufacturers instruction (Promega, Madison, WI, USA; G7570). The cell viability (% viability) of the drug treated wells were normalised to the DMSO control and the growth inhibition was calculated using the combenefit software (Di Veroli et al., 2016).

Synergy scores and excess activity over each monotherapy alone were calculated by comparison to the HSA model of additivity (Borisy et al., 2003; Tan et al., 2012). In the represented plots error bars are mean ± SD.

**Combination assay - PARP inhibitor with ionizing radiation (IR)**

To evaluate the combination activity of Compound **20** with IR, a colony formation assay was employed with measures the growth of cells from a single colonies over 10-14 days allowing them to undergo at least 5 replication cell cycles. Here, cells were seeded at low density (500 cells per well) in 24-well plates coated with poly-L-lysine to facilitate cell attachment. After cell attachment, the plates were pre-treated with PARP inhibitor with an automated digital D300 HP dispenser (Tecan) for an hour, after which the cells were irradiated with 0.9 Gy via a high-voltage X-ray-generator tube

(Faxitron X-Ray Corporation). PARP inhibitor was added from compound stocks dissolved in DMSO (10 mM stock), at the titration dilutions indicated, where each concentration was tested in triplicate in each plate. DMSO served as a vehicle control. Plates were incubated at 37°C, 5% $CO_2$ for indicated times. Cell growth and absence of contamination was regularly checked at microscope. Next, cells were fixed and stained using the Sulforhodamin B acid form (SRB) [Sigma, 341738-5g] reconstituted in 1% Acetic Acid as the stain is added straight after fixation without any washing step which limits cell detachment. Plates with stained colonies were scanned with the GelCount colony counter (Oxford OPTRONIX) at 600 dpi resolution. Colony formation was scored by quantifying the total optical density measured with ImageJ software, using a 24-well plate Region of Interest (ROI) mask (CFU, colony formation units). Data analysis was performed by normalisation on the vehicle treated of the respective plate set as = 100. Data were normalised to respective vehicle-control (set as = 100) no IR, plotted and $IC_{50}$ were calculated with Prism GraphPad software. In the represented plots error bars are mean ± SD.

<u>**U87MG xenograft studies**</u>

U87MG cells (2.5 million in serum-free MEM medium) were implanted subcutaneously (SC) into the dorsal flank of female Nude mice (weight greater than 18 g). Tumours were measured (length x width) two times weekly by bilateral Vernier calliper measurements and tumour volume was calculated using elliptical formula ($\pi$/6×width×width×length). Animal bodyweight and tumour condition were monitored throughout the study. Mice were randomised into treatment groups when mean tumour volume reached approximately 0.2 $cm^3$.

Animals were treated orally (PO) from the day after the randomisation. Control animals were treated with vehicle (water/ methane sulfonic acid (MSA) pH3-3.2) once daily for 10 days (QD x10). PARP inhibitor in water / MSA pH3-3.2 was dosed in the monotherapy and combination groups at 3 mg/kg QD x10 or QD x33, respectively. Temozolomide (TMZ) in OraPlus Perrigo was dosed in the monotherapy group at 25 mg/kg QD on days 1-5 and 29-33, and in monotherapy and combination group at 6.25 mg/kg QD on days 1-5 and 29-33.

Tumour growth inhibition from start of treatment was assessed by comparison of the mean change in tumour volume (TV) of the control and treated groups and represented as percent tumour growth inhibition (TGI, when tumour volume (TV) ≥ starting TV) or tumour regression (reg, when TV < starting TV). Statistical significance was evaluated using a one-tailed t test. Statistical significance is indicated as follows: * p≤0.05, ** p≤0.01, *** p≤0.001.

**Treatment using PARP inhibitor in ATRX mutant GBM models**

We investigated the Compound **20** for monotherapy activity in IDHmt and ATRXmt cell lines (SJ-G2 and U87 GBM models). ATRXmt GBM SJ-G2 showed sensitivity to Compound **20** monotherapy in a clinically achievable dose range and the addition of IDH1 mutation did not further the sensitivity of this model. SJ-G2 ATRXmt model exhibited about 40-fold higher sensitivity to Compound **20** compared to the ATRXwt U87 model. The results are provided below in **Table 5** and shown in **Figure 7.**

**Table 5:** Monotherapy treatment in ATRX mutant GBM models

| Cell line | Compound 20 IC50 (μM) | Fold change IC50 compared to U87 |
|---|---|---|
| U87 IDH1wt ATRXwt | >100 | |
| U87 IDH1 mt ATRXwt | >100 | |
| SJ-G2 IDH1wt ATRXmt | 2.341 | ~ 43 |
| SJ-G2 IDH1 mt ATRXmt | 1.748 | ~ 57 |

These results demonstrate that Compound **20** has potent monotherapy activity in ATRX mutant cell lines and that the additional presence of an IDH1 mutation did not further sensitivity of this model.

**Combination treatment using PARP inhibitor and TMZ**

The combination of Compound **20** and TMZ was evaluated in IDH1 wild type (IDH1wt) and IDH1 mutant (IDH1mt) glioblastoma (GBM) cells. Compound **20** shows increased synergy with TMZ at multiple concentrations of Compound **20,** which are well above the IC95 for PARylation inhibition (0.008 μM; data not shown) of Compound **20** in the U87 isogenic pair **(Figure 8).** Importantly, Compound **20** shows potent trapping of PARP1 at concentrations as low as 10 nM while sparing PARP2 (data not shown), suggesting that the combination benefit observed here with Compound **20** and TMZ is driven by the catalytic inhibition of PARP1 as well as increased DNA damage due to trapped PARP1 on the DNA.

Furthermore, Compound **20** synergises with TMZ at a 10 fold lower concentration (10 μM in IDH1wt compared to 1 μM in IDH1mt) in the IDH1mt GBM line compared to the wild type, suggesting that IDH1 mt GBM is more sensitive to TMZ treatment.

Furthermore, the Compound **20** and TMZ combination was investigated in SJ-G2 GBM isogenic IDH1mt and IDH1mt models, which are ATRX mutant and p53 mutant. These IDH1wt and IDH1mt SJG2 models more closely represent the GBM patient population (Ohba, Kuwahara, Yamada, Abe, & Hirose, 2020). Importantly, Compound **20** significantly enhanced the TMZ effect in the SJ-G2 isogenic pairs at multiple concentrations of the PARP inhibitor even as low as 10 nM **(Figure 9).** In addition, TMZ was significantly synergistic with Compound **20** at the same concentration in the IDH1wt and IDH1mt model.

The anti-tumour efficacy of Compound **20** in combination with TMZ was also investigated in a U87MG xenograft model using the method described above. TMZ dosed at 6.25mg/kg QD on days 1-5 and 29-33 resulted in 28% tumour regression (2/8 tumours regressed), while its combination with Compound **20** (3 mg/kg QD) delivered further anti-tumour benefit of 95% tumour regression (8/8 tumours regressed) **(Figure 10A).** The difference was statistically significant. The effect of the higher dose of TMZ (25 mg/kg QD on days 1-5 and 29-33), 92% tumour regression (7/8 tumours regressed), was comparable to the combination treatment effect. Body weight loss and animal welfare was monitored on treatment as a surrogate for toxicity. The high dose of temozolomide (25 mg/kg) resulted in more body weight loss than the combination of the lower dose of temozolomide (6.25 mg/kg) with 3mg/kg of Compound **20 (Figure 10B).**

**Combination treatment using PARP inhibitor and VAL-083**

The combination of Compound **20** and alkylating agent VAL-083 was evaluated in U87 IDH1wt and IDH1mt isogenic pairs. The results shown in **Figure 11** demonstrate that Compound **20** synergises with VAL-083 in these cells. Notably, Compound **20** combination benefit with VAL-083 is not dependent on the IDH1 mutational status of the GBM model as there was no dose reduction of the alkylating agent required in the IDH1 mt model to synergise with the PARP inhibitor.

Similarly, the ATRX mutant SJ-G2 IDH1wt and IDH1mt isogenic pairs shows enhanced combination benefit of Compound **20** and VAL-083 which is independent of IDH1mt status **(Figure 12).** These results also demonstrate that 1 μM of Compound **20** has activity in ATRX mutant cells lines in the absence of VAL-083. This is consistent with the results reported above and shown in **Figure 7,** demonstrating that Compound **20** has a monotherapy effect across multiple concentrations. Thus, as well as demonstrating the potential suitability of this PARP inhibitor in a monotherapy (i.e. without chemotherapy or radiotherapy) this suggests the PARP inhibitor has a tumour reducing effect that is independent of its ability to potentiate the efficacy of alkylating chemotherapeutic agents such as VAL-083 and TMZ.

**Combination treatment using PARP inhibitor and ionizing radiation therapy**

H3K27M-mutant diffuse midline gliomas are paediatric gliomas for which radiotherapy is the standard of care treatment. However, the prognosis of these types of tumours is relatively poor. In this study, the combination of ionizing radiation and Compound **20** was evaluated in a model of H3K27M-mutant glioma.

The results shown in **Figure 13** demonstrate that the studied PARP inhibitor shows enhanced combination benefit with ionizing radiation at multiple doses of the inhibitor together with a reduced clinically relevant dose of 0.9 Gy (clinically relevant dose is 1.8 Gy). Importantly, the IC95 for Compound **20** induced PARylation inhibition in the paediatric model is 0.004 μM, therefore the compound is within its maximal activity range. These results suggest that the disclosed PARP inhibitors may be used in combination with ionizing radiation to treat challenging gliomas such as H3K27M-mutant glioma. Furthermore, the results suggest that it may be possible treat tumours using the PARP inhibitors with reduced levels of ionizing radiation, which may reduce the harmful effects associated with exposure to high doses of radiation.

**References**

A number of publications are cited above in order to more fully describe and disclose the invention and the state of the art to which the invention pertains. Full citations for these references are provided below.


WO2004/080976

EP 699 754

EP 705 903

American Cancer Society (2021). Treating Neuroblastoma. American Cancer Society website. https://www.cancer.org/content/dam/CRC/PDF/Public/8761.00.pdf Bai P. Biology of poly(ADP-ribose) polymerases: the factotums of cell maintenance. Mol Cell 2015;58:947-958, doi: 10.1016/j.molcel.2015.01.034

Bartkova J, Hamerlik P, Stockhausen MT, Ehrmann J, Hlobilkova A, Laursen H, Kalita O, Kolar Z, Poulsen HS,

Broholm H, Lukas J, Bartek J. Replication stress and oxidative damage contribute to aberrant constitutive activation of DNA damage signalling in human gliomas. Oncogene. 2010 Sep 9;29(36):5095-5102. doi: 10.1038/onc.2010.249. Epub 2010 Jun 28. PMID: 20581868.

Borisy, A. A., Elliott, P. J., Hurst, N. W., Lee, M. S., Lehar, J., Price, E. R., ... Keith, C. T. (2003). Systematic discovery of multicomponent therapeutics. Proc Natl Acad Sci U S A, 100(13), 7977-7982. doi:10.1073/pnas.1337088100

Chappnis, P.O. and Foulkes, W.O., Cancer Treat Res, 107, 29-59 (2002), doi: 10.1007/978-1-4757-3587-1_2

Di Veroli, G. Y., Fornari, C., Wang, D., Mollard, S., Bramhall, J. L., Richards, F. M., & Jodrell, D. I. (2016). Combenefit: an interactive platform for the analysis and visualization of drug combinations. Bioinformatics, 32(18), 2866-2868. doi:10.1093/bioinformatics/btw230

George SL, Lorenzi F, King D, Hartlieb S, Campbell J, Pemberton H, Toprak UH, Barker K, Tall J, da Costa BM, van den Boogaard ML, Dolman MEM, Molenaar JJ, Bryant HE, Westermann F, Lord CJ, Chesler L. Therapeutic vulnerabilities in the DNA damage response for the treatment of ATRX mutant neuroblastoma. EBioMedicine. 2020 Sep;59:102971. doi: 10.1016/j.ebiom.2020.102971. Epub 2020 Aug 23. PMID: 32846370; PMCID: PMC7452577

Gupta, S. K., Smith, E. J., Mladek, A. C., Tian, S., Decker, P. A., Kizilbash, S. H., . . . Sarkaria, J. N. (2018). PARP Inhibitors for Sensitization of Alkylation Chemotherapy in Glioblastoma: Impact of Blood-Brain Barrier and Molecular Heterogeneity. Front Oncol, 8, 670. doi:10.3389/fonc.2018.00670 Haase, S., Garcia-Fabiani, M. B., Carney, S., Altshuler, D., Núñez, F. J., Méndez, F. M., Núñez, F., Lowenstein, P. R., & Castro, M. G. (2018). Mutant ATRX: uncovering a new therapeutic target for glioma. Expert opinion on therapeutic targets, 22(7), 599-613. doi: 10.1080/14728222.2018.1487953 Hartmann C, Meyer J, Balss J, Capper D, Mueller W, Christians A, Felsberg J, Wolter M, Mawrin C, Wick W, Weller M, Herold-Mende C, Unterberg A, Jeuken JW, Wesseling P, Reifenberger G, von Deimling A. Type and frequency of IDH1 and IDH2 mutations are related to astrocytic and oligodendroglial differentiation and age: a study of 1,010 diffuse gliomas. Acta Neuropathol. 2009 Oct;118(4):469-74. doi: 10.1007/s00401-009-0561-9. Epub 2009 Jun 25. PMID: 19554337.

Higuchi, F., Nagashima, H., Ning, J., Koerner, M. V. A., Wakimoto, H., & Cahill, D. P. (2020). Restoration of Temozolomide Sensitivity by PARP Inhibitors in Mismatch Repair Deficient Glioblastoma is Independent of Base Excision Repair. Clin Cancer Res, 26(7), 1690-1699. doi:10.1158/1078-0432.CCR-19-2000

Himes, B. T., Zhang, L., & Daniels, D. J. (2019). Treatment Strategies in Diffuse Midline Gliomas With the H3K27M Mutation: The Role of Convection-Enhanced Delivery in Overcoming Anatomic Challenges. Front Oncol, 9, 31. doi:10.3389/fonc.2019.00031

Hughes-Davies, et al., Cell, 115, 523-535 (2003) doi: 10.1016/s0092-8674(03)00930-9 Janatova M., et al., Neoplasma, 50(4), 246-250 (2003)

Jancarkova, N., Ceska Gynekol., 68(1), 11-6 (2003), PMID: 12708108

Jannetti, S. A., Zeglis, B. M., Zalutsky, M. R., & Reiner, T. (2020). Poly(ADP-Ribose)Polymerase (PARP) Inhibitors and Radiation Therapy. Front. Pharmacol., 11:170. doi: 10.3389/fphar.2020.00170 Jimenez-Alcazar, M., Curiel-Garcia, A., Nogales, P., Perales-Paton, J., Schuhmacher, A. J., Galan-Ganga, M., ... Squatrito, M. (2021). Dianhydrogalactitol Overcomes Multiple Temozolomide Resistance Mechanisms in Glioblastoma. Mol Cancer Ther, 20(6), 1029-1038. doi:10.1158/1535-7163.MCT-20-0319

Koschmann, C., Calinescu, A. A., Nunez, F. J., Mackay, A., Fazal-Salom, J., Thomas, D., Mendez, F., Kamran, N., Dzaman, M., Mulpuri, L., Krasinkiewicz, J., Doherty, R., Lemons, R., Brosnan-Cashman, J. A., Li, Y., Roh, S., Zhao, L., Appelman, H., Ferguson, D., Gorbunova, V., ... Castro, M. G. (2016). ATRX loss promotes tumor growth and impairs nonhomologous end joining DNA repair in glioma. Science translational medicine, 8(328), 328ra28. doi: 10.1126/scitranslmed.aac8228

Mansouri, A., Hachem, L. D., Mansouri, S., Nassiri, F., Laperriere, N. J., Xia, D., Lindeman, N. I., Wen, P. Y., Chakravarti, A., Mehta, M. P., Hegi, M. E., Stupp, R., Aldape, K. D., & Zadeh, G. (2019). MGMT promoter methylation status testing to guide therapy for glioblastoma: refining the approach based on emerging evidence and current challenges. Neuro-oncology, 21(2), 167-178. doi:10.1093/neuonc/noy132

Martinez-Ricarte F, Mayor R, Martínez-Sáez E, Rubio-Pérez C, Pineda E, Cordero E, Cicuéndez M, Poca MA, López-Bigas N, Ramon Y Cajal S, Vieito M, Carles J, Tabernero J, Vivancos A, Gallego S, Graus F, Sahuquillo J, Seoane J. Molecular Diagnosis of Diffuse Gliomas through Sequencing of Cell-Free Circulating Tumor DNA from Cerebrospinal Fluid. Clin Cancer Res. 2018 Jun 15;24(12):2812-2819. doi: 10.1158/1078-0432.CCR-17-3800. Epub 2018 Apr 3. PMID: 29615461.

Murai, J., Huang, S. Y., Das, B. B., Renaud, A., Zhang, Y., Doroshow, J. H., . . . Pommier, Y. (2012). Trapping of PARP1 and PARP2 by Clinical PARP Inhibitors. Cancer Res, 72(21), 5588-5599. doi:10.1158/0008-5472.CAN-12-2753

Murai, J., Zhang, Y., Morris, J., Ji, J., Takeda, S., Doroshow, J. H., & Pommier, Y. (2014). Rationale for poly(ADP-ribose) polymerase (PARP) inhibitors in combination therapy with camptothecins or temozolomide based on PARP trapping versus catalytic inhibition. J Pharmacol Exp Ther, 349(3), 408-416. doi:10.1124/jpet.113.210146

Nabors, LB et al., (2020). Central Nervous System Cancers, Version 3.2020, NCCN Clinical Practice Guidelines in

Oncology. Journal of the National Comprehensive Cancer Network, 18(11), 1537-1570. DOI: 10.6004/jnccn.2020.0052.
J Neuhausen, S.L. and Ostrander, E.A., Genet. Test, 1, 75-83 (1997), doi:10.1089/gte.1997.1.75
Ohba, S., Kuwahara, K., Yamada, S., Abe, M., & Hirose, Y. (2020). Correlation between IDH, ATRX, and TERT promoter mutations in glioma. Brain Tumor Pathol, 37(2), 33-40. doi:10.1007/s10014-020-00360-4
Patel, M. M., Expert Opin. Drug Deliv., 2011, 8(10), 1247-1258, doi: 10.1517/17425247.2011.597739 Reisz, J. A., Bansal, N., Qian, J., Zhao, W., & Furdui, C. M. (2014). Effects of ionizing radiation on biological molecules--mechanisms of damage and emerging methods of detection. Antioxid Redox Signal, 21(2), 260-292. doi:10.1089/ars.2013.5489
Singh, N., Miner, A., Hennis, L., & Mittal, S. (2021). Mechanisms of temozolomide resistance in glioblastoma - a comprehensive review. Cancer Drug Resist, 4, 17-43. doi:10.20517/cdr.2020.79 SongTao, Q., Lei, Y., Si, G., YanQing, D., HuiXia, H., XueLin, Z., . . . Fei, Y. (2012). IDH mutations predict longer survival and response to temozolomide in secondary glioblastoma. Cancer Sci, 103(2), 269-273. doi:10.1111/j.1349-7006.2011.02134.x
Stupp, R., Brada, M., van den Bent, M. J., Tonn, J. C., Pentheroudakis, G., & Group, E. G. W. (2014). High-grade glioma: ESMO Clinical Practice Guidelines for diagnosis, treatment and follow-up. Ann Oncol, 25 Suppl 3, iii93-101. doi:10.1093/annonc/mdu050
Stupp, R., Tonn, J. C., Brada, M., Pentheroudakis, G., & Group, E. G. W. (2010). High-grade malignant glioma: ESMO Clinical Practice Guidelines for diagnosis, treatment and follow-up. Ann Oncol, 21 Suppl 5, v190-193. doi:10.1093/annonc/mdq187
Tan, X., Hu, L., Luquette, L. J., 3rd, Gao, G., Liu, Y., Qu, H., . . . Elledge, S. J. (2012). Systematic identification of synergistic drug pairs targeting HIV. Nat Biotechnol, 30(11), 1125-1130.
doi:10.1038/nbt.2391
van Vuurden DG, Hulleman E, Meijer OL, Wedekind LE, Kool M, Witt H, Vandertop PW, Würdinger T, Noske DP, Kaspers GJ, Cloos J. PARP inhibition sensitizes childhood high grade glioma, medulloblastoma and ependymoma to radiation. Oncotarget. 2011 Dec;2(12):984-996. doi: 10.18632/oncotarget.362. PMID: 22184287; PMCID: PMC3282104.
Weller M, Stupp R, Reifenberger G, Brandes AA, van den Bent MJ, Wick W, Hegi ME. MGMT promoter methylation in malignant gliomas: ready for personalized medicine? Nat Rev Neurol. 2010 Jan;6(1):39-51. doi: 10.1038/nrneurol.2009.197. Epub 2009 Dec 8. PMID: 19997073.

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

## Claims

1. A PARP inhibitor for use in a method of treating a brain tumour or a neuroblastoma in a patient in need thereof, the method comprising administering to the patient a therapeutically effective amount of a poly(ADP-ribose) polymerase (PARP) inhibitor,

   wherein the brain tumour or neuroblastoma comprises an alpha thalassemia/mental retardation syndrome X-linked (ATRX) deficient phenotype,
   wherein the PARP inhibitor is a compound of Formula I

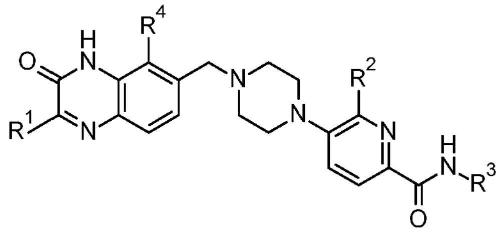

(I)

   wherein:

   $R^1$ is independently selected from H, $C_{1-4}$ alkyl, $C_{3-6}$ cycloalkyl, $C_{1-4}$ fluoroalkyl, and $C_{1-4}$ alkyloxy;
   $R^2$ is independently selected from H, halo, $C_{1-4}$ alkyl, and $C_{1-4}$ fluoroalkyl; and
   $R^3$ is H or $C_{1-4}$ alkyl;

$R^4$ is halo or $C_{1-4}$ alkyl,
or a pharmaceutically acceptable salt thereof.

**2.** The PARP inhibitor for use according to claim 1, wherein the brain tumour or neuroblastoma further comprises an isocitrate dehydrogenase 1 and/or 2 (IDH1 and/or IDH2) deficient phenotype.

**3.** The PARP inhibitor for use according to claim 1, wherein the brain tumour or neuroblastoma does not comprise an IDH1 deficient phenotype.

**4.** The PARP inhibitor for use according to any one of claims 1 to 3, wherein the brain tumour or neuroblastoma comprises $O^6$-methylguanine-DNA methyltransferase (MGMT) promoter methylation.

**5.** The PARP inhibitor for use according to any one of the preceding claims, further comprising a step of diagnosing the patient as having the brain tumour or neuroblastoma comprising the ATRX deficient phenotype prior to administering the PARP inhibitor.

**6.** The PARP inhibitor for use according to claim 5, wherein the step of diagnosing the patient further comprises determining whether the brain tumour or neuroblastoma further comprises the IDH1 or IDH2 deficient phenotype and/or MGMT promoter methylation.

**7.** The PARP inhibitor for use according to any one of the preceding claims, wherein the PARP inhibitor is administered to the patient in combination with

i) an alkylating chemotherapeutic agent; and/or
ii) ionizing radiation.

**8.** A PARP inhibitor for use in a method of treating a brain tumour or a neuroblastoma in a patient in need thereof, the method comprising administering to the patient a therapeutically effective amount of a poly(ADP-ribose) polymerase (PARP) inhibitor, and

i) an alkylating chemotherapeutic agent; and/or
ii) ionizing radiation administered at a dose of 10 Gy or above,

wherein the PARP inhibitor is a compound of Formula I

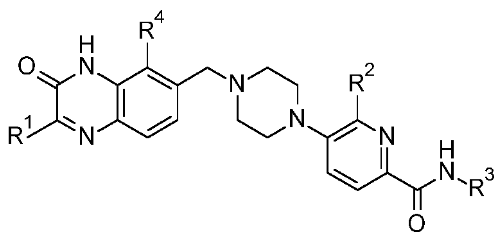

(I)

wherein:

$R^1$ is independently selected from H, $C_{1-4}$ alkyl, $C_{3-6}$ cycloalkyl, $C_{1-4}$ fluoroalkyl, and $C_{1-4}$ alkyloxy;
$R^2$ is independently selected from H, halo, $C_{1-4}$ alkyl, and $C_{1-4}$ fluoroalkyl; and
$R^3$ is H or $C_{1-4}$ alkyl;
$R^4$ is halo or $C_{1-4}$ alkyl,
or a pharmaceutically acceptable salt thereof.

**9.** The PARP inhibitor for use according to claim 8, wherein the brain tumour or neuroblastoma comprises:

(i) an alpha thalassemia/mental retardation syndrome X-linked (ATRX) deficient phenotype;
(ii) an isocitrate dehydrogenase 1 or 2 (IDH1 or IDH2) deficient phenotype; and/or
(iii) $O^6$-methylguanine-DNA methyltransferase (MGMT) promoter methylation.

**10.** The PARP inhibitor for use according to any one of claims 7 to 9, wherein the alkylating chemotherapeutic agent is temozolomide (TMZ) or dianhydrogalactitol (VAL-083).

**11.** The PARP inhibitor for use according any one of claims 1 to 10 wherein the PARP inhibitor is selected from selected from:

5-[4-[(2,5-dimethyl-3-oxo-4H-quinoxalin-6-yl)methyl]piperazin-1-yl]-6-fluoro-N-methyl-pyridine-2-carboxamide,
5-[4-[(2,5-dimethyl-3-oxo-4H-quinoxalin-6-yl)methyl]piperazin-1-yl]-N-methyl-pyridine-2-carboxamide,
6-chloro-5-[4-[(2,5-dimethyl-3-oxo-4H-quinoxalin-6-yl)methyl]piperazin-1-yl]-N-methyl-pyridine-2-carboxamide,
5-[4-[(2,5-dimethyl-3-oxo-4H-quinoxalin-6-yl)methyl]piperazin-1-yl]-N,6-dimethyl-pyridine-2-carboxamide,
5-[4-[(2,5-dimethyl-3-oxo-4H-quinoxalin-6-yl)methyl]piperazin-1-yl]-6-fluoro-pyridine-2-carboxamide,
5-[4-[(5-fluoro-2-methyl-3-oxo-4H-quinoxalin-6-yl)methyl]piperazin-1-yl]-6-methyl-pyridine-2-carboxamide,
5-[4-[(2,5-dimethyl-3-oxo-4H-quinoxalin-6-yl)methyl]piperazin-1-yl]-6-methyl-pyridine-2-carboxamide,
6-chloro-5-[4-[(5-chloro-2-ethyl-3-oxo-4H-quinoxalin-6-yl)methyl]piperazin-1-yl]-N-methyl-pyridine-2-carboxamide,
5-[4-[(5-chloro-2-ethyl-3-oxo-4H-quinoxalin-6-yl)methyl]piperazin-1-yl]-6-fluoro-N-methyl-pyridine-2-carboxamide,
5-[4-[(5-chloro-2-ethyl-3-oxo-4H-quinoxalin-6-yl)methyl]piperazin-1-yl]-N-methyl-pyridine-2-carboxamide,
5-[4-[(5-chloro-2-ethyl-3-oxo-4H-quinoxalin-6-yl)methyl]piperazin-1-yl]-N,6-dimethyl-pyridine-2-carboxamide,
6-fluoro-5-[4-[[5-fluoro-2-[(1S and 1R)-1-fluoroethyl]-3-oxo-4H-quinoxalin-6-yl]methyl]piperazin-1-yl]-N-methyl-pyridine-2-carboxamide,
5-[4-[[5-fluoro-2-[(1S and 1R)-1-fluoroethyl]-3-oxo-4H-quinoxalin-6-yl]methyl]piperazin-1-yl]-N,6-dimethyl-pyridine-2-carboxamide,
5-[4-[(5-chloro-2-methyl-3-oxo-4H-quinoxalin-6-y)methyl]piperazin-1-yl]-N-methyl-pyridine-2-carboxamide,
5-[4-[(5-chloro-2-methyl-3-oxo-4H-quinoxalin-6-yl)methyl]piperazin-1-yl]-6-fluoro-N-methyl-pyridine-2-carboxamide,
5-[4-[(5-chloro-2-methyl-3-oxo-4H-quinoxalin-6-yl)methyl]piperazin-1-yl]-N,6-dimethyl-pyridine-2-carboxamide,
5-[4-[[2-(1,1-difluoroethyl)-5-fluoro-3-oxo-4H-quinoxalin-6-yl]methyl]piperazin-1-yl]-N,6-dimethylpyridine-2-carboxamide,
6-fluoro-5-[4-[(5-fluoro-2-methyl-3-oxo-4H-quinoxalin-6-yl)methyl]piperazin-1-yl]-N-methyl-pyridine-2-carboxamide,
6-(difluoromethyl)-5-[4-[(5-fluoro-2-methyl-3-oxo-4H-quinoxalin-6-yl)methyl]piperazin-1-yl]-N-methylpyridine-2-carboxamide,
6-fluoro-5-[4-[(5-fluoro-2-methyl-3-oxo-4H-quinoxalin-6-yl)methyl]piperazi n-1-yl]pyridine-2-carboxamide,
5-[4-[(2-ethyl-5-fluoro-3-oxo-4H-quinoxalin-6-yl)methyl]piperazin-1-yl]-N,6-dimethyl-pyridine-2-carboxamide,
6-(difluoromethyl)-5-[4-[(2-ethyl-5-fluoro-3-oxo-4H-quinoxalin-6-yl)methyl]piperazin-1-yl]-N-methylpyridine-2-carboxamide,
5-[4-[(2-ethyl-5-fluoro-3-oxo-4H-quinoxalin-6-yl)methyl]piperazin-1-yl]pyridine-2-carboxamide,
5-[4-[(2-ethyl-5-fluoro-3-oxo-4H-quinoxalin-6-yl)methyl]piperazin-1-yl]-6-methyl-pyridine-2-carboxamide,
5-[4-[(2-ethyl-5-fluoro-3-oxo-4H-quinoxalin-6-yl)methyl]piperazin-1-yl]-6-fluoro-N-methyl-pyridine-2-carboxamide,
6-chloro-5-[4-[(2-ethyl-5-fluoro-3-oxo-4H-quinoxalin-6-yl)methyl]piperazin-1-yl]-N-methyl-pyridine-2-carboxamide,
5-[4-[(2-ethyl-5-fluoro-3-oxo-4H-quinoxalin-6-yl]methyl]piperazin-1-yl]-N-methyl-pyridine-2-carboxamide,
6-chloro-5-[4-[(5-fluoro-2-methyl-3-oxo-4H-quinoxalin-6-yl)methyl]piperazin-1-yl]-N-methyl-pyridine-2-carboxamide,
5-[4-[(5-fluoro-2-methyl-3-oxo-4H-quinoxalin-6-yl)methyl]piperazin-1-yl]-N,6-dimethyl-pyridine-2-carboxamide,
5-[4-[(5-fluoro-2-methyl-3-oxo-4H-quinoxalin-6-yl)methyl]piperazin-1-yl]-N-methyl-pyridine-2-carboxamide,
5-[4-[(5-fluoro-3-oxo-4H-quinoxalin-6-yl)methyl]piperazin-1-yl]-N-methyl-pyridine-2-carboxamide,
6-chloro-5-[4-[(5-fluoro-3-oxo-4H-quinoxalin-6-yl)methyl]piperazin-1-yl]-N-methyl-pyridine-2-carboxamide,
5-[4-[(5-fluoro-3-oxo-4H-quinoxalin-6-yl)methyl]piperazin-1-yl]-N,6-dimethyl-pyridine-2-carboxamide,
6-fluoro-5-[4-[(5-fluoro-3-oxo-4H-quinoxalin-6-yl)methyl]piperazin-1-yl]-N-methyl-pyridine-2-carboxamide,
5-[4-[[2-(difluoromethyl)-5-fluoro-3-oxo-4H-quinoxalin-6-yl]methyl]piperazin-1-yl]-N,6-dimethylpyridine-2-carboxamide,

5-[4-[(5-fluoro-2-methoxy-3-oxo-4H-quinoxalin-6-yl)methyl]piperazin-1-yl]-N-methyl-pyridine-2-carboxamide,
6-fluoro-5-[4-[(5-fluoro-2-methoxy-3-oxo-4H-quinoxalin-6-yl)methyl]piperazin-1-yl]-N-methyl-pyridine-2-carboxamide,
5-[4-[(5-fluoro-2-methoxy-3-oxo-4H-quinoxalin-6-yl)methyl]piperazin-1-yl]-N,6-dimethyl-pyridine-2-carboxamide,
6-chloro-5-[4-[(5-fluoro-2-methoxy-3-oxo-4H-quinoxalin-6-yl)methyl]piperazin-1-yl]-N-methyl-pyridine-2-carboxamide,
5-[4-[(2-ethyl-5-methyl-3-oxo-4H-quinoxalin-6-yl)methyl]piperazin-1-yl]-N,6-dimethyl-pyridine-2-carboxamide,
5-[4-[(2-ethyl-5-methyl-3-oxo-4H-quinoxalin-6-yl)methyl]piperazin-1-yl]-6-fluoro-N-methyl-pyridine-2-carboxamide,
5-[4-[(2-ethyl-5-methyl-3-oxo-4H-quinoxalin-6-yl)methyl]piperazin-1-yl]-N-methyl-pyridine-2-carboxamide,
N-ethyl-6-fluoro-5-[4-[(5-fluoro-2-methyl-3-oxo-4H-quinoxalin-6-yl)methyl]piperazin-1-yl]pyridine-2-carboxamide,
N-ethyl-5-[4-[(5-fluoro-2-methyl-3-oxo-4H-quinoxalin-6-yl]methyl]piperazin-1-yl]-6-methyl-pyridine-2-carboxamide,
5-[4-[[5-fluoro-3-oxo-2-(trifluoromethyl)-4H-quinoxalin-6-yl]methyl]piperazin-1-yl]-N,6-dimethylpyridine-2-carboxamide,
6-fluoro-5-[4-[[5-fluoro-3-oxo-2-(trifluoromethyl)-4H-quinoxalin-6-yl]methyl]piperazin-1-yl]-N-methylpyridine-2-carboxamide,
6-chloro-5-[4-[[5-fluoro-3-oxo-2-(trifluoromethyl)-4H-quinoxalin-6-yl]methyl]piperazin-1-yl]-N-methylpyridine-2-carboxamide,
5-[4-[[5-fluoro-3-oxo-2-(trifluoromethyl)-4H-quinoxalin-6-yl]methyl]piperazin-1-yl]-N-methyl-pyridine-2-carboxamide,
6-fluoro-5-[4-[(5-fluoro-2-isopropyl-3-oxo-4H-quinoxalin-6-yl)methyl]piperazin-1-yl]-N-methyl-pyridine-2-carboxamide,
5-[4-[(5-fluoro-2-isopropyl-3-oxo-4H-quinoxalin-6-yl)methyl]piperazin-1-yl]-N,6-dimethyl-pyridine-2-carboxamide,
5-[4-[(5-fluoro-2-isopropyl-3-oxo-4H-quinoxalin-6-yl)methyl]piperazin-1-yl]-N-methyl-pyridine-2-carboxamide,
5-[4-[(2-cyclopropyl-5-fluoro-3-oxo-4H-quinoxalin-6-yl)methyl]piperazin-1-yl]-6-fluoro-N-methylpyridine-2-carboxamide,
5-[4-[(2-cyclopropyl-5-fluoro-3-oxo-4H-quinoxalin-6-yl)methyl]piperazin-1-yl]-N,6-dimethyl-pyridine-2-carboxamide,
5-[4-[(2-cyclopropyl-5-fluoro-3-oxo-4H-quinoxalin-6-yl)methyl]piperazin-1-yl]-N-methyl-pyridine-2-carboxamide,
5-[4-[(2-methoxy-5-methyl-3-oxo-4H-quinoxalin-6-yl)methyl]piperazin-1-yl]-N,6-dimethyl-pyridine-2-carboxamide,
6-fluoro-5-[4-[(2-methoxy-5-methyl-3-oxo-4H-quinoxalin-6-yl)methyl]piperazin-1-yl]-N-methyl-pyridine-2-carboxamide,
6-(difluoromethyl)-5-[4-[(2-methoxy-5-methyl-3-oxo-4H-quinoxalin-6-yl)methyl]piperazin-1-yl]-N-methyl-pyridine-2-carboxamide, and
6-(difluoromethyl)-5-[4-[(2,5-dimethyl-3-oxo-4H-quinoxalin-6-yl)methyl]piperazin-1-yl]-N-methylpyridine-2-carboxamide,
or a pharmaceutically acceptable salt thereof.

**12.** The PARP inhibitor for use according to any one of claims 1 to 10 wherein the PARP inhibitor is:
6-fluoro-5-[4-[(5-fluoro-2-methyl-3-oxo-4H-quinoxalin-6-yl)methyl]piperazin-1-yl]-N-methyl-pyridine-2-carboxamide, or a pharmaceutically acceptable salt thereof.

**13.** The PARP inhibitor for use according to any one of claims 1 to 10 wherein the PARP inhibitor is:
6-fluoro-5-[4-[(5-fluoro-2-methyl-3-oxo-4H-quinoxalin-6-yl)methyl]piperazin-1-yl]-N-methyl-pyridine-2-carboxamide.

**14.** The PARP inhibitor for use according to any one of the claims 1 to 13, wherein the brain tumour is a glioma or an ependymoma.

**15.** The PARP inhibitor for use according to claim 14, wherein the brain tumour is a glioma.

**Patentansprüche**

1. PARP-Inhibitor zur Verwendung in einem Verfahren zum Behandeln eines Gehirntumors oder eines Neuroblastoms bei einem Patienten, der dies benötigt, das Verfahren umfassend ein Verabreichen einer therapeutisch wirksamen Menge eines Poly(ADP-Ribose)-Polymerase-Inhibitors (PARP-Inhibitor) an den Patienten,

   wobei der Hirntumor oder das Neuroblastom einen Alpha-Thalassämie/Mentales-Retardierungssyndrom-X-chromosomalen-Mangelphänotyp (ATRX-Mangelphänotyp) umfasst,
   wobei der PARP-Inhibitor eine Verbindung der Formel I ist

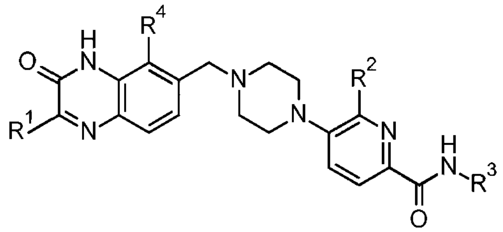

(I)

   wobei:

   $R^1$ unabhängig ausgewählt ist aus H, $C_{1-4}$-Alkyl, $C_{3-6}$-Cycloalkyl, $C_{1-4}$-Fluoralkyl und $C_{1-4}$-Alkyloxy;
   $R^2$ unabhängig ausgewählt ist aus H, Halogen, $C_{1-4}$-Alkyl und $C_{1-4}$-Fluoralkyl; und
   $R^3$ H oder $C_{1-4}$-Alkyl ist;
   $R^4$ Halogen oder $C_{1-4}$-Alkyl ist,
   oder ein pharmazeutisch unbedenkliches Salz davon.

2. PARP-Inhibitor zur Verwendung nach Anspruch 1, wobei der Hirntumor oder das Neuroblastom ferner einen Isocitrat-Dehydrogenase-1- und/oder 2-Mangelphänotyp (IDH1- und/oder IDH2-Mangelphänotyp) umfasst.

3. PARP-Inhibitor zur Verwendung nach Anspruch 1, wobei der Hirntumor oder das Neuroblastom keinen IDH1-Mangelphänotyp umfasst.

4. PARP-Inhibitor zur Verwendung nach einem der Ansprüche 1 bis 3, wobei der Hirntumor oder das Neuroblastom $Q^6$-Methylguanin-DNA-Methyltransferase-Promotormethylierung (MGMT-Promotormethylierung) umfasst.

5. PARP-Inhibitor zur Verwendung nach einem der vorstehenden Ansprüche, ferner umfassend einen Schritt, bei dem vor dem Verabreichen des PARP-Inhibitors bei dem Patienten ein Hirntumor oder Neuroblastom, umfassend den ATRX-Mangelphänotyp, diagnostiziert wird.

6. PARP-Inhibitor zur Verwendung nach Anspruch 5, wobei der Schritt des Diagnostizierens des Patienten ferner das Bestimmen umfasst, ob der Hirntumor oder das Neuroblastom ferner den IDH1- oder IDH2-Mangelphänotyp und/oder eine MGMT-Promotormethylierung umfasst.

7. PARP-Inhibitor zur Verwendung nach einem der vorstehenden Ansprüche, wobei der PARP-Inhibitor dem Patienten in Kombination verabreicht wird mit

   i. einem alkylierenden chemotherapeutischen Mittel; und/oder
   ii. ionisierender Strahlung.

8. PARP-Inhibitor zur Verwendung in einem Verfahren zum Behandeln eines Gehirntumors oder eines Neuroblastoms bei einem Patienten, der dies benötigt, das Verfahren umfassend das Verabreichen einer therapeutisch wirksamen Menge eines Poly(ADP-Ribose)-Polymerase-Inhibitors (PARP-Inhibitor) an den Patienten, und

   i. ein alkylierendes chemotherapeutisches Mittel; und/oder
   ii. ionisierende Strahlung, verabreicht mit einer Dosis von 10 Gy oder mehr, wobei der PARP-Inhibitor eine Verbindung der Formel I ist

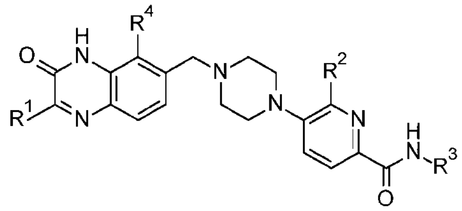

(I)

wobei:

$R^1$ unabhängig ausgewählt ist aus H, $C_{1-4}$-Alkyl, $C_{3-6}$-Cycloalkyl, $C_{1-4}$-Fluoralkyl und $C_{1-4}$-Alkyloxy;
$R^2$ unabhängig ausgewählt ist aus H, Halogen, $C_{1-4}$-Alkyl und $C_{1-4}$-Fluoralkyl; und
$R^3$ H oder $C_{1-4}$-Alkyl ist;
$R^4$ Halogen oder $C_{1-4}$-Alkyl ist,
oder ein pharmazeutisch unbedenkliches Salz davon.

**9.** PARP-Inhibitor zur Verwendung nach Anspruch 8, wobei der Hirntumor oder das Neuroblastom umfasst:

(i. einen Alpha-Thalassämie/Mentales-Retardierungssyndrom-X-chromosomalen-Mangelphänotyp (ATRX-Mangelphänotyp);
(ii. einen Isocitrat-Dehydrogenase-1- oder 2-Mangelphänotyp (IDH1- oder IDH2-Mangelphänotyp); und/oder
(iii. $O^6$-Methylguanin-DNA-Methyltransferase-Promotormethylierung (MGMT-Promotormethylierung).

**10.** PARP-Inhibitor zur Verwendung nach einem der Ansprüche 7 bis 9, wobei das alkylierende chemotherapeutische Mittel Temozolomid (TMZ) oder Dianhydrogalactitol (VAL-083) ist.

**11.** PARP-Inhibitor zur Verwendung nach einem der Ansprüche 1 bis 10, wobei der PARP-Inhibitor ausgewählt ist aus:

5-[4-[(2,5-Dimethyl-3-oxo-4H-chinoxalin-6-yl)methyl]piperazin-1-yl]-6-fluor-N-methyl-pyridin-2-carboxamid,
5-[4-[(2,5-Dimethyl-3-oxo-4H-chinoxalin-6-yl)methyl]piperazin-1-yl]-N-methyl-pyridin-2-carboxamid,
6-Chlor-5-[4-[(2,5-dimethyl-3-oxo-4H-chinoxalin-6-yl)methyl]piperazin-1-yl]-N-methyl-pyridin-2-carboxamid,
5-[4-[(2,5-Dimethyl-3-oxo-4H-chinoxalin-6-yl)methyl]piperazin-1-yl]-N,6-dimethyl-pyridin-2-carboxamid,
5-[4-[(2,5-Dimethyl-3-oxo-4H-chinoxalin-6-yl)methyl]piperazin-1-yl]-6-fluor-pyridin-2-carboxamid,
5-[4-[(5-Fluor-2-methyl-3-oxo-4H-chinoxalin-6-yl)methyl]piperazin-1-yl]-6-methyl-pyridin-2-carboxamid,
5-[4-[(2,5-Dimethyl-3-oxo-4H-chinoxalin-6-yl)methyl]piperazin-1-yl]-6-methyl-pyridin-2-carboxamid,
6-Chlor-5-[4-[(5-chlor-2-ethyl-3-oxo-4H-chinoxalin-6-yl)methyl]piperazin-1-yl]-N-methyl-pyridin-2-carboxamid,
5-[4-[(5-Chlor-2-ethyl-3-oxo-4H-chinoxalin-6-yl)methyl]piperazin-1-yl]-6-fluor-N-methyl-pyridin-2-carboxamid,
5-[4-[(5-Chlor-2-ethyl-3-oxo-4H-chinoxalin-6-yl)methyl]piperazin-1-yl]-N-methyl-pyridin-2-carboxamid,
5-[4-[(5-Chlor-2-ethyl-3-oxo-4H-chinoxalin-6-yl)methyl]piperazin-1-yl]-N,6-dimethyl-pyridin-2-carboxamid,
6-Fluor-5-[4-[[5-fluor-2-[(1S und 1R)-1-fluorethyl]-3-oxo-4H-chinoxalin-6-yl]methyl]piperazin-1-yl]-N-methyl-pyridin-2-carboxamid,
5-[4-[[5-Fluor-2-[(1S und 1R)-1-fluorethyl]-3-oxo-4H-chinoxalin-6-yl]methyl]piperazin-1-yl]-N,6-dimethyl-pyridin-2-carboxamid,
5-[4-[(5-Chlor-2-methyl-3-oxo-4H-chinoxalin-6-yl)methyl]piperazin-1-yl]-N-methyl-pyridin-2-carboxamid,
5-[4-[(5-Chlor-2-methyl-3-oxo-4H-chinoxalin-6-yl)methyl]piperazin-1-yl]-6-fluor-N-methyl-pyridin-2-carboxamid,
5-[4-[(5-Chlor-2-methyl-3-oxo-4H-chinoxalin-6-yl)methyl]piperazin-1-yl]-N,6-dimethyl-pyridin-2-carboxamid,
5-[4-[[2-(1,1-Difluorethyl)-5-fluor-3-oxo-4H-chinoxalin-6-yl]methyl]piperazin-1-yl]-N,6-dimethyl-pyridin-2-carboxamid,
6-Fluor-5-[4-[(5-fluor-2-methyl-3-oxo-4H-chinoxalin-6-yl)methyl]piperazin-1-yl]-N-methyl-pyridin-2-carboxamid,
6-(Difluormethyl)-5-[4-[(5-fluor-2-methyl-3-oxo-4H-chinoxalin-6-yl)methyl]piperazin-1-yl]-N-methyl-pyridin-2-carboxamid,
6-Fluor-5-[4-[(5-fluor-2-methyl-3-oxo-4H-chinoxalin-6-yl)methyl]piperazin-1-yl]pyridin-2-carboxamid,
5-[4-[(2-Ethyl-5-fluor-3-oxo-4H-chinoxalin-6-yl)methyl]piperazin-1-yl]-N,6-dimethyl-pyridin-2-carboxamid,
6-(Difluormethyl)-5-[4-[(2-ethyl-5-fluor-3-oxo-4H-chinoxalin-6-yl) methyl]piperazin-1-yl]-N-methyl-pyridin-2-carboxamid,

5-[4-[(2-Ethyl-5-fluor-3-oxo-4H-chinoxalin-6-yl)methyl]piperazin-1-yl]pyridin-2-carboxamid,
5-[4-[(2-Ethyl-5-fluor-3-oxo-4H-chinoxalin-6-yl)methyl]piperazin-1-yl]-6-methyl-pyridin-2-carboxamid,
5-[4-[(2-Ethyl-5-fluor-3-oxo-4H-chinoxalin-6-yl)methyl]piperazin-1-yl]-6-fluor-N-methyl-pyridin-2-carboxamid,
6-Chlor-5-[4-[(2-ethyl-5-fluor-3-oxo-4H-chinoxalin-6-yl)methyl]piperazin-1-yl]-N-methyl-pyridin-2-carboxamid,
5-[4-[(2-Ethyl-5-fluor-3-oxo-4H-chinoxalin-6-yl)methyl]piperazin-1-yl]-N-methyl-pyridin-2-carboxamid,
6-Chlor-5-[4-[(5-fluor-2-methyl-3-oxo-4H-chinoxalin-6-yl)methyl]piperazin-1-yl]-N-methyl-pyridin-2-carboxamid,
5-[4-[(5-Fluor-2-methyl-3-oxo-4H-chinoxalin-6-yl)methyl]piperazin-1-yl]-N,6-dimethyl-pyridin-2-carboxamid,
5-[4-[(5-Fluor-2-methyl-3-oxo-4H-chinoxalin-6-yl)methyl]piperazin-1-yl]-N-methyl-pyridin-2-carboxamid,
5-[4-[(5-Fluor-3-oxo-4H-chinoxalin-6-yl)methyl]piperazin-1-yl]-N-methyl-pyridin-2-carboxamid,
6-Chlor-5-[4-[(5-fluor-3-oxo-4H-chinoxalin-6-yl)methyl]piperazin-1-yl]-N-methyl-pyridin-2-carboxamid,
5-[4-[(5-Fluor-3-oxo-4H-chinoxalin-6-yl)methyl]piperazin-1-yl]-N,6-dimethyl-pyridin-2-carboxamid,
6-Fluor-5-[4-[(5-fluor-3-oxo-4H-chinoxalin-6-yl)methyl]piperazin-1-yl]-N-methyl-pyridin-2-carboxamid,
5-[4-[[2-(Difluormethyl)-5-fluor-3-oxo-4H-chinoxalin-6-yl]methyl]piperazin-1-yl]-N,6-dimethyl-pyridin-2-carboxamid,
5-[4-[(5-Fluor-2-methoxy-3-oxo-4H-chinoxalin-6-yl)methyl]piperazin-1-yl]-N-methyl-pyridin-2-carboxamid,
6-Fluor-5-[4-[(5-fluor-2-methoxy-3-oxo-4H-chinoxalin-6-yl)methyl]piperazin-1-yl]-N-methyl-pyridin-2-carboxamid,
5-[4-[(5-Fluor-2-methoxy-3-oxo-4H-chinoxalin-6-yl)methyl]piperazin-1-yl]-N,6-dimethyl-pyridin-2-carboxamid,
6-Chlor-5-[4-[(5-fluor-2-methoxy-3-oxo-4H-chinoxalin-6-yl) methyl]piperazin-1-yl]-N-methyl-pyridin-2-carboxamid,
5-[4-[(2-Ethyl-5-methyl-3-oxo-4H-chinoxalin-6-yl)methyl]piperazin-1-yl]-N,6-dimethyl-pyridin-2-carboxamid,
5-[4-[(2-Ethyl-5-methyl-3-oxo-4H-chinoxalin-6-yl)methyl]piperazin-1-yl]-6-fluor-N-methyl-pyridin-2-carboxamid,
5-[4-[(2-Ethyl-5-methyl-3-oxo-4H-chinoxalin-6-yl)methyl]piperazin-1-yl]-N-methyl-pyridin-2-carboxamid,
N-Ethyl-6-fluor-5-[4-[(5-fluor-2-methyl-3-oxo-4H-chinoxalin-6-yl)methyl]piperazin-1-yl]pyridin-2-carboxamid,
N-Ethyl-5-[4-[(5-fluor-2-methyl-3-oxo-4H-chinoxalin-6-yl)methyl]piperazin-1-yl]-6-methyl-pyridin-2-carboxamid,
5-[4-[[5-Fluor-3-oxo-2-(trifluormethyl)-4H-chinoxalin-6-yl]methyl]piperazin-1-yl]-N,6-dimethyl-pyridin-2-carboxamid,
6-Fluor-5-[4-[[5-fluor-3-oxo-2-(trifluormethyl)-4H-chinoxalin-6-yl]methyl]piperazin-1-yl]-N-methyl-pyridin-2-carboxamid,
6-Chlor-5-[4-[[5-fluor-3-oxo-2-(trifluormethyl)-4H-chinoxalin-6-yl]methyl]piperazin-1-yl]-N-methyl-pyridin-2-carboxamid,
5-[4-[[5-Fluor-3-oxo-2-(trifluormethyl)-4H-chinoxalin-6-yl]methyl]piperazin-1-yl]-N-methyl-pyridin-2-carboxamid,
6-Fluor-5-[4-[(5-fluor-2-isopropyl-3-oxo-4H-chinoxalin-6-yl)methyl]piperazin-1-yl]-N-methyl-pyridin-2-carboxamid,
5-[4-[(5-Fluor-2-isopropyl-3-oxo-4H-chinoxalin-6-yl)methyl]piperazin-1-yl]-N,6-dimethyl-pyridin-2-carboxamid,
5-[4-[(5-Fluor-2-isopropyl-3-oxo-4H-chinoxalin-6-yl)methyl]piperazin-1-yl]-N-methyl-pyridin-2-carboxamid,
5-[4-[(2-Cyclopropyl-5-fluor-3-oxo-4H-chinoxalin-6-yl)methyl]piperazin-1-yl]-6-fluor-N-methyl-pyridin-2-carboxamid,
5-[4-[(2-Cyclopropyl-5-fluor-3-oxo-4H-chinoxalin-6-yl)methyl]piperazin-1-yl]-N,6-dimethyl-pyridin-2-carboxamid,
5-[4-[(2-Cyclopropyl-5-fluor-3-oxo-4H-chinoxalin-6-yl)methyl]piperazin-1-yl]-N-methyl-pyridin-2-carboxamid,
5-[4-[(2-Methoxy-5-methyl-3-oxo-4H-chinoxalin-6-yl)methyl]piperazin-1-yl]-N,6-dimethyl-pyridin-2-carboxamid,
6-Fluor-5-[4-[(2-methoxy-5-methyl-3-oxo-4H-chinoxalin-6-yl)methyl]piperazin-1-yl]-N-methyl-pyridin-2-carboxamid,
6-(Difluormethyl)-5-[4-[(2-methoxy-5-methyl-3-oxo-4H-chinoxalin-6-yl) methyl]piperazin-1-yl]-N-methyl-pyridin-2-carboxamid und
6-(Difluormethyl)-5-[4-[(2,5-dimethyl-3-oxo-4H-chinoxalin-6-yl) methyl]piperazin-1-yl]-N-methyl-pyridin-2-carboxamid,
oder ein pharmazeutisch unbedenkliches Salz davon.

**12.** PARP-Inhibitor zur Verwendung nach einem der Ansprüche 1 bis 10, wobei der PARP-Inhibitor ist:
6-Fluor-5-[4-[(5-Fluor-2-methyl-3-oxo-4H-chinoxalin-6-yl)methyl]piperazin-1-yl]-N-methyl-pyridin-2-carboxamid oder ein pharmazeutisch unbedenkliches Salz davon.

**13.** PARP-Inhibitor zur Verwendung nach einem der Ansprüche 1 bis 10, wobei der PARP-Inhibitor ist: 6-Fluor-5-[4-[(5-fluor-2-methyl-3-oxo-4H-chinoxalin-6-yl)methyl]piperazin-1-yl]-N-methyl-pyridin-2-carboxamid.

**14.** PARP-Inhibitor zur Verwendung nach einem der Ansprüche 1 bis 13, wobei der Hirntumor ein Gliom oder ein Ependymom ist.

**15.** PARP-Inhibitor zur Verwendung nach Anspruch 14, wobei der Hirntumor ein Gliom ist.

## Revendications

**1.** Inhibiteur de PARP pour utilisation dans un procédé de traitement d'une tumeur cérébrale ou d'un neuroblastome chez un patient en ayant besoin, le procédé comprenant l'administration au patient d'une quantité thérapeutiquement efficace d'un inhibiteur de poly(ADP-ribose) polymérase (PARP),

la tumeur cérébrale ou le neuroblastome comprenant un phénotype déficient de syndrome alpha thalassémie/-retard mental lié à l'X (ATRX),
l'inhibiteur de PARP étant un composé de Formule I

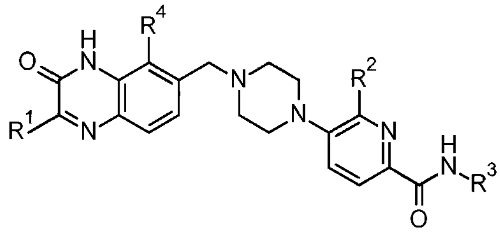

(I)

dans laquelle :

$R^1$ est choisi indépendamment parmi H, alkyle en $C_{1-4}$, cycloalkyle en $C_{3-6}$, fluoroalkyle en $C_{1-4}$, et alkyloxy en $C_{1-4}$ ;
$R^2$ est choisi indépendamment parmi H, halo, alkyle en $C_{1-4}$, et fluoroalkyle en $C_{1-4}$ ; et
$R^3$ est H ou alkyle en $C_{1-4}$ ;
$R^4$ est halo ou alkyle en $C_{1-4}$,
ou un sel pharmaceutiquement acceptable de celui-ci.

**2.** Inhibiteur de PARP pour utilisation selon la revendication 1, la tumeur cérébrale ou le neuroblastome comprenant en outre un phénotype déficient d'isocitrate déshydrogénase 1 et/ou 2 (IDH1 et/ou IDH2).

**3.** Inhibiteur de PARP pour utilisation selon la revendication 1, la tumeur cérébrale ou le neuroblastome ne comprenant pas de phénotype déficient IDH1.

**4.** Inhibiteur de PARP pour utilisation selon l'une quelconque des revendications 1 à 3, la tumeur cérébrale ou le neuroblastome comprenant une méthylation du promoteur de la $Q^6$-méthylguanine-ADN méthyltransférase (MGMT).

**5.** Inhibiteur de PARP pour utilisation selon l'une quelconque des revendications précédentes, comprenant en outre une étape de diagnostic du patient comme ayant une tumeur cérébrale ou un neuroblastome comprenant le phénotype déficient ATRX avant l'administration de l'inhibiteur de PARP.

**6.** Inhibiteur de PARP pour utilisation selon la revendication 5, l'étape de diagnostic du patient comprenant en outre le fait de déterminer si la tumeur cérébrale ou le neuroblastome comprend en outre un phénotype déficient IDH1 ou IDH2 et/ou une méthylation du promoteur MGMT.

**7.** Inhibiteur de PARP pour utilisation selon l'une quelconque des revendications précédentes, l'inhibiteur de PARP étant administré au patient en association avec

i. un agent chimiothérapeutique alkylant ; et/ou

ii. un rayonnement ionisant.

**8.** Inhibiteur de PARP pour utilisation dans un procédé de traitement d'une tumeur cérébrale ou d'un neuroblastome chez un patient en ayant besoin, le procédé comprenant l'administration au patient d'une quantité thérapeutiquement efficace d'un inhibiteur de poly(ADP-ribose) polymérase (PARP), et

i. un agent chimiothérapeutique alkylant ; et/ou
ii. un rayonnement ionisant administré à une dose de 10 Gy ou plus, l'inhibiteur de PARP étant un composé de Formule I

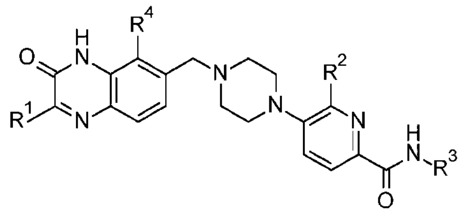

(I)

dans laquelle :

$R^1$ est choisi indépendamment parmi H, alkyle en $C_{1-4}$, cycloalkyle en $C_{3-6}$, fluoroalkyle en $C_{1-4}$, et alkyloxy en $C_{1-4}$ ;
$R^2$ est choisi indépendamment parmi H, halo, alkyle en $C_{1-4}$, et fluoroalkyle en $C_{1-4}$ ; et
$R^3$ est H ou alkyle en $C_{1-4}$ ;
$R^4$ est halo ou alkyle en $C_{1-4}$,
ou un sel pharmaceutiquement acceptable de celui-ci.

**9.** Inhibiteur de PARP pour utilisation selon la revendication 8, la tumeur cérébrale ou le neuroblastome comprenant :

(i) un phénotype déficient de syndrome alpha thalassémie/retard mental lié à l'X (ATRX) ;
(ii) un phénotype déficient d'isocitrate déshydrogénase 1 ou 2 (IDH1 ou IDH2) ; et/ou
(iii) méthylation du promoteur de la $O^6$-méthylguanine-ADN méthyltransférase (MGMT).

**10.** Inhibiteur de PARP pour utilisation selon l'une quelconque des revendications 7 à 9, l'agent chimiothérapeutique alkylant étant le témozolomide (TMZ) ou le dianhydrogalactitol (VAL-083).

**11.** Inhibiteur de PARP pour utilisation selon l'une quelconque des revendications 1 à 10, l'inhibiteur de PARP étant choisi parmi :

5-[4-[(2,5-diméthyl-3-oxo-4H-quinoxalin-6-yl)méthyl]pipérazin-1-yl]-6-fluoro-N-méthyl-pyridine-2-carboxamide,
5-[4-[(2,5-diméthyl-3-oxo-4H-quinoxalin-6-yl)méthyl]pipérazin-1-yl]-N-méthyl-pyridine-2-carboxamide,
6-chloro-5-[4-[(2,5-diméthyl-3-oxo-4H-quinoxalin-6-yl)méthyl]pipérazin-1-yl]-N-méthyl-pyridine-2-carboxamide,
5-[4-[(2,5-diméthyl-3-oxo-4H-quinoxalin-6-yl)méthyl]pipérazin-1-yl]-N,6-diméthyl-pyridine-2-carboxamide,
5-[4-[(2,5-diméthyl-3-oxo-4H-quinoxalin-6-yl)méthyl]pipérazin-1-yl]-6-fluoro-pyridine-2-carboxamide,
5-[4-[(5-fluoro-2-méthyl-3-oxo-4H-quinoxalin-6-yl)méthyl]pipérazin-1-yl]-6-méthyl-pyridine-2-carboxamide,
5-[4-[(2,5-diméthyl-3-oxo-4H-quinoxalin-6-yl)méthyl]pipérazin-1-yl]-6-méthyl-pyridine-2-carboxamide,
6-chloro-5-[4-[(5-chloro-2-éthyl-3-oxo-4H-quinoxalin-6-yl)méthyl]pipérazin-1-yl]-N-méthyl-pyridine-2-carboxamide,
5-[4-[(5-chloro-2-éthyl-3-oxo-4H-quinoxalin-6-yl)méthyl]pipérazin-1-yl]-6-fluoro-N-méthyl-pyridine-2-carboxamide,
5-[4-[(5-chloro-2-éthyl-3-oxo-4H-quinoxalin-6-yl)méthyl]pipérazin-1-yl]-N-méthyl-pyridine-2-carboxamide,
5-[4-[(5-chloro-2-éthyl-3-oxo-4H-quinoxalin-6-yl)méthyl]pipérazin-1-yl]-N,6-diméthyl-pyridine-2-carboxamide,
6-fluoro-5-[4-[[5-fluoro-2-[(1S et 1R)-1-fluoroéthyl]-3-oxo-4H-quinoxalin-6-yl]méthyl]pipérazin-1-yl]-N-méthyl-pyridine-2-carboxamide,
5-[4-[[5-fluoro-2-[(1S et 1R)-1-fluoroéthyl]-3-oxo-4H-quinoxalin-6-yl]méthyl]pipérazin-1-yl]-N,6-diméthyl-pyridine-2-carboxamide,
5-[4-[(5-chloro-2-méthyl-3-oxo-4H-quinoxalin-6-yl)méthyl]pipérazin-1-yl]-N-méthyl-pyridine-2-carboxamide,
5-[4-[(5-chloro-2-méthyl-3-oxo-4H-quinoxalin-6-yl)méthyl]pipérazin-1-yl]-6-fluoro-N-méthyl-pyridine-2-carboxamide,
5-[4-[(5-chloro-2-méthyl-3-oxo-4H-quinoxalin-6-yl)méthyl]pipérazin-1-yl]-N,6-diméthyl-pyridine-2-carboxamide,
5-[4-[[2-(1,1-difluoroéthyl)-5-fluoro-3-oxo-4H-quinoxalin-6-yl]méthyl]pipérazin-1-yl]-N,6-diméthyl-pyridine-2-carboxamide,
6-fluoro-5-[4-[(5-fluoro-2-méthyl-3-oxo-4H-quinoxalin-6-yl)méthyl]pipérazin-1-yl]-N-méthyl-pyridine-2-carboxamide,
6-(difluorométhyl)-5-[4-[(5-fluoro-2-méthyl-3-oxo-4H-quinoxalin-6-yl)méthyl]pipérazin-1-yl]-N-méthyl-pyridine-2-carboxamide,
6-fluoro-5-[4-[(5-fluoro-2-méthyl-3-oxo-4H-quinoxalin-6-yl)méthyl]pipérazin-1-yl]pyridine-2-carboxamide,
5-[4-[(2-éthyl-5-fluoro-3-oxo-4H-quinoxalin-6-yl)méthyl]pipérazin-1-yl]-N,6-diméthyl-pyridine-2-carboxamide,
6-(difluorométhyl)-5-[4-[(2-éthyl-5-fluoro-3-oxo-4H-quinoxalin-6-yl)méthyl]pipérazin-1-yl]-N-méthyl-pyridine-2-carboxamide,
5-[4-[(2-éthyl-5-fluoro-3-oxo-4H-quinoxalin-6-yl)méthyl]pipérazin-1-yl]pyridine-2-carboxamide,
5-[4-[(2-éthyl-5-fluoro-3-oxo-4H-quinoxalin-6-yl)méthyl]pipérazin-1-yl]-6-méthyl-pyridine-2-carboxamide,
5-[4-[(2-éthyl-5-fluoro-3-oxo-4H-quinoxalin-6-yl)méthyl]pipérazin-1-yl]-6-fluoro-N-méthyl-pyridine-2-carboxamide,
6-chloro-5-[4-[(2-éthyl-5-fluoro-3-oxo-4H-quinoxalin-6-yl)méthyl]pipérazin-1-yl]-N-méthyl-pyridine-2-carboxamide,
5-[4-[(2-éthyl-5-fluoro-3-oxo-4H-quinoxalin-6-yl)méthyl]pipérazin-1-yl]-N-méthyl-pyridine-2-carboxamide,
6-chloro-5-[4-[(5-fluoro-2-méthyl-3-oxo-4H-quinoxalin-6-yl)méthyl]pipérazin-1-yl]-N-méthyl-pyridine-2-carboxamide,
5-[4-[(5-fluoro-2-méthyl-3-oxo-4H-quinoxalin-6-yl)méthyl]pipérazin-1-yl]-N,6-diméthyl-pyridine-2-carboxamide,
5-[4-[(5-fluoro-2-méthyl-3-oxo-4H-quinoxalin-6-yl)méthyl]pipérazin-1-yl]-N-méthyl-pyridine-2-carboxamide,
5-[4-[(5-fluoro-3-oxo-4H-quinoxalin-6-yl)méthyl]pipérazin-1-yl]-N-méthyl-pyridine-2-carboxamide,
6-chloro-5-[4-[(5-fluoro-3-oxo-4H-quinoxalin-6-yl)méthyl]pipérazin-1-yl]-N-méthyl-pyridine-2-carboxamide,
5-[4-[(5-fluoro-3-oxo-4H-quinoxalin-6-yl)méthyl]pipérazin-1-yl]-N,6-diméthyl-pyridine-2-carboxamide,
6-fluoro-5-[4-[(5-fluoro-3-oxo-4H-quinoxalin-6-yl)méthyl]pipérazin-1-yl]-N-méthyl-pyridine-2-carboxamide,
5-[4-[[2-(difluorométhyl)-5-fluoro-3-oxo-4H-quinoxalin-6-yl]méthyl]pipérazin-1-yl]-N,6-diméthyl-pyridine-2-carboxamide,
5-[4-[(5-fluoro-2-méthoxy-3-oxo-4H-quinoxalin-6-yl)méthyl]pipérazin-1-yl]-N-méthyl-pyridine-2-carboxamide,
6-fluoro-5-[4-[(5-fluoro-2-méthoxy-3-oxo-4H-quinoxalin-6-yl)méthyl]pipérazin-1-yl]-N-méthyl-pyridine-2-carboxamide,
5-[4-[(5-fluoro-2-méthoxy-3-oxo-4H-quinoxalin-6-yl)méthyl]pipérazin-1-yl]-N,6-diméthyl-pyridine-2-carboxamide,
6-chloro-5-[4-[(5-fluoro-2-méthoxy-3-oxo-4H-quinoxalin-6-yl)méthyl]pipérazin-1-yl]-N-méthyl-pyridine-2-carboxamide,
5-[4-[(2-éthyl-5-méthyl-3-oxo-4H-quinoxalin-6-yl)méthyl]pipérazin-1-yl]-N,6-diméthyl-pyridine-2-carboxamide,
5-[4-[(2-éthyl-5-méthyl-3-oxo-4H-quinoxalin-6-yl)méthyl]pipérazin-1-yl]-6-fluoro-N-méthyl-pyridine-2-carboxamide,
5-[4-[(2-éthyl-5-méthyl-3-oxo-4H-quinoxalin-6-yl)méthyl]pipérazin-1-yl]-N-méthyl-pyridine-2-carboxamide,
N-éthyl-6-fluoro-5-[4-[(5-fluoro-2-méthyl-3-oxo-4H-quinoxalin-6-yl)méthyl]pipérazin-1-yl]pyridine-2-carboxamide,
N-éthyl-5-[4-[(5-fluoro-2-méthyl-3-oxo-4H-quinoxalin-6-yl)méthyl]pipérazin-1-yl]-6-méthyl-pyridine-2-carboxamide,
5-[4-[[5-fluoro-3-oxo-2-(trifluorométhyl)-4H-quinoxalin-6-yl]méthyl]pipérazin-1-yl]-N,6-diméthyl-pyridine-2-carboxamide,
6-fluoro-5-[4-[[5-fluoro-3-oxo-2-(trifluorométhyl)-4H-quinoxalin-6-yl]méthyl]pipérazin-1-yl]-N-méthyl-pyridine-2-carboxamide,
6-chloro-5-[4-[[5-fluoro-3-oxo-2-(trifluorométhyl)-4H-quinoxalin-6-yl]méthyl]pipérazin-1-yl]-N-méthyl-pyridine-2-carboxamide,
5-[4-[[5-fluoro-3-oxo-2-(trifluorométhyl)-4H-quinoxalin-6-yl]méthyl]pipérazin-1-yl]-N-méthyl-pyridine-2-carboxamide,
6-fluoro-5-[4-[(5-fluoro-2-isopropyl-3-oxo-4H-quinoxalin-6-yl)méthyl]pipérazin-1-yl]-N-méthyl-pyridine-2-carboxamide,
5-[4-[(5-fluoro-2-isopropyl-3-oxo-4H-quinoxalin-6-yl)méthyl]pipérazin-1-yl]-N,6-diméthyl-pyridine-2-carboxamide,
5-[4-[(5-fluoro-2-isopropyl-3-oxo-4H-quinoxalin-6-yl)méthyl]pipérazin-1-yl]-N-méthyl-pyridine-2-carboxamide,
5-[4-[(2-cyclopropyl-5-fluoro-3-oxo-4H-quinoxalin-6-yl)méthyl]pipérazin-1-yl]-6-fluoro-N-méthyl-pyridine-2-carboxamide,
5-[4-[(2-cyclopropyl-5-fluoro-3-oxo-4H-quinoxalin-6-yl)méthyl]pipérazin-1-yl]-N,6-diméthyl-pyridine-2-carboxamide,
5-[4-[(2-cyclopropyl-5-fluoro-3-oxo-4H-quinoxalin-6-yl)méthyl]pipérazin-1-yl]-N-méthyl-pyridine-2-carboxamide,
5-[4-[(2-méthoxy-5-méthyl-3-oxo-4H-quinoxalin-6-yl)méthyl]pipérazin-1-yl]-N,6-diméthyl-pyridine-2-carboxamide,
6-fluoro-5-[4-[(2-méthoxy-5-méthyl-3-oxo-4H-quinoxalin-6-yl)méthyl]pipérazin-1-yl]-N-méthyl-pyridine-2-carboxamide,
6-(difluorométhyl)-5-[4-[(2-méthoxy-5-méthyl-3-oxo-4H-quinoxalin-6-yl)méthyl]pipérazin-1-yl]-N-méthyl-pyridine-2-carboxamide, et
6-(difluorométhyl)-5-[4-[(2,5-diméthyl-3-oxo-4H-quinoxalin-6-yl)méthyl]pipérazin-1-yl]-N-méthyl-pyridine-2-carboxamide,
ou un sel pharmaceutiquement acceptable de celui-ci.

**12.** Inhibiteur de PARP pour utilisation selon l'une quelconque des revendications 1 à 10, l'inhibiteur de PARP étant : le 6-fluoro-5-[4-[(5-fluoro-2-méthyl-3-oxo-4H-quinoxalin-6-yl)méthyl]pipérazin-1-yl]-N-méthyl-pyridine-2-carboxamide, ou un sel pharmaceutiquement acceptable de celui-ci.

**13.** Inhibiteur de PARP pour utilisation selon l'une quelconque des revendications 1 à 10, l'inhibiteur de PARP étant : le 6-fluoro-5-[4-[(5-fluoro-2-méthyl-3-oxo-4H-quinoxalin-6-yl)méthyl]pipérazin-1-yl]-N-méthyl-pyridine-2-carboxamide.

**14.** Inhibiteur de PARP pour utilisation selon l'une quelconque des revendications 1 à 13, la tumeur cérébrale étant un gliome ou un épendymome.

**15.** Inhibiteur de PARP pour utilisation selon la revendication 14, la tumeur cérébrale étant un gliome.

**FIG. 1: XRPD diffractogram of 6-fluoro-5-[4-[(5-fluoro-2-methyl-3-oxo-4H-quinoxalin-6-yl)methyl]piperazin-1-yl]-N-methyl-pyridine-2-carboxamide Form B**

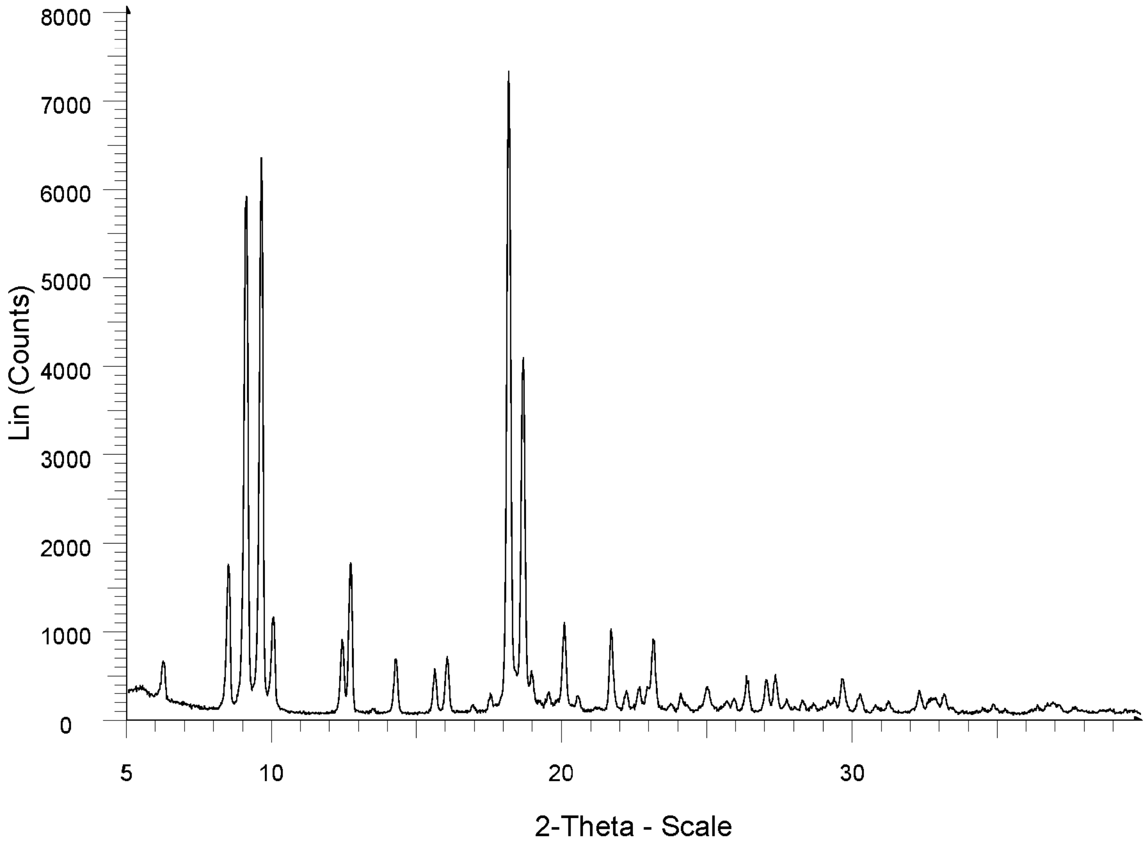

**FIG. 2: DSC trace of 6-fluoro-5-[4-[(5-fluoro-2-methyl-3-oxo-4H-quinoxalin-6-yl)methyl]piperazin-1-yl]-N-methyl-pyridine-2-carboxamide Form B**

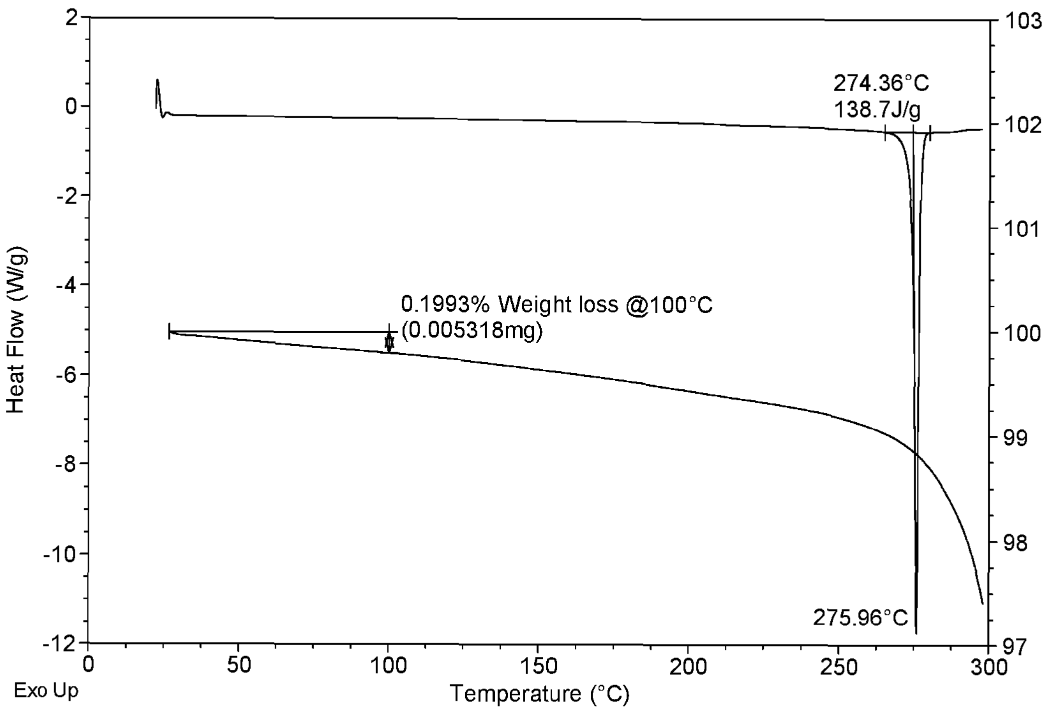

**FIG. 3 : XRPD diffractogram of 6-fluoro-5-[4-[(5-fluoro-2-methyl-3-oxo-4H-quinoxalin-6-yl)methyl]piperazin-1-yl]-N-methyl-pyridine-2-carboxamide Form D**

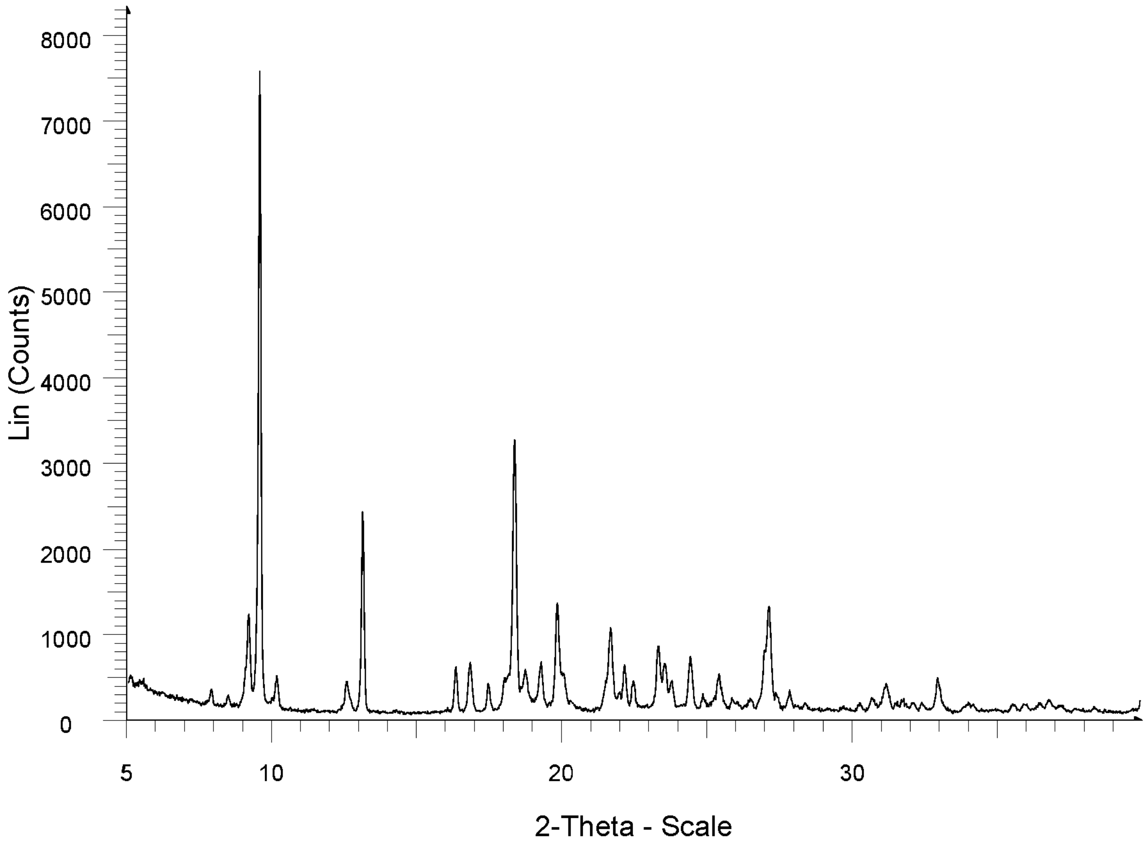

**FIG. 4:** Single crystal structure of **6-fluoro-5-[4-[(5-fluoro-2-methyl-3-oxo-4H-quinoxalin-6-yl)methyl]piperazin-1-yl]-N-methyl-pyridine-2-carboxamide** -Form D (ORTEP50)

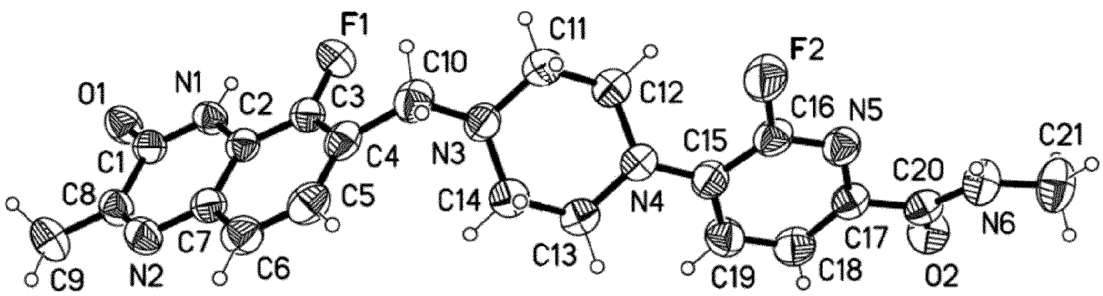

**FIG. 5: XRPD diffractogram of 6-fluoro-5-[4-[(5-fluoro-2-methyl-3-oxo-4H-quinoxalin-6-yl)methyl]piperazin-1-yl]-N-methyl-pyridine-2-carboxamide MSA salt Form C**

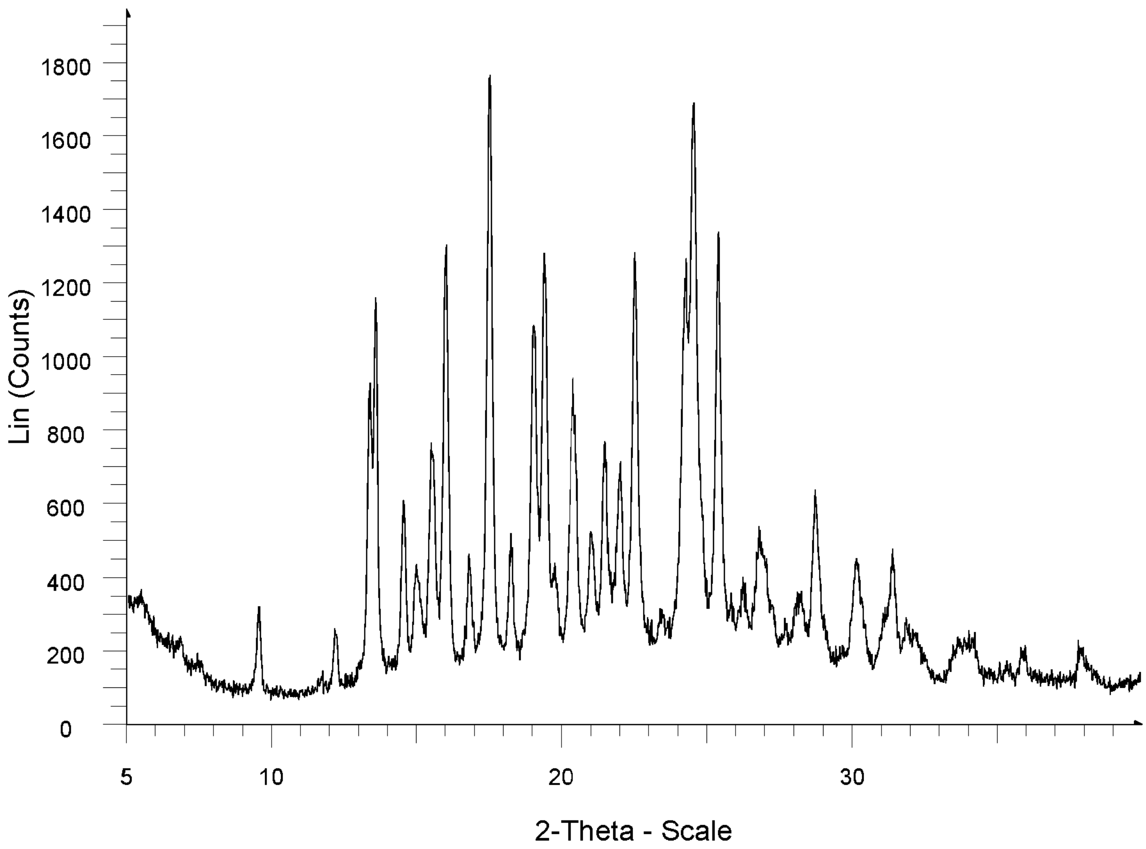

**FIG. 6: DSC trace of 6-fluoro-5-[4-[(5-fluoro-2-methyl-3-oxo-4H-quinoxalin-6-yl)methyl]piperazin-1-yl]-N-methyl-pyridine-2-carboxamide MSA salt Form C**

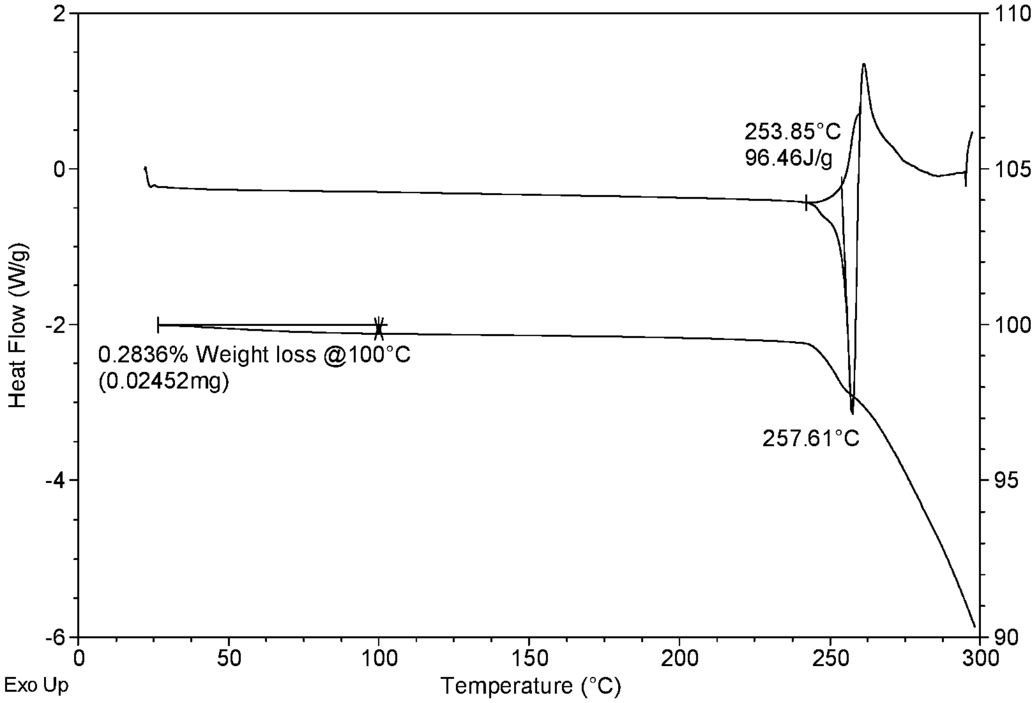

**FIG. 7: Cell confluence graphs representing the potent PARP inhibitor monotherapy activity in glioma cells with ATRX mutation (SJG2IDH1m and SJG2IDH1wt) but not wild type ATRX (U87 IDH1wt and U87 IDH1mt)**

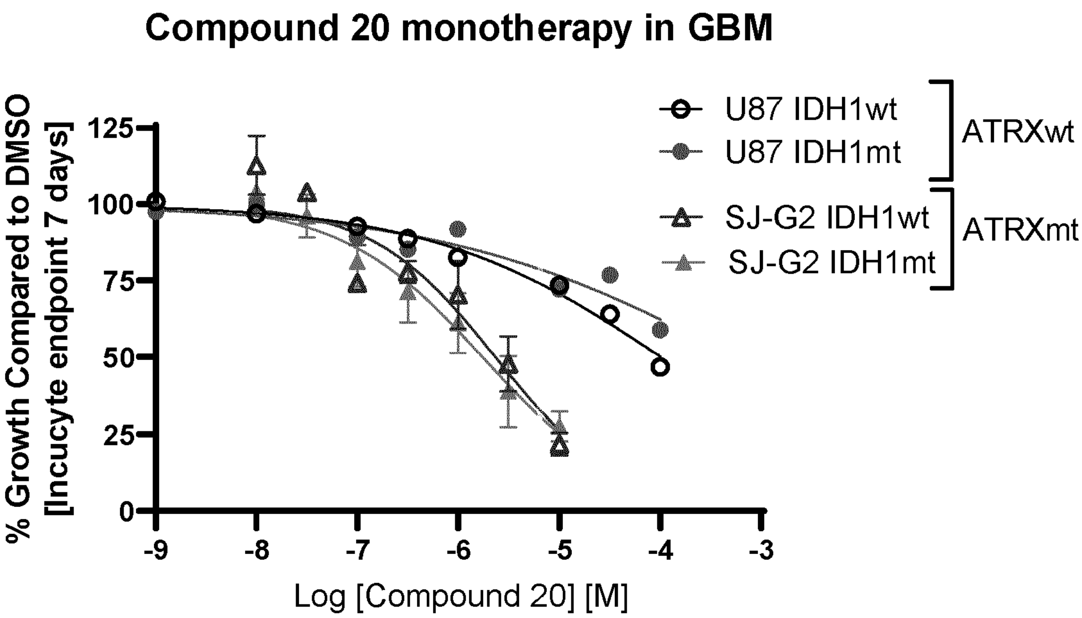

FIG. 8: Cell confluence graphs representing PARP inhibitor potentiation of TMZ efficacy in U87 IDH1wt (A) and U87 IDH1mt R132H (B) isogenic pairs. Representative graphs are shown from 3 biological replicates

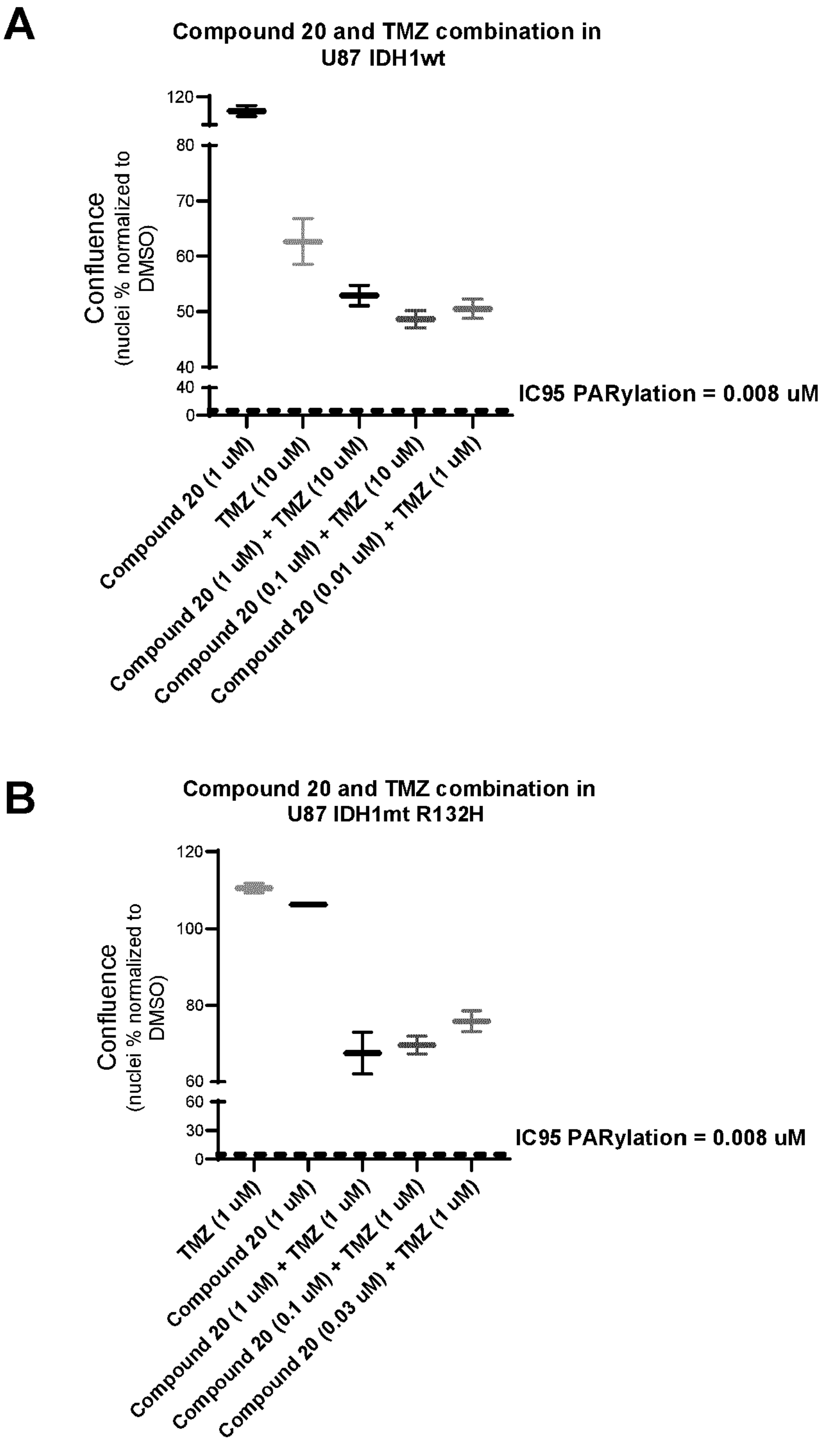

FIG. 9: Cell viability graphs representing PARP inhibitor potentiation of TMZ efficacy in SJ-G2 IDH1wt (A) and SJ-G2 IDH1mt R132H (B) isogenic pairs. Representative graphs are shown, error bars represent +/- SD

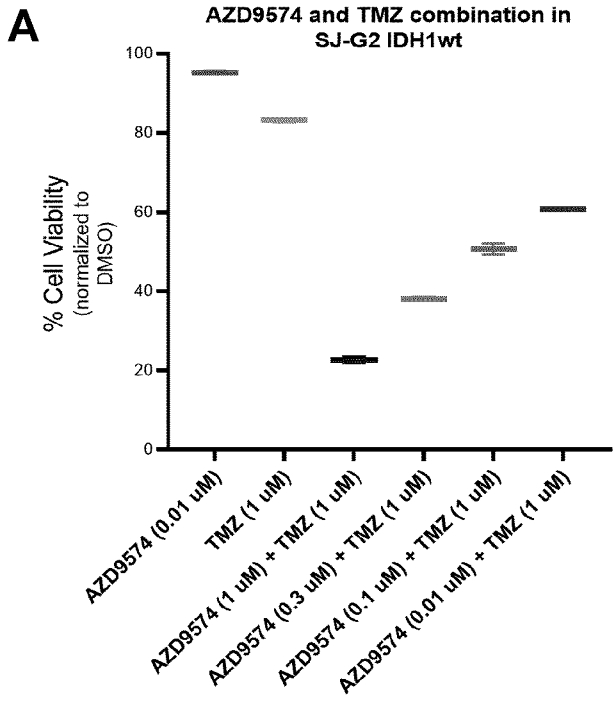


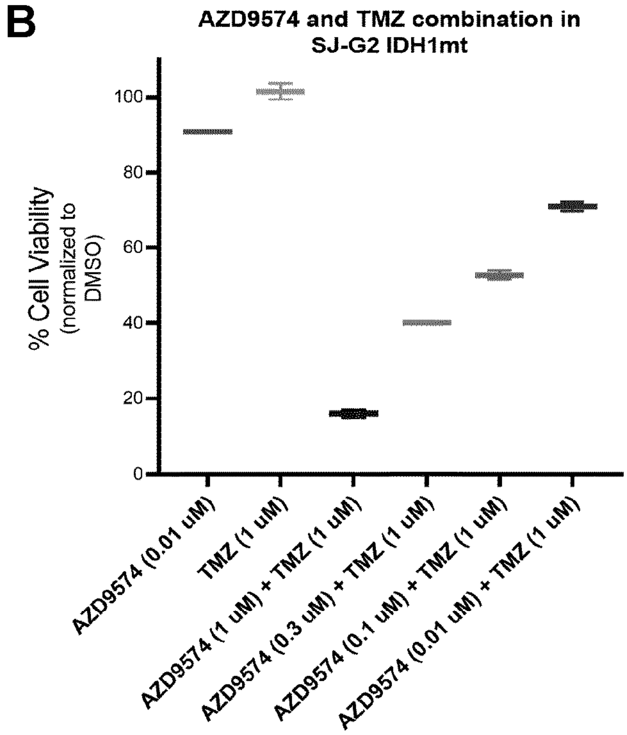

**FIG. 10: Anti-tumour efficacy (A) and change in body weight (B) of a U87MG xenograft model dosed with: i) vehicle; ii) Compound 20 alone (3 mg/kg QD); iii) TMZ at a lower dose (6.25 mg/kg QD on days 1-5 and 29-33); iv) TMZ at a higher dose (25 mg/kg QD on days 1-5 and 29-33); and v) the combination of Compound 20 and TMZ at the lower dose**

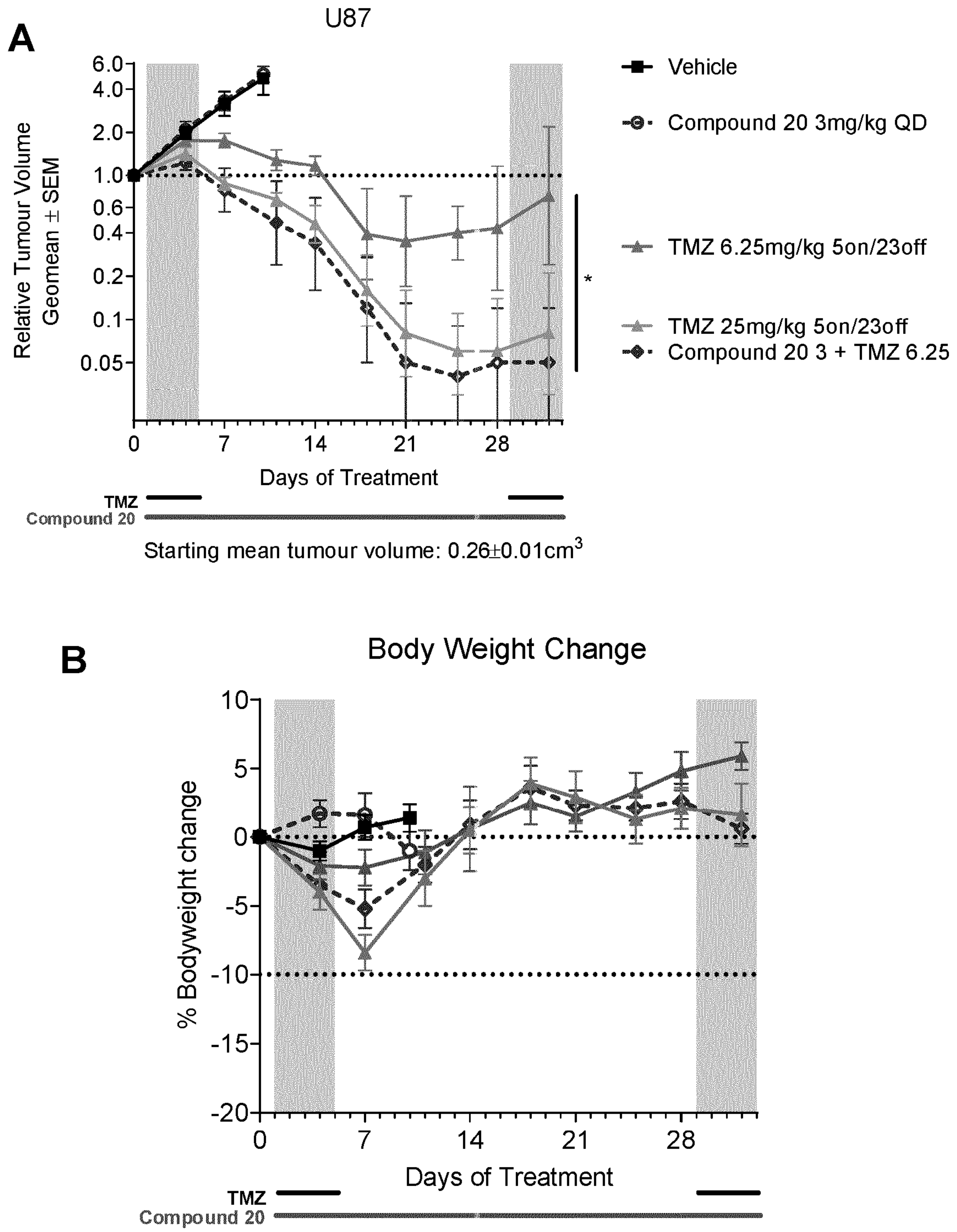

FIG. 11: Cell confluence graphs representing PARP inhibitor potentiation of VAL-083 efficacy in U87 IDH1wt (A) and U87 IDH1mt R132H (B) isogenic pairs. Representative graphs are shown, error bars represent +/- SD

A

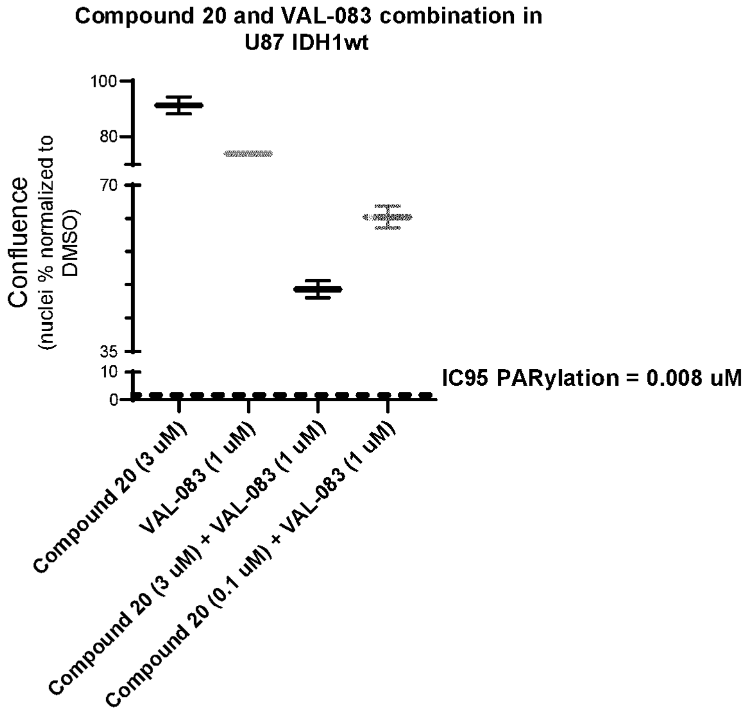


B

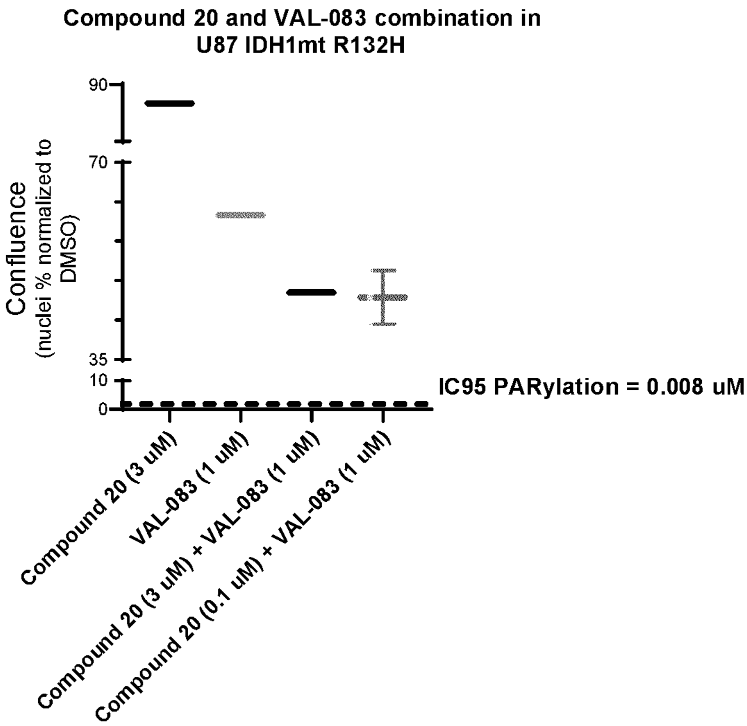

**FIG. 12: Cell confluence graphs representing PARP inhibitor potentiation of VAL-083 efficacy in SJ-G2 IDH1wt (A) and SJ-G2 IDH1mt R132H (B) isogenic pairs. Representative graphs are shown, error bars represent +/- SD**

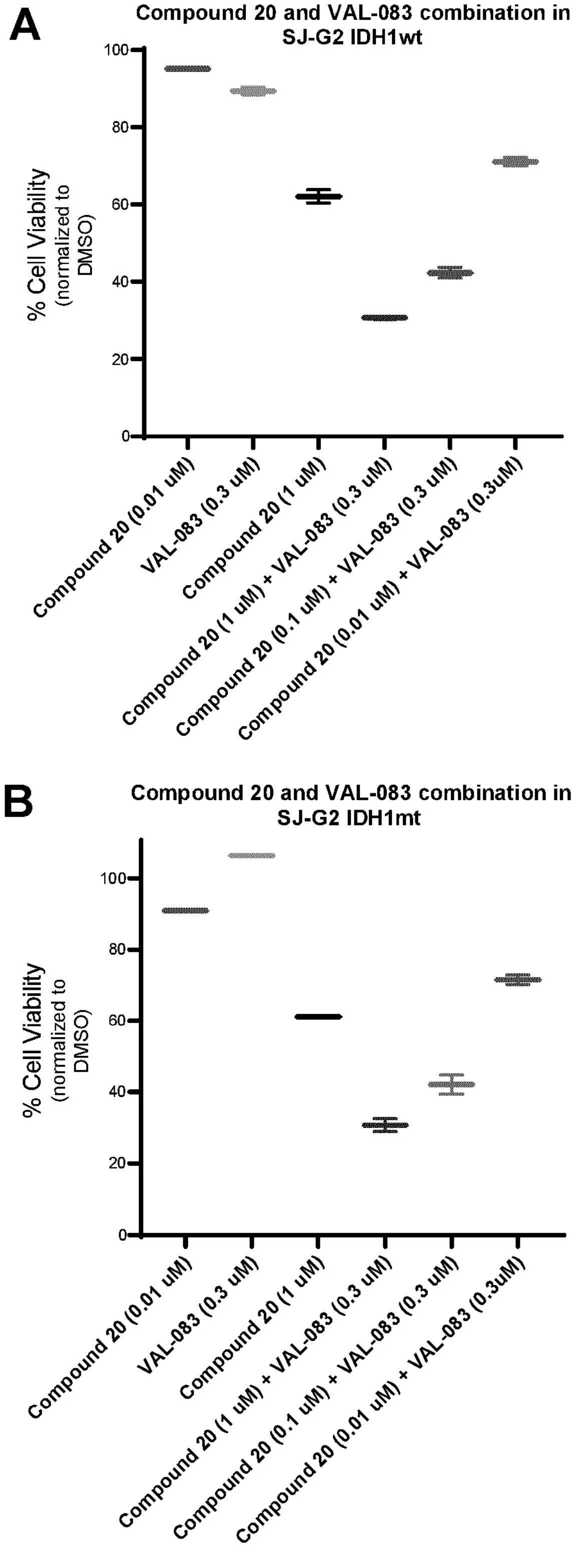

**FIG. 13 shows cell confluence graph representing PARP inhibitor potentiation of IR efficacy in BT245 H3K27M paediatric gliomas**

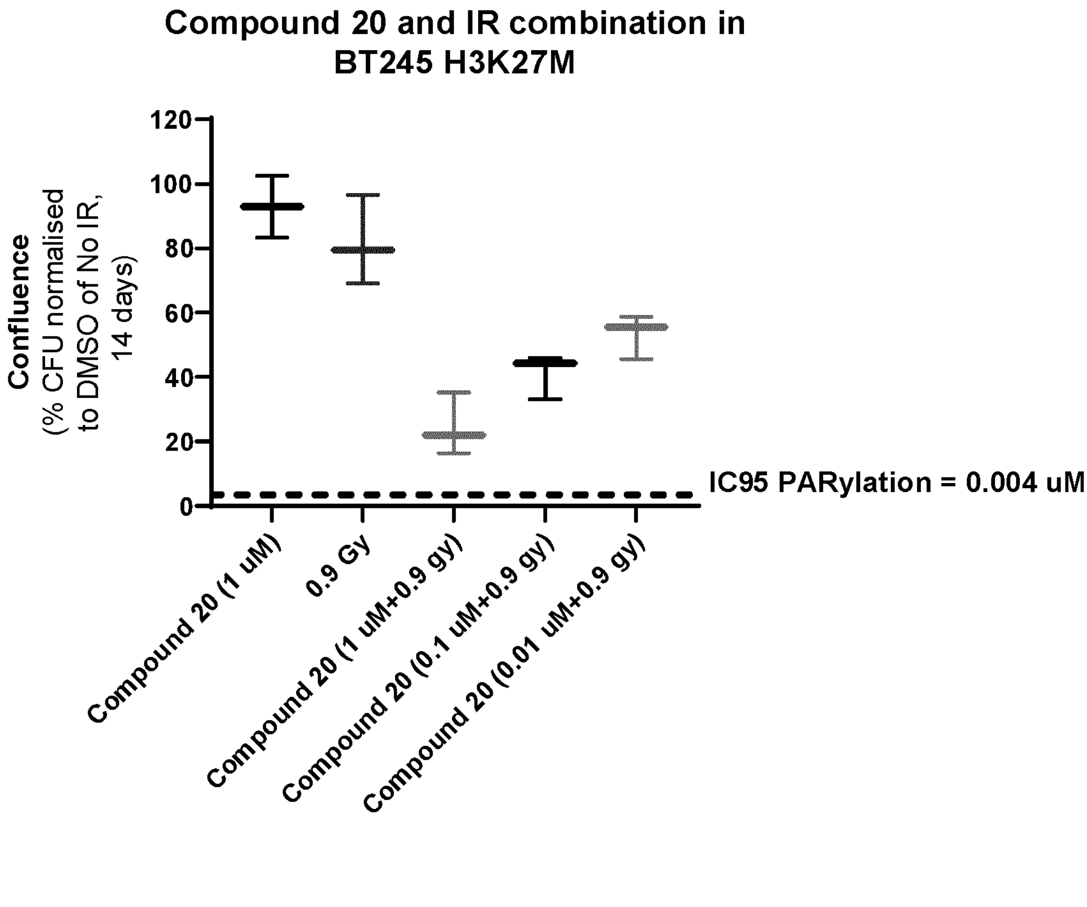

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004080976 A
- EP 699754 A
- EP 705903 A


### Non-patent literature cited in the description


- **NABORS et al.** National Comprehensive Cancer Network. American Cancer Society, 2020
- Remington's Pharmaceutical Sciences. Mack Publishing Company, 1985
- **GIACOVAZZO, C. et al.** Fundamentals of Crystallography. Oxford University Press, 1995
- **JENKINS, R.** ; **SNYDER, R. L.** Introduction to X-Ray Powder Diffractometry. John Wiley & Sons, 1996
- **KLUG, H. P.** ; **ALEXANDER, L. E.** X-ray Diffraction Procedures. John Wiley and Sons, 1974
- *Anal Biochem.*, 01 September 1980, vol. 107 (1), 220-39
- **FRIDÉN et al.** *Drug Metabolism and Disposition*, 2009, vol. 37 (6), 1226-1233
- **AMERICAN CANCER SOCIETY** ; **AMERICAN CANCER SOCIETY WEBSITE**. Treating Neuroblastoma. 2021
- **BAI P.** Biology of poly(ADP-ribose) polymerases: the factotums of cell maintenance. *Mol Cell*, 2015, vol. 58, 947-958
- **BARTKOVA J** ; **HAMERLIK P** ; **STOCKHAUSEN MT** ; **EHRMANN J** ; **HLOBILKOVA A** ; **LAURSEN H** ; **KALITA O** ; **KOLAR Z** ; **POULSEN HS** ; **BROHOLM H**. Replication stress and oxidative damage contribute to aberrant constitutive activation of DNA damage signalling in human gliomas. *Oncogene*, 28 June 2010, vol. 29 (36), 5095-5102
- **BORISY, A. A., ELLIOTT, P. J., HURST, N. W., LEE, M. S., LEHAR, J., PRICE, E. R., ... KEITH, C. T.** Systematic discovery of multicomponent therapeutics. *Proc Natl Acad Sci U S A*, 2003, vol. 100 (13), 7977-7982
- **CHAPPNIS, P.O.** ; **FOULKES, W.O.** *Cancer Treat Res*, 2002, vol. 107, 29-59
- **DI VEROLI, G. Y** ; **FORNARI, C.** ; **WANG, D.** ; **MOLLARD, S.** ; **BRAMHALL, J. L** ; **RICHARDS, F. M.** ; **JODRELL, D. I.** Combenefit: an interactive platform for the analysis and visualization of drug combinations. *Bioinformatics*, 2016, vol. 32 (18), 2866-2868
- **GEORGE SL** ; **LORENZI F** ; **KING D** ; **HARTLIEB S** ; **CAMPBELL J** ; **PEMBERTON H** ; **TOPRAK UH** ; **BARKER K** ; **TALL J** ; **DA COSTA BM**. Therapeutic vulnerabilities in the DNA damage response for the treatment of ATRX mutant neuroblastoma. *EBioMedicine*, 23 August 2020, vol. 59, 102971
- **GUPTA, S. K** ; **SMITH, E. J.** ; **MLADEK, A. C** ; **TIAN, S.** ; **DECKER, P. A.** ; **KIZILBASH, S. H.** ; **SARKARIA, J. N.** PARP Inhibitors for Sensitization of Alkylation Chemotherapy in Glioblastoma: Impact of Blood-Brain Barrier and Molecular Heterogeneity. *Front Oncol*, 2018, vol. 8, 670
- **HAASE, S.** ; **GARCIA-FABIANI, M. B.** ; **CARNEY, S** ; **ALTSHULER, D.** ; **NÚÑEZ, F. J.** ; **MÉNDEZ, F. M.** ; **NÚÑEZ, F.** ; **LOWENSTEIN, P. R** ; **CASTRO, M. G.** Mutant ATRX: uncovering a new therapeutic target for glioma. *Expert opinion on therapeutic targets*, 2018, vol. 22 (7), 599-613
- **HARTMANN C, MEYER J** ; **BALSS J** ; **CAPPER D** ; **MUELLER W** ; **CHRISTIANS A** ; **FELSBERG J** ; **WOLTER M** ; **MAWRIN C** ; **WICK W** ; **WELLER M**. Type and frequency of IDH1 and IDH2 mutations are related to astrocytic and oligodendroglial differentiation and age: a study of 1,010 diffuse gliomas. *Acta Neuropathol.*, 25 June 2009, vol. 118 (4), 469-74
- **HIGUCHI, F.** ; **NAGASHIMA, H.** ; **NING, J.** ; **KOERNER, M. V. A.** ; **WAKIMOTO, H.** ; **CAHILL, D. P.** Restoration of Temozolomide Sensitivity by PARP Inhibitors in Mismatch Repair Deficient Glioblastoma is Independent of Base Excision Repair.. *Clin Cancer Res*, 2020, vol. 26 (7), 1690-1699
- **HIMES, B. T.** ; **ZHANG, L.** ; **DANIELS, D. J.** Treatment Strategies in Diffuse Midline Gliomas With the H3K27M Mutation: The Role of Convection-Enhanced Delivery in Overcoming Anatomic Challenges.. *Front Oncol*, 2019, vol. 9, 31
- **HUGHES-DAVIES et al.** *Cell*, 2003, vol. 115, 523-535
- **JANATOVA M. et al.** *Neoplasma*, 2003, vol. 50 (4), 246-250
- **JANCARKOVA, N.** *Ceska Gynekol.*, 2003, vol. 68 (1), 11-6

- **JANNETTI, S. A.** ; **ZEGLIS, B. M.** ; **ZALUTSKY, M. R.** ; **REINER, T.** Poly(ADP-Ribose)Polymerase (PARP) Inhibitors and Radiation Therapy. *Front. Pharmacol.*, 2020, vol. 11, 170
- **JIMENEZ-ALCAZAR, M.** ; **CURIEL-GARCIA, A.** ; **NOGALES, P.** ; **PERALES-PATON, J.** ; **SCHUHMACHER, A. J.** ; **GALAN-GANGA, M.** ; **SQUATRITO, M.** Dianhydrogalactitol Overcomes Multiple Temozolomide Resistance Mechanisms in Glioblastoma. *Mol Cancer Ther*, 2021, vol. 20 (6), 1029-1038
- **KOSCHMANN, C.** ; **CALINESCU, A. A.** ; **NUNEZ, F. J.** ; **MACKAY, A.** ; **FAZAL-SALOM, J.** ; **THOMAS, D.** ; **MENDEZ, F.** ; **KAMRAN, N.** ; **DZAMAN, M** ; **MULPURI, L.** ATRX loss promotes tumor growth and impairs nonhomologous end joining DNA repair in glioma. *Science translational medicine*, 2016, vol. 8 (328), 328-28
- **MANSOURI, A** ; **HACHEM, L. D.** ; **MANSOURI, S.** ; **NASSIRI, F.** ; **LAPERRIERE, N. J.** ; **XIA, D.** ; **LINDEMAN, N. I.** ; **WEN, P. Y.** ; **CHAKRAVARTI, A.** ; **MEHTA, M. P.** MGMT promoter methylation status testing to guide therapy for glioblastoma: refining the approach based on emerging evidence and current challenges. *Neuro-oncology*, 2019, vol. 21 (2), 167-178
- **MARTINEZ-RICARTE F** ; **MAYOR R** ; **MARTÍNEZ-SÁEZ E, RUBIO-PÉREZ C** ; **PINEDA E** ; **CORDERO E** ; **CICUÉNDEZ M** ; **POCA MA** ; **LÓPEZ-BIGAS N** ; **RAMON Y CAJAL S** ; **VIEITO M**. Molecular Diagnosis of Diffuse Gliomas through Sequencing of Cell-Free Circulating Tumor DNA from Cerebrospinal Fluid. *Clin Cancer Res.*, 03 April 2018, vol. 24 (12), 2812-2819
- **MURAI, J.** ; **HUANG, S. Y** ; **DAS, B. B.** ; **RENAUD, A.** ; **ZHANG, Y.** ; **DOROSHOW, J. H.** ; **POMMIER, Y.** Trapping of PARP1 and PARP2 by Clinical PARP Inhibitors. *Cancer Res*, 2012, vol. 72 (21), 5588-5599
- **MURAI, J.** ; **ZHANG, Y** ; **MORRIS, J.** ; **JI, J.** ; **TAKEDA, S.** ; **DOROSHOW, J. H.** ; **POMMIER, Y.** Rationale for poly(ADP-ribose) polymerase (PARP) inhibitors in combination therapy with camptothecins or temozolomide based on PARP trapping versus catalytic inhibition. *J Pharmacol Exp Ther*, 2014, vol. 349 (3), 408-416
- **NABORS, LB et al.** Central Nervous System Cancers, Version 3.2020, NCCN Clinical Practice Guidelines in Oncology. *Journal of the National Comprehensive Cancer Network*, 2020, vol. 18 (11), 1537-1570
- **J NEUHAUSEN, S.L.** ; **OSTRANDER, E.A.** *Genet. Test*, 1997, vol. 1, 75-83
- **OHBA, S** ; **KUWAHARA, K.** ; **YAMADA, S.** ; **ABE, M.** ; **HIROSE, Y.** Correlation between IDH, ATRX, and TERT promoter mutations in glioma. *Brain Tumor Pathol*, 2020, vol. 37 (2), 33-40
- **PATEL, M. M.** *Expert Opin. Drug Deliv.*, 2011, vol. 8 (10), 1247-1258
- **REISZ, J. A** ; **BANSAL, N.** ; **QIAN, J.** ; **ZHAO, W.** ; **FURDUI, C. M.** Effects of ionizing radiation on biological molecules--mechanisms of damage and emerging methods of detection. *Antioxid Redox Signal*, 2014, vol. 21 (2), 260-292
- **SINGH, N.** ; **MINER, A** ; **HENNIS, L.** ; **MITTAL, S.** Mechanisms of temozolomide resistance in glioblastoma - a comprehensive review. *Cancer Drug Resist*, 2021, vol. 4, 17-43
- **SONGTAO, Q.** ; **LEI, Y.** ; **SI, G.** ; **YANQING, D.** ; **HUIXIA, H.** ; **XUELIN, Z.** ; **FEI, Y.** IDH mutations predict longer survival and response to temozolomide in secondary glioblastoma.. *Cancer Sci*, 2012, vol. 103 (2), 269-273
- **STUPP, R.** ; **BRADA, M.** ; **VAN DEN BENT, M. J.** ; **TONN, J. C.** ; **PENTHEROUDAKIS, G.** ; **GROUP, E. G. W.** High-grade glioma: ESMO Clinical Practice Guidelines for diagnosis, treatment and follow-up.. *Ann Oncol*, 2014, vol. 25 (3), 93-101
- **STUPP, R.** ; **TONN, J. C.** ; **BRADA, M.** ; **PENTHEROUDAKIS, G.** ; **GROUP, E. G. W.** High-grade malignant glioma: ESMO Clinical Practice Guidelines for diagnosis, treatment and follow-up. *Ann Oncol*, 2010, vol. 21 (5), 190-193
- **TAN, X.** ; **HU, L** ; **LUQUETTE, L. J.** ; **GAO, G.** ; **LIU, Y.** ; **QU, H** ; **ELLEDGE, S. J.** Systematic identification of synergistic drug pairs targeting HIV. *Nat Biotechnol*, 2012, vol. 30 (11), 1125-1130
- **VAN VUURDEN DG** ; **HULLEMAN E** ; **MEIJER OL** ; **WEDEKIND LE** ; **KOOL M** ; **WITT H** ; **VANDERTOP PW** ; **WÜRDINGER T** ; **NOSKE DP** ; **KASPERS GJ**. PARP inhibition sensitizes childhood high grade glioma, medulloblastoma and ependymoma to radiation. *Oncotarget*, December 2011, vol. 2 (12), 984-996
- **WELLER M** ; **STUPP R** ; **REIFENBERGER G** ; **BRANDES AA** ; **VAN DEN BENT MJ** ; **WICK W** ; **HEGI ME.** MGMT promoter methylation in malignant gliomas: ready for personalized medicine?. *Nat Rev Neurol.*, 08 December 2009, vol. 6 (1), 39-51
- **SAMBROOK, J.** ; **RUSSEL, D.W.** Molecular Cloning, A Laboratory Manual.. Cold Spring Harbor Laboratory Press, 2001